# EUROPEAN PATENT SPECIFICATION

(11) **EP 1 641 759 B1**
(45) Date of publication and mention of the grant of the patent: **12.03.2014**
(21) Application number: 04740026.2
(22) Date of filing: 18.06.2004
(51) Int. Cl.: C07D 213/68, C07D 413/12, A61K 31/44, A61P 35/00

(54) **MALONAMIDE DERIVATIVES**
MALONAMIDDERIVATE
DERIVES DE MALONAMIDE

(30) Priority: 07.07.2003 EP 03014556
(43) Date of publication of application: 05.04.2006
(73) Proprietor: Merck Patent GmbH, 64293 Darmstadt (DE)
(72) Inventor: BRUGE, David, 60599 Frankfurt (DE); BUCHSTALLER, Hans-Peter, 64347 griesheim (DE); WIESNER, Matthias, 64342 Seeheim-Jugenheim (DE); FINSINGER, Dirk, 64291 Darmstadt (DE); BAUMGARTH, Manfred, 64297 Darmstadt (DE); SIRRENBERG, Christian, 64289 Darmstadt (DE); ZENKE, Frank, 64291 Darmstadt (DE); AMENDT, Christiane, 64289 Darmstadt (DE); GRELL, Matthias, 64291 Darmstadt (DE)
(86) International application number: PCT/EP2004/006573
(87) International publication number: WO 2005/005389

(56) References cited:
- EP-A- 0 156 456
- WO-A-00/26197
- WO-A-02/074730
- WO-A-2004/019941
- WO-A-2004/037789
- NEHRING; SEELIGER ET AL: "Umsetzung von 2-Alkyl-oxazolinen mit Phenylisocyanat" JUSTUS LIEBIGS ANNALEN DER CHEMIE, vol. 698, 1966, pages 167-173, XP009041896
- JERCHEL, DIETRICH ET AL: "N-Pyridylaminophenole aus Pyridylaminophenyläthern; eine neue Umlagerungsreaktion" CHEMISCHE BERICHTE, vol. 92, 1959, pages 724-731, XP009041907
- MUSSER ET AL.: "N-Arylmethoxyphenyl- and N-Arylmethoxynaphthyl-sulfonamides" J.MED.CHEM., vol. 32, no. 6, 1989, pages 1176-1183, XP002300915

## Description

The present invention relates to malonamide derivatives, malonamide derivatives as medicaments, malonamide derivatives as inhibitors of one or more kinases, preferably of raf-kinase, the use of malonamide derivatives for the manufacture of a pharmaceutical, a method for producing a pharmaceutical composition containing said malonamide derivatives, the pharmaceutical composition obtainable by said method which can be employed in a method of treatment, comprising administering said pharmaceutical composition.

Protein phosphorylation is a fundamental process for the regulation of cellular functions. The coordinated action of both protein kinases and phosphatases controls the levels of phosphorylation and, hence, the activity of specific target proteins. One of the predominant roles of protein phosphorylation is in signal transduction, where extracellular signals are amplified and propagated by a cascade of protein phosphorylation and dephosphorylation events, e.g. in the p21^{ras}/raf pathway.

The p21^{ras} gene was discovered as an oncogene of the Harvey (rasH) and Kirsten (rasK) rat sarcoma viruses. In humans, characteristic mutations in the cellular ras gene (c-ras) have been associated with many different types of cancers. These mutant alleles, which render Ras constitutively active, have been shown to transform cells, such as the murine cell line NIH 3T3, in culture.

The p21^{ras} oncogene is a major contributor to the development and progression of human solid cancers and is mutated in 30 % of all human cancers (Bolton et al. (1994) Ann. Rep. Med. Chem., 29, 165-74; Bos. (1989) Cancer Res., 49, 4682-9). In its normal, unmutated form; the ras protein is a key element of the signal transduction cascade directed by growth factor receptors in almost all tissues (Avruch et al. (1994) Trends Biochem. Sci., 19, 279-83).

Biochemically, ras is a guanine nucleotide binding protein, and cycling between a GTP-bound activated and a GDP-bound resting form is strictly controlled by ras endogenous GTPase activity and other regulatory proteins. The ras gene product binds to guanine triphosphate (GTP) and guanine diphosphate (GDP) and hydrolyzes GTP to GDP. It is the GTP-bound state of Ras that is active. In the ras mutants in cancer cells, the endogenous GTPase activity is alleviated and, therefore, the protein delivers constitutive growth signals to downstream effectors such as the enzyme raf kinase. This leads to the cancerous growth of the cells which carry these mutants (Magnuson et al. (1994) Semin. Cancer Biol., 5, 247-53). The ras proto-oncogene requires a functionally intact c-raf1 proto-oncogene in order to transduce growth and differentiation signals initiated by receptor and non-receptor tyrosine kinases in higher eukaryotes.

Activated Ras is necessary for the activation of the c-raf1 proto-oncogene, but the biochemical steps through which Ras activates the Raf-1 protein (Ser/Thr) kinase are now well characterized. It has been shown that inhibiting the effect of active ras by inhibiting the raf kinase signaling pathway by administration of deactivating antibodies to raf kinase or by co-expression of dominant negative raf kinase or dominant negative MEK (MAPKK), the substrate of raf kinase, leads to the reversion of transformed cells to the normal growth phenotype see: Daum et al. (1994) Trends Biochem. Sci., 19, 474-80; Fridman et al. (1994) J Biol. Chem., 269, 30105-8. Kolch et al. (1991) Nature, 349, 426-28) and for review Weinstein-Oppenheimer et al. Pharm. & Therap. (2000), 88, 229-279.

Similarly, inhibition of raf kinase (by antisense oligodeoxynucleotides) has been correlated in vitro and in vivo with inhibition of the growth of a variety of human tumor types (Monia et al., Nat. Med. 1996, 2, 668-75).

Raf serine- and threonine-specific protein kinases are cytosolic enzymes that stimulate cell growth in a variety of cell systems (Rapp, U.R., et al. (1988) in The oncogene handbook; T. Curran, E.P. Reddy, and A. Skalka (ed.) Elsevier Science Publishers; The Netherlands, pp. 213-253; Rapp, U.R., et al. (1988) Cold Spring Harbor Sym. Quant. Biol. 53:173-184; Rapp, U.R., et al. (1990) Inv Curr. Top. Microbiol. Amunol. Potter and Melchers (eds), Berlin, Springer-Verlag 166:129-139).

Three isozymes have been characterized:
c-Raf (Raf-1) (Bonner, T.I., et al. (1986) Nucleic Acids Res. 14:1009-1015). A-Raf (Beck, T.W., et al. (1987) Nucleic Acids Res. 15:595-609), and B-Raf (Qkawa, S., et al. (1998) Mol. Cell. Biol. 8:2651-2654; Sithanandam, G. et a. (1990) Oncogene:1775). These enzymes differ in their expression in various tissues. Raf-1 is expressed in all organs and in all cell lines that have been examined, and A- and B-Raf are expressed in urogenital and brain tissues, respectively (Storm, S.M. (1990) Oncogene 5:345-351).

Raf genes are proto-oncogenes: they can initiate malignant transformation of cells when expressed in specifically altered forms. Genetic changes that lead to oncogenic activation generate a constitutively active protein kinase by removal or interference with an N-terminal negative regulatory domain of the protein (Heidecker, G., et al. (1990) Mol. Cell. Biol. 10:2503-2512; Rapp, U.R., et al. (1987) in Oncogenes and cancer S. A. Aaronson, J. Bishop, T. Sugimura, M. Terada, K. Toyoshima, and P. K. Vogt (ed). Japan Scientific Press, Tokyo). Microinjection into NIH 3T3 cells of oncogenically activated but not wild-type versions of the Raf-protein prepared with Escherichia coli expression vectors results in morphological transformation and stimulates DNA synthesis (Rapp, U.R., et al. (1987) in Oncogenes and cancer; S. A. Aaronson, J. Bishop, T. Sugimura, M. Terada, K. Toyoshima, and P. K. Vogt (ed.) Japan Scientific Press, Tokyo; Smith, M. R., et al (1990) Mol. Cell. Biol. 10:3828-3833). Activating mutants of B-Raf have been identified in a wide range of human cancers e.g. colon, ovarien, melanomas and sarcomas (Davies, H., et al. (2002), Nature 417 949-945. Published online June 9, 2002,
10.1038/nature00766). The preponderant mutation is a single phosphomimetic substitution in the kinase activation domain (V599E), leading to constitutive kinase activity and transformation of NIH3T3 cells.

Thus, activated Raf-1 is an intracellular activator of cell growth. Raf-1 protein serine kinase in a candidate downstream effector of mitogen signal transduction, since Raf oncogenes overcome growth arrest resulting from a block of cellular ras activity due either to a cellular mutation (ras revertant cells) or microinjection of anti-ras antibodies (Rapp, U.R., et al. (1988) in The Oncogene Handbook, T. Curran, E.P. Reddy, and A. Skalka (ed.), Elsevier Science Publishers; The Netherlands, pp. 213-253; Smith, M.R., et al. (1986) Nature (London) 320:540-543).

c-Raf function is required for transformation by a variety of membrane-bound oncogenes and for growth stimulation by mitogens contained in serums (Smith, M.R., et al. (1986) Nature (London) 320:540-543). Raf-1 protein serine kinase activity is regulated by mitogens via phosphorylation (Morrison, D.K., et al. (1989) Cell 58:648-657), which also effects sub cellular distribution (Olah, Z., et al. (1991) Exp. Brain Res. 84:403; Rapp, U.R., et al. (1988) Cold Spring Harbor Sym. Quant. Biol. 53:173-184. Raf-1 activating growth factors include platelet-derived growth factor (PDGF) (Morrison, D.K., et al. (1988) Proc. Natl. Acad. Sci. USA 85:8855-8859), colony-stimulating factor (Baccarini, M., et al. (1990) EMBO J. 9:3649-3657), insulin (Blackshear, P.J., et al. (1990) J. Biol. Chem. 265:12115-12118), epidermal growth factor (EGF) (Morrison, R.K., et al. (1988) Proc. Natl. Acad. Sci. USA 85:8855-8859), interleukin 2 (Turner, B.C., et al (1991) Proc. Natl. Acad. Sci. USA 88:1227), and interleukin 3 and granulocytemacrophage colony-stimulating factor (Carroll, M.P., et al (1990) J. Biol. Chem. 265:19812-19817).

Upon mitogen treatment of cells, the transiently activated Raf-1 protein serine kinase translocates to the perinuclear area and the nucleus (Olah, Z., et al. (1991) Exp. Brain Res. 84:403; Rapp, U.R., et al. (1988) Cold Spring Habor Sym. Quant. Biol. 53:173-184). Cells containing activated Raf are altered in their pattern of gene expression (Heidecker, G., et al. (1989) in Genes and signal transduction in multistage carcinogenesis, N. Colburn (ed.), Marcel Dekker, Inc., New York, pp. 339-374), and Raf oncogenes activate transcription from Ap-I/PEA3-dependent promoters in transient transfection assays (Jamal, S., et al (1990) Science 344:463-466; Kaibuchi, K., et al (1989) J. Biol. Chem. 264:20855-20858; Wasylyk, C., et al. (1989) Mol. Cell. Biol. 9:2247-2250).

There are at least two independent pathways for Raf-1 activation by extracellular mitogens: one involving protein kinase C (KC) and a second initiated by protein tyrosine kinases (Blackshear, P.J., et al. (1990) J. Biol. Chem. 265:12131-12134; Kovacina, K.S., et al (1990) J. Biol. Chem. 265:12115-12118; Morrison, D.K., et al. (1988) Proc. Natl. Acad. Sci. USA 85:8855-8859; Siegel, J.N., et al (1990) J. Biol. Chem. 265:18472-18480; Turner, B.C., et al (1991) Proc. Natl. Acad. Sci. USA 88:1227). In either case, activation involves Raf-1 protein phosphorylation. Raf-1 phosphorylation may be a consequence of a kinase cascade amplified by autophosphorylation or may be caused entirely by autophosphorylation initiated by binding of a putative activating ligand to the Raf-1 regulatory domain, analogous to PKC activation by diacylglycerol (Nishizuka, Y. (1986) Science 233:305-312).

The process of angiogenesis is the development of new blood vessels, generally capillaries, from pre-existing vasculature. Angiogenesis is defined as involving one or more of the following steps: (i) activation of endothelial cells; (ii) increased vascular permeability; (iii) subsequent dissolution of the basement membrane and extravisation of plasma components leading to formation of a provisional fibrin gel extracellular matrix; (iv) proliferation and mobilization of endothelial cells; (v) reorganization of mobilized endothelial cells to form functional capillaries; (vi) capillary loop formation; and (vii) deposition of basement membrane and recruitment of perivascular cells to newly formed vessels.

Normal angiogenesis is activated during tissue growth, from embryonic development through maturity, and then enters a period of relative quiescence during adulthood.

Normal angiogensesis is also activated during wound healing, and at certain stages of the female reproductive cycle. Inappropriate or pathological angiogenesis has been associated with several disease states including various retinopathies; ischemic disease; atherosclerosis; chronic inflammatory disorders; rheumatoid arthritis, and cancer. The role of angiogenesis in disease states is discussed, for instance, in Fan et al, Trends in Pharmacol Sci. 16:54 66; Shawver et al, DOT Vol. 2, No. 2 February 1997; Folkmann, 1995, Nature Medicine 1:27-31.

In cancer the growth of solid tumors has been shown to be angiogenesis dependent. (See Folkmann, J., J. Nat'l. Cancer Inst., 1990, 82, 4-6.) Consequently, the targeting of pro-angiogenic pathways is a strategy being widely pursued in order to provide new therapeutics in these areas of great, unmet medical need.

Raf is involved in angiogenic processes. Endothelial growth factors (e.g. vascular endothelial growth factor VEGF) activates receptor tyrosine kinases (e.g. VEGFR-2) and signal through the Ras/Raf/Mek/Erk kinase cascade. Activation of VEGFR-2 by VEGF is a critical step in the signal transduction pathway that initiates tumor angiogenesis. VEGF expression may be constitutive to tumor cells and can also be upregulated in response to certain stimuli. One such stimuli is hypoxia, where VEGF expression is upregulated in both tumor and associated host tissues. The VEGF ligand activates VEGFR-2 by binding with its extracellular VEGF binding site. This leads to receptor dimerization of VEGFRs and autophosphorylation of tyrosine residues at the intracellular kinase domain of VEGFR- 2. The kinase domain operates to transfer a phosphate from ATP to the tyrosine residues, thus providing binding sites for signaling proteins downstream of VEGFR-2 leading ultimately to initiation of angiogenesis (McMahon, G., The Oncologist, Vol. 5, No. 90001, 3-10, April 2000).

Mice with a targeted disruption in the Braf gene die of vascular defects during development (Wojnowski, L. et al. 1997, Nature genetics 16, page 293- 296). These mice show defects in the formation of the vascular system and in angiogenesis e.g. enlarged blood vessels and increased apoptotic death of differentiated endothelial cells.

For the identification of a signal transduction pathway and the detection of cross talks with other signaling pathways suitable models or model systems have been generated by various scientists, for example cell culture models (e.g. Khwaja et al., EMBO, 1997, 16, 2783-93) and transgenic animal models (e.g. White et al., Oncogene, 2001, 20, 7064-7072). For the examintion of particular steps in the signal transduction cascade, interfering compounds can be used for signal modulation (e.g. Stephens et al., Biochemical J., 2000, 351, 95-105). The compounds according to the invention may also be useful as reagents for the examination of kinase dependent signal transduction pathways in animal and/or cell culture models or any of the clinical disorders listed throughout this application.

The measurement of kinase activity is a well known technique feasible for each person skilled in the art. Generic test systems for kinase activity detection with substrates, for example histone (e.g. Alessi et al., FEBS Lett. 1996, 399, 3, page 333-8) or myelin basic protein are well described in the literature (e.g. Campos-González, R. and Glenney, Jr., J.R. 1992 J. Biol. Chem. 267, Page 14535).

For the identification of kinase inhibitors various assay systems are available (see for example Walters et al., Nature Drug Discovery 2003, 2; page 259-266). For example, in scintillation proximity assays (e.g. Sorg et al., J. of. Biomolecular Screening, 2002, 7, 11-19) or flashplate assays the radioactive phosphorylation of a protein or peptide as substrate with γATP can be measured. In the presence of an inhibitory compound no signal or a decreased radioactive signal is detectable. Furthermore homogeneous time-resolved fluorescence resonance energy transfer (HTR-FRET), and fluorescence polarization (FP) technologies are useful for assay methods (for example Sills et al., J. of Biomolecular Screening, 2002, 191-214).

Other non-radioactive ELISA based assay methods use specific phospho-antibodies (AB). The phospho-AB binds only the phosphorylated substrate. This binding is detectable with a secondary peroxidase conjugated antibody, measured for example by chemiluminescence (for exaple Ross et al., Biochem. J., 2002, 366, 977-981).

The present invention provides compounds generally described as malonamide derivatives, including both aryl and/or heteroaryl derivatives which are preferably inhibitors of the enzyme raf kinase. Since the enzyme is a downstream effector of p21^{ras}, the inhibitors are useful in pharmaceutical compositions for human or veterinary use where inhibition of one or more kinase pathways, preferably of the raf kinase pathway, is indicated, e.g., in the treatment of tumors and/or cancerous cell growth mediated by raf kinase. In particular, the compounds are useful in the treatment of human or animal solid cancers, e.g. murine cancer, since the progression of these cancers is dependent upon the ras protein signal transduction cascade and therefore susceptible to treatment by interruption of the cascade, i.e., by inhibiting one or more kinases, preferably by inhibiting raf kinase. Accordingly, the compound of Formula II or a pharmaceutically acceptable salt thereof can be administered for the treatment of diseases mediated by one or more kinase pathways, preferably by the raf kinase pathway, especially cancers, including solid cancers, such as, for example, carcinomas (e.g., of the lungs, pancreas, thyroid, bladder or colon), myeloid disorders (e.g., myeloid leukemia) or adenomas (e.g., villous colon adenoma), pathological angiogenesis and metastatic cell migration. Furthermore the compounds are useful in the treatment of complement activation dependent chronic inflammation (Niculescu et al. (2002) Immunol. Res., 24:191-199) and HIV-1 (human immunodeficiency virus type1) induced immunodeficiency (Popik et al. (1998)J Virol, 72: 6406-6413).

Therefore, subject of the present invention are malonamide derivatives of formula II, 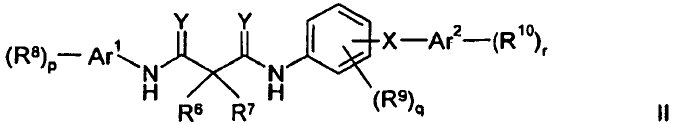 wherein
- Ar¹: is phenyl, pyridinyl, oxazolyl, isoxazolyl, pyrazolyl or imidazolyl,
- Ar²: is pyridinyl,
- R¹⁰: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)nO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³,
- k: is 0, 1 or 2,
- r: is 0, 1 or 2;
- R⁶, R⁷: are independently selected from the meanings given for R⁸, R⁹,
or R⁶ and R⁷ together form a carbocyclic residue comprising 3 to 7 carbon atoms or a heterocyclic residue comprising 1, 2 or 3 hetero atoms, selected from the group consisting of O, N and S, and 2 to 6 carbon atoms, said carbocyclic or heterocyclic residue being unsubstituted or comprising 1, 2 or 3 substituents, selected from the meanings given for R⁸, R⁹ and R¹⁰,
- R⁸, R⁹: are independently selected from a group consisting of H, A, cycloalkyl comprising 3 to 7 carbon atoms, Hal, CH₂Hal, CH(Hal)₂, C(Hal)₃, NO₂, (CH₂)ₙCN, (CH₂)ₙNR¹¹R¹², (CH₂)ₙOR¹¹, (CH₂)ₙO(CH₂)ₖNR¹¹R¹², (CH₂)ₙCOOR¹², (CH₂)ₙCONR¹¹R¹², (CH₂)ₙNR¹¹COR¹³, (CH₂)ₙNR¹¹CONR¹¹R¹², (CH₂)ₙNR¹¹SO₂A, (CH₂)ₙSO₂NR¹¹R¹², (CH₂)ₙS(O)ᵤR¹³, (CH₂)ₙOC(O)R¹³, (CH₂)ₙCOR¹³, (CH₂)ₙSR¹¹, CH=N-OA, CH₂CH=N-OA, (CH₂)ₙNHOA, (CH₂)ₙCH=N-R¹¹, (CH₂)ₙOC(O)NR¹¹R¹², (CH₂)ₙNR¹¹COOR¹², (CH₂)ₙN(R¹¹)CH₂CH₂OR¹³, (CH₂)ₙN(R¹¹)CH₂CH₂OCF₃, (CH₂)ₙN(R¹¹)C(R¹³)HCOOR¹², C(R¹³)HCOR¹², (CH₂)ₙN(R¹¹)CH₂CH₂N(R¹²)CH₂COOR¹², (CH₂)ₙN(R¹¹)CH₂CH₂NR¹¹R¹², CH=CHCOOR¹¹, CH=CHCH₂NR¹¹R¹², CH=CHCH₂NR¹¹R¹², CH=CHCH₂OR¹³, (CH₂)ₙN(COOR¹¹)COOR¹², (CH₂)ₙN(CONH₂)COOR¹¹, (CH₂)ₙN(CONH₂)CONH₂, (CH₂)ₙN(CH₂COOR¹¹)COOR¹², (CH₂)ₙN(CH₂CONH₂)COOR¹¹, (CH₂)ₙN(CH₂CONH₂)CONH₂, (CH₂)ₙCHR¹³COR¹¹, (CH₂)ₙCHR¹³COOR¹¹, (CH₂)ₙCHR¹³CH₂OR¹⁴, (CH₂)ₙOCN and (CH₂)ₙNCO, wherein
- R¹¹, R¹²: are independently selected from a group consisting of H, A and (CH₂)ₘAr³ or in NR¹¹R¹²,
- R¹¹ and R¹²: form, together with the N-Atom they are bound to, a 5-, 6- or 7-membered heterocyclus which optionally contains 1 or 2 additional hetero atoms, selected from N, O an S,
- R¹³, R¹⁴: are independently selected from a group consisting of H, Hal, A, (CH₂)ₘAr⁴ and (CH₂)ₘHet,
- A: is selected from the group consisting of alkyl, alkenyl, cycloalkyl, alkylenecycloalkyl, alkoxy and alkoxyalkyl,
- Ar³, Ar⁴: are independently from one another aromatic hydrocarbon residues comprising 5 to 12 and preferably 5 to 10 carbon atoms which are optionally substituted by one or more substituents, selected from a group consisting of A, Hal, NO₂, CN, OR¹⁵, NR¹⁵R¹⁶, COOR¹⁵, CONR¹⁵R¹⁶, NR¹⁵COR¹⁶, NR¹⁵CONR¹⁵R¹⁶, NR¹⁶SO₂A, COR¹⁵, SO₂R¹⁵R¹⁶, S(O)ᵤA and OOCR¹⁵,
- Het: is a saturated, unsaturated or aromatic heterocyclic residue which is optionally substituted by one ore more substituents, selected from a group consisting of A, Hal, NO₂, CN, OR¹⁵, NR¹⁵R¹⁶, COOR¹⁵, CONR¹⁵R¹⁶, NR¹⁵COR¹⁶, NR¹⁵CONR¹⁵R¹⁶, NR¹⁶SO₂A, COR¹⁵, SO₂R¹⁵R¹⁶, S(O)ᵤA and OOCR¹⁵,
- R¹⁵, R¹⁶: are independently selected from a group consisting of H, A, and (CH₂)ₘAr⁶, wherein
- Ar⁶: is a 5- or 6-membered aromatic hydrocarbon which is optionally substituted by one or more substituents selected from a group consisting of methyl, ethyl, propyl, 2-propyl, tert.-butyl, Hal, CN, OH, NH₂ and CF₃,
- m and n: are independently of one another 0, 1, 2, 3, 4, or 5,
- X: is selected from the group consisting of O, S, NR¹¹, CHOR¹¹, CH₂, CH₂CH₂, OCH₂, CH₂O, OCH₂CH₂, CH₂CH₂O
- h, i: are independently from each other 0, 1, 2, 3, 4, 5, or 6, and
- j: is 1, 2, 3, 4, 5, or 6,
- Y: is selected from O, S, NR²¹, C(R²²)-NO₂, C(R²²)-CN and C(CN)₂, wherein
- R²¹: is independently selected from the meanings given for R¹³, R¹⁴ and
- R²²: is independently selected from the meanings given for R¹¹, R¹²,
- p: is 0, 1, 2, 3, 4 or 5,
- q: is 0, 1, 2, 3 or 4, preferably 0, 1 or 2,
- u: is 0, 1, 2 or 3, preferably 0, 1 or 2,
and
- Hal: is independently selected from a group consisting of F, Cl, Br and I;
and the pharmaceutically acceptable solvates, salts and stereoisomers thereof, including mixtures thereof in all ratios, and more preferred the salts and/or solvates thereof, and especially preferred the physiologically acceptable salts and/or solvates thereof.

As used herein, the term "effective amount" means that amount of a drug or pharmaceutical agent that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

As used herein, the term "halogen" or "hal" preferably refers to fluorine (F), chlorine (Cl), bromine (Br) or iodine (I).

As used herein, the term "oxo" preferably refers to the group =O.

As used herein, the term "mercapto" preferably refers to the group -SH.

As used herein, the term "carboxy" preferably refers to the group -COOH.

As used herein, the term "cyano" preferably refers to the group -CN.

As used herein, the term "aminosulfonyl" preferably refers to the group -SO₂NH₂.

As used herein, the term "carbamoyl" preferably refers to the group -C(O)NH₂.

As used herein, the term "sulfanyl" shall refer to the group -S-.

As used herein, the term "sulfenyl" shall refer to the group -S(O)-.

As used herein, the term "sulfonyl" shall refer to the group -S(O)₂- or -SO₂-.

As used herein, the term "acyl" preferably refers to the group R_{F}C(O)-, where R_{F} is alkyl, cycloalkyl or heterocyclyl as defined herein.

As used herein, the term "aroyl" preferably refers to the group R_{C}C(O)-, where R_{C} is aryl as defined herein.

As used herein, the term "heteroaroyl" preferably refers to the group R_{E}C(O)-, where R_{E} is heteroaryl as defined herein.

As used herein, the term "alkoxycarbonyl" preferably refers to the group R_{A}OC(O)-, where R_{A} is alkyl as defined herein.

As used herein, the term "acyloxy" preferably refers to the group R_{F}C(O)O-, where R_{F} is alkyl, cycloalkyl, or heterocyclyl as defined herein.

As used herein, the term "aroyloxy" preferably refers to the group R_{C}C(O)O-, where R_{C} is aryl as defined herein.

As used herein, the term "heteroaroyloxy" preferably refers to the group R_{E}C(O)O-, where R_{E} is heteroaryl as defined herein.

As used herein, the term "carbonyl" or "carbonyl moiety" preferably refers to the group C=O.

As used herein, the term "thiocarbonyl" or "thiocarbonyl moiety" preferably refers to the group C=S.

As used herein, the term "amino", "amino group" or "amino moiety" preferably refers to the group NR_{G}R_{G'}, wherein R_{G} and R_{G'}, are preferably selected, independently from one another, from the group consisting of hydrogen, alkyl, haloalkyl, alkenyl, cycloalkyl, alkylenecycloalkyl, cyanoalkyl, aryl, aralkyl, heteroaryl, acyl and aroyl. If both R_{G} and R_{G'} are hydrogen, NR_{G}R_{G'} is also referred to as "unsubstituted amino moiety" or "unsubstituted amino group". If R_{G} and/or R_{G'} are other than hydrogen, NR_{G}R_{G'} is also referred to as "substituted amino moiety" or "substituted amino group".

As used herein, the term "imino" or "imino moiety" preferably refers to the group C=NR_{G}, wherein R_{G} is preferably selected from the group consisting of hydrogen, alkyl, haloalkyl, alkenyl, cycloalkyl, alkylenecycloalkyl, cyanoalkyl, aryl, aralkyl, heteroaryl, acyl and aroyl. If R_{G} is hydrogen, C=NR_{G} is also referred to as "unsubstituted imino moiety". If R_{G} is a residue other than hydrogen, C=NR_{G} is also referred to as "substituted imino moiety".

As used herein, the term "ethene-1,1-diyl moiety" preferably refers to the group C=CR_{K}R_{L}, wherein R_{K} and R_{L} are preferably selected, independently from one another, from the group consisting of hydrogen, halogen, alkyl, haloalkyl, alkenyl, cycloalkyl, nitro, alkylenecycloalkyl, cyanoalkyl, aryl, aralkyl, heteroaryl, acyl and aroyl. If both hydrogen R_{K} and R_{L} are hydrogen, C=CR_{K}R_{L} is also referred to as "unsubstituted ethene-1,1-diyl moiety". If one of R_{K} and R_{L} or both are a residue other than hydrogen, C=CR_{K}R_{L} is also referred to as "substituted ethene-1,1-diyl moiety".

As used herein, the terms "group", "residue" and "radical" or "groups", "residues" and "radicals" are usually used as synonyms, respectively, as it is common practice in the art.

As used herein, the term "optionally" means that the subsequently described event(s) may or may not occur, and includes both event(s), which occur, and events that do not occur.

As used herein, the term "pharmaceutically acceptable derivative" preferably refers to any physiologically functional derivative of a compound of the present invention, for example, an ester or an amide, which upon administration to a mammal is capable of providing (directly or indirectly) a compound of the present invention or an active metabolite thereof. Such derivatives are clear to those skilled in the art, without undue experimentation, and with reference to the teaching of Burger's Medicinal Chemistry And Drug Discovery, 5th Edition, Vol 1: Principles and Practice, which is incorporated herein by reference to the extent that it teaches physiologically functional derivatives. Such derivatives preferably include so-called prodrug-compounds, for example compounds according to the invention that are derivatized with alkyl groups, acyl groups, sugars or peptides, such as oligopeptides, and that are easily degraded or metabolized to the active compounds according to the invention. Such derivatives preferably include biodegradable polymer derivatives of the compounds according to the invention. Suitable polymers and methods for producing biodegradable polymeric derivatives are known in the art, for example from Int. J. Pharm. 115, 61-67 (1995).

As used herein, the term "solvate" preferably refers to a complex of variable stoichiometry formed by a solute (in this invention, a compound of formula II or a salt or physiologically functional derivative thereof) and a solvent. Such solvents for the purpose of the invention may not interfere with the biological activity of the solute. Examples of suitable solvents include, but are not limited to, water, methanol, ethanol and acetic acid. Preferably the solvent used is a pharmaceutically acceptable solvent. Examples of suitable pharmaceutically acceptable solvents include, without limitation, water, ethanol and acetic acid. Most preferably the solvent used is water. Examples for suitable solvates are the mono- or dihydrates or alcoholates of the compounds according to the invention.

As used herein, the term "substituted" preferably refers to substitution with the named substituent or substituents, multiple degrees of substitution being allowed unless otherwise stated.

Certain of the compounds described herein may contain one or more chiral atoms, or may otherwise be capable of existing as two or more stereoisomers, which are usually enantiomers and/or diastereomers. Accordingly, the compounds of this invention include mixtures of stereoisomers, especially mixtures of enantiomers, as well as purified stereoisomers, especially purified enantiomers, or stereoisomerically enriched mixtures, especially enantiomerically enriched mixtures. Also included within the scope of the invention are the individual isomers of the compounds represented by formula II above as well as any wholly or partially equilibrated mixtures thereof. The present invention also covers the individual isomers of the compounds represented by the formulas above as mixtures with isomers thereof in which one or more chiral Centers are inverted. Also, it is understood that all tautomers and mixtures of tautomers of the compounds of formula (II) are included within the scope of the compounds of formula (II) and preferably the formulae and subformulae corresponding thereto.

Racemates obtained can be resolved into the isomers mechanically or chemically by methods known per se. Diastereomers are preferably formed from the racemic mixture by reaction with an optically active resolving agent. Examples of suitable resolving agents are optically active acids, such as the D and L forms of tartaric acid, diacetyltartaric acid, dibenzoyltartaric acid, mandelic acid, malic acid, lactic acid or the various optically active camphorsulfonic acids, such as β-camphorsulfonic acid. Also advantageous is enantiomer resolution with the aid of a column filled with an optically active resolving agent (for example dinitrobenzoylphenyl-glycine); an example of a suitable eluent is a hexane/isopropanol/ acetonitrile mixture.
The diastereomer resolution can also be carried out by standard purification processes, such as, for example, chromatography or fractional crystallization.

It is of course also possible to obtain optically active compounds of the formula II by the methods described above by using starting materials which are already optically active.

Unless indicated otherwise, it is to be understood that reference to the compounds of formula II preferably includes the reference to the sub formulae corresponding thereto, for example the sub formulae II.1 to II.20 and preferably formulae IIa to IIh.

Subject of the present invention are especially compounds of formula II, in which one or more substituents or groups, preferably the major part of the substituents or groups has a meaning which is indicated as preferred, more preferred, even more preferred or especially preferred.

In compounds of formula II, the term alkyl preferably refers to an unbranched or branched alkyl residue, preferably an unbranched alkyl residue comprising 1, 2, 3, 4, 5, 6, 7, 8, 9 or 10, preferably 1, 2, 3, 4, 5 or 6, more preferred 1, 2, 3 or 4 and especially 1 or 2 carbon atoms, or a branched alkyl residue comprising 3, 4, 5, 6, 7, 8 ,9 or 10, preferably 3, 4, 5 or 6 more preferred 3 or 4 carbon atoms. The alkyl residues can be optionally substituted, especially by one or more halogen atoms, for example up to perhaloalkyl, by one or more hydroxy groups or by one or more amino groups, all of which can optionally be substituted by alkyl. If an alkyl residue is substituted by halogen, it usually comprises 1, 2, 3, 4 or 5 halogen atoms, depending on the number of carbon atoms of the alkyl residue. For example, a methyl group can comprise, 1, 2 or 3 halogen atoms, an ethyl group (an alkyl residue comprising 2 carbon atoms) can comprise 1, 2, 3, 4 or 5 halogen atoms. If an alkyl residue is substituted by hydroxy groups, it usually comprises one or two, preferably one hydroxy groups. If the hydroxy group is substituted by alkyl, the alkyl substituent comprises preferably 1 to 4 carbon atoms and is preferably unsubstituted or substituted by halogen and more preferred unsubstituted. If an alkyl residue is substituted by amino groups, it usually comprises one or two, preferably one amino groups. If the amino group is substituted by alkyl, the alkyl substituent comprises preferably 1 to 4 carbon atoms and is preferably unsubstituted or substituted by halogen and more preferred unsubstituted. According to compounds of formula II, alkyl is preferably selected from the group consisting of methyl, ethyl, trifluoro methyl, pentafluoro ethyl, isopropyl, tert.-butyl, 2-amino ethyl, N-methyl-2-amino ethyl, N,N-dimethyl-2-amino ethyl, N-ethyl-2-amino ethyl, N,N-diethyl-2-amino ethyl, 2-hydroxy ethyl, 2-methoxy ethyl and 2-ethoxy ethyl, further preferred of the group consisting of 2-butyl, n-pentyl, neo-nentyl, isopentyl, hexyl and n-decyl, more preferred of methyl, ethyl, trifluoro methyl, isoproply and tert.-butyl.

In compounds of formula II, alkenyl is preferably selected from the group consisting of allyl, 2- or 3-butenyl, isobutenyl, sec-butenyl, furthermore preferably 4-pentenyl, isopentenyl and 5-hexenyl.

In compounds of formula II, alkylene is preferably unbranched and is more preferably methylene or ethylene, furthermore preferably propylene or butylene.

In compounds of formula II, alkylenecycloalkyl preferably has 5 to 10 carbon atoms and is preferably methylenecyclopropyl, methylenencyclobutyl, furthermore preferably methylenecyclopentyl, methylenecyclohexyl or methylenecycloheptyl, furthermore alternatively ethylenecyclopropyl, ethylenecyclobutyl, ethylenecyclopentyl, ethylenecyclohexyl or ethylenencycloheptyl, propylenecyclopentyl, propylenecyclohexyl, butylenecyclopentyl or butylenecyclohexyl.

In compounds of formula II, the term "alkoxy" preferably comprises groups of formula O-alkyl, where alkyl is an alkyl group as defined above. More preferred, alkoxy is selected from group consisting of methoxy, ethoxy, n-propoxy, isopropoxy, 2-butoxy, tert.-butoxy and halogenated, especially perhalogenated, derivatives thereof. Preferred perhalogenated derivatives are selected from the group consisting of O-CCl₃, O-CF₃, O-C₂Cl₅, O-C₂F₅, O-C(CCl₃)₃ and O-C(CF₃)₃.

In compounds of formula II, the term "alkoxyalkyl" preferably comprises branched and unbranched residues, more preferred unbranched residues, of formula CᵤH₂ᵤ₊₁-O-(CH₂)ᵥ, wherein u and v are independently from each other 1 to 6. Especially preferred is u = 1 and v = 1 to 4.
In compounds of formula II the term "alkoxyalkyl" includes alkoxyalkyl groups as defined above, wherein one or more of the hydrogen atoms are substituted by halogen, for example up to perhalo alkoxyalkyl.

In compounds of formula II, cycloalkyl preferably has 3 - 7 carbon atoms and is preferably cyclopropyl or cyclobutyl, furthermore preferably cyclopentyl or cyclohexyl, furthermore also cycloheptyl, particularly preferably cyclopentyl.

In compounds of formula II, Ar³ to Ar⁶ are preferably selected independently from one another from phenyl, naphthyl and biphenyl which is optionally substituted by one or more substituents, selected from the group consisting of A, Hal, NO₂, CN, OR¹⁵, NR¹⁵R¹⁶, COOR¹⁵, CONR¹⁵R¹⁶, NR¹⁵COR¹⁶, NR¹⁵CONR¹⁵R¹⁶, NR¹⁶SO₂A, COR¹⁵, SO₂R¹⁵R¹⁶, S(O)ᵤA and OOCR¹⁵.

In compounds of formula II, het is preferably an optionally substituted aromatic heterocyclic residue and even more preferred and optionally substituted saturated heterocyclic residue, wherein the substituents are preferably selected from A, CN and Hal. Even more preferred, het is selected from the group consisting of 1-piperidyl, 1-piperazyl, 1-(4-methyl)-piperazyl, 4-methylpiperazin-1-yl amine, 4-morpholinyl, 1-4pyrrolidinyl, 1-pyrazolidinyl 1-(2-methyl)-pyrazolidinyl, 1-imidazolidinyl or 1-(3-methyl)-imidazolidinyl, thiophen-2-yl, thiophen-3-yl, 2-pyridyl, 3-pyridyl, 4-pyridyl, 2-oxazolyl, 4-oxazolyl, 5-oxazolyl, 2-thiazolyl, 4-thiazolyl, 5-thiazolyl, chinolinyl, isochinolinyl, 2-pyridazyl, 4-pyridazyl, 2-pyrimidyl, 4-pyrimidyl, 5-pyrimidyl, 2-pyrazinyl and 3-pyrazinyl.
Preferably, the sum of h and i exceeds 0.

A preferred aspect of the instant invention relates to compounds of formula II, wherein n is 0 or 1 and especially 0.

Another preferred aspect of the instant invention relates to compounds of formula II, wherein n is 0 in the residues R⁸ and/or R⁹.

Another preferred aspect of the instant invention relates to compounds of formula II, wherein n is 0 in the residues R⁶ and/or R⁷.

The invention relates in particular to compounds of the formula II in which at least one of said radicals has one of the preferred meanings given above.

Some more preferred groups of compounds may be expressed by the following sub-formulae II.1) to II.20), which correspond to the formula II and in which radicals not denoted in greater detail are as defined in the formula II, but in which
II.1)
   - Ar¹: is phenyl, pyridinyl, oxazolyl, isoxazolyl, pyrazolyl or imidazolyl, preferably phenyl, pyridinyl or isoxazolyl and especially phenyl or oxazolyl;
   II.2)
   - Ar¹: is phenyl, pyridinyl, oxazolyl, isoxazolyl, pyrazolyl or imidazolyl, preferably phenyl, pyridinyl or isoxazolyl and especially phenyl or oxazolyl, and
   - p: is 1, 2 or 3;
II.3)
   - Ar¹: is phenyl, pyridinyl, oxazolyl, isoxazolyl, pyrazolyl or imidazolyl, preferably phenyl, pyridinyl or isoxazolyl and especially phenyl or oxazolyl,
   - p: is 1, 2 or 3, and
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³;
   II.4)
   - Ar¹: is phenyl, pyridinyl, oxazolyl, isoxazolyl, pyrazolyl or imidazolyl, preferably phenyl, pyridinyl or isoxazolyl and especially phenyl or oxazolyl,
   - p: is 1, 2 or 3,
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³;
II.5)
   - Ar¹: is phenyl, pyridinyl, oxazolyl, isoxazolyl, pyrazolyl or imidazolyl, preferably phenyl, pyridinyl or isoxazolyl and especially phenyl or oxazolyl,
   - p: is 1, 2 or 3,
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³,
   - n: is 0, 1 or 2, preferably 0 or 1;
II.6)
   - Ar¹: is phenyl, pyridinyl, oxazolyl, isoxazolyl, pyrazolyl or imidazolyl, preferably phenyl, pyridinyl or isoxazolyl and especially phenyl or oxazolyl,
   - p: is 1, 2 or 3,
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³,
   - n: is 0, 1 or 2, preferably 0 or 1, and
   - q: is 0 or 1;
II.7)
   - Ar¹: is phenyl, pyridinyl, oxazolyl, isoxazolyl, pyrazolyl or imidazolyl, preferably phenyl, pyridinyl or isoxazolyl and especially phenyl or oxazolyl,
   - p: is 1, 2 or 3,
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³,
   - n: is 0, 1 or 2, preferably 0 or 1,
   - q: is 0 or 1, and
   - X: is selected from the group consisting of O, S, NR¹¹, CHOR¹¹, CH₂, CH₂CH₂, OCH₂, CH₂O, OCH₂CH₂, CH₂CH₂O, preferably O, S and CH₂ and especially O and S;
II.8)
   - Ar¹: is phenyl, pyridinyl, oxazolyl, isoxazolyl, pyrazolyl or imidazolyl, preferably phenyl, pyridinyl or isoxazolyl and especially phenyl or oxazolyl,
   - p: is 1, 2 or 3,
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³,
   - n: is 0, 1 or 2, preferably 0 or 1,
   - q: is 0 or 1, and
   - X: is selected from the group consisting of O, S, NR¹¹, CHOR¹¹, CH₂, CH₂CH₂, OCH₂, CH₂O, OCH₂CH₂, CH₂CH₂O, preferably O, S and CH₂ and especially O and S,
   - Ar²: is pyridinyl;
II.9)
   - Ar¹: is phenyl, pyridinyl, oxazolyl, isoxazolyl, pyrazolyl or imidazolyl, preferably phenyl, pyridinyl or isoxazolyl and especially phenyl or oxazolyl,
   - p: is 1, 2 or 3,
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³,
   - n: is 0, 1 or 2, preferably 0 or 1,
   - q: is 0 or 1, and
   - X: is selected from the group consisting of O, S, NR¹¹, CHOR¹¹, CH₂, CH₂CH₂, OCH₂, CH₂O, OCH₂CH₂, CH₂CH₂O, preferably O, S and CH₂ and especially O and S,
   - Ar²: is pyridinyl, and
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, preferably selected from the group consisting of alkyl comprising 1 to 4 carbon atoms, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹² and especially (CH₂)ₙCONR¹¹R¹²;
II.10)
   - Ar¹: is phenyl, pyridinyl, oxazolyl, isoxazolyl, pyrazolyl or imidazolyl, preferably phenyl, pyridinyl or isoxazolyl and especially phenyl or oxazolyl,
   - p: is 1, 2 or 3,
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³,
   - n: is 0, 1 or 2, preferably 0 or 1,
   - q: is 0 or 1, and
   - X: is selected from the group consisting of O, S, NR¹¹, CHOR¹¹, CH₂, CH₂CH₂, OCH₂, CH₂O, OCH₂CH₂, CH₂CH₂O, preferably O, S and CH₂ and especially O and S,
   - Ar²: is pyridinyl, and
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, preferably selected from the group consisting of alkyl comprising 1 to 4 carbon atoms, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹² and especially (CH₂)ₙCONR¹¹R¹²,
   - k: is 0, 1 or 2, preferably 0 or 2;
II.11)
   - Ar¹: is phenyl, pyridinyl, oxazolyl, isoxazolyl, pyrazolyl or imidazolyl, preferably phenyl, pyridinyl or isoxazolyl and especially phenyl or oxazolyl,
   - p: is 1, 2 or 3,
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³,
   - n: is 0, 1 or 2, preferably 0 or 1,
   - q: is 0 or 1, and
   - X: is selected from the group consisting of 0, S, NR¹¹, CHOR¹¹, CH₂, CH₂CH₂, OCH₂, CH₂O, OCH₂CH₂, CH₂CH₂O, preferably O, S and CH₂ and especially O and S,
   - Ar²: is pyridinyl, and
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, preferably selected from the group consisting of alkyl comprising 1 to 4 carbon atoms, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹² and especially (CH₂)ₙCONR¹¹R¹²,
   - k: is 0, 1 or 2, preferably 0 or 2 and
   - r: is 0, 1 or 2, preferably 0 or 1;
II.12)
   - p: is 1, 2 or 3,
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³,
   - n: is 0, 1 or 2, preferably 0 or 1,
   - q: is 0 or 1, and
   - X: is selected from the group consisting of O, S, NR¹¹, CHOR¹¹, CH₂, CH₂CH₂, OCH₂, CH₂O, OCH₂CH₂, CH₂CH₂O, preferably O, S and CH₂ and especially O and S,
   - Ar²: is pyridinyl, and
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, preferably selected from the group consisting of alkyl comprising 1 to 4 carbon atoms, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹² and especially (CH₂)ₙCONR¹¹R¹²,
   - k: is 0, 1 or 2, preferably 0 or 2 and
   - r: is 0, 1 or 2, preferably 0 or 1;
II.13)
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³,
   - n: is 0, 1 or 2, preferably 0 or 1,
   - q: is 0 or 1, and
   - X: is selected from the group consisting of O, S, NR¹¹, CHOR¹¹, CH₂, CH₂CH₂, OCH₂, CH₂O, OCH₂CH₂, CH₂CH₂O, preferably O, S and CH₂ and especially O and S,
   - Ar²: is pyridinyl, and
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, preferably selected from the group consisting of alkyl comprising 1 to 4 carbon atoms, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹² and especially (CH₂)ₙCONR¹¹R¹²,
   - k: is 0, 1 or 2, preferably 0 or 2 and
   - r: is 0, 1 or 2, preferably 0 or 1;
II.14)
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹². (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³,
   - X: is selected from the group consisting of O, S, NR¹¹, CHOR¹¹, CH₂, CH₂CH₂, OCH₂, CH₂O, OCH₂CH₂, CH₂CH₂O, preferably O, S and CH₂ and especially O and S,
   - Ar²: is pyridinyl, and
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, preferably selected from the group consisting of alkyl comprising 1 to 4 carbon atoms, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹² and especially (CH₂)ₙCONR¹¹R¹²,
   - k: is 0, 1 or 2, preferably 0 or 2 and
   - r: is 0, 1 or 2, preferably 0 or 1;
II.15)
   - R⁸: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³,
   - q: is 0 or 1, and
   - X: is selected from the group consisting of O, S, NR¹¹, CHOR¹¹, CH₂, CH₂CH₂, OCH₂, CH₂O, OCH₂CH₂, CH₂CH₂O, preferably O, S and CH₂ and especially O and S,
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ₙR¹³, preferably selected from the group consisting of alkyl comprising 1 to 4 carbon atoms, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹² and especially (CH₂)ₙCONR¹¹R¹²,
   - k: is 0, 1 or 2, preferably 0 or 2 and
   - r: is 0, 1 or 2, preferably 0 or 1;
II.16)
   - q: is 0 or 1, and
   - X: is selected from the group consisting of O, S, NR¹¹, CHOR¹¹, CH₂, CH₂CH₂, OCH₂, CH₂O, OCH₂CH₂, CH₂CH₂O, preferably O, S and CH₂ and especially O and S,
   - Ar²: is pyridinyl, and
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, preferably selected from the group consisting of alkyl comprising 1 to 4 carbon atoms, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹² and especially (CH₂)ₙCONR¹¹R¹²,
   - k: is 0, 1 or 2, preferably 0 or 2 and
   - r: is 0, 1 or 2, preferably 0 or 1;
II.17)
   - X: is selected from the group consisting of 0, S, NR¹¹, CHOR¹¹, CH₂, CH₂CH₂, OCH₂, CH₂O, OCH₂CH₂, CH₂CH₂O, preferably O, S and CH₂ and especially O and S,
   - Ar²: is pyridinyl, and
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, preferably selected from the group consisting of alkyl comprising 1 to 4 carbon atoms, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹² and especially (CH₂)ₙCONR¹¹R¹²,
   - k: is 0, 1 or 2, preferably 0 or 2 and
   - r: is 0, 1 or 2, preferably 0 or 1;
II.18)
   - Ar²: is pyridinyl, and
   - R¹⁰: is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, preferably selected from the group consisting of alkyl comprising 1 to 4 carbon atoms, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹² and especially (CH₂)ₙCONR¹¹R¹²,
   - k: is 0, 1 or 2, preferably 0 or 2 and
   - r: is 0, 1 or 2, preferably 0 or 1;
II.19)
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, preferably selected from the group consisting of alkyl comprising 1 to 4 carbon atoms, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹² and especially (CH₂)ₙCONR¹¹R¹²,
   - k: is 0, 1 or 2, preferably 0 or 2 and
   - r: is 0, 1 or 2, preferably 0 or 1;
II.20)
   - R¹⁰: is selected from the group consisting of H, alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³, preferably selected from the group consisting of alkyl comprising 1 to 4 carbon atoms, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹² and especially (CH₂)ₙCONR¹¹R¹², and
   - r: is 0, 1 or 2, preferably 0 or 1.

One preferred embodiment of the instant invention relates to compounds of formula II and preferably one or more of sub formulae II.1) to II.20), wherein p is 1, 2 or 3 and R⁸ is independently selected from the group consisting of methyl, ethyl, isopropyl, tert.-butyl, F, Cl, Br, CF₃, C(CF₃)₃, methoxy, ethoxy, tert.-butoxy, perfluoro tert.-butoxy (OC(CF₃)₃), methyl sulfanyl (SCH₃), ethyl sulfanyl (SCH₂CH₃), acetyl (COCH₃), propionyl (COCH₂CH₃), butyryl (COCH₂CH₂CH₃) and SO₂CF₃. If p is 2 or 3, all substituents can be the same or different.

Another preferred embodiment of the instant invention relates to compounds of formula II and preferably one or more of sub formulae II.1) to II.20), wherein X is selected from the group consisting of S, N-R²¹, CH₂, CH₂CH₂, OCH₂ and CH₂O.

Another preferred embodiment of the instant invention relates to compounds of formula II and preferably one or more of sub formulae II.1) to II.20), wherein X is selected from the group consisting of S, CH₂.

Another even more preferred embodiment of the instant invention relates to compounds of formula II and preferably one or more of sub formulae II.1) to II.20), wherein X is O.

Another preferred aspect of the instant invention relates to compounds of formula II, wherein n is 0 in the residues R⁶ and/or R⁷.

Another preferred embodiment of the instant invention relates to compounds of formula II and preferably one or more of sub formulae II.1) to II.20), wherein Y is selected from the group consisting of C(R²²)-NO₂, C(R²²)-CN and C(CN)₂.

Another more preferred embodiment of the instant invention relates to compounds of formula II and preferably one or more of sub formulae II.1) to II.20), wherein Y is selected from the group consisting of O, S and NR²¹.

Another even more preferred embodiment of the instant invention relates to compounds of formula II and preferably one or more of sub formulae II.1) to 11.20), wherein Y is selected from the group consisting of O and S.

Another even more preferred embodiment of the instant invention relates to compounds of formula II and preferably one or more of sub formulae II.1) to II.20), wherein Y is O.

Another preferred embodiment of the instant invention relates to compounds of formula II and preferably one or more of sub formulae II.1) to II.20), wherein R⁶ and R⁷ both are hydrogen.

Another preferred embodiment of the instant invention relates to compounds of formula II and preferably one or more of sub formulae II.1) to II.20), wherein R⁶ or R⁷ is a residue other than hydrogen. In this embodiment, the residue other than hydrogen is preferably selected from the meanings given for R⁸, R⁹ and R¹⁰, more preferably from A, and a especially preferred from substituted or preferably unsubstituted alkyl, substituted or preferably unsubstituted alkenyl, substituted or preferably unsubstituted cycloalkyl and substituted or preferably unsubstituted alkylenecycloalkyl, even more preferred substituted or unsubstituted alkyl with 1 to 6 carbon atoms, for example methyl, ethyl, n-propyl, isopropyl, n-butyl, 2-butyl, tert.-butyl, optionally substituted by one or more hydroxy groups, preferably one or two hydroxy groups and/or one or more halogen atoms, up to perhalo. Examples for preferred substituted alkyl groups are CH₂Hal, especially CH₂F, CH₂Cl and CH₂Br, CHal₃, especially CF₃, CCl₃ and CBr₃, and (CH₂)_{z}OH, wherein Z is 1 to 6, especially CH₂OH and CH₂CH₂OH.

Another preferred embodiment of the instant invention relates to compounds of formula II and preferably one or more of sub formulae II.1) to II.20), wherein R⁶ and R⁷ are residues other than hydrogen. In this embodiment, R⁶ and R⁷ are preferably selected, independently from one another, from the meanings given for R⁸, R⁹ and R¹⁰, more preferably from the meanings given for A, and a specially preferred from substituted or preferably unsubstituted alkyl, substituted or preferably unsubstituted alkenyl, substituted or preferably unsubstituted cycloalkyl and substituted or preferably unsubstituted alkylenecycloalkyl, even more preferred substituted or unsubstituted alkyl with 1 to 6 carbon atoms, for example methyl, ethyl, n-propyl, isopropyl, n-butyl, 2-butyl, tert.-butyl, optionally substituted by one or more hydroxy groups, preferably one or two hydroxy groups and/or one or more halogen atoms, up to perhalo. Examples for preferred substituted alkyl groups are CH₂Hal, especially CH₂F, CH₂Cl and CH₂Br, CHal₃, especially CF₃, CCl₃ and CBr₃, and (CH₂)_{z}OH, wherein Z is 1 to 6, especially CH₂OH and CH₂CH₂OH. In this embodiment, R⁶ and R⁷ even more preferred form, together with the carbon atom they are bound to (i. e. the carbon atom of the methylene moiety of the malonamide moiety), a carbocyclic residue comprising 3 to 6 carbon atoms or a heterocyclic residue comprising one or two heteroatoms, selected from the group consisting of O, N and S, and 2 to 5 carbon atoms, wherein the carbocyclic residue respectively the heterocyclic residue can be substituted by one or more substituents, preferably one or two substituents, selected, independently from one another, from the meanings given for R⁸, R⁹ and R¹⁰. If R⁶ and R⁷ form a cyclic residue together with the carbon atom they are bound to, carbocyclic residues are preferred. Even more preferred are carbocyclic residues comprising 3, 4 or 5 carbon atoms, especially 3 carbon atoms which can be substituted once or twice as given above and preferably are unsubstituted. In this respect, one preferred embodiment of the instant invention relates to compounds, wherein R⁶ and R⁷ form, together with the carbon atom they are bound to, a cyclopropane moiety.

Another preferred embodiment of the instant invention relates to compounds of formula II and preferably one or more of sub formulae II.1) to II.20), wherein R⁶ is hydrogen and R⁷ is methyl, or R⁶ is methyl and R⁷ is hydrogen.

Another preferred embodiment of the instant invention relates to compounds of formula II and preferably one or more of sub formulae II.1) to II.20), wherein one of the residues R⁶ or R⁷ or both residues R⁶ and R⁷ are other than hydrogen and are preferably as defined in the preferred embodiments relating to R⁶ and R⁷ given above.

Another preferred embodiment of the instant invention relates to compounds of formula II and preferably one or more of sub formulae II.1) to II.20), wherein r is either 0 or 1. If r is 1, R¹⁰ is preferably (CH₂)ₙCONR¹¹R¹² and especially (CH₂)ₙCONR¹¹R¹², wherein n is 0. In this embodiment, R¹¹ is preferably selected from the group consisting of H and A, more preferred from H and alkyl and especially is H, and R¹² is preferably selected from the group consisting of H and A and more preferred from H, unsubsitituted alkyl and substituted alkyl, preferably comprising 1 to 6 and especially 1 or 2 carbon atoms. Suitable for substituents include amino groups, such as NH₂, NHCH₃, NHCH₂CH₃, N(CH₃)₂ and NH(CH₂CH₃), and carboxyl groups and derivatives thereof, such as COOH, COOCH₃, CONH₂, and CONHCH₃. Especially preferred as residue R¹⁰ are CONHCH₃, CONHCH₂CH₂NH₂, CONHCH₂CH₂NHCH₃, CONHCH₂CH₂N(CH₃)₂, CONHCH₂COOH and CONHCH₂CH₂COOH. This embodiment is especially preferred when Ar² is pyridinyl. When Ar² is pyridinyl, R¹⁰ is preferably bonded in a vicinal position to the nitrogen atom of the pyrindiyl residue, i.e. in 2- and/or 6-position of the pyridinyl residue.

Another preferred embodiment of the instant invention relates to compounds of formula II and preferably one or more of sub formulae II.1) to II.20), wherein Ar¹ comprises two or more substituents R⁸, wherein one or more, preferably one substituent R⁸ is selected from the group consisting of (CH₂)ₙNR¹¹R¹², (CH₂)ₙO(CH₂)ₖNR¹¹R¹², (CH₂)ₙNR¹¹(CH₂)ₖOR¹², (CH₂)ₙNR¹¹(CH₂)ₖNR¹²R¹², (CH₂)ₙCOOR¹³ and (CH₂)ₙS(O)ᵤR¹³ wherein R¹¹, R¹² and R¹³ are defined as above and n is as defined above, preferably n is 0, 1 or 2 and especially is 0, k is 1 to 4 and preferably 1 or 2, and u is preferably 2. In this embodiment R¹¹, R¹² and R¹³ are more preferably selected independently from each other from the group consisting of H, methyl and ethyl. In this embodiment, one or two substituents R⁸ and preferably one substituent R⁸ is especially preferably selected from the group consisting of NH₂, N(CH₃)₂, N(C₂H₅)₂, NHCH₂CH₂NH₂, N(CH₃)CH₂CH₂NH₂, N(CH₃)CH₂CH₂N(CH₃)₂, N(CH₃)CH₂CH₂N(CH₃)₂, N(CH₃)CH₂CH₂OCH₃, OCH₂CH₂N(CH₃)₂, SCH₃, SC₂H₅, SO₂CH₃, COOCH₃ and COOH. Accordingly, in this embodiment Ar¹ especially preferably comprises at least one substituent R⁸ other than (CH₂)ₙNR¹¹R¹², (CH₂)ₙO(CH₂)ₖNR¹¹R¹², (CH₂)ₙNR¹¹(CH₂)ₖOR¹², (CH₂)ₙNR¹¹(CH₂)ₖNR¹²R¹², (CH₂)ₙCOOR¹³ and(CH₂)ₙS(O)ᵤR¹³ as defined in this paragraph and especially other than NH₂, N(CH₃)₂, N(C₂H₅)₂, NHCH₂CH₂NH₂, N(CH₃)CH₂CH₂NH₂, N(CH₃)CH₂CH₂N(CH₃)₂, N(CH₃)CH₂CH₂N(CH₃)₂, N(CH₃)CH₂CH₂OCH₃, OCH₂CH₂N(CH₃)₂, SCH₃, SC₂H₅, SO₂CH₃, COOCH₃ and COOH.

Another preferred embodiment of the instant invention relates to compounds of formula II and preferably one or more of sub formulae II.1) to II.20), wherein q is 1, i.e. the phenylen moiety bound to the malonamide group and the radical X is substituted once, preferably by a substituent selected from the group consisting of alkyl and halogen and more preferred from methyl, ethyl, F, Cl and Br.

Another preferred embodiment of the instant invention relates to compounds of formula II and preferably one or more of sub formulae II.1) to II.20), wherein q is 0, i.e. the phenyten moiety bound to the malonamide group and the radical X is unsubstituted.

Another preferred embodiment of the instant invention relates to compounds of formula II and preferably one or more of formulae II.1) to II.20), wherein (R⁸)ₚ-Ar¹ is selected from the group consisting of 3-acetyl-phenyl, 4-acetyl-phenyl, 2-bromo-phenyl, 3-bromo-phenyl, 4-bromo-phenyl, 4-bromo-2-chloro-phenyl, 4-bromo-3-methyl-phenyl, 4-bromo-3-trifluoromethyl-phenyl, 2-chloro-phenyl, 2-chloro-4-trifluoromethyl-phenyl, 2-chloro-5-trifluoromethyl-phenyl, 3-chloro-phenyl, 3-chloro-4-methyl-phenyl, 3-chloro-4-methoxy-phenyl, 3-chloro-4-methoxy-phenyl, 4-chloro-phenyl, 4-chloro-2-trifluoromethyl-phenyl, 4-chloro-3-trifluoromethyl-phenyl, 4-chloro-2-methyl-phenyl, 5-chloro-2-methyl-phenyl, 5-chloro-2-methoxy-phenyl, 4-chloro-2-methoxy-5-methyl-phenyl, 4-chloro-2-methoxy-5-trifluoromethyl-phenyl, 2,3-dichloro-phenyl, 2,4-dichloro-phenyl, 2,5-dichloro-phenyl, 3,4-dichloro-phenyl, 3,5-dichloro-phenyl, 2,4,5-trichloro-phenyl, 4-fluoro-phenyl, 4-fluoro-3-trifluoromethyl-phenyl, 4-ethoxy-phenyl, 2-methoxy-phenyl, 2-methoxy-5-trifluoromethyl-phenyl, 4-methoxy-phenyl, 2,5-dimethoxy-phenyl, 2-trifluoromethyl-phenyl, 3-trifluoromethyl-phenyl, 3-trifluoromethoxy-phenyl, 4-trifluoromethyl-phenyl, 4-trifluoromethoxy-phenyl, 3,5-bis-trifluoromethyl-phenyl, 3-methoxy-phenyl, 3-methylsulfanyl-phenyl, 4-methylsulfanyl-phenyl, o-tolyl (2-methyl-phenyl), m-tolyl (3-methyl-phenyl), p-tolyl (4-methyl-phenyl), 2,3-dimethyl-phenyl, 2,3-di-methyl-phenyl, 2,5-dimethyl-phenyl, 3,4-dimethyl-phenyl, 3,5-dimethyl-phenyl, 2-ethyl-phenyl, 3-ethyl-phenyl, 4-ethyl-phenyl, 4-isopropyl-phenyl, 4-tert-butyl-phenyl and 5-tert-butyl-isoxazol-3-yl.

Another preferred embodiment of the instant invention relates to compounds of formula II and preferably one or more of sub formulae II.1) to 11.20), wherein (R⁸)ₚ-Ar¹ is as defined above, but comprises one or more additional residues, preferably one additional residue. The additional residues are preferably selected from the meanings given for R⁸ and more preferably selected from the group consisting of (CH₂)ₙNR¹¹R¹², (CH₂)ₙO(CH₂)ₖNR¹¹R¹², (CH₂)ₙNR¹¹(CH₂)ₖOR¹², (CH₂)ₙNR¹¹(CH₂)ₖNR¹²R¹², (CH₂)ₙCOOR¹³ and (CH₂)ₙS(O)ᵤR¹³ wherein R¹¹, R¹² and R¹³ are defined as above and n is as defined above, preferably n is 0, 1 or 2 and especially is 0, k is 1 to 4 and preferably 1 or 2, and u is preferably 2. In this embodiment R¹¹, R¹² and R¹³ are more preferably selected independently from each other from the group consisting of H, methyl and ethyl. Even more preferred, the additional residue(s) is/are selected from the group consisting of NH₂, N(CH₃)₂, N(C₂H₅)₂, NHCH₂CH₂NH₂, N(CH₃)CH₂CH₂NH₂, N(CH₃)CH₂CH₂N(CH₃)₂, N(CH₃)CH₂CH₂N(CH₃)₂, N(CH₃)CH₂CH₂OCH_{3,} OCH₂CH₂N(CH₃)₂, SCH₃, SC₂H₅, SO₂CH₃, COOCH₃ and COOH.

Another preferred embodiment of the instant invention relates to compounds of formula II and preferably one or more of sub formulae II.1) to II.20), wherein X is bonded in the para- (p-) or metha- (m-)position to the phenyl residue that is bonded directly to the malonamide moiety.

Another preferred embodiment of the instant invention relates to compounds of formula II and preferably one or more of sub formulae II.1) to II.20), wherein Ar² is a pyridinyl residue and wherein said pyridinyl residue is bonded to X in the 3- or 4-position, preferably the 4-position, relative to the nitrogen atom of the pyridinyl residue.

Another preferred embodiment of the instant invention relates to compounds of formula II and preferably one or more of sub formulae II.1) to II.20), wherein Ar² comprises one or more substituents R¹⁰ and wherein one or two, preferably one substituent R¹⁰ is selected from unsubstituted or substituted carbamoyl moieties. Substituted carbamoyl moieties are preferably selected from CONHR²³ or CONR²³R²⁴, preferably CONHR²³, wherein R²³ and R²⁴ are independently selected from the definitions given for R⁸, more preferably selected from alkyl, preferably methyl, ethyl, propyl and butyl, (CH₂)ₙNR¹¹R¹² and (CH₂)ₙOR¹², wherein R¹¹, R¹² and n are as defined above. In this embodiment, n is preferably not 0 and more preferred 1 to 3 and especially 1 or 2. Preferred examples for R²³ are selected from the group consisting of methyl, ethyl, CH₂CH₂NH₂, CH₂CH₂N(CH₃)₂, CH₂CH₂N(CH₂CH₃)_{2,} CH₂CH₂OH, CH₂CH₂OCH₃ and CH₂CH₂OCH₂CH₃.

Another preferred embodiment of the instant invention relates to compounds of formula II and preferably one or more of sub formulae II.1) to II.20), wherein Ar² comprises one or more substituents R¹⁰ and wherein one or two, preferably one substituent R¹⁰ is selected from substituted carbamoyl moieties. Substituted carbamoyl moieties are preferably selected from CONHR²³, wherein R²³ is preferably unsubstituted C₁-C₄-alkyl and especially methyl.

Another preferred embodiment of the instant invention relates to compounds of formula II and preferably one or more of sub formulae II.1) to II.20), wherein Ar² comprises one or more substituents R¹⁰ and wherein one or two, preferably one substituent R¹⁰ is selected from substituted carbamoyl moieties. Substituted carbamoyl moieties are preferably selected from CONHR²³, wherein R²³ is selected from (CH₂)ₙNR¹¹R¹² and (CH₂)ₙOR¹², wherein R¹¹, R¹² and n are as defined above. In this embodiment, n is preferably not 0 and more preferred 1 to 3 and especially 1 or 2. Preferred examples for R²³ are selected from the group consisting of CH₂CH₂NH₂, CH₂CH₂N(CH₃)₂, CH₂CH₂N(CH₂CH₃)₂, CH₂CH₂OH, CH₂CH₂OCH₃ and CH₂CH₂OCH₂CH₃.

Another preferred embodiment of the instant invention relates to compounds of formula II and preferably one or more of sub formulae II.1) to II.20), wherein the benzimidazole-moiety comprises one or more substituents R⁸ and wherein one or two, preferably one substituent R⁸ is selected from the group consisting of NH₂, N(CH₃)₂, NHCH₃, N(C₂H₅)₂, HNCH₂CH₂NH₂, OCH₂CH₂NH₂, HOCH₂CH₂NH, OCH₂CH₂NHCH₃, N(CH₃)CH₂CH₂NH₂, HN(CH₃)CH₂CH₂NH, N(CH₃)CH₂CH₂N(CH₃)₂, N(CH₃)CH₂CH₂N(CH₃)₂, N(CH₃)CH₂CH₂OCH₃, OCH₂CH₂N(CH₃)₂, OCH₂CH₂N(CH₂CH₃)₂, SCH₃, SC₂H₅, and compounds of the formulae 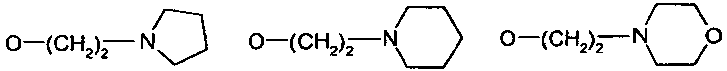 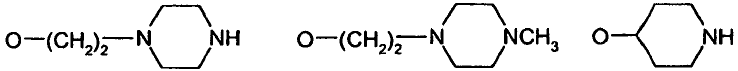 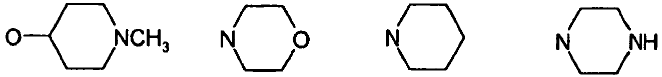 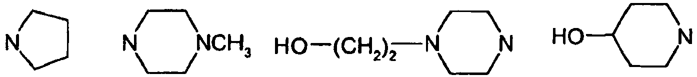 and/or Ar² comprises one or more substituents R¹⁰ and wherein one or two, preferably one substituent R¹⁰ is independently selected from the meanings given for R⁸ in this paragraph. In this embodiment, Ar¹ and/or Ar² preferably additionally comprise one or more substituents R⁸ and R¹⁰, respectively, which are other than the residues defined in this paragraph.

Another preferred embodiment of the instant invention relates to compounds of formula II and preferably one or more of sub formulae II.1) to II.20), wherein -Ar²-(R¹⁰) is selected from the formulae 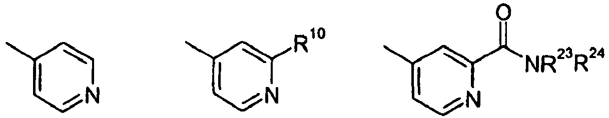 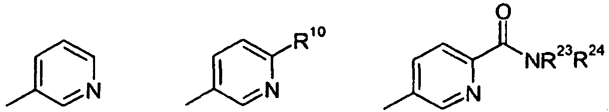 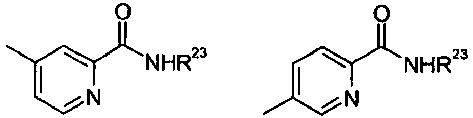 wherein R¹⁰, R²³ and R²⁴ are as defined above and below.

Another especially preferred embodiment of the instant invention relates to compounds of formula II and preferably one or more of sub formulae II.1) to II.20), wherein one or more features of the above and below mentioned embodiments are combined in one compound.

Subject of the present invention are therefore especially preferred compounds of formula II according to one or more of the formulae IIa, IIb, IIc, IId, IIe IIf, IIg and IIh, 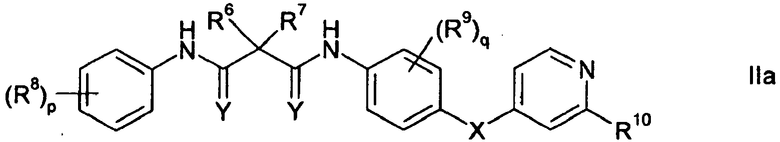 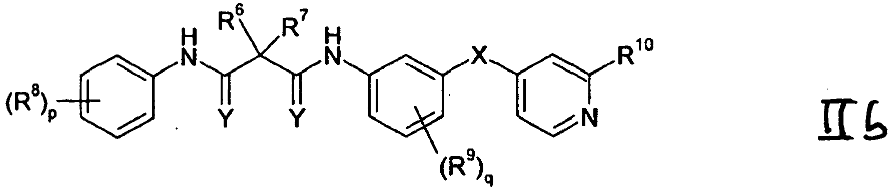 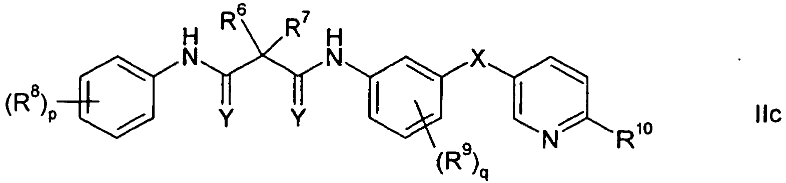 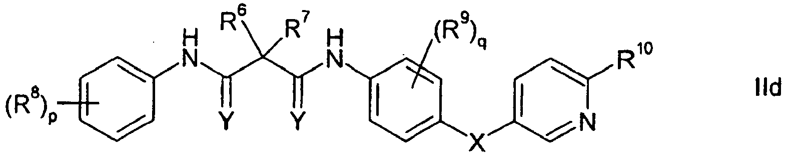 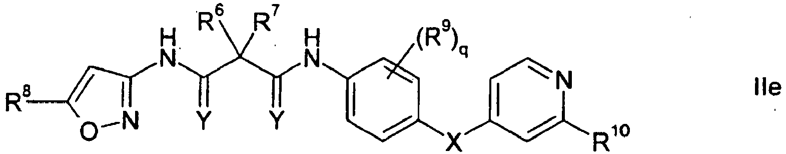 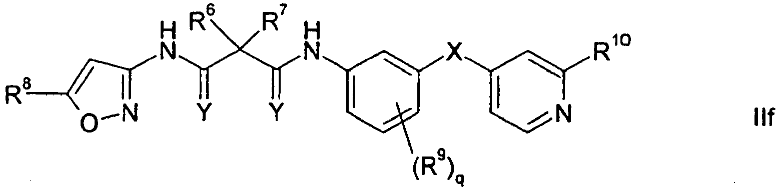 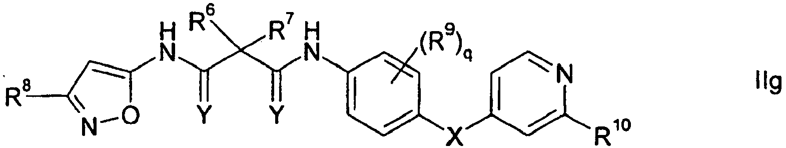 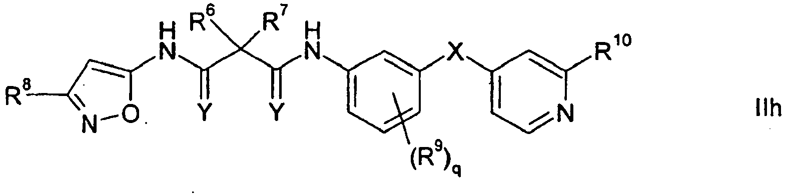 wherein R⁶, R⁷, R⁸, p, X, Y, R⁹, q are as defined above and below and preferably as defined in sub formulae II.1) to II.20) and/or the embodiments related thereto, and R¹⁰ is H or as defined ss defined above and below, and preferably as defined in sub formulae II.1) to II.20) and/or the embodiments related thereto.

One preferred aspect of the invention relates to compounds of formula II and especially to compounds of one or more of formulae IIa to IIh, wherein both R⁶ and R⁷ are hydrogen.

Another preferred aspect of the invention relates to compounds of formula II and especially to compounds of one or more of formulae IIa to IIh, wherein R⁶ and/or R⁷ are residues other than hydrogen.

Another preferred embodiment of the instant invention relates to compounds of formula II and preferably one or more of sub formulae II.1) to II.20) and IIa to IIh, wherein R¹⁰ is a substituted carbamoyl moiety CONHR²³ or CONR²³R²⁴, preferably CONHR²³, wherein R²³ and R²⁴ are independently selected from the definitions given for R⁸, more preferably selected from (CH₂)ₙNR¹¹R¹² and (CH₂)ₙOR¹², wherein R¹¹, R¹² and n are as defined above. In this embodiment, n is preferably not 0 and more preferred 1 to 4 and especially 1 or 2. Preferred examples for R²³ are selected from the group consisting of CH₂CH₂NH₂, CH₂CH₂N(CH₃)₂, CH₂CH₂N(CH₂CH₃)₂, CH₂CH₂OH, CH₂CH₂OCH₃, CH₂CH₂OCH₂CH₃ and from the formulae

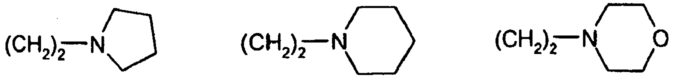

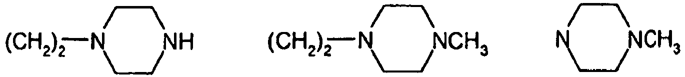

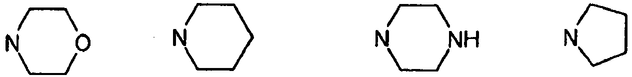

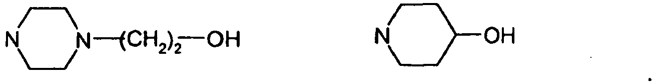

It is understood that when a residue, for example R⁸, R⁹, R¹⁰ or R¹⁴ or R²³, is comprised twice or more times in II and the sub formulae corresponding thereto, it is in each case independently from one another selected from the meanings given for the respective residue. For example, R¹¹ and R¹² are defined to be independently selected from a group consisting of H, A, (CH₂)ₘAr³ and (CH₂)ₘHet. Then (CH₂)ₙNR¹¹(CH₂)ₘNR¹²R¹² can be (CH₂)ₙNA(CH₂)ₘNA₂ (if R¹¹ = A, R¹² = A and R¹² = H) as well as (CH₂)ₙNA(CH₂)ₘNHA (if R¹¹ = A, R¹² = H and R¹² = A or (CH₂)ₙNA(CH₂)ₘNH(CH₂)ₘHet (if R¹¹ = A, R¹² = H and R¹² = (CH₂)ₘHet). Accordingly, if a compound of formula II comprises one residue R⁸, R⁹ and R¹⁰, then for example R⁸, R⁹ and R¹⁰ can all be (CH₂)ₙCOOR¹³, wherein all residues R¹³ are the same (for example CH₂Hal, wherein Hal is Cl; then all residues R⁸, R⁹ and R¹⁰ are the same) or different (for example CH₂Hal, wherein in R⁸ Hal is Cl; in R⁹ Hal is F; and in R¹⁰ Hal is Br; then all residues R⁸, R⁹ and R¹⁰ are different); or for example R⁸ is (CH₂)ₙCOOR¹³, R⁹ is NO₂ and R¹⁰ is (CH₂)ₙSR¹¹, wherein R¹¹ and R¹³ can be the same (for example both can be H or both can be A which is methyl) of different (for example R¹¹ can be H and R¹³ can be A which is methyl).

If not stated otherwise, reference to compounds of formula II also includes the sub formulae related thereto, especially sub formulae II.1) to II.20) and IIa to IIh.

Subject of the instant invention are especially those compounds of formula formula II, in which at least one of the residues mentioned in said formulae has one of the preferred or especially preferred meanings given above and below.

The present invention further relates to compounds (1) to (228) of formula A-NH-CO-CH₂-CO-NH-B, wherein A and B are as given in the table below:

| | A | B |
|---|---|---|
| (1) | 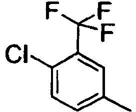 | 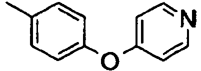 |
| (2) | 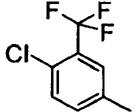 | 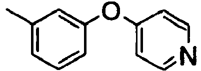 |
| (3) | 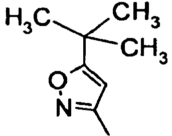 | 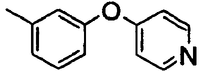 |
| (4) | 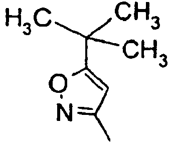 | 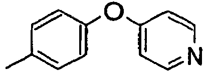 |
| (5) | 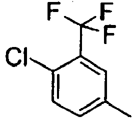 | 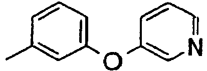 |
| (6) | 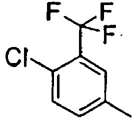 | 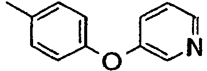 |
| (7) | 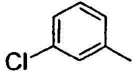 | 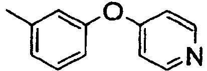 |
| (8) | 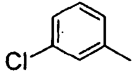 | 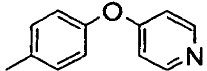 |
| (9) | 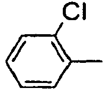 | 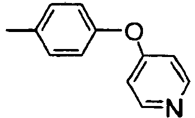 |
| (10) | 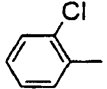 | 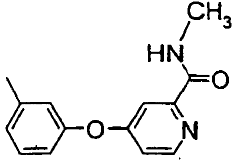 |
| (11) | 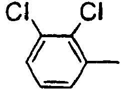 | 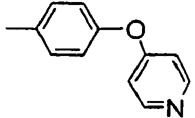 |
| (12) | 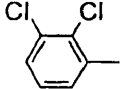 | 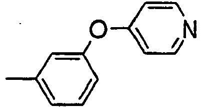 |
| (13) | 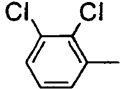 | 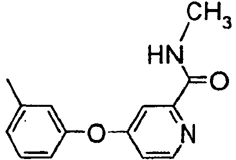 |
| (14) | 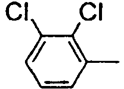 | 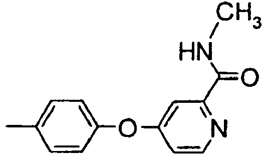 |
| (15) | 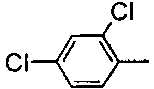 | 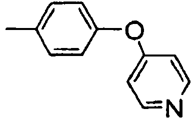 |
| (16) | 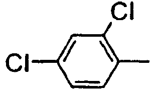 | 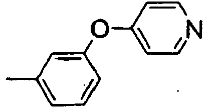 |
| (17) | 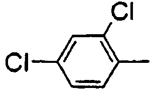 | 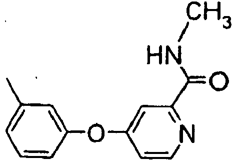 |
| (18) | 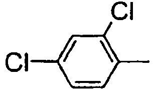 | 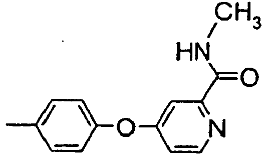 |
| (19) | 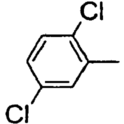 | 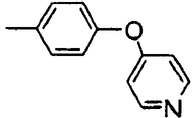 |
| (20) | 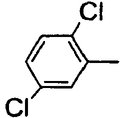 | 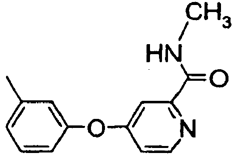 |
| (21) | 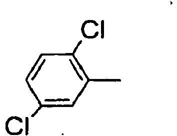 | 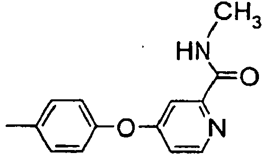 |
| (22) | 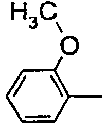 | 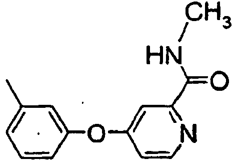 |
| (23) | 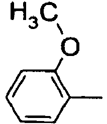 | 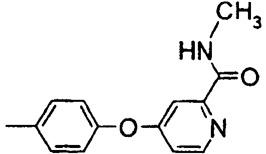 |
| (24) | 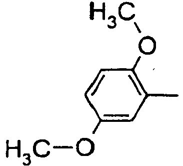 | 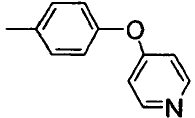 |
| (25) | 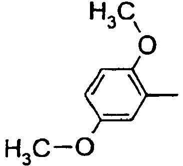 | 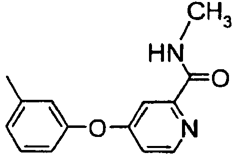 |
| (26) | 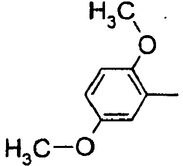 | 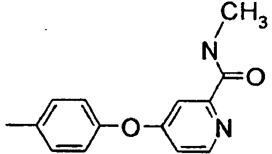 |
| (27) | 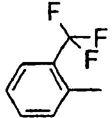 | 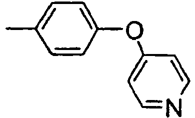 |
| (28) | 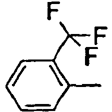 | 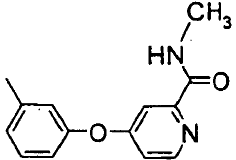 |
| (29) | 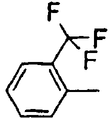 | 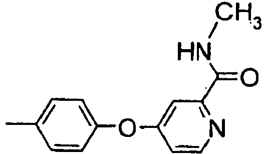 |
| (30) | 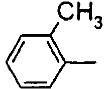 | 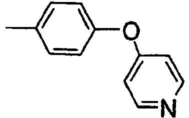 |
| (31) | 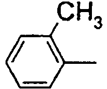 | 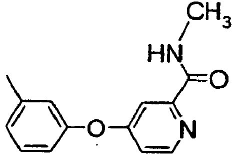 |
| (32) | 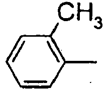 | 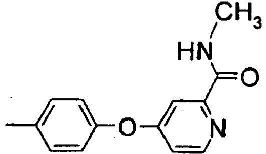 |
| (33) | 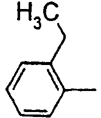 | 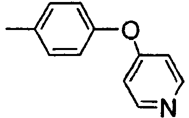 |
| (34) | 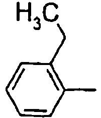 | 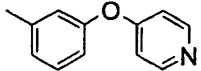 |
| (35) | 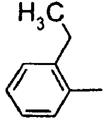 | 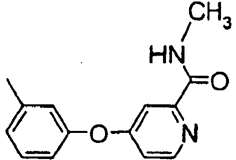 |
| (36) | 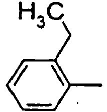 | 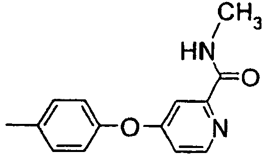 |
| (37) | 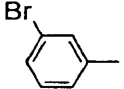 | 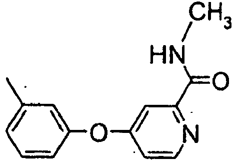 |
| (38) | 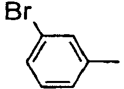 | 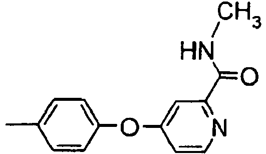 |
| (39) | 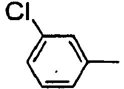 | 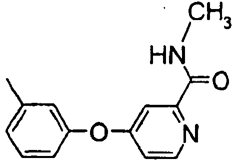 |
| (40) | 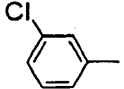 | 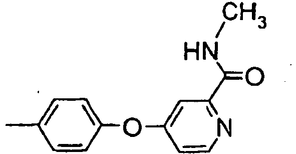 |
| (41) | 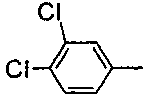 | 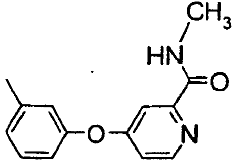 |
| (42) | 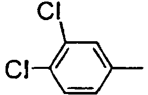 | 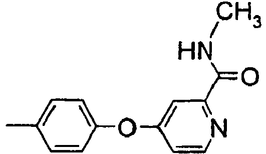 |
| (43) | 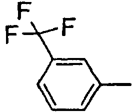 | 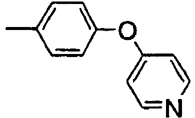 |
| (44) | 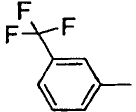 | 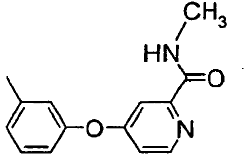 |
| (45) | 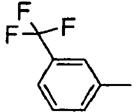 | 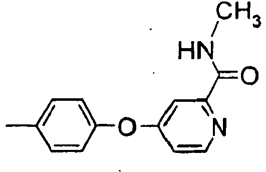 |
| (46) | 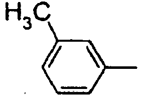 | 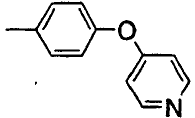 |
| (47) | 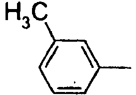 | 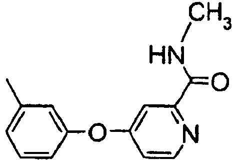 |
| (48) | 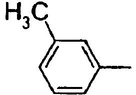 | 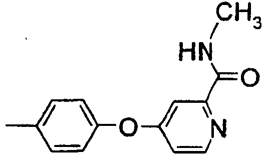 |
| (49) | 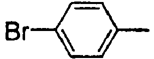 | 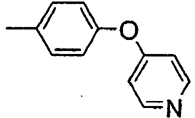 |
| (50) | 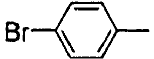 | 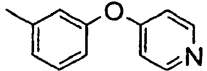 |
| (51) | 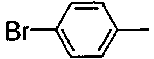 | 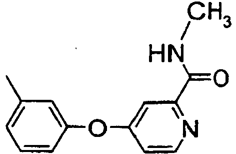 |
| (52) | 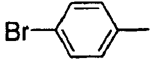 | 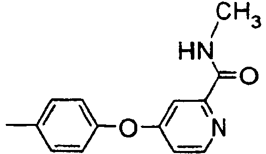 |
| (53) | 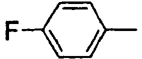 | 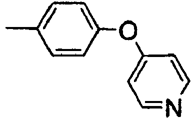 |
| (54) | 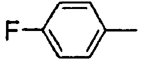 | 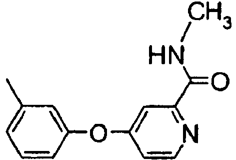 |
| (55) | 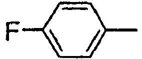 | 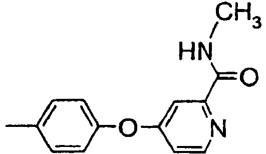 |
| (56) | 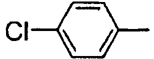 | 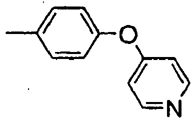 |
| (57) | 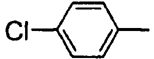 | 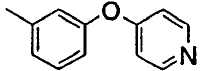 |
| (58) | 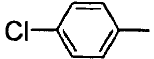 | 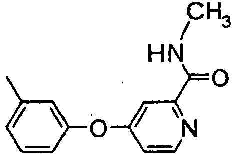 |
| (59) | 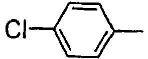 | 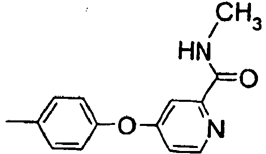 |
| (60) | 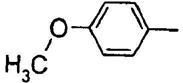 | 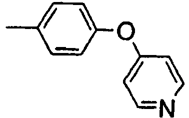 |
| (61) | 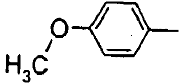 | 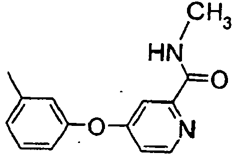 |
| (62) | 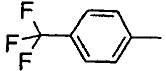 | 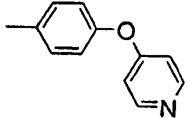 |
| (63) | 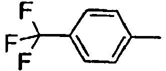 | 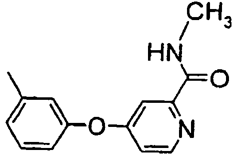 |
| (64) | 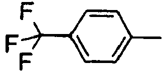 | 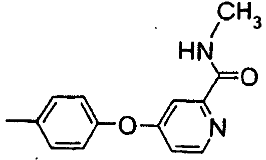 |
| (65) | 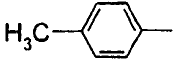 | 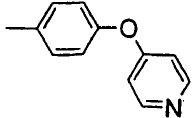 |
| (66) | 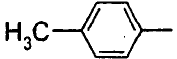 | 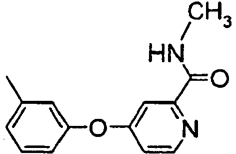 |
| (67) | 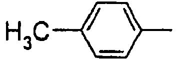 | 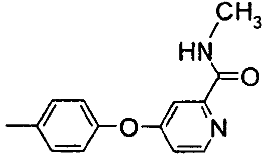 |
| (68) | 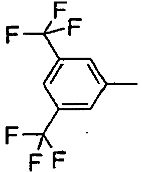 | 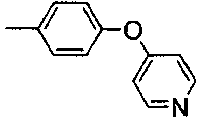 |
| (69) | 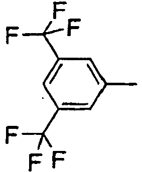 | 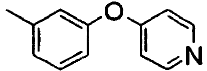 |
| (70) | 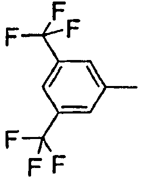 | 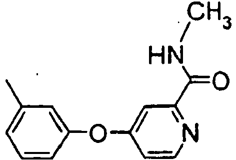 |
| (71) | 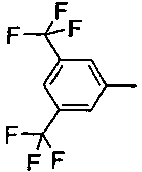 | 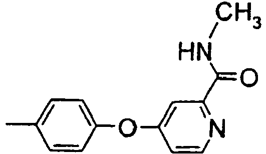 |
| (72) | 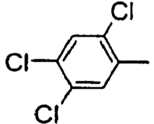 | 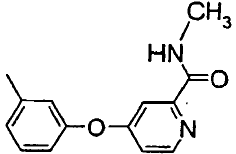 |
| (73) | 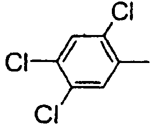 | 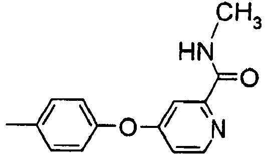 |
| (74) | 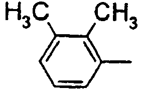 | 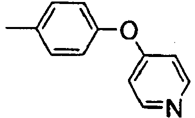 |
| (75) | 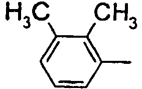 | 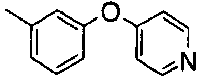 |
| (76) | 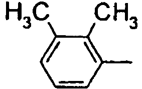 | 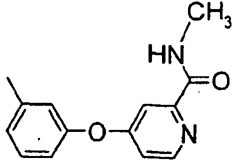 |
| (77) | 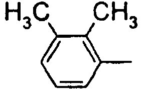 | 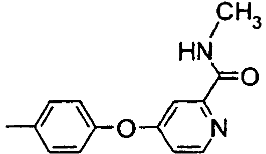 |
| (78) | 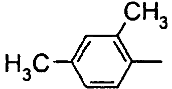 | 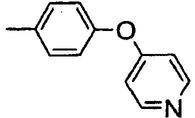 |
| (79) | 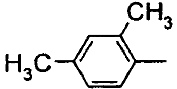 | 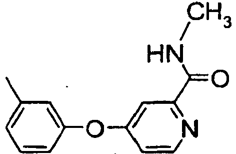 |
| (80) | 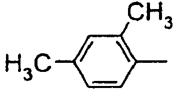 | 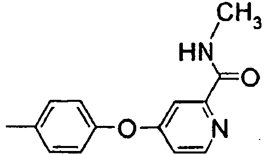 |
| (81) | 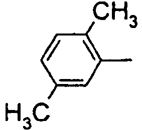 | 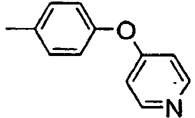 |
| (82) | 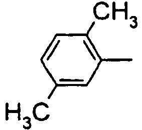 | 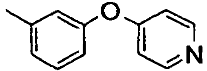 |
| (83) | 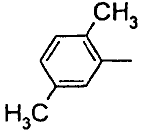 | 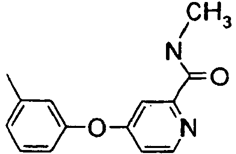 |
| (84) | 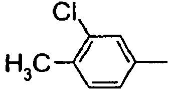 | 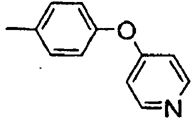 |
| (85) | 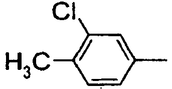 | 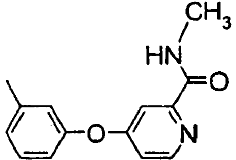 |
| (86) | 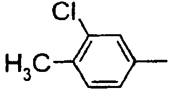 | 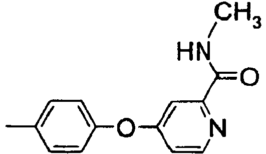 |
| (87) | 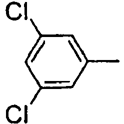 | 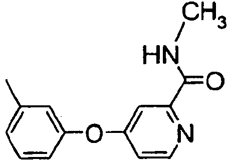 |
| (88) | 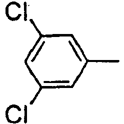 | 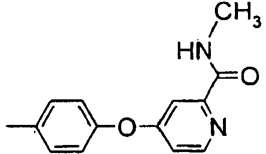 |
| (89) | 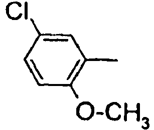 | 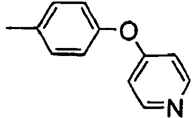 |
| (90) | 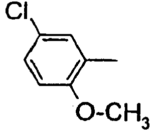 | 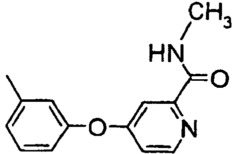 |
| (91) | 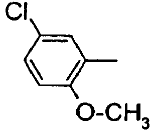 | 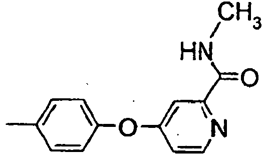 |
| (92) | 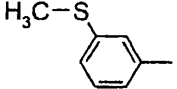 | 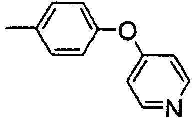 |
| (93) | 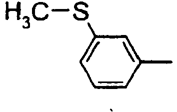 | 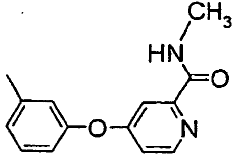 |
| (94) | 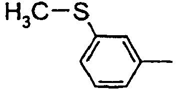 | 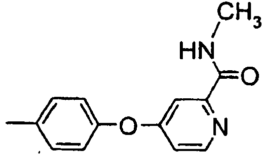 |
| (95) | 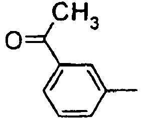 | 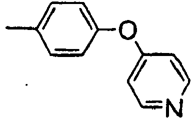 |
| (96) | 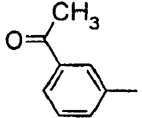 | 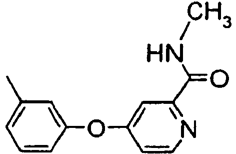 |
| (97) | 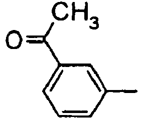 | 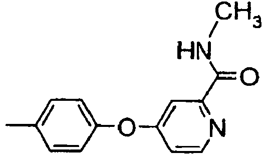 |
| (98) | 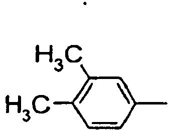 | 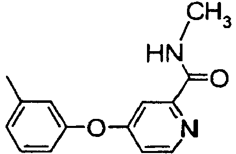 |
| (99) | 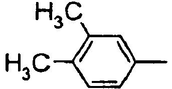 | 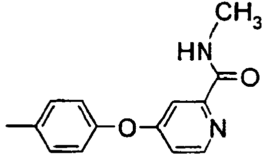 |
| (100) | 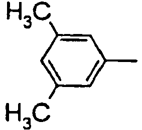 | 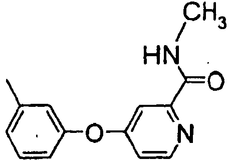 |
| (101) | 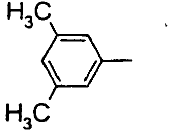 | 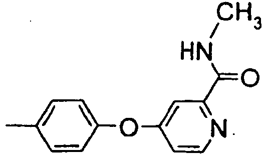 |
| (102) | 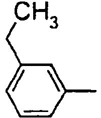 | 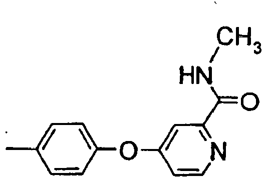 |
| (103) | 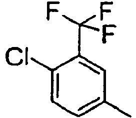 | 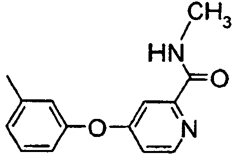 |
| (104) | 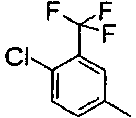 | 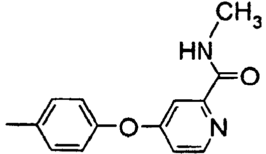 |
| (105) | 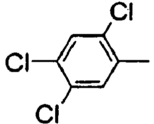 | 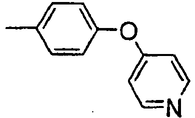 |
| (106) | 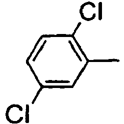 | 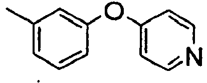 |
| (107) | 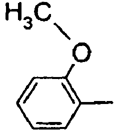 | 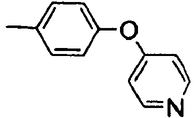 |
| (108) | 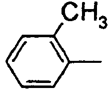 | 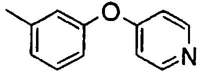 |
| (109) | 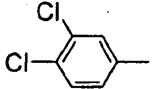 | 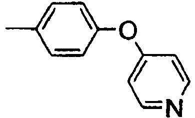 |
| (110) | 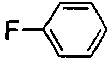 | 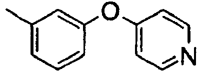 |
| (111) | 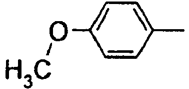 | 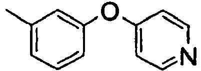 |
| (112) | 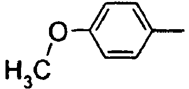 | 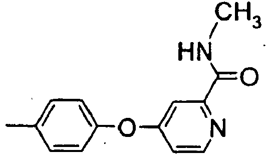 |
| (113) | 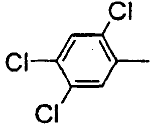 | 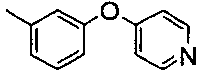 |
| (114) | 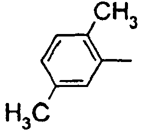 | 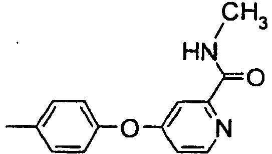 |
| (115) | 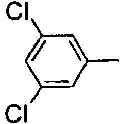 | 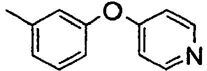 |
| (116) | 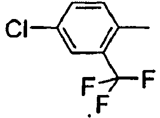 | 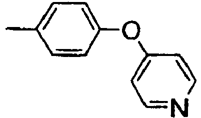 |
| (117) | 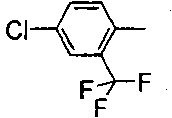 | 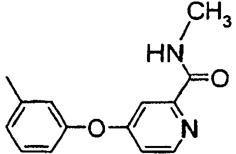 |
| (118) | 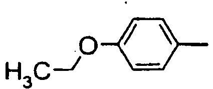 | 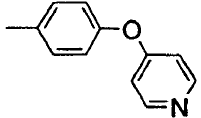 |
| (119) | 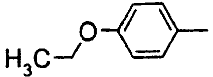 | 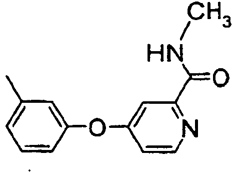 |
| (120) | 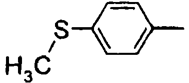 | 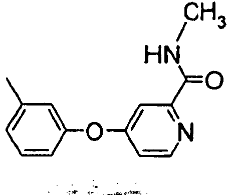 |
| (121) | 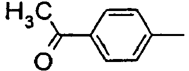 | 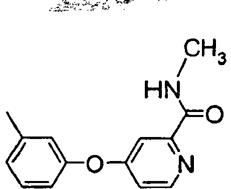 |
| (122) | 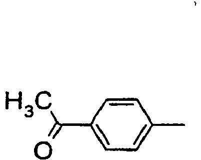 | 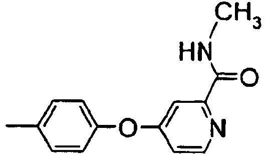 |
| (123) | 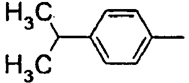 | 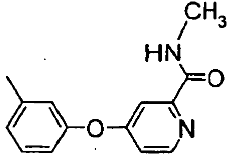 |
| (124) | 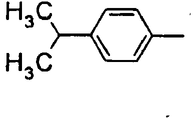 | 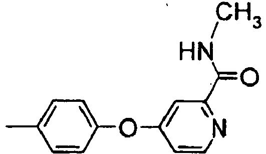 |
| (125) | 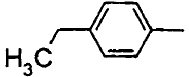 | 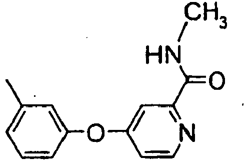 |
| (126) | 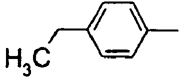 | 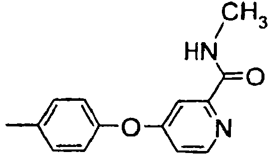 |
| (127) | 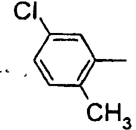 | 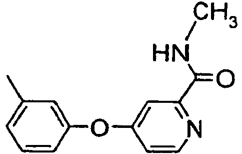 |
| (128) | 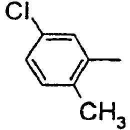 | 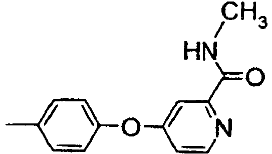 |
| (129) | 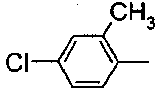 | 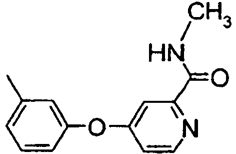 |
| (130) | 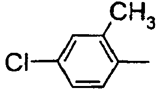 | 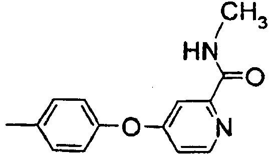 |
| (131) | 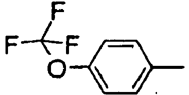 | 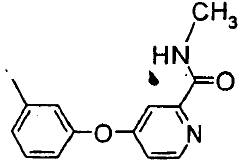 |
| (132) 2 | 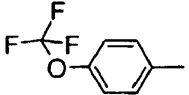 | 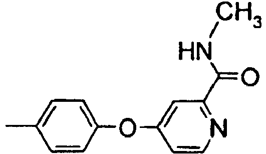 |
| (133) | 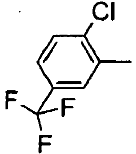 | 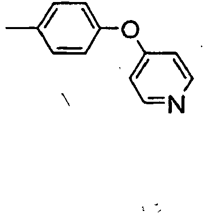 |
| (134) | 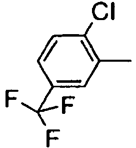 | 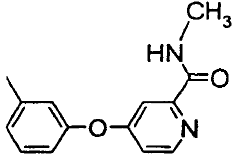 |
| (135) | 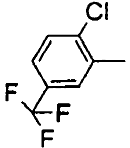 | 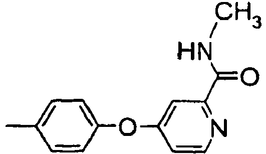 |
| (136) | 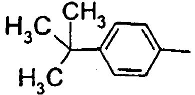 | 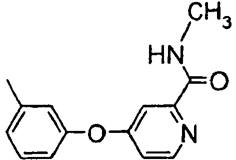 |
| (137) | 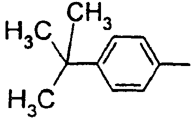 | 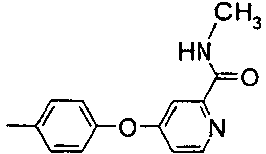 |
| (138) | 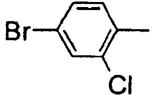 | 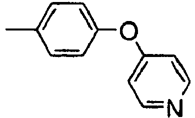 |
| (139) | 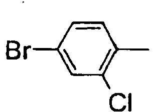 | 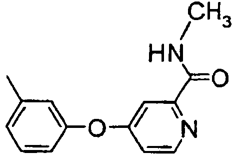 |
| (140) | 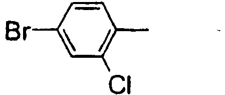 | 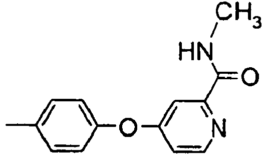 |
| (141) | 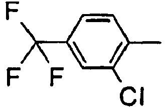 | 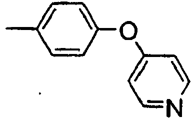 |
| (142) | 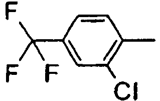 | 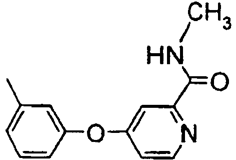 |
| (143) | 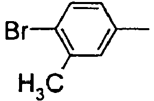 | 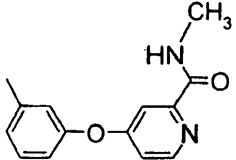 |
| (144) | 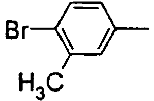 | 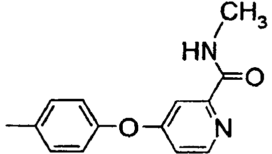 |
| (145) | 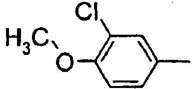 | 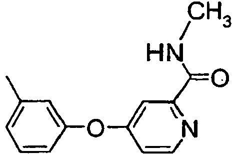 |
| (146) | 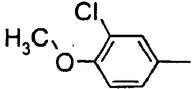 | 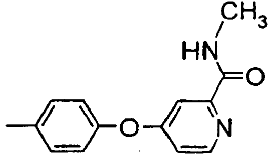 |
| (147) | 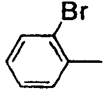 | 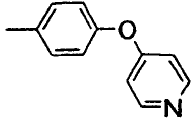 |
| (148) | 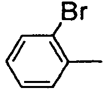 | 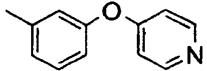 |
| (149) | 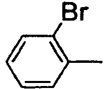 | 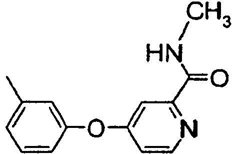 |
| (150) | 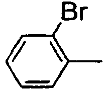 | 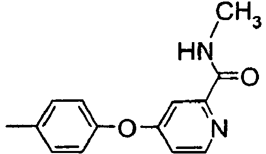 |
| (151) | 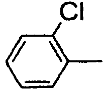 | 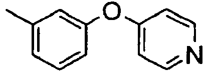 |
| (152) | 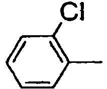 | 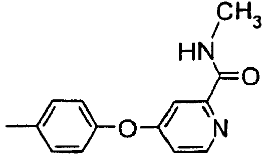 |
| (153) | 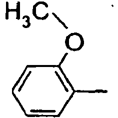 | 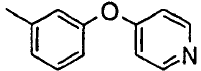 |
| (154) | 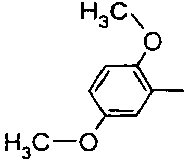 | 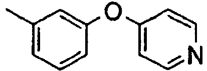 |
| (155) | 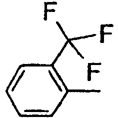 | 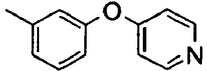 |
| (156) | 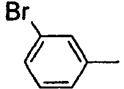 | 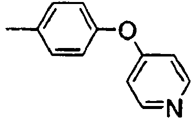 |
| (157) | 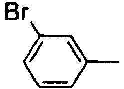 | 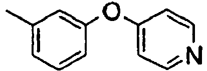 |
| (158) | 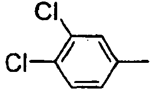 | 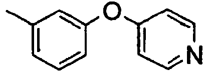 |
| (159) | 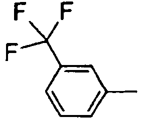 | 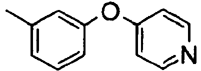 |
| (160) | 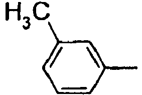 | 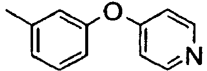 |
| (161) | 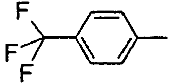 | 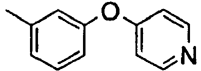 |
| (162) | 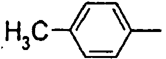 | 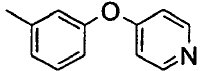 |
| (163) | 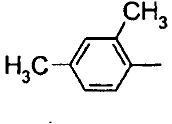 | 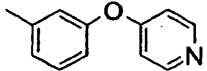 |
| (164) | 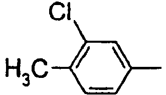 | 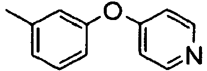 |
| (165) | 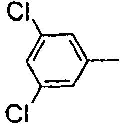 | 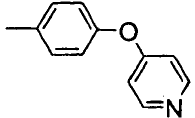 |
| (166) | 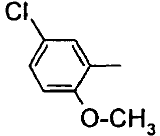 | 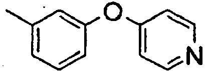 |
| (167) | 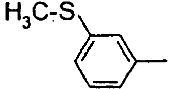 | 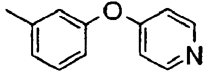 |
| (168) | 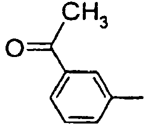 | 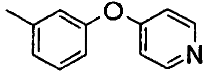 |
| (169) | 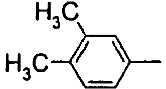 | 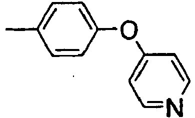 |
| (170) | 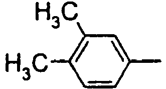 | 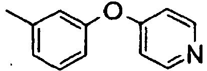 |
| (171) | 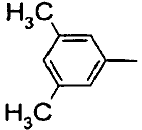 | 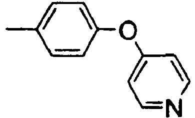 |
| (172) | 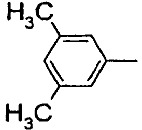 | 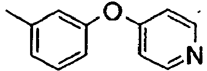 |
| (173) | 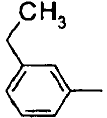 | 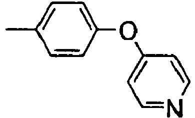 |
| (174) | 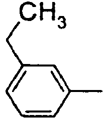 | 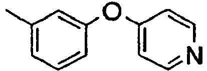 |
| (175) | 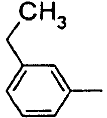 | 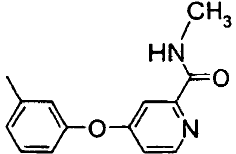 |
| (176) | 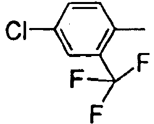 | 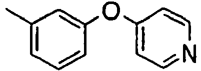 |
| (177) | 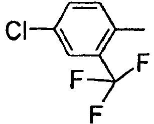 | 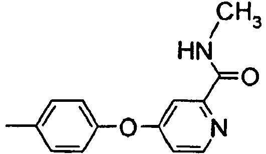 |
| (178) | 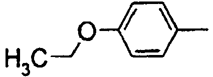 | 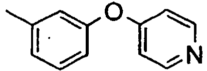 |
| (179) | 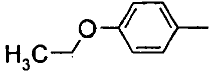 | 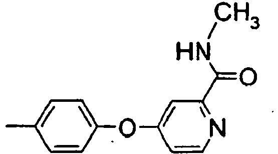 |
| (180) | 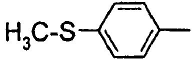 | 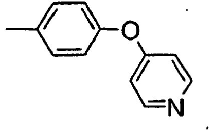 |
| (181) | 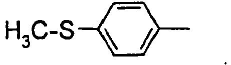 | 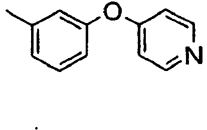 |
| (182) | 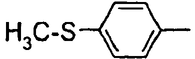 | 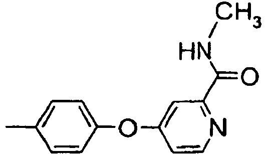 |
| (183) | 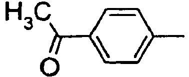 | 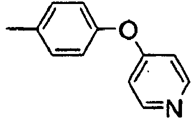 |
| (184) | 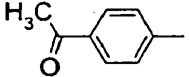 | 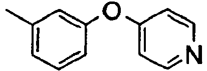 |
| (185) | 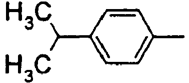 | 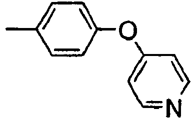 |
| (186) | 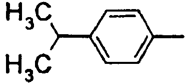 | 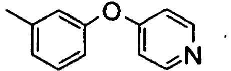 |
| (187) | 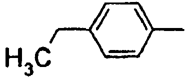 | 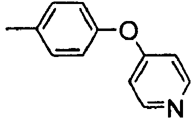 |
| (188) | 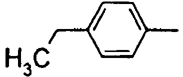 | 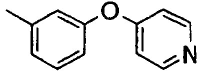 |
| (189) | 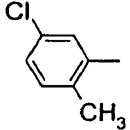 | 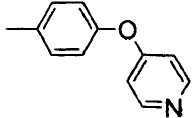 |
| (190) | 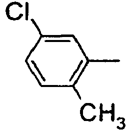 | 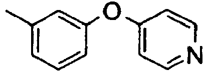 |
| (191) | 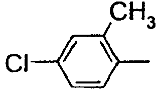 | 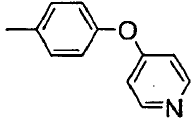 |
| (192) | 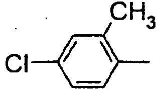 | 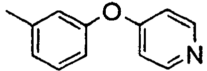 |
| (193) | 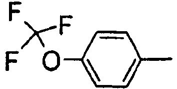 | 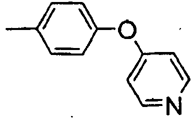 |
| (194) | 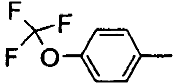 | 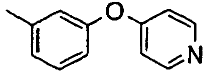 |
| (195) | 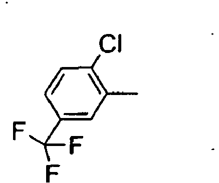 | 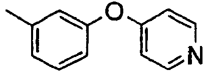 |
| (196) | 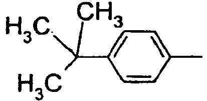 | 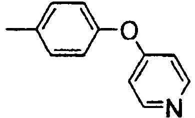 |
| (197) | 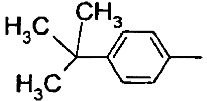 | 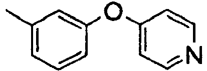 |
| (198) | 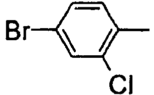 | 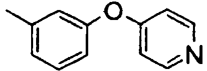 |
| (199) | 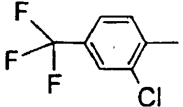 | 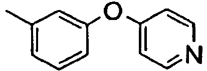 |
| (200) | 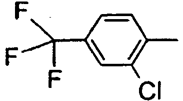 | 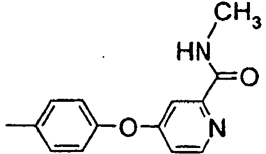 |
| (201) | 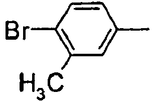 | 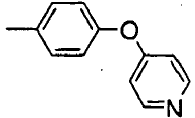 |
| (202) | 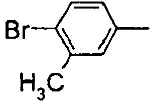 | 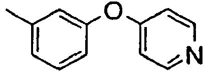 |
| (203) | 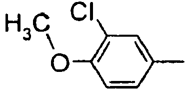 | 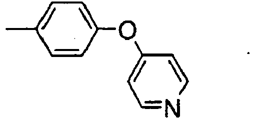 |
| (204) | 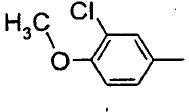 | 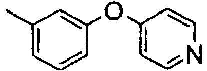 |
| (205) | 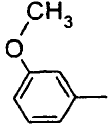 | 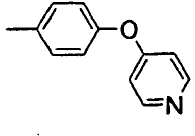 |
| (206) | 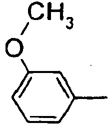 | 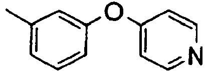 |
| (207) | 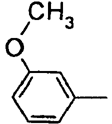 | 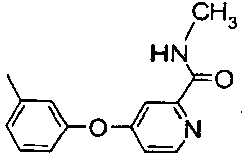 |
| (208) | 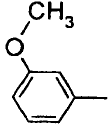 | 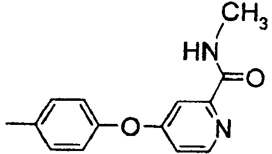 |
| (209) | 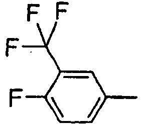 | 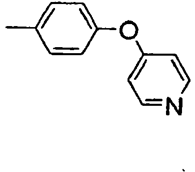 |
| (210) | 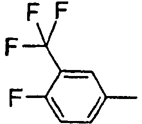 | 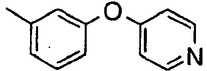 |
| (211) | 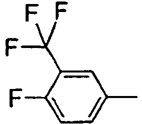 | 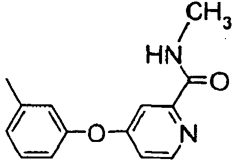 |
| (212) | 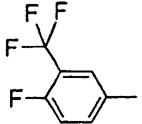 | 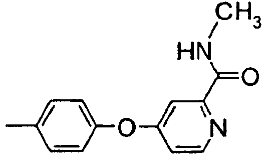 |
| (213) | 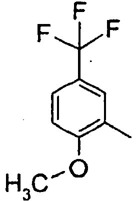 | 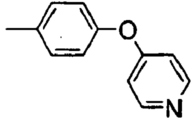 |
| (214) | 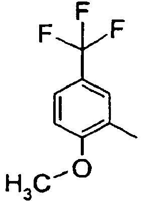 | 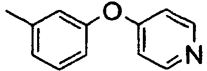 |
| (215) | 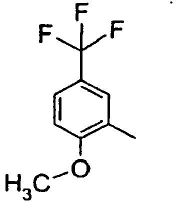 | 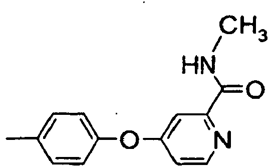 |
| (216) | 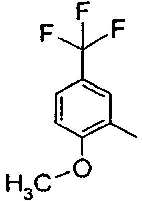 | 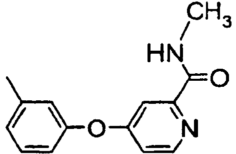 |
| (217) | 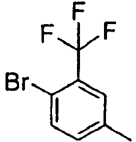 | 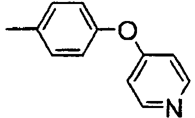 |
| (218) | 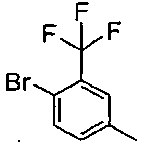 | 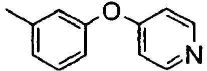 |
| (219) | 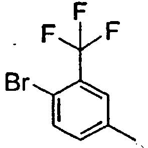 | 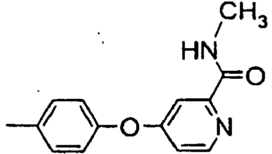 |
| (220) | 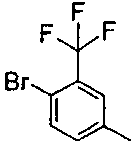 | 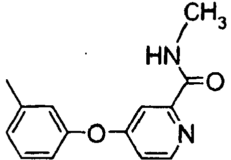 |
| (221) | 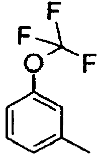 | 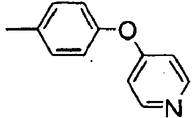 |
| (222) | 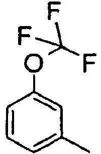 | 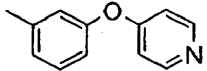 |
| (223) | 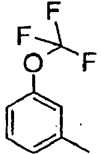 | 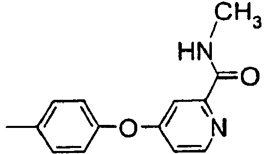 |
| (224) | 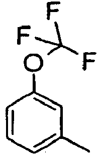 | 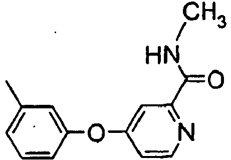 |
| (225) | 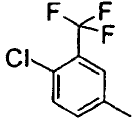 | 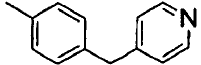 |
| (226) | 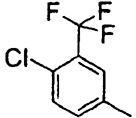 | 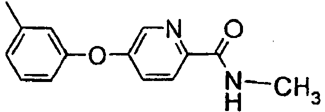 |
| (227) | 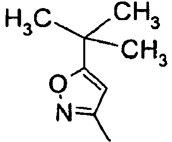 | 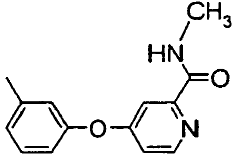 |
| (228) | 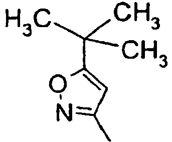 | 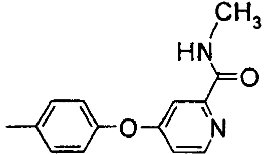 |

The present invention further relates to compounds of formula A-NH-CO-CR⁶R⁷-CO-NH-B, wherein A and B are as given for compounds (1) to (228) in the table above, and wherein C⁶ and/or C⁷ are residues other than hydrogen. These compounds are hereinafter referred to as compounds of formula (1') to (228').

In a special embodiment, the malonamide derivatives according to sub formulae IIa, Ib, IIc, IId, IIe, IIg, IIh and/or compounds (1) to (228) and/or ompounds of formula (1') to (228') additionally comprise one or two substituents selected from the group consisting of O(CH₂)ₙNR¹¹R¹², NR¹¹(CH₂)ₙNR¹¹R¹², O(CH²)ₙOR¹² and NR¹¹(CH₂)ₙOR¹²,
wherein
- R¹¹, R¹²: are independently selected from a group consisting of H, A, (CH₂)ₘAr³ and (CH₂)ₘHet, or in NR¹¹R¹²,
- R¹¹ and R¹²: form, together with the N-Atom they are bound to, a 5-, 6- or 7-membered heterocyclus which optionally contains 1 or 2 additional hetero atoms, selected from N, O an S, and
- n: is 1, 2, 3, 4, 5 or 6, preferably 2, 3 or 4.

In this embodiment, the substituents are preferably selected from the group consisting of HNCH₂CH₂NH₂, OCH₂CH₂NH₂, NHCH₂CH₂OH, OCH₂CH₂NHCH₃, N(CH₃)CH₂CH₂NH₂, HN(CH₃)CH₂CH₂NH, N(CH₃)CH₂CH₂N(CH₃)₂, N(CH₃)CH₂CH₂N(CH₃)₂, N(CH₃)CH₂CH₂OCH₃, OCH₂CH₂N(CH₃)₂, OCH₂CH₂N(CH₂CH₃)₂ and compounds of the formulae

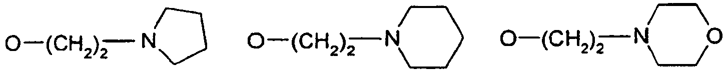

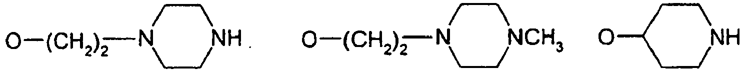

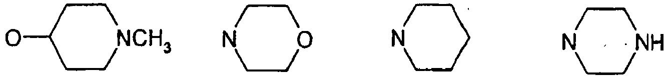

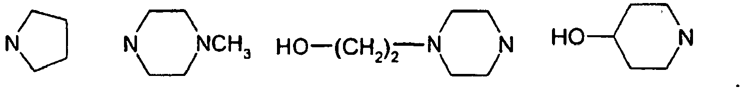

The nomenclature as used herein for defining compounds, especially the compounds according to the invention, is in general based on the rules of the IUPAC-organisation for chemical compounds and especially organic compounds.

Another aspect of the invention relates to a method for producing compounds of formula II, characterised in that
a) A compound of formula III 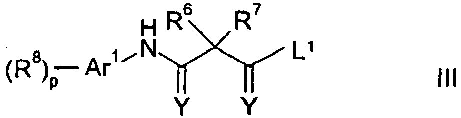 wherein
   - L¹: is Cl, Br, I, OH, an esterified OH-group or a diazonium moiety, and R⁸, p, Ar¹, Y are as defined above and below,
   is reacted
b) with a compound of formula IV, 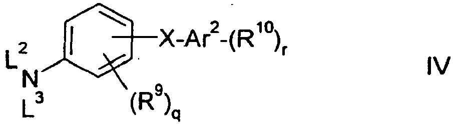 wherein
   - L², L³: are independently from one another H or a metal ion, and R⁹, q, X, Ar², R¹⁰ and r are as defined above and below,
   and optionally
c) isolating and/or treating the compound of formula II obtained by said reaction with an acid, to obtain the salt thereof.

The compounds of the formula II and also the starting materials for their preparation are, in addition, prepared by methods known per se, as described in the literature (for example in the standard works, such as Houben-Weyl, Methoden der organischen Chemie [Methods of Organic Chemistry], Georg-Thieme-Verlag, Stuttgart), to be precise under reaction conditions which are known and suitable for the said reactions. Use can also be made here of variants which are known per se, but are not mentioned here in greater detail.

If desired, the starting materials can also be formed in situ by not isolating them from the reaction mixture, but instead immediately converting them further into the compounds of the formula II, respectively. On the other hand, it is possible to carry out the reaction stepwise.

The compounds of formula II can preferably be obtained by reacting compounds of the formula III with compounds of the formula IV.

In detail, the reaction of the compounds of the formula III with the compounds of the formula IV is carried out in the presence or absence of a preferably inert solvent at temperatures between about -20° and about 200°, preferably between 0° and 150° and especially between room temperature (25°) and 120°. In some cases, it can be advantageous to combine one compound of formula III with one compound of formula IV at the lower end of the given temperature range, preferably between -20° and 75°, more preferred between 0° and 60° and especially between 10° and 40°, for example at about room temperature, and heat the mixture up to a temperature at the upper end of the given temperature range, preferably between 80° and 180°, more preferred between 90° and 150° and especially between 95° and 120°, for example at about 100° or at about 110°.

In general, the compounds of formula III and/or formula IV are new. In any case, they can be prepared according to methods known in the art.

In the compounds of formula III, L¹ is preferably Cl, Br, I, OH, a reactive derivatized OH-moiety, especially an esterified OH-moiety, for example an OR'-moiety wherein R' is an alkyl moiety, preferably an alkyl moiety as described above/below comprising 1 to 10 and more preferably 1 to 6 carbon atoms, or a reactive esterified OH-moiety, for example an alkylsulfonyloxy-moiety comprising 1 to 6 carbon atoms (preferably methylsulfonyloxy) or an arylsulfonyloxy-moiety comprising 6 to 10 carbon atoms (preferably phenyl- oder p-tolylsulfonyloxy), or diazonium moiety, more preferred Cl, Br or I and OR', wherein R' is as defined above/below, and even more preferred OH and OR', wherein R' is preferably selected from the group consisting of methyl, ethyl, n-propyl, isopropyl, n-butyl, isobutyl and t-butyl. Especially preferred as L¹ is OH.

In the compounds of formula IV, L² and/or L³ is preferably H or a moiety which activates the amino group it is bonded to, for example a metal ion. Suitable metal ions are preferably selected from the group consisting of alkaline metal ions, alkaline-earth metal ions and aluminium ions. Especially preferred metal ions are alkaline metal ions, of which Li, Na and K are especially preferred. In case of multi-valent metal ions, the metal ions and the compounds of formula IV form a complex containing one or more compounds of formula IV and one or more metal ions wherein the ratio between compounds of formula IV and metal ions is depending on the valency of the metal ion(s) according to the rules of stoichiometry and/or electroneutrality.

The reaction between the compounds of formula III and compounds of formula IV can in many cases advantageously be carried out in the presence of an acid binding means, for example one or more bases. Suitable acid binding means are known in the art. Preferred as acid binding means are inorganic bases and especially organic bases. Examples for inorganic bases are alkaline or alkaline-earth hydroxides, alkaline or alkaline-earth carbonates and alkaline or alkaline-earth bicarbonates or other salts of a weak acid and alkaline or alkaline-earth metals, preferably of potassium, sodium, calcium or cesium. Examples for organic bases are triethyl amine, diisopropyl ethyl amine (DIPEA), dimethyl aniline, pyridine or chinoline. If an organic base is used, it is advantageous in general to use a base with a boiling point that is higher than the highest reaction temperature employed during the reaction. Especially preferred as organic base is diisopropyl ethyl amine.

Reaction times are generally in the range between some minutes and several days, depending on the reactivity of the respective compounds and the respective reaction conditions. Suitable reaction times are readily determinable by methods known in the art, for example reaction monitoring. Based on the reaction temperatures given above, suitable reaction times generally lie in the range 10 min and 36 hrs, preferably 30 min and 24 hrs and especially between 45 min and 16 hrs, for example about 2 h, about 6 hrs, about 10 hrs or about 14 hrs.

Preferably, the reaction of the compounds of the formula III with the compounds of the formula IV is carried out in the presence of a suitable solvent, that is preferably inert under the respective reaction conditions. Examples of suitable solvents are hydrocarbons, such as hexane, petroleum ether, benzene, toluene or xylene; chlorinated hydrocarbons, such as trichlorethylene, 1,2-dichloroethane, tetrachloromethane, chloroform or dichloromethane; alcohols, such as methanol, ethanol, isopropanol, n-propanol, n-butanol or tert-butanol; ethers, such as diethyl ether, diisopropyl ether, tetrahydrofuran (THF) or dioxane; glycol ethers, such as ethylene glycol monomethyl or monoethyl ether or ethylene glycol dimethyl ether (diglyme); ketones, such as acetone or butanone; amides, such as acetamide, dimethylacetamide, dimethylformamide (DMF) or N-methyl pyrrolidinone (NMP); nitriles, such as acetonitrile; sulfoxides, such as dimethyl sulfoxide (DMSO); nitro compounds, such as nitromethane or nitrobenzene; esters, such as ethyl acetate, or mixtures of the said solvents. Polar solvents are in general preferred. Examples for suitable polar solvents are chlorinated hydrocarbons, alcohols, glycol ethers, nitriles, amides and sulfoxides or mixtures thereof. More preferred are amides, especially dimethylformamide (DMF).

If compounds of formula II are desired wherein Y is other than O, it can be advantageous, however, to carry out the reaction of a compound of formula III, wherein Y is O, and a compound of formula IV according to the invention to obtain a compound of formula II, wherein Y is O, and to modify or convert the corresponding C=O group (i. e. the C=Y group, wherein Y is O) in the compound of formula II into a C=NR²¹, C=C(R²²)-NO₂, C=C(R²²)-CN or C=C(CN)₂ group according to methods known in the art, for example from Houben-Weyl, Methods of Organic Chemistry.

The compounds of formula III can be obtained according to methods known in the art. In an advantageous manner, they can be readily obtained by reacting a compound of formula V 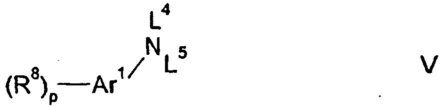 wherein R⁸, p, and Ar¹ are as defined above/below and L⁴ and L⁵ are selected independently from each other from the meanings given for L² and L³ and more preferred are hydrogen, with a compound of formula VI 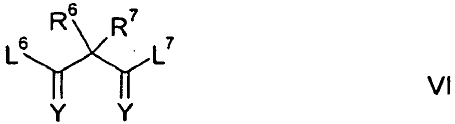 wherein each Y is independently from one another as defined above/below, R⁶ and R⁷ are as defined above/below and L⁶ and L⁷ are selected independently from each other from the meanings given for L¹. Preferably, one of L⁶ and L⁷ is halogen and one of L⁶ and L⁷ is an OH-moiety and more preferred a derivatized OH-moiety. Preferably, derivatized OH-moieties are OR'-moieties, wherein R' is selected from the meanings given above/below. More preferred, L⁶ is selected from the group consisting of Cl, Br and I. Especially preferred, L⁶ is Cl. L⁷ is more preferred an OH-moiety and even more preferred a derivatized OH-moiety as defined above. Especially preferred, L⁷ is an OR'-moiety, wherein R' is selected from the group consisting of methyl, ethyl, n-propyl, isopropyl, n-butyl, isobutyl and t-butyl, and especially is OCH₃.

If L⁷ in the compounds of formula III is OR', wherein R' is as defined above, it is in many cases advantageous, to transfer said compound into a compound of formula III, wherein L¹ is OH before reacting it with a compound of formula IV. Methods for transferring a compound of formula III, wherein L¹ is OR' as defined above, into a compound of formula III, wherein L¹ is OH are known in the art, for example ester cleavages. An ester cleavage can be carried out in an acidic or basic medium according to methods known per se. Preferably an ester cleavage is carried out in a basic medium, for example in the presence of one or more bases, preferably inorganic bases such as alkaline or alkaline-earth hydroxides, more preferably NaOH or KOH, in a preferably polar solvent such as water or alcohol, for example alcohols as described above/below, or mixtures thereof. Suitable reaction temperatures usually lie in the range between 0 °C and the boiling point of the solvent chosen and especially at about room temperature.

Some of the starting materials of the formula V and/or the formula VI are known and preferably commercially available. If they are not known, they can be prepared by methods known per se.

Suitable reaction conditions for carrying out the reaction of a compound of formula V with a compound of formula VI are known in the art. In detail, the reaction of the compounds of the formula V with the compounds of the formula VI is carried out in the presence or absence of a preferably inert solvent and in the presence or absence of a suitable base at temperatures between about -40° and about 180°, preferably between -20 °C and 100° and especially between -10° and 50°, for example at about 0° and/or about room temperature (25°).

The reaction between compounds of formula V and compounds of formula VI is preferably carried out in the presence of an acid binding means, for example one or more bases. Suitable acid binding means are known in the art. Preferred as acid binding means are organic bases and especially inorganic bases. Examples for inorganic bases are alkaline or alkaline-earth hydroxides, alkaline or alkaline-earth carbonates and alkaline or alkaline-earth bicarbonates or other salts of a weak acid and alkaline or alkaline-earth metals, preferably of potassium, sodium, calcium or cesium. Examples for organic bases are triethyl amine, diisopropyl ethal amine (DIPEA), dimethyl aniline, pyridine or chinoline. If an organic base is used, it is advantageous in general to use a base with a boiling point that is higher than the highest reaction temperature employed during the reaction. Especially preferred as base is KOH.

The reaction between compounds of formula V and compounds of formula VI can be carried out in the presence of a suitable solvent, that is preferably polar and preferably inert at the chosen reaction conditions. Suitable solvents are known in the art. Examples for suitable polar solvents are chlorinated hydrocarbons, alcohols, glycol ethers, nitriles, amides and sulfoxides or mixtures thereof. More preferred are amides and alcohols, especially preferred is methanol.

In many cases, it is advantageous to carry out the reaction of a compound of formula V with a compound of formula VI in the presence of one or more compounds that promote the reaction between the said compounds, for example one or more catalysts and/or one or more compounds that are acting as condensing agents. Suitable compounds in this respect are O-(Benzotriazole-1-yl)-N,N,N',N'-tetramethyluronium hexafluorophosphat tetrafluoroborate (TBTU), O-(Benzotriazole-1-yl)-N,N,N',N'-tetramethyluronium tetrafluoroborate and 1-Hydroxy-1H-benzotriazole (HOBT).

Especially preferred, the reaction between a compound of formula V, wherein L⁴ and L⁵ preferably are hydrogen, and a compound of formula VI, wherein preferably Y both are O and wherein L⁶ is halogen and L⁷ is OH, is carried out in the presence of an inorganic base, such as KOH, a polar organic solvent, such as methanol, in the presence of TBTU and HOBT at a temperature between 0 °C and 60 °C, for example at about room temperature.

The compounds of formula IV can be obtained according to methods known in the art.

If the compound of formula IV is a compound according to formula IVa, 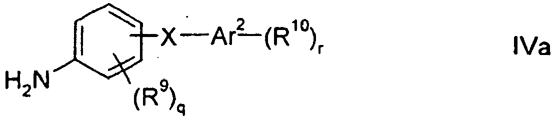 it can be readily obtained in an advantageous manner by reacting a compound of formula VIIa, 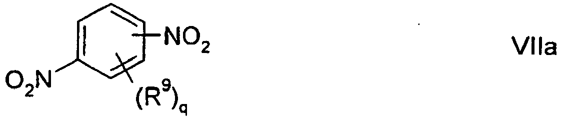 wherein R⁹ and q are as defined above/below,
with a compound of formula VIII,

L⁸-X-Ar²-(R¹⁰)ᵣ VIII

wherein L⁸ is H or a metal ion, preferably a metal ion selected from the group consisting of alkaline metal ions, alkaline-earth metal ions and aluminum ions, especially preferred alkaline metal ions, of which Li, Na and K are especially preferred, and even more preferred is H; and Ar², R¹⁰, r and X are as defined above/below, and especially wherein X is (CHR¹¹)ₕ-Q-(CHR¹²)ᵢ, wherein R¹¹, h, R¹² and i are defined above/below, and preferably wherein h and/or i are 0, and Q is selected from a group consisting of O, S, N-R¹⁷, (CHR¹⁸-O)ⱼ, (CHR¹⁸CHR¹⁹-O)ⱼ, CH=N-O, CH=N-NR¹⁷, SO₂NR¹⁷, wherein j, R¹⁷, R¹⁸ and R¹⁹ are as defined above/below;
optionally isolating the reaction product,
and transferring the obtained reaction product of formula IX 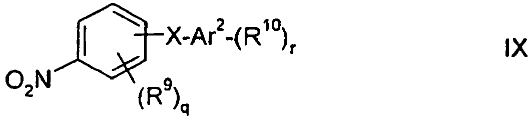 into a compound of formula IVa, preferably by hydrogenating the NO₂-moiety of the compound of formula IX into a NH₂-moiety. Methods and reaction conditions for hydrogenating said NO₂-moiety into a NH₂-moiety are known in the art. In general, it is advantageous to carry out the hydrogenation reaction in a hydrogen atmosphere in the presence of a suitable catalyst, preferably a Palladium catalyst, for example Pd/C. In general, such hydrogenation reactions are carried out in a suitable solvent. Suitable solvents for hydrogenation reactions are known in the art. Suitable solvents, for example, are alcohols, especially methanol and ethanol and ethers, especially THF, and mixtures thereof. In general, the hydrogenation reactions are carried out at about normal pressure or slightly elevated pressure, for example between normal pressure and 3 bar pressure (about 300 kPa). The hydrogenation reaction is usually carried out in the temperature range between -20° and 150°, preferably 0° and 50°.

Ar² is preferably pyridinyl. Accordingly, the compound of formula VIII is preferably selected from the group consisting of formulae VIIIa and Vlllb, 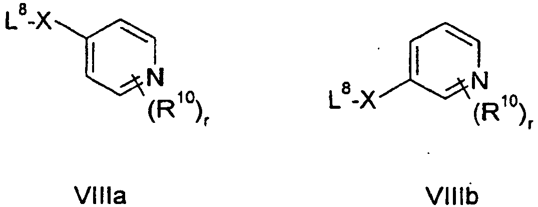 wherein L⁸, X, R¹⁰ and r are as defined above, and especially preferred from the group consisting of formulae VIIIc and VIIId, 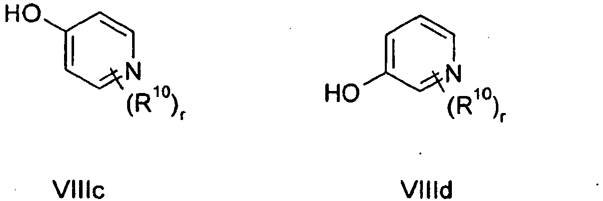 wherein R¹⁰, and r are as defined above, or the alkaline metal salts and especially the sodium or potasium salts thereof.

Accordingly, in formulae IVa, VIII, VIIIa, VIIIb and IX, the bridging group X is preferably O, S, OCH₂ and OCH₂CH₂ and especially is O.

In the formulae VIII, VIIIa and VIIIb, L⁸ is preferably H or selected from the group consisting of Na, K and Cs and especially preferred is H.

In general, this reaction is advantageous to produce compounds of formula IVaa, 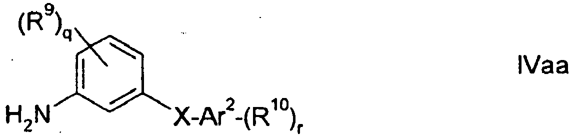 wherein R⁹, q, X, Ar², R¹⁰ and r are as defined above/below.

To obtain compounds of formula IVaa, it is reasonable to employ a compound of formula VII that is selected from the compounds of formula VIIa, 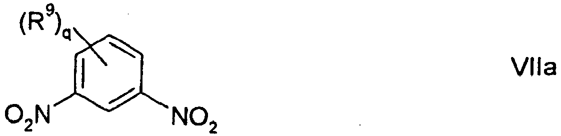 and proceed the reaction as described above/below.

Accordingly, by starting from a compound of formula VIIa and a compound of formula VIIIa, the reaction preferably leads to compounds of formula IVaaa, 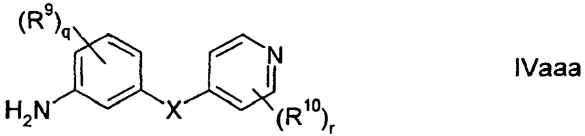 wherein R⁹, q, X, R¹⁰ and r are as defined above/below.

Accordingly, by starting from a compound of formula VIIa and a compound of formula Vlllb, the reaction preferably leads to compounds of formula IVaab, 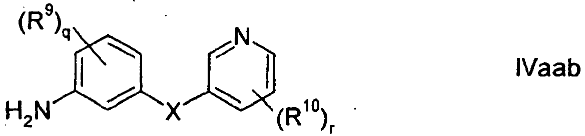 wherein R⁹, q, X, R¹⁰ and r are as defined above/below.

Accordingly, by starting from a compound of formula VIIa and a compound of formula VIIIc, the reaction preferably leads to compounds of formula IVaac, 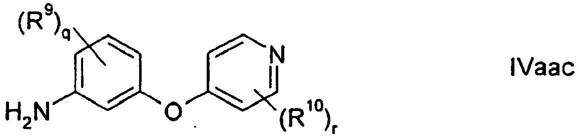 wherein R⁹, q, R¹⁰ and r are as defined above/below.

Accordingly, by starting from a compound of formula VIIa and a compound of formula Vllld, the reaction preferably leads to compounds of formula 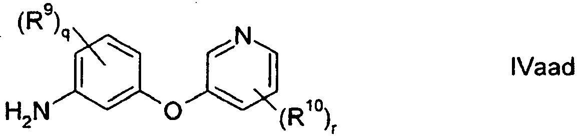 wherein R⁹, q, R¹⁰ and r are as defined above/below.

Some of the starting materials of the formula VII and/or the formula VIII are known and preferably commercially available. If they are not known, they can be prepared by methods known per se.

The reaction between the compound of formula VII and VIII is preferably carried out in the temperature range between 0° and 250°, more preferred room temperature and 200°, for example at about 120°, at about 150° or at about 180°. Reaction times depend on the respective reactants and the respective reaction temperature, but generally lie in the range between 30 min and 36 hrs, preferably 3 hrs and 24 hrs, more preferably 8 hrs and 20 hrs for example about 10 hrs, about 16 hrs or about 18 hrs.

The reaction can be carried out in the absence of solvent or preferably in the presence of an solvent, preferable a solvent that is inert under the respective reaction conditions. Suitable inert solvents for carrying out the reaction are known in the art. Examples for suitable solvents are high boiling aliphatic hydrocarbons, high boiling aromatic carbons, for example toluene, xylenes, high boiling chlorinated hydrocarbons, such as trichloroethylene, tetrachloroethanes, pentachloroethanes and hexachloroethanes; high boiling ethers, such as ethylene glycol and propylene glycols; glycol ethers, such as ethylene glycol monomethyl or monoethyl ether or ethylene glycol dimethyl ether (diglyme); amides, such as acetamide, dimethylacetamide, dimethylformamide (DMF) or N-Methyl pyrrolidone (NMP); sulfoxides, such as dimethyl sulfoxide (DMSO); or mixtures of the said solvents. Preferred are amides, especially dimethylformamide (DMF).

Preferably, the reaction is carried out in the presence of a base. Suitable bases are known in the art. Preferred bases are organic bases and especially inorganic bases. Examples for inorganic bases are alkaline or alkaline-earth hydroxides, alkaline or alkaline-earth carbonates and alkaline or alkaline-earth bicarbonates or other salts of a weak acid and alkaline or alkaline-earth metals, preferably of potassium, sodium, calcium or cesium. Preferred inorganic bases are K₂CO₃, Na₂CO₃, MgCO₃, CaCO₃, NaOH and KOH, especially preferred is K₂CO₃. Examples for organic bases are triethyl amine, diisopropyl ethyl amine (DIPEA), dimethyl aniline, pyridine or chinoline. If an organic base is used, it is advantageous in general to use a base with a boiling point that is higher than the highest reaction temperature employed during the reaction.

Alternatively, if the compound of formula IV is a compound according to formula IVb, 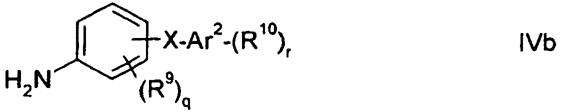 it can be readily obtained in an advantageous manner by reacting a compound of formula Vllb, 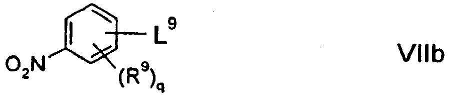 wherein R⁹ and q are as defined above/below and wherein L⁹ is selected independently from the meanings given for L¹. Preferably, L⁹ is halogen. More preferred, L⁹ is selected from the group consisting of Cl, Br and I. Especially preferred, L⁹ is Cl.
with a compound of formula Vlllb,

L¹⁰-X-Ar²-(R¹⁰)ᵣ VIIIb

wherein L¹⁰ is H or a metal ion, preferably a metal ion, more preferred a metal ion selected from the group consisting of alkaline metal ions, alkaline-earth metal ions and aluminium ions, especially preferred alkaline metal ions, of which Li, Na and K are especially preferred; and Ar², R¹⁰, r and X are as defined above/below, and especially wherein X is (CHR¹¹)ₕ-Q-(CHR¹²)ᵢ, CH=N-O, CH=N-NR¹⁷, SO₂NR¹⁷, wherein Q, R¹¹, R¹², h, i, R¹⁷, R¹⁸ and R¹⁹ are as defined above/below; and even more preferred wherein X is (CHR¹¹)ₕ-Q-(CHR¹²)_{¡}, Q is as defined above/below and h and/or i are 0;
optionally isolating the reaction product,
and transferring the obtained reaction product of formula IXb 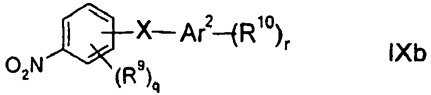 into a compound of formula Iva, preferably by hydrogenating the NO₂-moiety of the compound of formula IX into a NH₂-moiety. Methods and reaction conditions for hydrogenating said NO₂-moiety into a NH₂-moiety are known in the art. In general, it is advantageous to carry out the hydrogenation reaction in a hydrogen atmosphere in the presence of a suitable catalyst, preferably a Palladium catalyst, for example Pd/C. In general, such hydrogenation reactions are carried out in a suitable solvent. Suitable solvents for hydrogenation reactions are carried out in a suitable solvent. Suitable solvents for hydrogenation reactions are known in the art. Suitable solvents, for example, are alcohols, especially methanol and ethanol, ethers, especially THF, and mixtures thereof. In general, the hydrogenation reactions are carried out at about normal pressure or slightly elevated pressure, for example between normal pressure or slightly elevated pressure, for example between normal pressure and 3 bar pressure (about 300 kPa). The hydrogenation reaction is usually carried out in the temperature range between -20° and 150°, preferably 0° and 50°.

Ar² is preferably pyridinyl. Accordingly, the compound of formula VIIIb is preferably selected from the group consisting of formulae VIIIe and VIIIf, 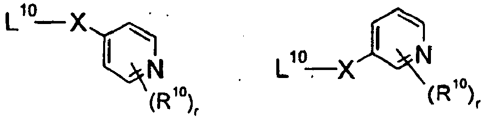 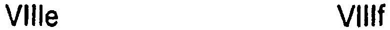 wherein L¹⁰, X, R¹⁰, and r are as defined above, and especially preferred from the group consisting of formulae VIIIg and Vlllh, 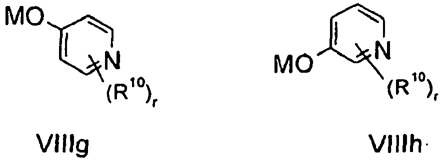 wherein R¹⁰ and r are as defined above, and wherein M is an alkaline metal ion and especially sodium or potassium, or the corresponding alcohols thereof.

Accordingly, in formulae IVb, VIIIb, VIIIe, Vlllf and IXb, the bridging group X is preferably O, S, OCH₂ and OCH₂CH₂ and especially is O.

In general, this alternative reaction is advantageous to produce compounds of formula IVbb, 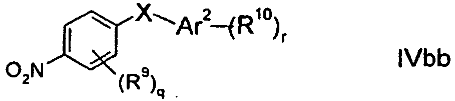 wherein R⁹, q, X, Ar², R¹⁰ and r are as defined above/below.

To obtain compounds of formula IVbb, it is reasonable to employ a compound of formula VIIb that is selected from the compounds of formula VIIbb, 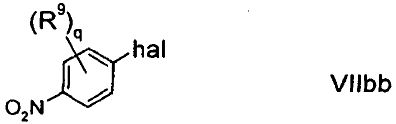 wherein hal is as defined above/below and especially is Cl, and proceed the alternative reaction as described above/below.

Accordingly, by starting from a compound a formula VIIbb and a compound of formula VIIe, the reaction preferably leads to compounds of formula IVbbe, 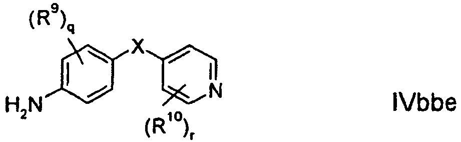 wherein R⁹, q, X, R¹⁰ and r are as defined above/below.

Accordingly, by starting from a compound of formula Vllbb and a compound of formula VIIIf, the reaction preferably leads to compounds of formula IVbbf, 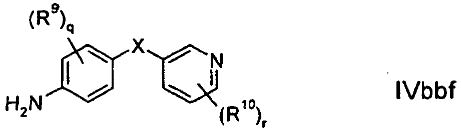 wherein R⁹, q, X, R¹⁰ and r are as defined above/below.

Accordingly, by starting from a compound of formula Vllbb and a compound of formula VIIIg, the reaction preferably leads to compounds of formula IVbbg, 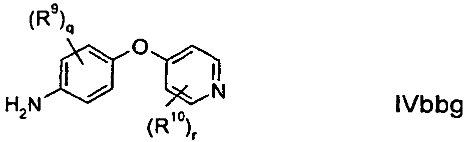 wherein R⁹, q, R¹⁰ and r are as defined above/below.

Accordingly, by starting from a compound of formula VIIb and a compound of formula VIIIh, the reaction preferably leads to compounds of formula IVbbh, 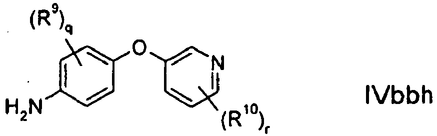 wherein R⁹, q, R¹⁰ and r are as defined above/below.

Some of the starting materials of the formula VIIb and/or the formula VIIIb are known and preferably commercially available. If they are not known, they can be prepared by methods known per se.

The reaction between the compound of formula Vllb and VIIIb is preferably carried out in the temperature range between 0° and 250°, more preferred 50° and 220°, for example at about 90°, at about 120°, at about 160°, at about 180° or at about 200°. Reaction times depend on the respective reactants and the respective reaction temperature, but generally lie in the range between 10 min and 24 hrs, preferably 30 min and 12 hrs, more preferably 1 h and 6 hrs for example about 1,5 hrs, about 3 hrs, about 4 hrs or about 5 hrs.

The reaction can be carried out in the absence or the presence of a solvent, preferable a solvent that is inert under the respective reaction conditions. Suitable inert solvents for carrying out the reaction are known in the art. Examples for suitable solvents are alipathic hydrocarbons, aromatic carbons, for example toluene and xylenes, chlorinated hydrocarbons, such as dichlormethane, trichloromethane trichloroethylene, tetrachloroethanes, pentachloroethanes and hexachloroethanes; ethers, such as diethylether, tert.-butyl methyl ether, ethylene glycol and propylene glycols; glycol ethers, such as ethylene glycol monomethyl or monoethyl ether or ethylene glycol dimethyl ether (diglyme); nitriles, such as acetonitrile, amides such as acetamide, dimethylformamide (DMF) or N-methyl pyrrolidone (NMP); sulfoxides, such as dimethyl sulfoxide (DMSO); or mixtures of the said solvents..

Preferably, the reaction is carried out in the presence of a catalyst. Suitable catalysts are known in the art. Preferred catalytic active metals and especially copper.

Preferably, the reaction is carried out by heating up a reaction mixture comprising one compound of formula VIIb and one compound of formula VIIIb to a suitable reaction temperature, which preferably lies at the upper end of the given temperature ranges and more preferred is in the range between 150° and 200°, for example at about 180°, preferably in the presence of the suitable catalyst and especially in the presence of copper. Reaction times at this temperature are preferably as given above and especially in the range between 1 h and 5 hrs, for example about 3 hrs. Preferably, the reaction mixture is then allowed to cool down to a temperature in the lower range of the given temperature, more preferred to a temperature in the range between 50° and 150°, for example to about 90°. Preferably, a suitable solvent, especially tert.-butyl methyl ether, is then added and the reaction mixture is preferably kept at about the same temperature for some more time, preferably for 30 min to 2 hrs and more preferred for about one hour.

If the compound IV is a compound according to formula IVc, 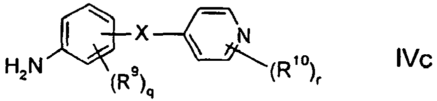 it can be readily obtained in an advantageous manner by reacting a compound of formula XI 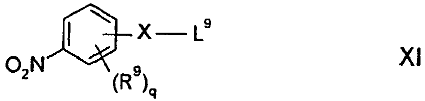 wherein L⁹ is H or a metal ion, preferably a metal ion selected from the group consisting of alkaline metal ions, alkaline-earth metal ions and aluminium ions, especially preferred alkaline metal ions, of which Li, Na and K are especially preferred, and even more preferred is H; and R⁹, q and X are as defined above/below, and especially wherein X is (CHR¹¹)ₕ-Q-(CHR¹²)ᵢ, wherein R¹¹, h, R¹² and i are defined above/below, and wherein h and/or i preferably are 0, and Q is selected from a group consisting of O, S, N-R¹⁷, (CHR¹⁸-O)ⱼ, (CHR¹⁸CHR¹⁹-O)ⱼ, CH=N-O, CH=N-NR¹⁷, SO₂NR¹⁷, wherein j, R¹⁷, R¹⁸ and R¹⁹ are as defined above/below;
with a compound of formula XII, 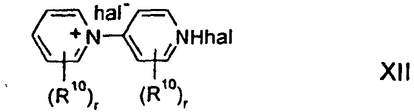 wherein hal is independently select selected from the group consisting of Cl, Br and I, the residue R¹⁰ are the same or different and have the meanings given above/below and preferably have both the same meaning, and the indices r are the same or different and have the meanings given above/below and preferably are the same,
optionally isolating the reaction product, and transferring the obtained reaction product of formula XIII 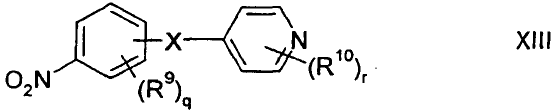 into a compound of formula IVc, preferably by hydrogenating the NO₂-moiety of the compound of formula XIII into a NH₂-moiety, for example as described above for the compound of formula IX.

In the compounds IVc, XII and XIII, r is preferably in each case identical and even more preferred in each case 0.

In formulae IVc, XI and XIII, the bridging group X is preferably O, S, OCH₂ and OCH₂CH₂ and especially is O.

In the formula XI, L⁹ is preferably H or selected from the group consisting of Na and K and especially preferred is H.

The reaction between the compound of formula XI and XII is preferably carried out in the temperature range between 0° and 250°, more preferred room temperature and 200°, for example at about 120°, at about 150° or at about 180°. Reaction times depend on the respective reactants and the respective reaction temperature, but generally lie in the range between 30 min and 24 hrs, preferably one hour and 12 hrs, for example about 2 hrs, about 3 hrs or about 6 hrs. The reaction can be carried out in the absence of solvent or in the presence of an solvent, preferable a solvent that is inert under the respective reaction conditions. Suitable inert solvents for carrying out the reaction are known in the art.

Some of the starting materials of the formula XI and/or the formula XII are known and preferably commercially available. If they are not known, they can be prepared by methods known per se.

Independently of the choosen reaction route, it is in many cases possible or even feasible to introduce residues R⁸, R⁹ and/or R¹⁰ into one or more of the compounds described above, or, if the compound already comprises one or more residues R⁸, R⁹ and/or R¹⁰, to introduce additional residues R⁸, R⁹ and/or R¹⁰ into said compound. The introduction of additional residues can be readily performed by methods known in the art and especially by aromatic substitution, for example nucleophilic aromatic substitution or electrophilic aromatic substitution. For example, in compounds comprising Ar¹, wherein Ar¹ comprises one or more halogen and preferably fluorine substituents, one or more of the halogen/fluorine substituents can be easily substituted by hydroxy, thio and/or amino substituted hydrocarbons, preferably selected from the group consisting of HO(CH₂)ₙNR¹¹R¹², HO(CH₂)ₙO(CH₂)ₖNR¹¹R¹², HO(CH₂)ₙNR¹¹(CH₂)ₖOR¹², HO(CH₂)ₙNR¹¹(CH₂)ₖNR¹¹R¹², HO(CH₂)ₙCOOR¹³, HO(CH₂)ₙS(O)ᵤR¹³ HNR¹¹(CH₂)ₙNR¹¹R¹², HNR¹¹(CH₂)ₙO(CH₂)ₖNR¹¹R¹², HNR¹¹(CH₂)ₙNR¹¹(CH₂)ₖOR¹², HNR¹¹(CH₂)ₙNR¹¹(CH₂)ₖNR¹¹R¹², HNR¹¹(CH₂)ₙCOOR¹³ and HNR¹¹(CH₂)ₙS(O)ᵤR¹³ wherein R¹¹, R¹² and R¹³ are defined as above and n is as defined above, preferably n is 0, 1 or 2 and especially is 0, k is 1 to 4 and preferably 1 or 2, and u is preferably 2. In this embodiment R¹¹, R¹² and R¹³ are more preferably selected independently from each other from the group consisting of H, methyl and ethyl. Even more preferred, the hydroxy, thio and/or amino substituted hydrocarbons are selected from the group consisting of NH₃, HN(CH₃)₂, NH₂CH₃, HN(C₂H₅)₂, H₂NCH₂CH₂NH₂, HOCH₂CH₂NH₂, HOCH₂CH₂NHCH₃, HN(CH₃)CH₂CH₂NH₂, HN(CH₃)CH₂CH₂N(CH₃)₂, HN(CH₃)CH₂CH₂N(CH₃)₂, HN(CH₃)CH₂CH₂0CH₃, HOCH₂CH₂N(CH₃)₂, HOCH₂CH₂N(CH₂CH₃)₂, HSCH₃, HSC₂H₅, and compounds of the formulae 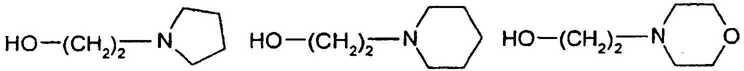 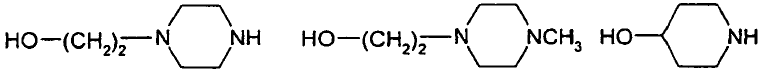 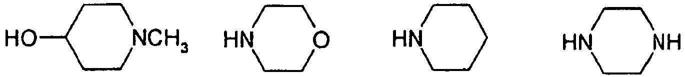 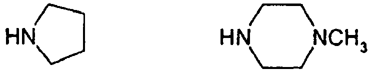 or salts and especially metal salts thereof.

On the other hand, it is in many cases possible or even feasible to modify or derivatize one or more of the residue is R⁸, R⁹ and R¹⁰ into residues R⁸, R⁹ and/or R¹⁰ other than the ones originally present. For example, CH₃-groups can be oxidised into aldehyde groups or carbonic acid groups, thio atom containing groups, for example S-alkyl or S-aryl groups, can be oxidised into SO₂-alkyl or SO₂-aryl groups, respectively, carbonic acid groups can be derivatized to carbonic acid ester groups or carbon amide groups and carbonic acid ester groups or carbon amide groups can be hydrolysed into the corresponding carbonic acid groups. Methods for performing such modifications or derivatizations are known in the art, for example from Houben-Weyl, Methods of Organic Chemistry.

Every reaction step described herein can optionally be followed by one or more working up procedures and/or isolating procedures. Suitable such procedures are known in the art, for example from standard works, such as Houben-Weyl, Methoden der organischen Chemie [Methods of Organic Chemistry], Georg-Thieme-Verlag, Stuttgart). Examples for such procedures include, but are not limited to evaporating a solvent, distilling, crystallization, fractionised crystallization, extraction procedures, washing procedures, digesting procedures, filtration procedures, chromatography, chromatography by HPLC and drying procedures, especially drying procedures in vacuo and/or elevated temperature.

A base of the formula II can be converted into the associated acid-addition salt using an acid, for example by reaction of equivalent amounts of the base and the acid in a preferably inert solvent, such as ethanol, followed by evaporation. Suitable acids for this reaction are, in particular, those which give physiologically acceptable salts. Thus, it is possible to use inorganic acids, for example sulfuric acid, sulfurous acid, dithionic acid, nitric acid, hydrohalic acids, such as hydrochloric acid or hydrobromic acid, phosphoric acids, such as, for example, orthophosphoric acid, sulfamic acid, furthermore organic acids, in particular aliphatic, alicyclic, araliphatic, aromatic or heterocyclic monobasic or polybasic carboxylic, sulfonic or sulfuric acids, for example formic acid, acetic acid, propionic acid, hexanoic acid, octanoic acid, decanoic acid, hexadecanoic acid, octadecanoic acid, pivalic acid, diethylacetic acid, malonic acid, succinic acid, pimelic acid, fumaric acid, maleic acid, lactic acid, tartaric acid, malic acid, citric acid, gluconic acid, ascorbic acid, nicotinic acid, isonicotinic acid, methane- or ethanesulfonic acid, ethanedisulfonic acid, 2-hydroxyethanesulfonic acid, benzenesulfonic acid, trimethoxybenzoic acid, adamantanecarboxylic acid, p-toluenesulfonic acid, glycolic acid, embonic acid, chlorophenoxyacetic acid, aspartic acid, glutamic acid, proline, glyoxylic acid, palmitic acid, parachlorophenoxyisobutyric acid, cyclohexanecarboxylic acid, glucose 1-phosphate, naphthalenemono- and -disulfonic acids or laurylsulfuric acid. Salts with physiologically unacceptable acids, for example picrates, can be used to isolate and/or purify the compounds of the formula II. On the other hand, compounds of the formula II can be converted into the corresponding metal salts, in particular alkali metal salts or alkaline earth metal salts, or into the corresponding ammonium salts, using bases (for example sodium hydroxide, potassium hydroxide, sodium carbonate or potassium carbonate). Suitable salts are furthermore substituted ammonium salts, for example the dimethyl-, diethyl- and diisopropyl-ammonium salts, monoethanol-, diethanol- and diisopropanolammonium salts, cyclohexyl- and dicyclohexylammonium salts, dibenzylethylenediammonium salts, furthermore, for example, salts with arginine or lysine.

On the other hand, if desired, the free bases of the formula II can be liberated from their salts using bases (for example sodium hydroxide, potassium hydroxide, sodium carbonate or potassium carbonate).

The invention relates to compounds of the formula II and the pharmaceutically acceptable solvates, salts and stereoisomers thereof, including mixtures thereof in all ratios, and more preferred the salts and/or solvates thereof, and especially preferred the physiologically acceptable salts and/or solvates thereof as medicaments.

The invention also relates to the compounds of the formula II and the pharmaceutically acceptable solvates, salts and stereoisomers thereof, including mixtures thereof in all ratios, and more preferred the salts and/or solvates thereof, and especially preferred the physiologically acceptable salts and/or solvates thereof which can be used as kinase inhibitors.

The invention furthermore relates to the use of the compounds of the formula II and the pharmaceutically acceptable solvates, salts and stereoisomers thereof, including mixtures thereof in all ratios, and more preferred the salts and/or solvates thereof, and especially preferred the physiologically acceptable salts and/or solvates thereof for the preparation of pharmaceutical compositions and/or pharmaceutical preparations, in particular by non-chemical methods. The invention furthermore relates to the use of the compounds of the formula II and/or physiologically acceptable salts and/or solvates thereof for the preparation of pharmaceutical compositions and/or pharmaceutical preparations, in particular by non-chemical methods. In this cases, one or more compounds according to the invention can be converted into a suitable dosage form together with at least one solid, liquid and/or semi-liquid excipient or adjuvant and, if desired, in combination with one or more further active ingredients.

The invention further relates to the use of one or more of the compounds according to the invention, selected from the group consisting of compounds of the formula II as free bases, solvates of compounds of the formula II and salts of compounds of formula II, for the production of pharmaceutical compositions and/or pharmaceutical preparations, in particular by a non-chemical route. In general, non-chemical routes for the production of pharmaceutical compositions and/or pharmaceutical preparations comprise processing steps on suitable mechanical means known in the art that transfer one or more compounds according to the invention into a dosage form suitable for administration to a patient in need of such a treatment. Usually, the transfer of one or more compounds according to the invention into such a dosage form comprises the addition of one or more compounds, selected from the group consisting of carriers, excipients, auxiliaries and pharmaceutical active ingredients other than the compounds according to the invention. Suitable processing steps include, but are not limited to combining, milling, mixing, granulating, dissolving, dispersing, homogenizing, casting and/or compressing the respective active and non-active ingridients. In this respect, active ingredients are preferably at least one compound according to this invention and one or more additional compounds other than the compounds according to the invention, which show valuable pharmaceutical properties, preferably those pharmaceutical active agents other than the compounds according to invention which are disclosed herein.

The process for preparing pharmaceutical compositions and/or pharmaceutical preparations preferably comprises one or more processing steps, selected from the group consisting of combining, milling, mixing, granulating, dissolving, dispersing, homogenizing and compressing. The one or more processing steps are preferably performed on one or more of the ingredients which are to form the pharmaceutical composition and/or pharmaceutical preparation preferably according to invention. Even more preferred, said processing steps are performed on two or more of the ingredients which are to form the pharmaceutical composition and/or pharmaceutical preparation, said ingredients comprising one or more compounds according to the invention and, additionally, one or more compounds, preferably selected from the group consisting of active ingredients other than the compounds according to the invention, excipients, auxiliaries, adjuvants and carriers. Mechanical means for performing said processing steps are known in the art, for example from Ullmann's Encyclopedia of Industrial Chemistry, 5th Edition.

Preferably, one or more compounds according to the invention are converted into a suitable dosage form together with at least one compound selected from the group consisting of excipients, auxiliaries, adjuvants and carriers, especially solid, liquid and/or semi-liquid excipients, auxiliaries, adjuvants and carriers, and, if desired, in combination with one or more further active ingredients.

Suitable dosage forms include, but are not limited to tablets, capsules, semi-solids, suppositories, aerosols, which can be produced according to methods known in the art, for example as described below:
- tablets: mixing of active ingredient/s and auxiliaries, compression of said mixture into tablets (direct compression), optionally granulation of part of mixture before compression
- capsules: mixing of active ingredient/s and auxiliaries to obtain a flowable powder, optionally granulating powder, filling powders/granulate into opened capsules, capping of capsules
- semi-solids (ointments, gels, creams): dissolving/dispersing active ingredient/s in an aqueous or fatty carrier; subsequent mixing of aqueous/fatty phase with complementary fatty resp. aqueous phase, homogenisation (creams only)
- suppositories (rectal and vaginal): dissolving/dispersing active ingredient/s in carrier material liquified by heat (rectal: carrier material normally a wax; vaginal: carrier normally a heated solution of a gelling agent), casting said mixture into suppository forms, annealing and withdrawal suppositories from the forms
- aerosols:: dispersing/dissolving active agent/s in a propellant, bottling said mixture into an atomizer

The invention thus relates to pharmaceutical compositions and/or pharmaceutical preparations comprising at least one compound of the formula II and/or one of its physiologically acceptable salts and/or solvates.

Preferably, the pharmaceutical compositions and/or pharmaceutical preparations according to the invention contain a therapeutic effective amount of one or more compounds according to the invention. Said therapeutic effective amount of one or more of the compounds according to the invention is known to the skilled artisan or can be easily determined by standard methods known in the art. For example, the compounds according to the invention can be administered to a patient in an analogous manner to other compounds that are effective as raf-kinase inhibitors. Usually, suitable doses that are therapeutically effective lie in the range between 0.0005 mg and 1000 mg, preferably between 0.005 mg and 500 mg and especially between 0.5 and 100 mg per dose unit. The daily dose comprises preferably more than 0.001 mg, more preferred more than 0.01 milligram, even more preferred more than 0.1 mg and especially more than 1.0 mg, for example more than 2.0 mg, more than 5 mg, more than 10 mg, more than 20 mg, more than 50 mg or more than 100 mg, and preferably less than 1500 mg, more preferred less than 750 mg, even more preferred less than 500 mg, for example less than 400 mg, less than 250 mg, less than 150 mg, less than 100 mg, less than 50 mg or less than 10 mg.

The specific dose for the individual patient depends, however, on the multitude of factors, for example on the efficacy of the specific compounds employed, on the age, body weight, general state of health, the sex, the kind of diet, on the time and route of administration, on the excretion rate, the kind of administration and the dosage form to be administered, the pharmaceutical combination and severity of the particular disorder to which the therapy relates. The specific therapeutic effective dose for the individual patient can readily be determined by routine experimentation, for example by the doctor or physician which advises or attends the therapeutic treatment.

However, the specific dose for each patient depends on a wide variety of factors, for example on the efficacy of the specific compound employed, on the age, body weight, general state of health, sex, on the diet, on the time and method of administration, on the rate of excretion, medicament combination and severity of the particular illness to which the therapy applies. Parenteral administration is preferred. Oral administration is especially preferred.
These compositions and/or preparations can be used as medicaments in human or veterinary medicine. Suitable excipients are organic or inorganic substances which are suitable for enteral (for example oral), parenteral or topical administration and do not react with the novel compounds, for example water, vegetable oils, benzyl alcohols, alkylene glycols, polyethylene glycols, glycerol triacetate, gelatine, carbohydrates, such as lactose or starch, magnesium stearate, talc or vaseline. Examples for suitable dosage forms, which are especially suitable for oral administration are, in particular, tablets, pills, coated tablets, capsulees, powders, granules, syrups, juices or drops. Further examples for suitable dosage forms, which are especially suitable for rectal administration are suppositories, further examples for suitable dosage forms, which are especially suitable for parenteral administration are solutions, preferably oil-based or aqueous solutions, furthermore suspensions, emulsions or implants, and suitable for topical application are ointments, creams or powders. The novel compounds may also be lyophilised and the resultant lyophilisates used, for example, for the preparation of injection preparations. The compositions and/or preparations indicated may be sterilized and/or comprise assistants, such as lubricants, preservatives, stabilizers and/or wetting agents, emulsifiers, salts for modifying the osmotic pressure, buffer substances, dyes and flavors and/or one or more further active ingredients, for example one or more vitamins.

For administration as an inhalation spray, it is possible to use sprays in which the active ingredient is either dissolved or suspended in a propellant gas or propellant gas mixture (for example CO₂ or chlorofluorocarbons). The active ingredient is advantageously used here in micronized form, in which case one or more additional physiologically acceptable solvents may be present, for example ethanol. Inhalation solutions can be administered with the aid of conventional inhalers.

The compounds of formula II and their physiologically acceptable salts and solvates can be employed for combating one or more diseases, for example allergic diseases, psoriasis and other skin diseases, especially melanoma, autoimmune diseases, such as, for example, rheumatoid arthritis, multiple sclerosis, Crohn's disease, diabetes mellitus or ulcerative colitis.

In general, the substances according to the invention are preferably administered in doses corresponding to the compound rolipram of between 1 and 500 mg, in particular between 5 and 100 mg per dosage unit. The daily dose is preferably between about 0.02 and 10 mg/kg of body weight.

However, the specific dose for each patient depends on a wide variety of factors, for example on the efficacy of the specific compound employed, on the age, body weight, general state of health, sex, on the diet, on the time and method of administration, on the excretion rate, medicament combination and severity of the particular illness to which the therapy applies.

The compounds of the formula II according to claim 1 and/or their physiologically acceptable salts are also suitable for in pathological processes which are maintained or propagated by angiogenesis, in particular in tumours, restenoses, diabetic retinopathy, macular degenerative disease or rheumatoid arthritis.

Those of skill will readily appreciate that dose levels can vary as a function of the specific compound, the severity of the symptoms and the susceptibility of the subject to side effects. Some of the specific compounds are more potent than others. Preferred dosages for a given compound are readily determinable by those of skill in the art by a variety of means. A preferred means is to measure the physiological potency of a given compound.
For use in the subject methods, the subject compounds may be formulated with pharmaceutically active agents other than the compounds according to the invention, particularly other anti-metastatic, antitumor or anti-angiogenic agents. Angiostatic compounds of interest include angiostatin, enclostatin, carboxy terminal peptides of collagen alpha (XV), etc. Cytotoxic and cytostatic agents of interest include adriamycin, aleran, Ara-C, BICNU, busulfan, CNNU, cisplatinum, cytoxan, daunorubicin, DTIC, 5-FU, hydrea, ifosfamicle, methotrexate, mithramycin, mitomycin, mitoxantrone, nitrogen mustard, velban, vincristine, vinblastine, VP-16, carboplatinum, fludarabine, gemcitabine, idarubicin, irinotecan, leustatin, navelbine, taxol, taxotere, topotecan, etc.

The compounds of the invention have been shown to have antiproliferative effects in an in vivo xenograft tumor model. The subject compounds are administered to a subject having a hyperproliferative disorders, e.g., to inhibit tumor growth, to decrease inflammation associated with a lymphoproliferative disorder, to inhibit graft rejection, or neurological damage due to tissue repair, etc. The present compounds are useful for prophylactic or therapeutic purposes. As used herein, the term "treating" is used to refer to both prevention of disease, and treatment of pre-existing conditions. The prevention of proliferation is accomplished by administration of the subject compounds prior to development of overt disease, e.g., to prevent the regrowth of tumors, prevent metastatic growth, diminish restenosis associated with cardiovascular surgery, etc. Alternatively the compounds are used to treat ongoing disease, by stabilizing or improving the clinical symptoms of the patient.
Furthermore, the compounds according the invention preferably can be utilized in the treatment of infectious diseases of diverse genesis.

Infections according the invention include, but are not limited to infections caused by pathogenic microorganisms, such as bacteria, fungi, viruses and protozoans, for example influenza (Pleschka, S. et al. Nature Cell Biol. 2001, 3, page 301-305), retroviruses, for example HIV infection (Yang, X. et al. J. Biol. Chem. 1999, 274, page 27981-27988; Popik, W et al Mol Cel Biol. 1996, 16, page 6532-6541), Hepatitis B (Benn, J et al., Proc. Natl. Acad. Sci. 1995, 92, page 11215-11219), Hepatitis C (Aoki et al. J. Virol. 2000, 74, page 1736-1741), papillomavirus, parainfluenza, rhinoviruses, adenoviruses, Heliobacter pylori, and viral and bacterial infections of the skin (e.g. cold sores, warts, chickenpox, molluscum, contagiosum, herpes zoster, boils, cellulitis, erysipelas, impetigo, tinea, Althlete's foot and ringworm).

Furthermore, the compounds according the invention preferably show anti-angiogenic properties.

Thus, compounds of the present invention can be advantageously employed in the treatment of one or more diseases afflicting mammals which are characterized by cellular proliferation in the area of disorders associated with neo- vascularization and/or vascular permeability including blood vessel proliferative disorders including arthritis and restenosis; fibrotic disorders including hepatic cirrhosis and atherosclerosis; mesangial cell proliferative disorders include glomerulonephritis, diabetic nephropathy, malignant nephrosclerosis, thrombotic microangiopathy syndromes, organ transplant rejection and glomerulopathies; and metabolic disorders include psoriasis, diabetes mellitus, chronic wound healing, inflammation and neurodegenerative diseases.

The host, or patient, may be from any mammalian species, e.g., primate sp., particularly human; rodents, including mice, rats and hamsters; rabbits; equines, bovines, canines, felines; etc. Animal models are of interest for experimental investigations, providing a model for treatment of human disease.

The susceptibility of a particular cell to treatment with the subject compounds may be determined by in vitro testing. Typically a culture of the cell is combined with a subject compound at varying concentrations for a period of time sufficient to allow the active agents to induce cell death or inhibit migration, usually between about one hour and one week. For in vitro testing, cultured cells from a biopsy sample may be used. The viable cells left after treatment are then counted.

The dose will vary depending on the specific compound utilized, specific disorder, patient status, etc. Typically a therapeutic dose will be sufficient to substantially decrease the undesirable cell population in the targeted tissue, while maintaining patient viability. Treatment will generally be continued until there is a substantial reduction, e.g., at least about 50 %, decrease in the cell burden, and may be continued until there are essentially none of the undesirable cells detected in the body.

The compounds according to the invention are preferably administered to human or nonhuman animals, more preferred to mammalian animals and especially to humans.

The compounds also find use in the specific inhibition of a signaling pathway mediated by protein kinases. Protein kinases are involved in signaling pathways for such important cellular activities as responses to extracellular signals and cell cycle checkpoints. Inhibition of specific protein kinases provided a means of intervening in these signaling pathways, for example to block the effect of an extracellular signal, to release a cell from cell cycle checkpoint, etc. Defects in the activity of protein kinases are associated with a variety of pathological or clinical conditions, where there is a defect in the signaling mediated by protein kinases. Such conditions include those associated with defects in cell cycle regulation or in response to extracellular signals, e.g., immunological disorders, autoimmune and immunodeficiency diseases; hyperproliferative disorders, which may include psoriasis, arthritis, inflammation, endometriosis, scarring, cancer, etc. The compounds of the present invention are active in inhibiting purified kinase proteins preferably raf kinases, e.g., there is a decrease in the phosphorylation of a specific substrate in the presence of the compound. The compounds of the invention may also be useful as reagents for studying signal transduction or any of the clinical disorders listed throughout this application.

There are many disorders associated with a dysregulation of cellular proliferation. The conditions of interest include, but are not limited to, the following conditions. The subject compounds are useful in the treatment of a variety of conditions where there is proliferation and/or migration of smooth muscle cells, and/or inflammatory cells into the intimal layer of a vessel, resulting in restricted blood flow through that vessel, e.g., neointimal occlusive lesions. Occlusive vascular conditions of interest include atherosclerosis, graft coronary vascular disease after transplantation, vein graft stenosis, peri-anastomatic prothetic graft stenosis, restenosis after angioplasty or stent placement, and the like.

Diseases where there is hyperproliferation and tissue remodelling or repair or reproductive tissue, e.g., uterine, testicular and ovarian carcinomas, endometriosis, squamous and glandular epithelial carcinomas of the cervix, etc. are reduced in cell number by administration of the subject compounds. The growth and proliferation of neural cells is also of interest.

Tumor cells are characterized by uncontrolled growth, invasion to surrounding tissues, and metastatic spread to distant sites. Growth and expansion requires an ability not only to proliferate, but also to down-modulate cell death (apoptosis) and activate angiogenesis to produce a tumor neovasculature.

Tumors of interest for treatment include carcinomas, e.g., colon, duodenal, prostate, breast, melanoma, ductal, hepatic, pancreatic, renal, endometrial, stomach, dysplastic oral mucosa, polyposis, invasive oral cancer, non-small cell lung carcinoma, transitional and squamous cell urinary carcinoma etc.; neurological malignancies; e.g. neuroplastoma, gliomas, etc.; hematological malignancies, e.g., childhood acute leukaemia, non-Hodgkin's lymphomas, chronic lymphocytic leukaemia, malignant cutaneous T-cells, mycosis fungoides, non-MF cutaneous T-cell-lymphoma, lymphomatoid papulosis, T-cell rich cutaneous lymphoid hyperplasia, bullous pemphigoid, discoid lupus erythematosus, lichen planus, etc.; and the like.

Tumors of neural tissue are of particular interest, e.g., gliomas, neuromas, etc. Some cancers of particular interest include breast cancers, which are primarily adenocarcinoma subtypes. Ductal carcinoma in situ is the most common type of noninvasive breast cancer. In DCIS, the malignant cells have not metastasized through the walls of the ducts into the fatty tissue of the breast. Infiltration (or invasive) ductal carcinoma (IDC) has metastasized through the wall of the duct and invaded the fatty tissue of the breast. Infiltrating (or invasive) lobular carcinoma (ILC) is similar to IDC, in that it has the potential to metastasize elsewhere in the body. About 10 % to 15 % of invasive breast cancers are invasive lobular carcinomas.

Also of interest is non-small cell lung carcinoma. Non-small cell lung cancer (NSCLC) is made up of three general subtypes of lung cancer. Epidermoid carcinoma (also called squamos cell carcinoma) usually starts in one of the larger bronchial tubes and grows relatively slowly. The size of these tumors can range from very small to quite large. Adenocarcinoma starts growing near the outside surface of the lung and may vary in both size and growth rate. Some slowly growing adenocarcinomas are described as alveolar cell cancer. Large cell carcinoma starts near the surface of the lung, grows rapidly, and the growth is usually fairly large when diagnosed. Other less common forms of lung cancer are carcinoid, cylindroma, mucoepidermoid, and malignant mesothelioma.

Melanoma is a malignant tumor of melanocytes. Although most melanomas arise in the skin, they also may arise from mucosal surfaces or at other sites to which neural crest cells migrate. Melanoma occurs predominantly in adults, and more than half of the cases arise in apparently normal areas of the skin. Prognosis is affected by clinical and histological factors and by anatomic location of the lesion. Thickness and/or level of invasion of the melanoma, mitotic index, tumor infiltrating lymphocytes, and ulceration or bleeding at the primary site affect the prognosis. Clinical staging is based on whether the tumor has spread to regional lymph nodes or distant sites. For disease clinically confined to the primary site, the greater the thickness and depth of local invasion of the melanoma, the higher the chance of lymph node metastases and the worse the prognosis. Melanoma can spread by local extension (through lymphatics) and/or by hematogenous routes to distant sites. Any organ may be involved by metastases, but lungs and liver are common sites.

Other hyperproliferative diseases of interest relate to epidermal hyperproliferation, tissue, remodeling and repair. For example, the chronic skin inflammation of psoriasis is associated with hyperplastic epidermal keratinocyctes as well as infiltrating mononuclear cells, including CD4+ memory T cells, neutrophils and macrophages.

The proliferation of immune cells is associated with a number of autoimmune and lymphoproliferative disorders. Diseases of interest include multiple sclerosis, rheumatoid arthritis and insulin dependent diabetes mellitus. Evidence suggests that abnormalities in apoptosis play a part in the pathogenesis of systemic lupus erythematosus (SLE). Other lymphoproliferative conditions the inherited disorder of lymphocyte apoptosis, which is an autoimmune lymphoproliferative syndrome, as well as a number of leukemia's and lymphomas. Symptoms of allergies to environmental and food agents, as well as inflammatory bowel disease, may also be alleviated by the compounds of the invention.

Surprisingly, it has been found that malonamide derivatives according to invention are able to interact with signaling pathways, especially the signaling pathways described herein and preferably the raf-kinase signaling pathway. Malonamide derivatives according to the invention preferably show advantageous biological activity which can easily be demonstrated according to methods known in the art, for example by enzyme based assays. Suitable assays are known in the art, for example from the literature cited herein and the references cited in the literature, or can be developed and/or performed in an analogous manner thereof. In such enzyme based assays, malonamide derivatives according to invention show an effect, preferably a modulating and especially an inhibiting effect which is usually documented by IC₅₀ values in a suitable range, preferably in the micromolar range and more preferred in the nanomolar range.

In general, compounds according to the invention are to be regarded as suitable kinase-modulators and especially suitable kinase-inhibitors according to the invention if they show an effect or an activity to one or more kinases, preferably to one or more kinase is as defined herein and more preferably to one or more raf-kinases, that preferably lies, determined as IC₅₀-value, in the range of 100 µmol or below, preferably 10 µmol or below, more preferably in the range of 3 µmol or below, even more preferably in the range of 1 µmol or below and most preferably in the nanomolar range. Especially preferred for use according to the invention are kinase-inhibitors as defined above/below, that show an activity, determined as IC₅₀-value, to one or more kinases, preferably kinases as defined herein and more preferably to one or more raf-kinases, even more preferably including A-raf, B-raf and c-raf1 or consisting of A-raf, B-raf and c-raf1 and especially preferred including c-raf1 or consisting of c-raf1, in the range of 0.5 µmol or below and especially in the range of 0.1 µmol or below. In many cases an IC₅₀-value at the lower end of the given ranges is advantageous and in some cases it is highly desirable that the IC₅₀-value is as small as possible or the he IC₅₀-values are as small as possible, but in general IC₅₀-values that lie between the above given upper limits and a lower limit in the range of 0.0001 µmol, 0.001 µmol, 0.01 µmol or even above 0.1 µmol are sufficient to indicate the desired pharmaceutical activity. However, the activities measured can vary depending on the respective testing system or assay chosen.

Alternatively, the advantageous biological activity of the compounds according to the invention can easily be demonstrated in *in vitro* assays, such as *in vitro* proliferation assays or *in vitro* growth assays. Suitable *in vitro* assays are known in the art, for example from the literature cited herein and the references cited in the literature or can be performed as described below, or can be developed and/or performed in an analogous manner thereof.

As an example for an *in vitro* growth assay, human tumor cell lines, for example HCT116, DLD-1 or MiaPaCa, containing mutated K-ras genes can be used in standard proliferation assays, for example for anchorage dependent growth on plastic or anchorage independent growth in soft agar. Human tumor cell lines are commercially available, for example from ATCC (Rockville MD), and can be cultured according to methods known in the art, for example in RPMI with 10% heat inactivated fetal bovine serum and 200 mM glutamine. Cell culture media, fetal bovine serum and additives are commercially available, for example from Invitrogen/Gibco/BRL (Karlsruhe, Germany) and/or QRH Biosciences (Lenexa, KS). In a standard proliferation assay for anchorage dependent growth, 3 X 10³ cells can be seeded into 96-well tissue culture plates and allowed to attach, for example overnight at 37 °C in a 5% CO₂ incubator. Compounds can be titrated in media in dilution series and added to 96 well cell cultures. Cells are allowed to grow, for example for 1 to 5 days, typically with a feeding of fresh compound containing media at about half of the time of the growing period, for example on day 3, if the cells are allowed to grow 5 days. Proliferation can be monitored by methods known in the art, such as measuring metabolic activity, for example with standard XTT colorimetric assay (Boehringer Mannheim) measured by standard ELISA plate reader at OD 490/560, by measuring ³H-thymidine incorporation into DNA following an 8 h culture with 1µCu ³H-thymidine, harvesting the cells onto glass fiber mats using a cell harvester and measuring ³H-thymidine incorporation by liquid scintillation counting, or by staining techniques, such as crystal violet staining. Other suitable cellular assay systems are known in the art.

Alternatively, for anchorage independent cell growth, cells can be plated at 1 x 10³ to 3 x 10³ in 0.4% Seaplaque agarose in RPMI complete media, overlaying a bottom layer containing only 0.64% agar in RPMI complete media, for example in 24-well tissue culture plates. Complete media plus dilution series of compounds can be added to wells and incubated, for example at 37 °C in a 5% CO₂ incubator for a sufficient time, for example 10-14 days, preferably with repeated feedings of fresh media containing compound, typically at 3-4 day intervals. Colony formation and total cell mass can be monitored, average colony size and number of colonies can be quantitated according to methods known in the art, for example using image capture technology and image analysis software. Image capture technology and image analysis software, such as Image Pro Plus or media Cybernetics.

As discussed herein, these signaling pathways are relevant for various disorders. Accordingly, by interacting with one or more of said signaling pathways, malonamide derivatives are useful in the prevention and/or the treatment of disorders that are dependent from said signaling pathways. The compounds according to the invention are preferably kinase modulators and more preferably kinase inhibitors. According to the invention, kinases include, but are not limited to one or more Raf-kinases, one or more Tie-kinases, one or more VEGFR-kinases, one or more PDGFR-kinases, p38-kinase and/or SAPK2alpha.

Preferably, kinases according to the invention are selected from Serine/Threonine kinases (STK) and Receptor-Tyrosine kinases (RTK).

Serine/Threonine kinases according to the invention are preferably selected from one or more Raf-kinases, p38-kinase and SAPK2alpha. Receptor-Tyrosine kinases according to the invention are preferably selected from one or more PDGFR-kinases, one or more VEGFR-kinases and one or more Tie-kinases.

Preferably, kinases according to the invention are selected from one or more Raf-kinases, one or more Tie-kinases, one or more VEGFR-kinases, one or more PDGFR-kinases, p38-kinase and SAPK2alpha.

Raf-kinases in this respect are respect preferably include or consist of A-Raf, B-Raf and c-Raf1.

Tie-kinases in this respect preferably include or consist of Tie-2 kinase.

VEGFR-kinases in this respect preferably include or consist of VEGFR-2 kinase.

Preferred signalling pathways according to the invention are signalling pathways, wherein one or more of the kinases given above are involved.

Due to the kinase modulating or inhibting properties of the compounds according to the invention, the compounds according to the invention preferably interact with one or more signalling pathways which are preferably cell signalling pathways, preferably by downregulating or inhibiting said signaling pathways. Examples for such signalling pathways include, but are not limited to the raf-kinase pathway, the Tie-kinase pathway, the VEGFR-kinase pathway, the PDGFR-kinase pathway, the p38-kinase pathway, the SAPK2alpha pathway and/or the Ras-pathway.

Modulation of the raf-kinase pathway plays an important role in various cancerous and noncancerous disorders, preferably cancerous disorders, such as dermatological tumors, haematological tumors, sarcomas, squamous cell cancer, gastric cancer, head cancer, neck cancer, oesophageal cancer, lymphoma, ovary cancer, uterine cancer and/or prostate cancer. Modulation of the raf-kinase pathway plays a even more important role in various cancer types which show a constitutive activation of the raf-kinase dependent signalling pathway, such as melanoma, colorectal cancer, lung cancer, brain cancer, pancreatic cancer, breast cancer, gynaecological cancer, ovarian cancar, thyroid cancer, chronic leukaemia and acute leukaemia, bladder cancer, hepatic cancer and/or renal cancer. Modulation of the raf-kinase pathway plays also an important role in infection diseases, preferably the infection diseases as mentioned above/below and especially in Helicobacter pylori infections, such as Helicobacter pylori infection during peptic ulcer disease.

One or more of the signalling pathways mentioned above/below and especially the VEGFR-kinase pathway plays an important role in angiogenesis. Accordingly, due to the kinase modulating or inhibting properties of the compounds according to the invention, the compounds according to the invention are suitable for the prophylaxis and/or treatment of pathological processes or disorders caused, mediated and/or propagated by angiogenesis, for example by inducing anti-angiogenesis. Pathological processes or disorders caused, mediated and/or propagated by angiogenesis include, but are not limited to tumors, especially solid tumors, arthritis, especially heumatic or rheumatoid arthritis, diabetic retinopathy, psoriasis, restenosis; fibrotic disorders; mesangial cell proliferative disorders, diabetic nephropathy, malignant nephrosclerosis, thrombotic microangiopathy syndromes, organ transplant rejection, glomerulopathies, metabolic disorders, inflammation and neurodegenerative diseases, and especially solid tumors, rheumatic arthritis, diabetic retinopathy and psoriasis.

Modulation of the p38-signalling pathway plays an important role in various cancerous and although in various noncancerous disorders, such as fibrosis, atherosclerosis, restenosis, vascular disease, cardiovascular disease, inflammation, renal disease and/or angiogenesis, and especially noncancerous disorders such as rheumatoid arthritis, inflammation, autoimmune disease, chronic obstructive pulmonary disease, asthma and/or inflammatory bowel disease.

Modulation of the PDGF-signalling pathway plays an important role in various cancerous and although in various noncancerous disorders, such as rheumatoid arthritis, inflammation, autoimmune disease, chronic obstructive pulmonary disease, asthma and/or inflammatory bowel disease, and especially noncancerous disorders such as fibrosis, atherosclerosis, restenosis, vascular disease, cardiovascular disease, inflammation, renal disease and/or angiogenesis.

Subject of the present invention are therefore malonamide derivatives according to the invention as promoters or inhibitors, preferably as inhibitors, of the signaling pathways described herein. Preferred subject of the invention are therefore malonamide derivatives according to the invention as promoters or inhibitors, preferably as inhibitors of the raf-kinase pathway. More preferred subject of the invention are therefore malonamide derivatives according to the invention as promoters or inhibitors, preferably as inhibitors of the raf-kinase. Even more preferred subject of the invention are malonamide derivatives according to invention as promoters or inhibitors, preferably as inhibitors of one or more raf-kinases, selected from the group consisting of A-raf, B-raf and c-raf1. Especially preferred subject of the invention are malonamide derivatives according to the invention as promoters or inhibitors, preferably as inhibitors of c-raf1.

Thus, subject of the present invention are malonamide derivatives according to the invention as medicaments. Subject of the present invention are malonamide derivatives according to the invention as medicament active ingredients. Further subject of the present invention is the use of one or more malonamide derivatives according to the invention as a pharmaceutical. Further subject of the present invention are one or more malonamide derivatives according to the invention for use in the treatment and/or the prophylaxis of disorders, preferably the disorders described herein, more preferred disorders that are caused, mediated and/ or propagated by signalling pathways discussed herein, even more preferred disorders that are caused, mediated and/or propagated by raf-kinases and especially disorders that are caused, mediated and/or propagated by raf-kinases, selected from the group consisting of A-raf, B-raf and c-raf1. Usually, the disorders discussed herein are divided into two groups, hyperproliferative and non hyperproliferative disorders. In this context, psioarsis, arthritis, inflammation, endometriosis, scarring, begnin prostatic hyperplasia, immunological diseases, autoimmune diseases and immunodeficiency diseases are to be regarded as noncancerous disorders, of which arthritis, inflammation, immunological diseases, autoimmune diseases and immunodeficiency diseases are usually regarded as non hyperproliferative disorders. In this context, brain cancer, lung cancer, squamous cell cancer, bladder cancer, gastric cancer, pancreatic cancer, hepatic cancer, renal cancer, colorectal cancer, breast cancer, head cancer, neck cancer, oesophageal cancer, gynaecological cancer, thyroid cancer, lymphoma, chronic leukaemia and acute leukaemia are to be regarded as cancerous disorders, all of which are usually regarded as hyperproliferative disorders. Especially cancerous cell growth and especially cancerous cell growth mediated by raf-kinase is a disorder which is a target of the present invention. Subject of the present invention therefore are malonamide derivatives according to the invention as medicaments and/or medicament active ingredients in the treatment and/or the prophylaxis of said disorders and the use of malonamide derivatives according to the invention for the manufacture of a pharmaceutical for the treatment and/or the prophylaxis of said disorders.

Accordingly, subject of the present invention are pharmaceutical compositions that contain one or more malonamide derivatives according to the invention. Subject of the present invention are especially pharmaceutical compositions that contain one or more malonamide derivatives according to the invention and one or more additional compounds (other than the compounds of the instant invention), preferably selected from the group consisting of physiologically acceptable excipients, auxiliaries, adjuvants, carriers and pharmaceutically active ingredients other than the compounds according to the invention.
Accordingly, subject of the present invention is a process for the manufacture of a pharmaceutical composition, wherein one or more malonamide derivatives according to the invention and one or more compounds (other than the compounds of the instant invention), preferably selected from the group consisting of carriers, excipients, auxiliaries, adjuvants and pharmaceutically active ingredients other than the compounds according to the invention.

Accordingly, the compounds according to the invention for use in the treatment of hyperproliferative disorders is a subject of the instant invention.

Accordingly, the use of the compounds according to the invention for producing a medicament for the treatment of hyperproliferative disorders is a subject of the instant invention.

The compounds according to the invention can be employed in a method for the treatment of cancerous cell growth mediated by one or more kinases and especially cancerous cell growth mediated by one or more raf-kinases

Above and below, all temperatures are given in °C. In the examples below, "conventional work-up" means that the organic phase is washed with saturated NaHCO₃ solution, if desired with water and saturated NaCl solution, the phases are separated, the organic phase is dried over sodium sulfate and evaporated, and the product is purified by chromatography on silica gel, by preparative HPLC and/or by crystallization.

### Examples

### Synthesis of the phenylamine moieties

### 4-(4-Pyridinyloxy)phenylamine

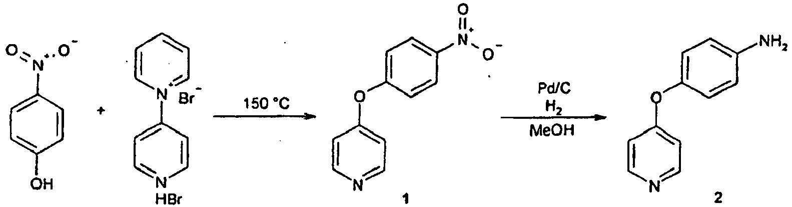

a) 195 g (1.4 mol) of 4-nitrophenol and 445.2 g (1.4 mol) of bipyridine are thoroughly mixed and slowly heated to 150°C. After the batch had been stirred at 150°C for 3 hours, it is poured while still hot into 5 I of ice-water. The mixture is acidified using hydrochloric acid, and the aqueous phase is washed 2' with 3 I of methyl tert-butyl ether. The aqueous phase is rendered basic (pH 12) using conc. sodium hydroxide solution and extracted 2' with 3 I of methyl tert-butyl ether. The combined organic phases are washed 4' with 1 I of water, dried using Na₂SO₄, filtered and evaporated. The residue is dissolved in 100 ml of ether, and the product is brought to crystallization in the ice bath by addition of 200 ml of petroleum ether. The crystals are filtered off with suction and dried under reduced pressure.
   Yield: 75 g (25%) of 1, brown crystals
b) Compound 1 is hydrogenated at room temperature using Pd/C in MeOH. The reaction solution is filtered through kieselguhr and rinsed with MeOH, and the filtrate is subsequently evaporated. The residue is digested with diethyl ether:petroleum ether = 2:1, filtered off with suction, rinsed with petroleum ether and dried overnight at 40°C under reduced pressure. Yield: 50.94 g (76%) of 2, brown crystals

### 3-(4-Pyridinyloxy)phenylamine

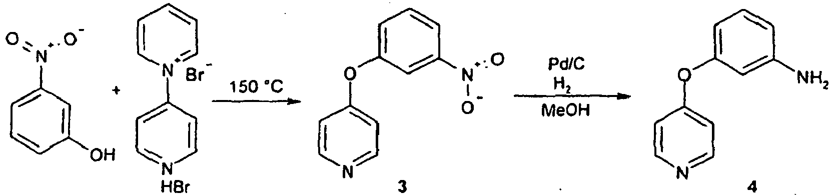

a) 200 g (1.44 mol) of 3-nitrophenol and 457.93 g (1.44 mol) of bipyridine are thoroughly mixed and slowly heated to 150°C. After the batch had been stirred at 150°C for 3 hours, it is poured while still hot into 5 I of ice-water. The mixture is acidified using hydrochloric acid, and the aqueous phase is washed 2' with 3 l of methyl tert-butyl ether. The aqueous phase is rendered basic (pH 12) using conc. sodium hydroxide solution and extracted 2' with 3 I of methyl tert-butyl ether. The combined organic phases are washed 4' with 1 I of water, dried over Na₂SO₄, filtered and evaporated. The residue is dissolved in 2 I of diethyl ether, 20 g of activated carbon are added, and the mixture is stirred for 1 hour and filtered. The filtrate is evaporated to about 200 ml, and the product is brought to crystallization in the ice bath by addition of 500 ml of petroleum ether. The crystals are filtered off with suction and dried under reduced pressure.
   Yield: 131 g (42%) of 3, beige crystals
b) Compound 3 is hydrogenated at room temperature using Pd/C in MeOH. The reaction solution is filtered through kieselguhr and rinsed with MeOH, and the filtrate is subsequently evaporated. The residue is digested with diethyl ether, filtered off with suction, rinsed with diethyl ether and dried overnight at 40°C under reduced pressure.
   Yield: 98.08 g (87%) of 4, pale-brown crystals

### 4-(3-Pyridinyloxy)phenylamine

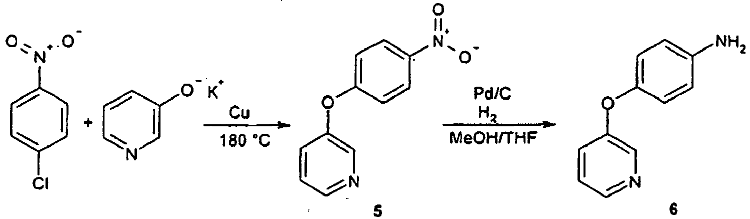

125 g (0.94 mol) of 3-hydroxypyridine, potassium salt, 300 g of 1-chloro-4-nitrobenzene and 15 g of copper are homogenized and heated to 180°C. The reaction mixture is stirred at 180°C for 6 hours and cooled to 90°C, and methyl tert-butyl ether is subsequently added rapidly. The suspension is stirred for 1 hour and filtered with suction. The filtrate is extracted 3' with 1 I of 10% HCl solution. The aqueous phase is rendered alkaline using NH₄OH solution and extracted with ethyl acetate. The combined organic phases are dried using Na₂SO₄, filtered and evaporated. The residue is purified by column chromatography (1 kg of silica gel, eluent: dichloromethane), taken up in 10% HCl solution and extracted with ethyl acetate. The aqueous phase is rendered alkaline using NH₄OH solution, and the deposited crystals are filtered off with suction, washed with a little cold water and dried in air for 4 days.
Yield: 44.7 g (22%) of 5, brown crystals
b) Compound 5 is hydrogenated at room temperature using Pd/C in MeOH/THF. The reaction solution is filtered through kieselguhr and rinsed with MeOH, and the filtrate is subsequently evaporated. The residue is digested with diethyl ether, filtered off with suction, rinsed with diethyl ether and dried overnight at 40°C under reduced pressure.
   Yield: 37.14 g (95%) of 6, pale-brown crystals

### 3-(3-Pyridinyloxy)phenylamine

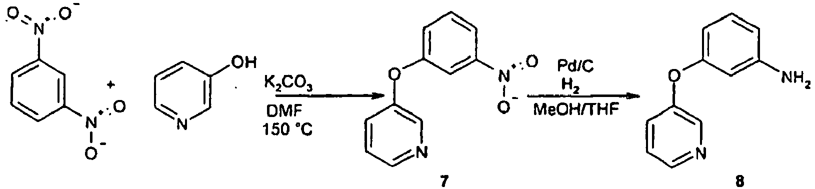

a) 50 g (0.53 mol) of 3-hydroxypyridine, 178.8 g (1.05 mol) of 1,3-dinitrobenzene and 159.9 g (1.16 mol) of K₂CO₃ are suspended in 1.4 I of DMF, and the suspension is heated to 150°C. After the reaction mixture had been stirred at 150°C for 16 hours, it is cooled to room temperature and evaporated. The residue is taken up in 1.5 I of ethyl acetate, stirred for 30 minutes and filtered. The filtrate is extracted with 10% HCl solution. The aqueous phase is rendered alkaline using NH₄OH solution and extracted with ethyl acetate. The combined organic phases are dried using Na₂SO₄, filtered and evaporated. The residue is purified by column chromatography (1 kg of silica gel, eluent: dichloromethane), taken up in 10% HCl solution and extracted with ethyl acetate. The aqueous phase is rendered alkaline using NH₄OH solution and extracted with ethyl acetate. The organic phase is dried using Na₂SO₄, filtered and evaporated.
   Yield: 98 g (86%) of 7, brown oil
b) Compound 7 is hydrogenated at room temperature using Pd/C in MeOH/THF. The reaction solution is filtered through kieselguhr and rinsed with MeOH, and the filtrate is subsequently evaporated. The residue is digested with 50 ml of diethyl ether:petroleum ether = 1:1, filtered off with suction and rinsed with petroleum ether. The mother liquor is evaporated to dryness, and the residue is stored overnight in the refrigerator. The crystals formed are digested with petroleum ether:diethyl ether = 9:1 and filtered off with suction. The combined crystal batches are dried overnight at 40°C under reduced pressure.
   Yield: 77.7 g (91%) of 8, pale-brown crystals

### 4-(4-Pyridinylmethyl)-phenylamine

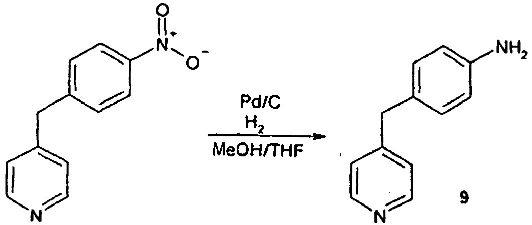

10 g 4-(4-Nitrobenzyl)-pyridin (46.7 mmol) are hydrogenated in MeOH/THF at room temperature in the presence of Pd/C. The reaction mixture is filtered over kieselguhr, washed with MeOH and the filtrate is evaporated. The residue is digested with 50 ml the diethylether:petrol ether (1:1), filtered and washed with petrol ether. The mother liquor is evaporated to dryness and the residue allowed to stand in the refrigerator overnight. The obtained residue are digested with petrol ether:diethylether (9:1) and filtered by suction. The combined crystals are dried in vacuo at 40 °C overnight. Yield: 7.96 g (93 %) 9, beige crystals

### 5-(3-Aminophenoxy)-pyridine-2-carbonic acid, methyl amide

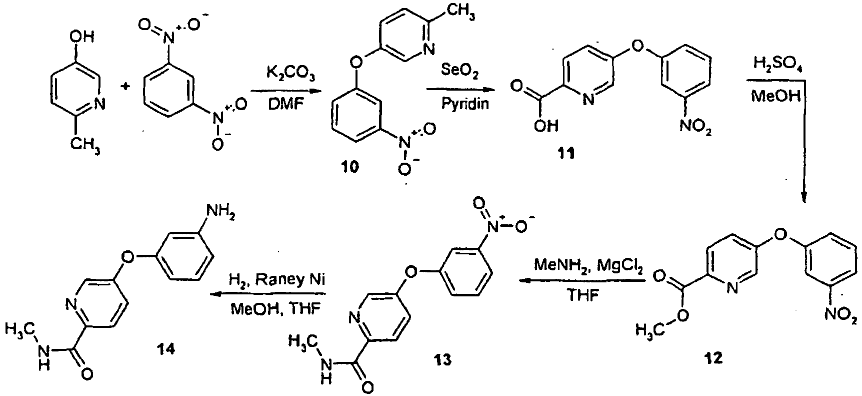

a) 5 g (0.045 mmol) 3-hydroxy-6-methyl pyridine, 15.25 g (0.091 mmol) 1,3-dinitrobenzene and 13,79 g (0.1 mmol) K₂CO₃ are suspended in 150 ml DMF and heated to 150 °C. After 16 hours at that temperature, the reaction mixture is cooled to room temperature and evaporated. The residue is taken up in 150 ml ethyl acetate, stirred for 15 minutes and filtered. The filtrate is extracted with hydrochloric acid-solution (10%). The water phase is neutralised with NH₄OH-solution and extracted with ethyl acetate. The combined organic phases are dried over Na₂SO₄, filtered and evaporated. Yield: 10.4 g (97%) 10, dark oil
b) 3.7 g (15.6 mmol) of 10 and 5.19 g (46.77 mmol) selenium oxide in 40 ml pyridine are heated to 126 °C. After four days at that temperature, the reaction mixture is cooled to room temperature. The obtained dark suspension is filtered by suction and the filtrate is evaporated. Yield: 7.55 g 11, dark oil.
c) 4.1 g (15.76 mmol) of 11 (as obtained above without further purification) in 25 ml MeOH is treated dropwise with 1.5 ml sulfuric acid and heated to reflux. After 3 hrs at that temperature, the reaction mixture is cooled to room temperature, treated with 11 ml 2M NaOH-solution under stirring and brought to a pH = 7 by addition of Na₂CO₃. The obtained crystals are filtered by suction, washed with water and dried. Yield 3.8 g (83%) 12, brownish pink crystals.
d) 3.7 g (12.82 mmol) 12 and 610 mg (6.41 mmol) magnesium chloride in 15 ml THF are stirred 5 min at room temperature. Then, 12.8 ml (25.63 mmol) methyl amine are added dropwise within 10 min and stirring is continued for three hours at room temperature. Water is added to the reaction mixture and the precipitated crystals are filtered by suction, washed with water and dried. Yield 2.8 g (80%) 13, brown crystals.
e) 3.5 g (12.81 mmol) 13 in MeOH/THF are hydrogenated in the presence of Raney nickel at room temperature. The reaction mixture is filtered over kieselguhr, the residue washed with MeOH and the filtrate evaporated. Yield: 2.7 g (87%) 14, brown oil.

### 4-[(4-Aminomethyl)phenoxy]-2-pyridine carbonic acid, methylamide

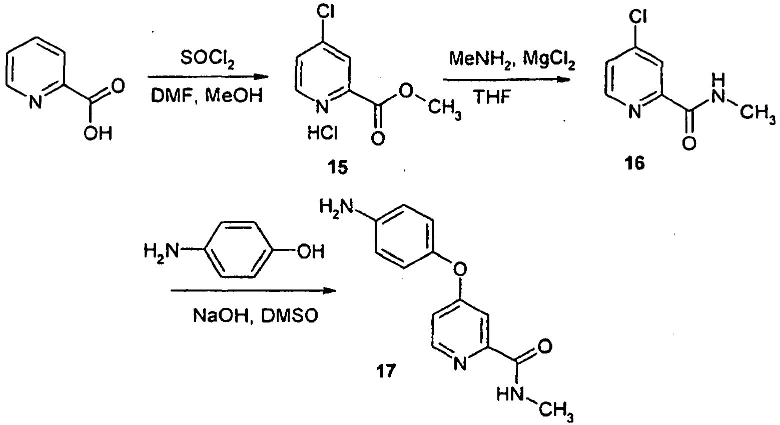

a) 750 ml Thionylchloride are heated to a temperature of 45 °C under a nitrogen atmosphere and 23 ml Dimethylformamide is added dropwise. 250 g Pyridine-2-carbonic acid is added to the solution in portions, the reaction mixture is stirred another 15 min at 45 °C and then heated to 80 °C for 24 hrs. The reaction mixture is evaporated and the resulting residue treated several times with dry toluene as a carrier and then evaporated. The resulting oil is dissolved in 180 ml toluene, cooled to 0 °C, slowly treated with 110 ml methanol and stirred for one hour. The resulting precipitate is filtered by suction, washed with toluene, recrystallised several times from acetone and dried.
   Yield: 140 g (33 %) **15,** colourless crystals
b) 140 g (0.673 mol) **15** are dissolved together with 32 g (0.336 mol) magnesiumchloride in 2 I THF. After 5 min 1.36 I (2.369 mol) methyl amine-solution are added dropwise within 20 min and the suspension stirred for 16 h at room temperature. 1.3 I water und 680 ml 1 M HCl-solution are added and the mixture is extracted with ethyl acetate (3 x 1 l). The combined organic phases are washed with brine, dried with Na₂SO₄, filtered and evaporated. The crude product is dissolved in 300 ml ethyl acetate and extracted with 200 ml 1N hydrochloric acid. The water phase is made alkaline (pH = 9) with 25% NH₄OH and extracted with ethyl acetate (2 x 400 ml). The organic phase is dried over Na₂SO₄, filtered and evaporated. Yield: 93 g (81%) **16,** brown oil.
c) 50 g (0.293 mol) **16** and 32.6 g (0.293 mol) 4-amino phenol, dissolved in DMSO, are treated slowly with 29.3 g (0.733 mol) sodium hydroxide. The resulting solution is heated under stirring to 100 °C overnight. After addition of another 29.3 g (0.733 mol) sodium hydroxide, the reaction mixture is heated under stirring to 100 °C for another night. The reaction mixture is cooled to room temperature, treated with icewater and extracted several times with the diethylether. The combined organic phases are dried over Na₂SO₄, filtered and evaporated. Yield: 36 g (51 %) **17,** brown oil.

### 4-(3-Aminophenoxy)-pyridine-2-carbonic acid, methyl amide

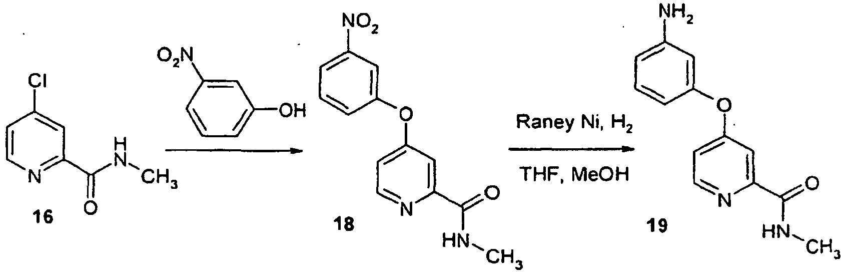

2.8 g (16.41 mmol) 16 and 4.6 g (32.83 mmol) 3-nitro phenol are stirred at 150 °C overnight. The reaction mixture is cooled to room temperature, treated with ethyl acetate and 2N NaOH-solution. The organic phase is separated. The water phase is again extracted to times with ethyl acetate. The combined organic phases are washed twice with brine, dried over Na₂SO₄, filtered and evaporated. The residue is put on silica gel and purified by column chromatography with n-heptane/ethyl acetate as eluent. Yield: 2.88 g (62%) **18,** pale yellow crystals
b) Compound **18** is hydrogenated in MeOH/THF solution in the presence of Raney nickel at room temperature. The reaction mixture is filtered through a Seitz-filter, washed with MeOH and the filtrate is evaporated. The residue is taken up in dichloromethane, dried with Na₂SO₄, filtered and evaporated.
   Yield: 2.29 g (92%) 19, brownish oil.

### Synthesis of the malonamides

### 3-[(4-Chloro-3-(trifluoromethyl)phenyl)-3-oxo-propionic acid, methyl ester

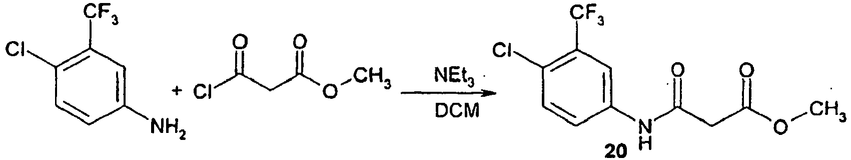

500 mg (2.557 mmol) 4-Chloro-3-(trifluoromethyl)aniline in 5 ml dichloromethane are cooled to 0 °C. Consecutively, 0.302 ml (2.813 mmol) malonic acid methylester chloride and 0.390 ml (2.813 mmol) triethyl amine are added slowly and the reaction mixture is stirred overnight at room temperature. After addition of brine, the reaction mixture is extracted three times with the dichloromethane. The combined organic phases are dried over Na₂SO₄, filtered and evaporated. The residue is put on silica gel and purified by column chromatography with n-heptane/ethyl acetate as eluent.
Yield: 622 mg (92%) **20,** pale yellow crystals

### 3-[(4-Chloro-3-(rifluoromethyl)phenyl)-2-oxo-propionic acid

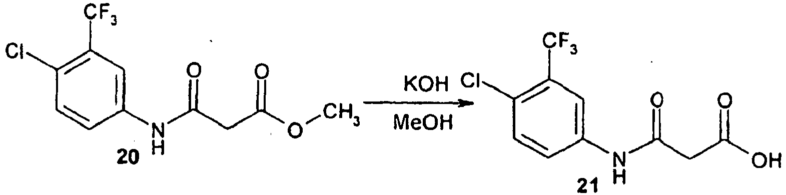

A solution of 645 mg (2.18 mmol) 20 in methanol is treated with 147 mg (2.62 mmol) KOH and the mixture is stirred overnight at room temperature. The reaction mixture is evaporated, the residue taken up in water and extracted three times with ethyl acetate. The combined organic phases are dried over Na₂SO₄, filtered and evaporated. The obtained residue is purified by column chromatography (10 g silica gel, eluent: ethyl acetate/MeOH).
Yield: 215 mg (90%) of **21,** yellow oil.

### N-[4-Chloro-3-(trifluoromethyl)phenyl]-N'-[4-(pyridine-4-yloxy)phenyl]-malonamide

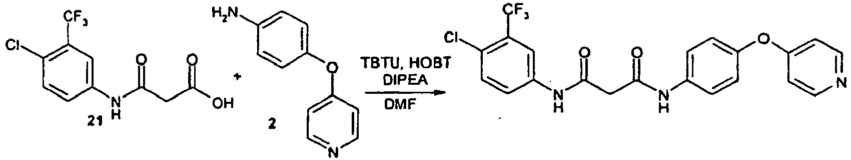

30 mg (0.107 mmol) of 21, 18.1 mg (0.097 mmol) of 2, 41 mg of TBTU (0.127 mmol) and 4.5 mg (0.029 mmol) of HOBT are dissolved in 3 ml dimethylformamide, 0.07 ml (0.39 mmol) of N-ethyldiisopropylamine is added at room temperature, and the mixture is stirred overnight. The reaction mixture is diluted with water and extracted several times with ethyl acetate. The combined organic phases are washed with water, dried over Na₂SO₄, filtered and evaporated. The residue is put on silica gel and purified by column chromatography (4 g silica gel, eluent: ethyl acetate/n-heptane).

Yield: 25 mg (57%), colourless solid.

### N-[4-Chloro-3-(trifluoromethyl)phenyl]-N'-[3-(pyridine-4-yloxy)phenyl]-malonamide

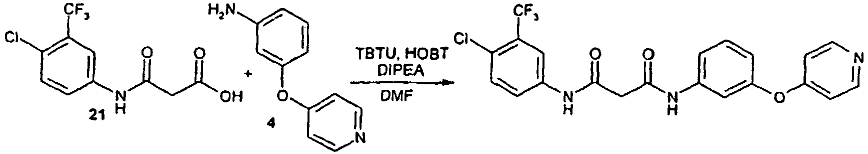

30 mg (0.107 mmol) of 21, 18.1 mg (0.097 mmol) of 4, 41 mg of TBTU (0.127 mmol) and 4.5 mg (0.029 mmol) of HOBT are dissolved in 3 ml dimethylformamide, 0.07 ml (0.39 mmol) of N-ethyldiisopropylamine is added at room temperature, and the mixture is stirred overnight. The reaction mixture is diluted with water and extracted several times with ethyl acetate. The combined organic phases are washed with water, dried over Na₂SO₄, filtered and evaporated. The residue is put on silica gel and purified by column chromatography (4 g silica gel, eluent: ethyl acetate/n-heptane).
Yield: 25 mg (57%), colourless solid.

### N-[4-Chloro-3-(trifluoromethyl)phenyl]-N'-[4-(pyridine-3-yloxy)phenyl]malonamide

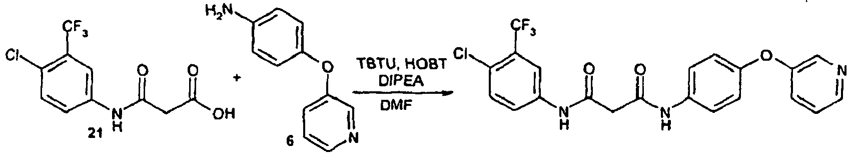

30 mg (0.107 mmol) of 21, 18.1 mg (0.097 mmol) of 6, 41 mg of TBTU (0.127 mmol) and 4.5 mg (0.029 mmol) of HOBT are dissolved in 3 ml dimethylformamide, 0.07 ml (0.39 mmol) of N-ethyldiisopropylamine is added at room temperature, and the mixture is stirred overnight. The reaction mixture is diluted with water and extracted several times with ethyl acetate. The combined organic phases are washed with water, dried over Na₂SO₄, filtered and evaporated. The residue is put on silica gel and purified by column chromatography (4 g silica gel, eluent: ethyl acetate/n-heptane).
Yield: 24 mg (55%), colourless solid.

### N-[4-Chloro-3-(trifluoromethyl)phenyl]-N'-[3-(pyridine-3-yloxy)phenyl]malonamide

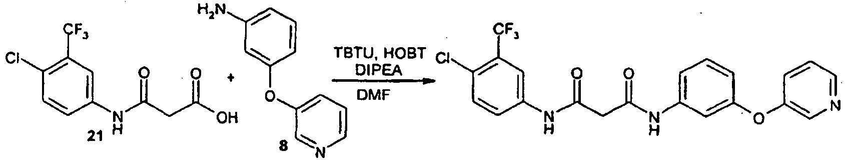

30 mg (0.107 mmol) of **21,** 18.1 mg (0.097 mmol) of **8,** 41 mg of TBTU (0.127 mmol) and 4.5 mg (0.029 mmol) of HOBT are dissolved in 3 ml dimethylformamide, 0.07 ml (0.39 mmol) of N-ethyldiisopropylamine is added at room temperature, and the mixture is stirred overnight. The reaction mixture is diluted with water and extracted several times with ethyl acetate. The combined organic phases are washed with water, dried over Na₂SO₄, filtered and evaporated. The residue is put on silica gel and purified by column chromatography (4 g silica gel, eluent: ethyl acetate/n-heptane).
Yield: 10 mg (23%), colourless solid.

### N-[4-Chloro-3-(trifluoromethyl)phenyl]-N'-[4-(pyridine-4-ylmethyl)phenyl]malonamide

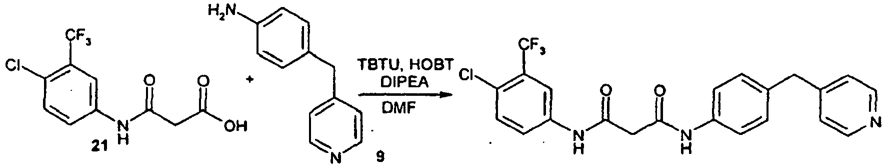

30 mg (0.107 mmol) of **21,** 17.9 mg (0.097 mmol) of **9, 41** mg of TBTU (0.127 mmol) and **4.5** mg (0.029 mmol) of HOBT are dissolved in 3 ml dimethylformamide, 0.07 ml (0.39 mmol) of N-ethyldiisopropylamine is added at room temperature, and the mixture is stirred overnight. The reaction mixture is diluted with water and extracted several times with ethyl acetate. The combined organic phases are washed with water, dried over Na₂SO₄, filtered and evaporated. The residue is put on silica gel and purified by column chromatography (4 g silica gel, eluent: ethyl acetate/n-heptane).
Yield: 10 mg (23%), colourless solid.

### N-[4-Chloro-3-(trifluoromethyl)phenyl]-N'-[4-(2-methylcarbamoyl-pyridine-4-yloxy)phenyl]malonamide

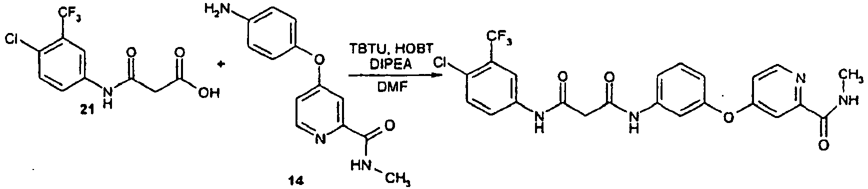

30 mg (0.107 mmol) **21,** 23.7 mg (0.097 mmol) **14,** 41 mg TBTU (0.13 mmol) and **4.5** mg (0.029 mmol) HOBT are dissolved in 3 ml dimethylformamide, 0.07 ml (0.39 mmol) of N-ethyldiisopropylamine is added at room temperature, and the mixture is stirred overnight. The reaction mixture is diluted with water and extracted several times with ethyl acetate. The combined organic phases are washed with water, dried over Na₂SO₄, filtered and evaporated. The residue is put on silica gel and purified by column chromatography (4 g silica gel, eluent: ethyl acetate/n-heptane).
Yield: 23 mg (45%), colourless solid.

### N-[4-Chloro-3-(trifluoromethyl)phenyl]-N'-[4-(2-methylcarbamoylpyridine-3-yloxy)phenyl]malonamide

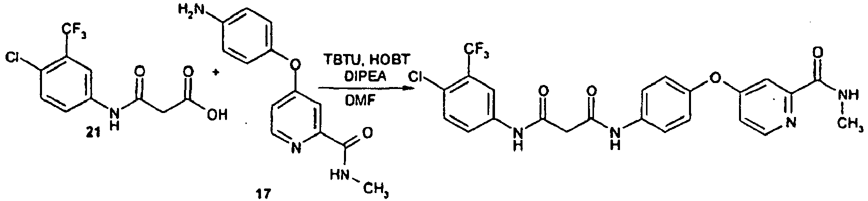

30 mg (0.107 mmol) **21,** 23.7 mg (0.097 mmol) **17**, 41 mg TBTU (0.13 mmol) and 4.5 mg (0.029 mmol) HOBT are dissolved in dimethylformamide, 0.07 ml (0.41 mmol) of N-ethyldiisopropylamine is added at room temperature, and the mixture is stirred overnight. The reaction mixture is diluted with water and extracted several times with ethyl acetate. The combined organic phases are washed with water, dried over Na₂SO₄, filtered and evaporated. The residue is put on silica gel and purified by column chromatography (4 g silica gel, eluent: ethyl acetate/n-heptane).
Yield: 26 mg (48%), colourless solid

### N-[4-Chloro-3-(trifluoromethyl)phenyl]-N'-[3-(2-methylcarbamoyl-pyridine-4-yloxy)-phenyl]-malonamide

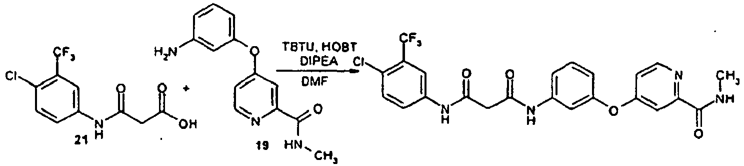

30 mg (0.107 mmol) **21,** 23.7 mg (0.097 mmol) **19, 41** mg TBTU (0.13 mmol) and 4.5 mg (0.029 mmol) HOBT are dissolved in dimethylformamide, 0.07 ml (0.41 mmol) of N-ethyldiisopropylamine is added at room temperature, and the mixture is stirred overnight. The reaction mixture is diluted with water and extracted several times with ethyl acetate. The combined organic phases are washed with water, dried over Na₂SO₄, filtered and evaporated. The residue is put on silica gel and purified by column chromatography (4 g silica gel, eluent: ethyl acetate/n-heptane).
Yield: 22 mg (43%), colourless solid

### Synthesis of the malonamide methylester

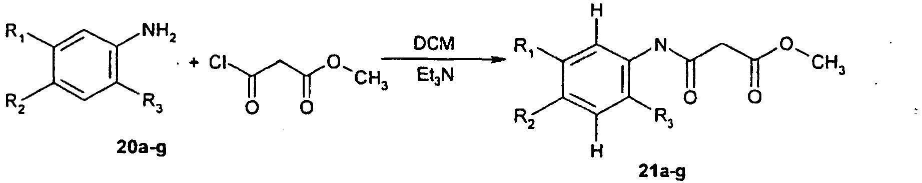

| | **R₁** | **R₂** | **R₃** |
|---|---|---|---|
| **20a** | **Cl** | **CH₃** | **H** |
| **20b** | **Cl** | **Cl** | **H** |
| **20c** | **CF₃** | **H** | **H** |
| **20d** | **CH₃** | **CH₃** | **H** |
| **20e** | **H** | **CF₃** | **H** |
| **20f** | **Cl** | **Cl** | **Cl** |
| **20g** | **OCF₃** | **H** | **H** |

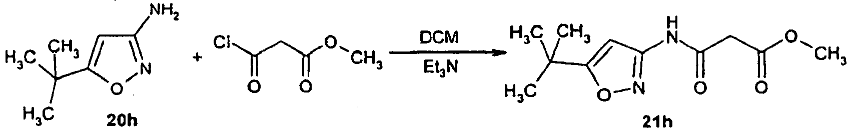

1.023 mmol amine 20a-h in 5 ml dichlormethane are cooled to 0 °C. Consecutively, 0.121 ml (1.125 mmol) malonic acid methylester chloride and 0.156 ml (1.125 mmol) triethyl amine are added slowly and the reaction mixture is stirred at room temperature overnight. The reaction mixture is treated with brine and extracted with dichlormethane (3x). The combined organic phases are dried over Na₂SO₄, filtered and evaporated. The residue is put on silica gel and purified by column chromatography (35g silica gel, Eluent: ethyl acetate/n-heptane).
Yield: 261 mg (98 %) **21a,** pale beige crystals; 282 mg (96 %) **21 b,** yellow crystals; 284 mg (97 %) **21c,** yellow crystals; 226 mg (90 %) **21d,** yellow crystals; 269 mg (71 %) **21e,** yellow crystals; 307 mg (88 %) **21f,** yellow crystals; 293 mg (100 %) **21g,** yellow crystals; 253 mg (96 %) **21 h,** yellow crystals.

### Synthesis of the malonic acid monoamides

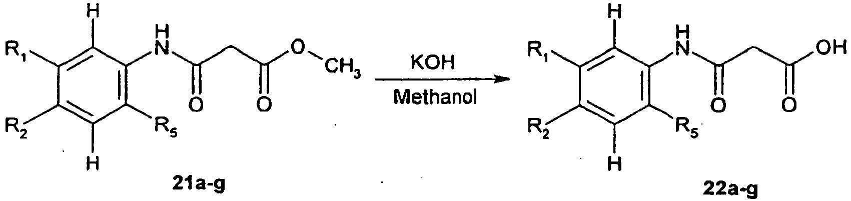

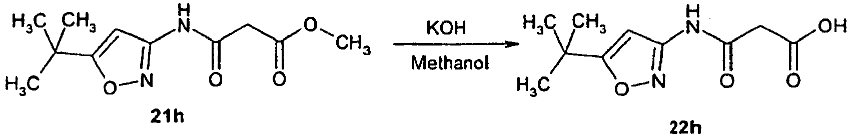

21a-h are dissolved in methanol, given into a closed PTFA-container together with 1.2 eq. KOH each and heated to 60 °C for 30 min in a Microwave field (Mars5, CEM). The reaction mixture is evaporated, the residue taken up in water and extracted with ethyl acetate (3x). The combined organic phases are dried over Na₂SO₄, filtered and evaporated. The residue of compound **22e** is purified by column chromatography (10 g silica gel, Eluent: ethyl acetate/MeOH).
Yield: 214 mg (81 %) **22a,** beige solid; 238 mg (87 %) **22b,** brown solid; 234 mg (85 %) **22c,** brown solid; 183 mg (80 %) **22d,** beige solid; 90 mg (50 %) **22e,** yellow oil; 193 mg (75 %) **22f,** colourless solid; 249 mg (85 %) **22g,** beige solid; 204 mg (81 %) **22h,** brown solid.

### Synthesis of the malonic acid diamides

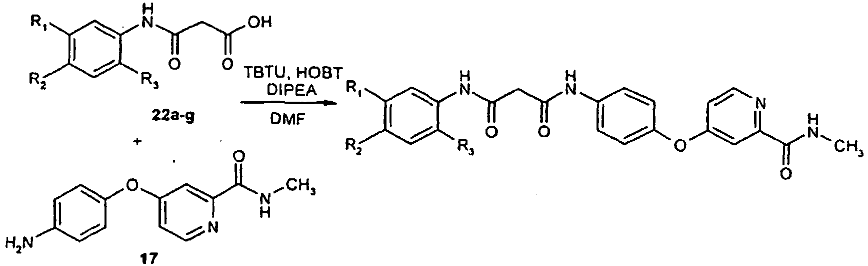

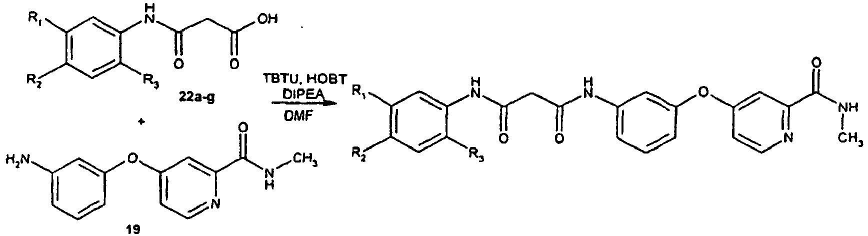

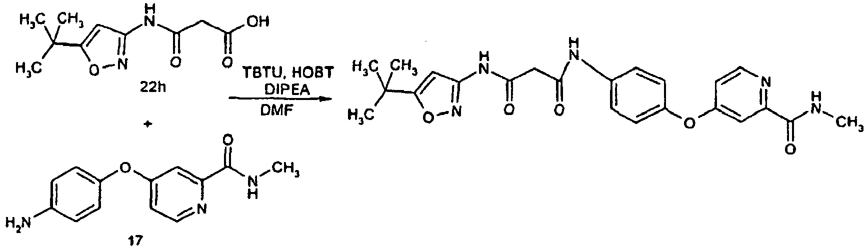

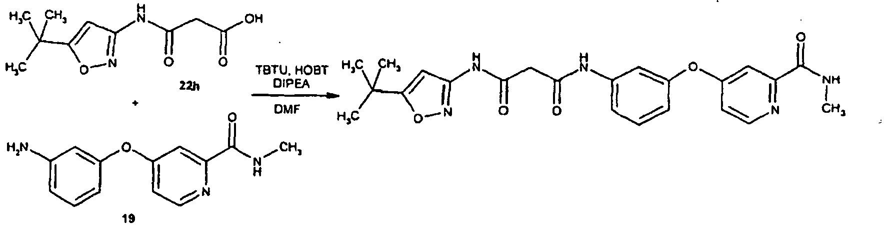

0.180 mmol 21 a-h, 0.164 mmol **17** and **19,** respectively, 68.5 mg TBTU (0.213 mmol) and 7.53 mg (0.049 mmol) HOBT are dissolved in 5 ml DMF, treated with 0.112 ml (0.656 mmol) N-ethyl-diisopropyl amine at room temperature and stirred overnight. The reaction mixture is diluted with water and extracted several times with ethyl acetate. The combined organic phases are washed with water, dried over Na₂SO₄, filtered and evaporated. The residue is put on silica gel and purified by column chromatography (4 g silica gel, Eluent: ethyl acetate/n-heptane).

**Table 2: Analytical data**

| **Structure*** | **MW** | **Rt^{a} (min)** |
|---|---|---|
| 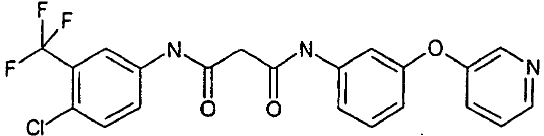 | 449.82 | 4,53 |
| 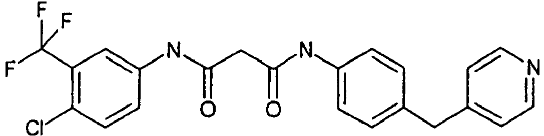 | 447.85 | 4,37 |
| 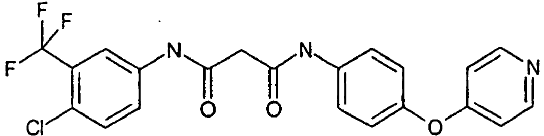 | 449.82 | 4,34 |
| 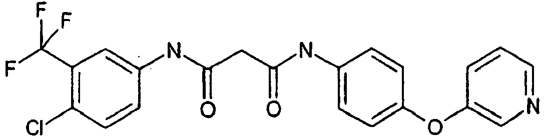 | 449.82 | 4,35 |
| 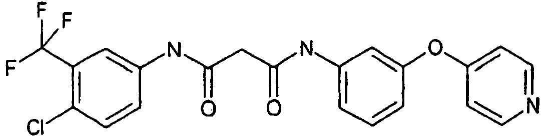 | 449.82 | 4,48 |
| 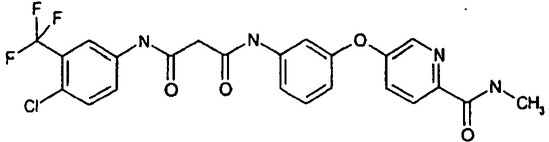 | 506.87 | 5,21 |
| 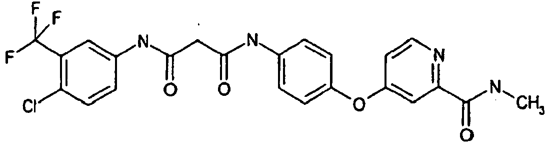 | 506.87 | 4,87 |
| 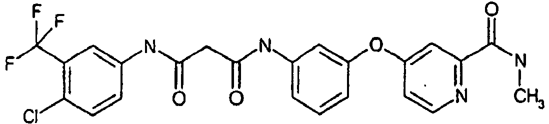 | 506.87 | 4,93 |
| 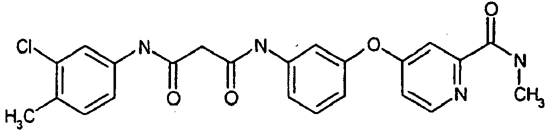 | 452.9 | 4,83 |
| 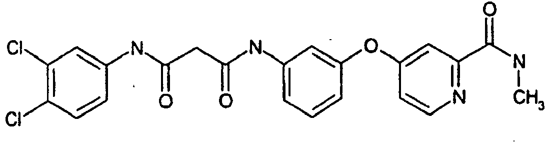 | 473.32 | 4,87 |
| 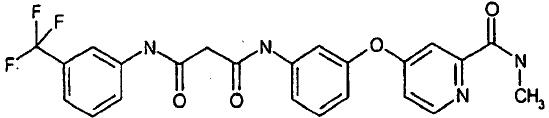 | 472.43 | 4,86 |
| 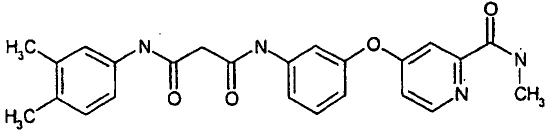 | 432.48 | 4,63 |
| 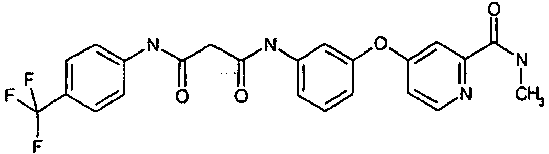 | 472.43 | 4,84 |
| 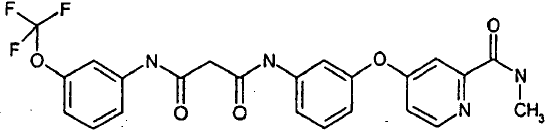 | 488.43 | 4,89 |
| 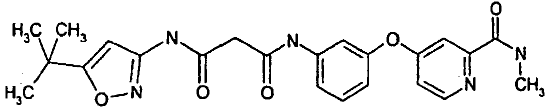 | 451.49 | 4,51 |
| 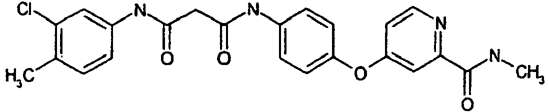 | 452.90 | 4,69 |
| 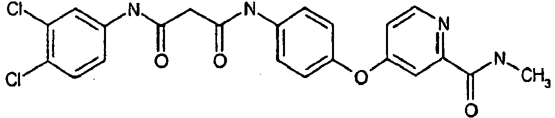 | 473.32 | 4,81 |
| 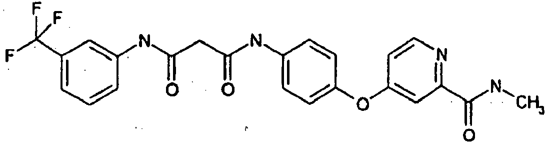 | 472.43 | 4,70 |
| 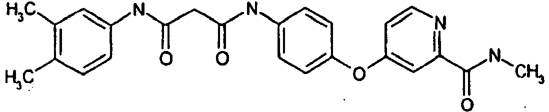 | 432.48 | 4,48 |
| 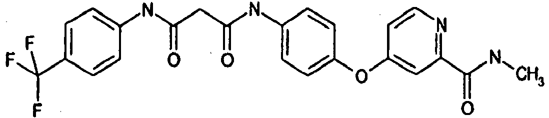 | 472.43 | 4,73 |
| 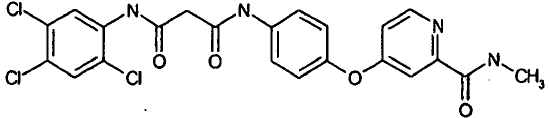 | 507.76 | 5,31 |
| 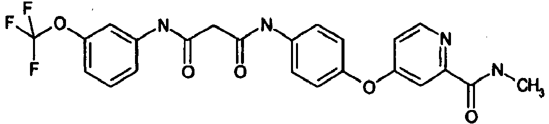 | 488.43 | 4,83 |
| 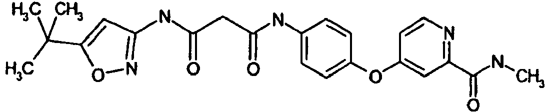 | 451.49 | 4,43 |

| | | |
|---|---|---|
| *: hydrogen atoms of the secondary amino groups not shown ^{a}HPLC method: Gradient: 9 min; flow rate: 1.5 ml/min from 80:20 to 0:100 H₂O/ACN Water + TFA (0.01 % by vol.); acetonitrile + TFA (0.01% by vol.) Column: Lichrospher RP-select-B (5 µm/125 mm) Wavelength: 220 nm; Rt=Retention time. | | |

The compounds (1) to (228) as described above can preferably be produced according to the procedures described herein or in an analogous manner thereof.

### Example A: Injection vials

A solution of 100 g of an active compound of the formula II and 5 g of disodium hydrogenphosphate is adjusted to pH 6.5 in 3 I of double-distilled water using 2N hydrochloric acid, sterile-filtered, dispensed into injection vials, lyophilized under sterile conditions and aseptically sealed. Each injection vial contains 5 mg of active compound.

### Example B: Suppositories

A mixture of 20 g of an active compound of the formula II is fused with 100 g of soya lecithin and 1400 g of cocoa butter, poured into moulds and allowed to cool. Each suppository contains 20 mg of active compound.

### Example C: Solution

A solution of 1 g of an active compound of the formula II 9.38 g of NaH₂PO₄ · 2 H₂O, 28.48 g of Na₂HPO₄ · 12 H₂O and 0.1 g of benzalkonium chloride in 940 ml of double-distilled water is prepared. It is adjusted to pH 6.8, made up to 1 I and sterilized by irradiation. This solution can be used in the form of eye drops.

### Example D: Ointment

500 mg of an active compound of the formula II is mixed with 99.5 g of petroleum jelly under aseptic conditions.

### Example E: Tablets

A mixture of 1 kg of active compound of the formula II, 4 kg of lactose, 1.2 kg of potato starch, 0.2 kg of talc and 0.1 kg of magnesium stearate is compressed to give tablets in a customary manner such that each tablet contains 10 mg of active compound.

### Example F: Coated tablets

Analogously to Example E, tablets are pressed and are then coated in a customary manner using a coating of sucrose, potato starch, talc, tragacanth and colourant.

### Example G: Capsules

2 kg of active compound of the formula II are dispensed into hard gelatin capsules in a customary manner such that each capsule contains 20 mg of the active compound.

### Example H: Ampoules

A solution of 1 kg of active compound of the formula II in 60 I of double-distilled water is sterile-filtered, dispensed into ampoules, lyophilized under sterile conditions and aseptically sealed. Each ampoule contains 10 mg of active compound.

## Claims

1. **Malonamide derivatives of formula II,** 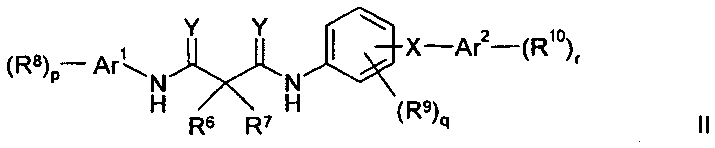 wherein
Ar¹ is phenyl, pyridinyl, oxazolyl, isoxazolyl, pyrazolyl or imidazolyl,
Ar² is pyridinyl,
R¹⁰ is selected from the group consisting of alkyl comprising 1 to 4 carbon atoms, alkoxy comprising 1 to 4 carbon atoms, Hal, CH₂Hal, CH(Hal)₂, perhaloalkyl comprising 1 to 4 carbon atoms, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² and (CH₂)ₙS(O)ᵤR¹³,
k is 0, 1 or 2,
r is 0, 1 or 2;
R⁶, R⁷ are independently selected from the meanings given for R⁸, R⁹,
or R⁶ and R⁷ together form a carbocyclic residue comprising 3 to 7 carbon atoms or a heterocyclic residue comprising 1, 2 or 3 hetero atoms, selected from the group consisting of O, N and S, and 2 to 6 carbon atoms, said carbocyclic or heterocyclic residue being unsubstituted or comprising 1, 2 or 3 substituents, selected from the meanings given for R⁸, R⁹ and R¹⁰,
R⁸, R⁹ are independently selected from a group consisting of H, A, cycloalkyl comprising 3 to 7 carbon atoms, Hal, CH₂Hal, CH(Hal)₂, C(Hal)₃, NO₂, (CH₂)ₙCN, (CH₂)ₙNR¹¹R¹², (CH₂)ₙOR¹¹, (CH₂)ₙO(CH₂)ₖNR¹¹R¹², (CH₂)ₙCOOR¹², (CH₂)ₙCONR¹¹R¹², (CH₂)ₙNR¹¹COR¹³, (CH₂)ₙNR¹¹CONR¹¹R¹², (CH₂)ₙNR¹¹SO₂A, (CH₂)ₙSO₂NR¹¹R¹², (CH₂)ₙS(O)ᵤR¹³, (CH₂)ₙOC(O)R¹³, (CH₂)ₙCOR¹³, (CH₂)ₙSR¹¹, CH=N-OA, CH₂CH=N-OA, (CH₂)ₙNHOA, (CH₂)ₙCH=N-R¹¹, (CH₂)ₙOC(O)NR¹¹R¹², (CH₂)ₙNR¹¹COOR¹², (CH₂)ₙN(R¹¹)CH₂CH₂OR¹³, (CH₂)N(R¹¹)CH₂CH₂OCF₃, (CH₂)ₙN(R¹¹)C(R¹³)HCOOR¹², C(R¹³)HCOR¹², (CH₂)ₙN(R¹¹)CH₂CH₂N(R¹²)CH₂COOR¹², (CH₂)ₙN(R¹¹)CH₂CH₂NR¹¹R¹², CH=CHCOOR¹¹, CH=CHCH₂NR¹¹R¹², CH=CHCH₂NR¹¹R¹², CH=CHCH₂OR¹³, (CH₂)ₙN(COOR¹¹)COOR¹², (CH₂)ₙN(CONH₂)COOR¹¹, (CH₂)ₙN(CONH₂)CONH₂, (CH₂)ₙN(CH₂COOR¹¹)COOR¹², (CH₂)ₙN(CH₂CONH₂)COOR¹¹, (CH₂)ₙN(CH₂CONH₂)CONH₂, (CH₂)ₙCHR¹³COR¹¹, (CH₂)ₙCHR¹³COOR¹¹, (CH₂)ₙCHR¹³CH₂OR¹⁴, (CH₂)ₙOCN and (CH₂)ₙNCO, wherein
R¹¹, R¹² are independently selected from a group consisting of H, A and (CH₂)ₘAr³ or in NR¹¹R¹²,
R¹¹ and R¹² form, together with the N-Atom they are bound to, a 5-, 6- or 7-membered heterocyclus which optionally contains 1 or 2 additional hetero atoms, selected from N, O an S,
R¹³, R¹⁴ are independently selected from a group consisting of H, Hal, A, (CH₂)ₘAr⁴ and (CH₂)ₘHet,
A is selected from the group consisting of alkyl, alkenyl, cycloalkyl, alkylenecycloalkyl, alkoxy and alkoxyalkyl,
Ar³, Ar⁴ are independently from one another aromatic hydrocarbon residues comprising 5 to 12 and preferably 5 to 10 carbon atoms which are optionally substituted by one or more substituents, selected from a group consisting of A, Hal, NO₂, CN, OR¹⁵, NR¹⁵R¹⁶, COOR¹⁵, CONR¹⁵R¹⁶, NR¹⁵COR¹⁶, NR¹⁵CONR¹⁵R¹⁶, NR¹⁶SO₂A, COR¹⁵, SO_{Z}R'^{S}R'⁶, S(O)ᵤA and OOCR¹⁵,
Het is a saturated, unsaturated or aromatic heterocyclic residue which is optionally substituted by one ore more substituents, selected from a group consisting of A, Hal, NO₂, CN, OR¹⁵, NR¹⁵R¹⁶, COOR¹⁵, CONR¹⁵R¹⁶, NR¹⁵COR¹⁶, NR¹⁵CONR¹⁵R¹⁶, NR¹⁶SO₂A, COR¹⁵, SO₂R¹⁵R¹⁶, S(O)ᵤA and OOCR¹⁵,
R¹⁵, R¹⁶ are independently selected from a group consisting of H, A, and (CH₂)ₘAr⁶, wherein
Ar⁶ is a 5- or 6-membered aromatic hydrocarbon which is optionally substituted by one or more substituents selected from a group consisting of methyl, ethyl, propyl, 2-propyl, tert.-butyl, Hal, CN, OH, NH₂ and CF₃,
m and n are independently of one another 0, 1, 2, 3, 4, or 5,
X is selected from the group consisting of O, S, NR¹¹, CHOR¹¹, CH₂, CH₂CH₂, OCH₂, CH₂O, OCH₂CH₂, CH₂CH₂O
h, i are independently from each other 0, 1, 2, 3, 4, 5, or 6, and
j is 1, 2, 3, 4, 5, or 6,
Y is selected from O, S, NR²¹, C(R²²)-NO₂, C(R²²)-CN and C(CN)₂, wherein
R²¹ is independently selected from the meanings given for R¹³, R¹⁴ and
R²² is independently selected from the meanings given for R¹¹, R¹²,
p is 0, 1, 2, 3, 4 or 5,
q is 0, 1, 2, 3 or 4, preferably 0, 1 or 2,
u is 0, 1, 2 or 3, preferably 0, 1 or 2,
and
Hal is independently selected from a group consisting of F, Cl, Br and I;
and the pharmaceutically acceptable salts and solvates thereof.

2. Malonamide derivative according to claim 1, selected from the compounds of formula IIa, IIb, IIc, IId, IIe, IIf, IIg and IIh, 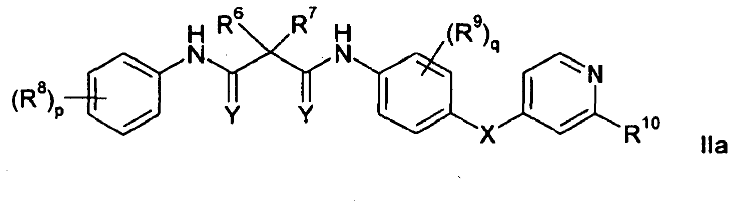 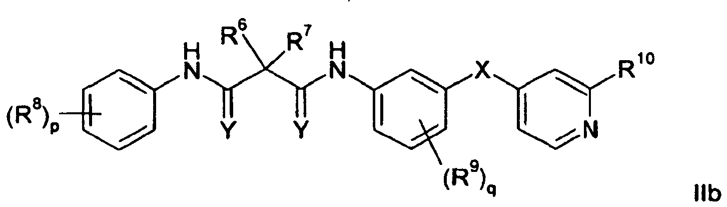 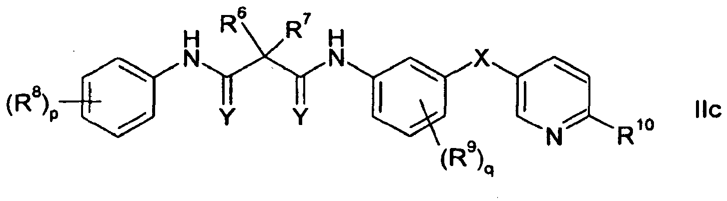 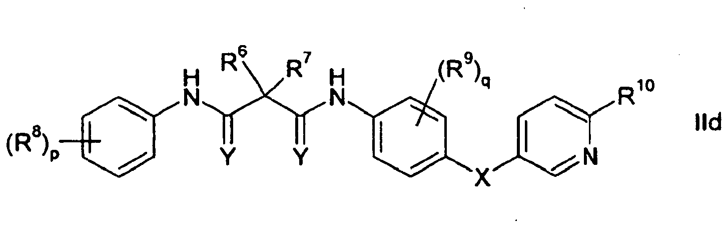 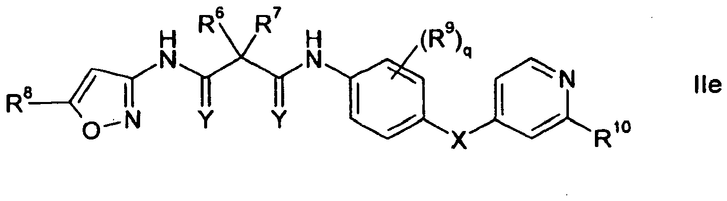 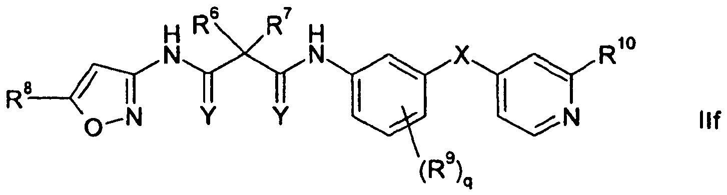 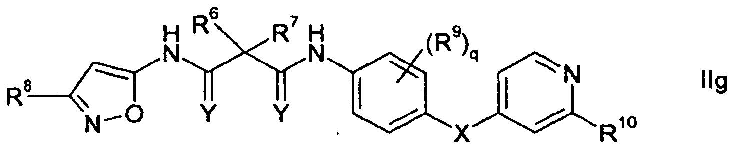 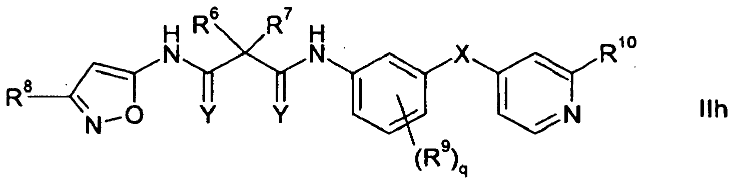 wherein R⁶, R⁷, R⁸, p, X, Y, R⁹, q are as defined in claim 1 and R¹⁰ is H or as defined in claim 1;
and the pharmaceutically acceptable salts and solvates thereof.

3. Malonamide derivative according to one of the claims 1 or 2, selected from the compounds (1) to (228) of table 1; compounds (1) to (228) of formula A-NH-CO-CH₂-CO-NH-B, wherein A and B are as given below:
| | A | B |
|---|---|---|
| (1) | 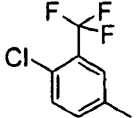 | 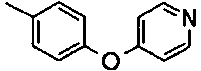 |
| (2) | 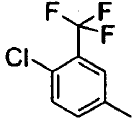 | 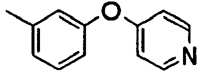 |
| (3) | 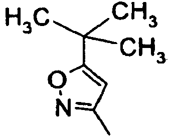 | 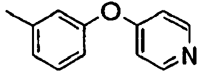 |
| (4) | 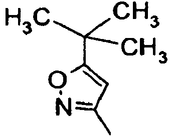 | 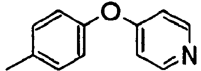 |
| (5) | 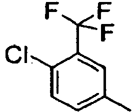 | 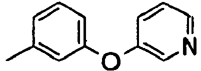 |
| (6) | 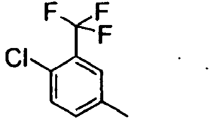 | 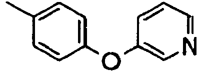 |
| (7) | 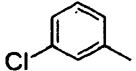 | 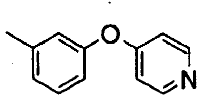 |
| (8) | 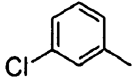 | 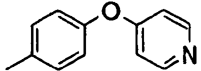 |
| (9) | 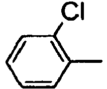 | 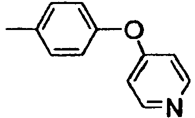 |
| (10) | 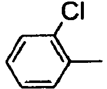 | 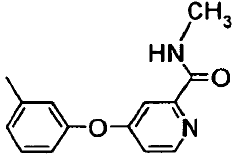 |
| (11) | 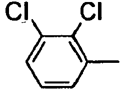 | 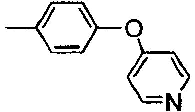 |
| (12) | 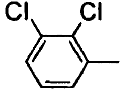 | 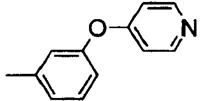 |
| (13) | 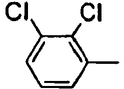 | 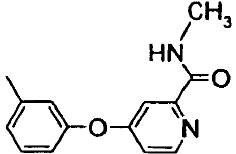 |
| (14) | 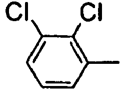 | 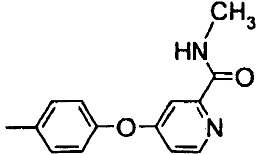 |
| (15) | 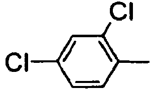 | 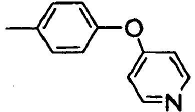 |
| (16) | 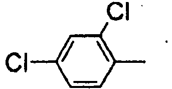 | 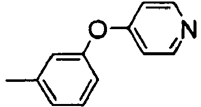 |
| (17) | 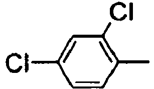 | 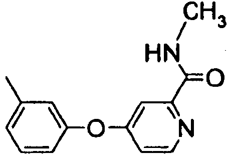 |
| (18) | 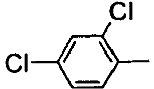 | 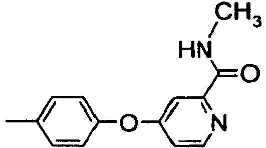 |
| (19) | 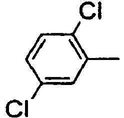 | 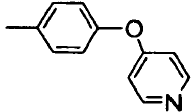 |
| (20) | 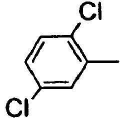 | 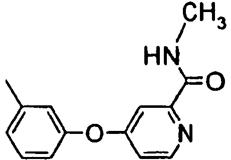 |
| (21) | 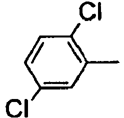 | 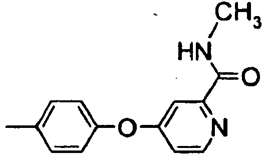 |
| (22) | 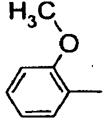 | 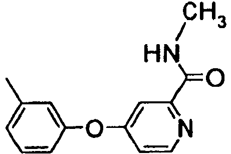 |
| (23) | 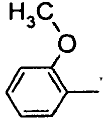 | 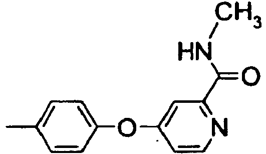 |
| (24) | 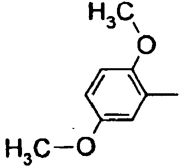 | 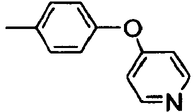 |
| (25) | 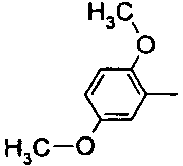 | 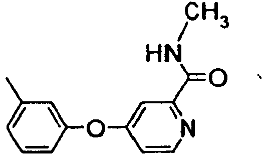 |
| (26) | 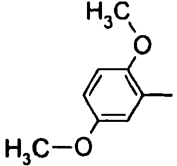 | 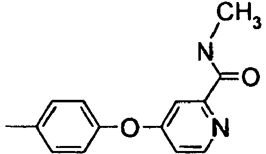 |
| (27) | 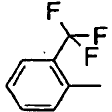 | 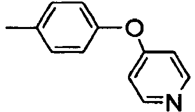 |
| (28) | 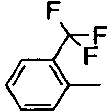 | 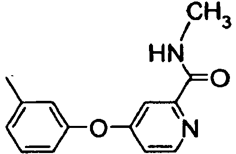 |
| (29) | 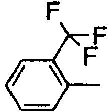 | 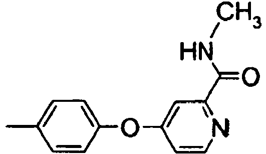 |
| (30) | 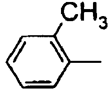 | 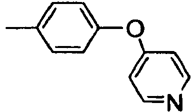 |
| (31) | 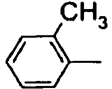 | 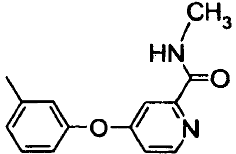 |
| (32) | 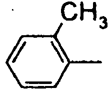 | 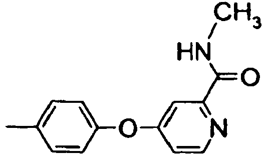 |
| (33) | 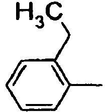 | 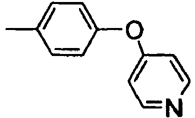 |
| (34) | 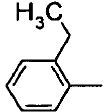 | 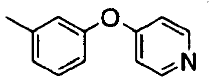 |
| (35) | 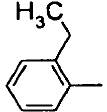 | 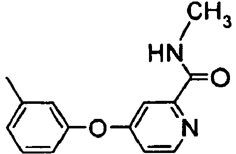 |
| (36) | 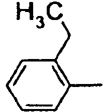 | 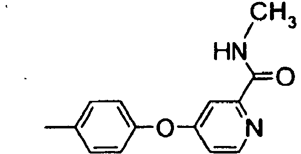 |
| (37) | 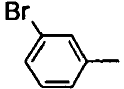 | 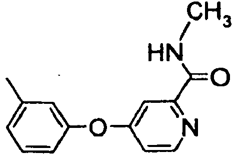 |
| (38) | 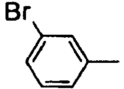 | 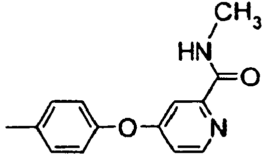 |
| (39) | 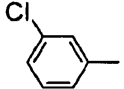 | 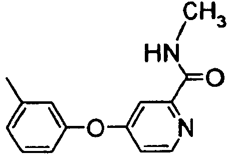 |
| (40) | 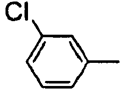 | 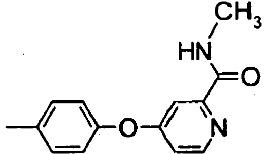 |
| (41) | 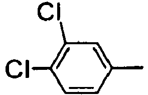 | 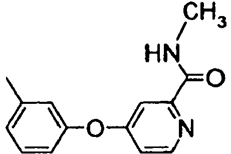 |
| (42) | 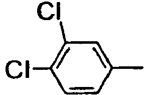 | 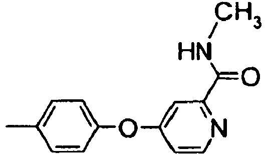 |
| (43) | 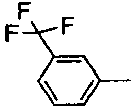 | 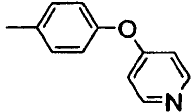 |
| (44) | 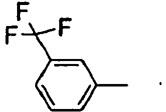 | 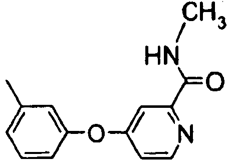 |
| (45) | 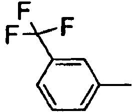 | 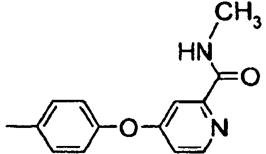 |
| (46) | 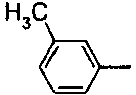 | 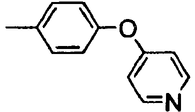 |
| (47) | 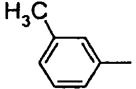 | 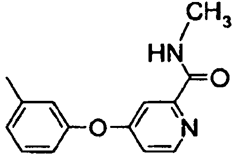 |
| (48) | 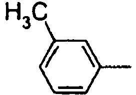 | 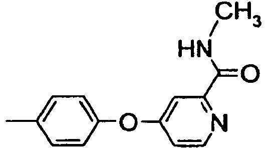 |
| (49) | 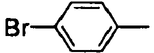 | 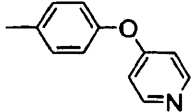 |
| (50) | 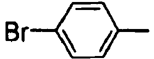 | 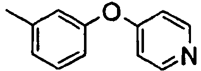 |
| (51) | 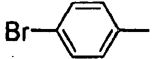 | 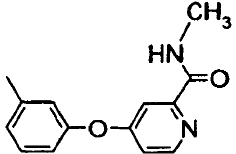 |
| (52) | 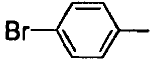 | 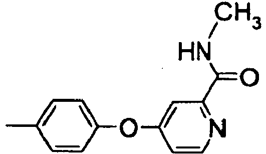 |
| (53) | 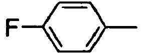 | 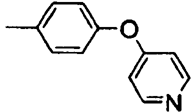 |
| (54) | 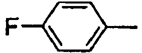 | 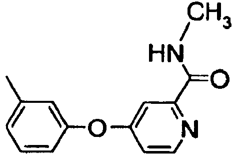 |
| (55) | 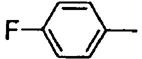 | 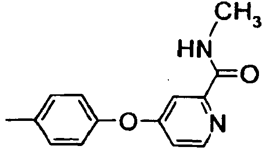 |
| (56) | 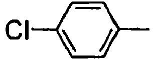 | 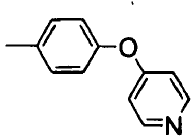 |
| (57) | 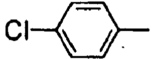 | 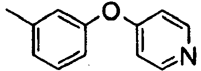 |
| (58) | 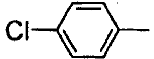 | 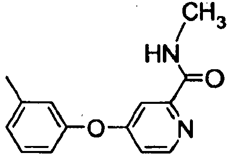 |
| (59) | 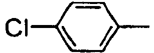 | 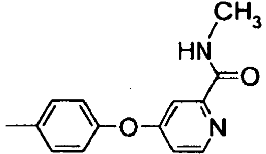 |
| (60) | 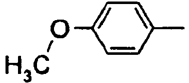 | 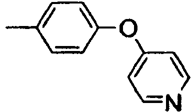 |
| (61) | 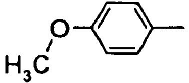 | 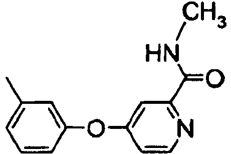 |
| (62) | 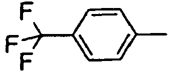 | 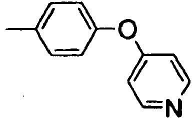 |
| (63) | 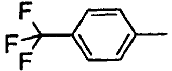 | 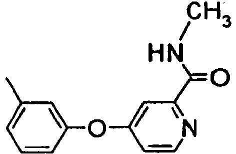 |
| (64) | 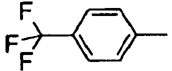 | 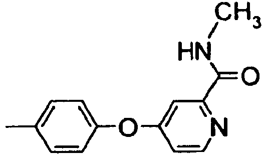 |
| (65) | 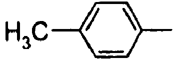 | 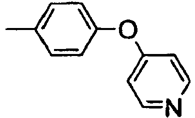 |
| (66) | 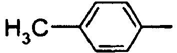 | 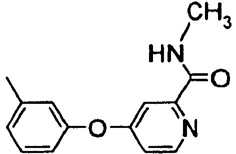 |
| (67) | 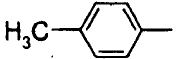 | 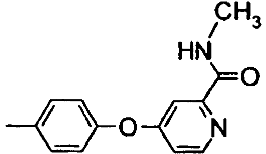 |
| (68) | 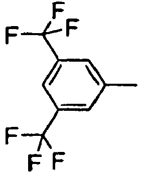 | 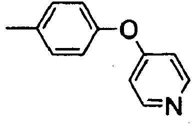 |
| (69) | 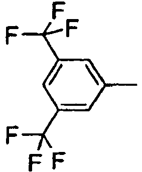 | 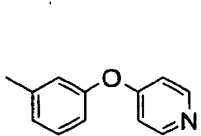 |
| (70) | 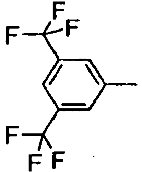 | 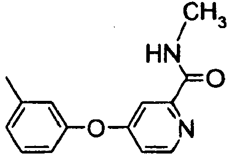 |
| (71) | 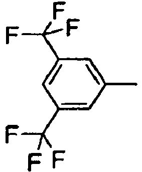 | 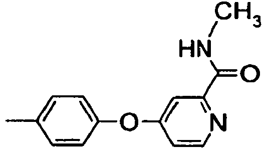 |
| (72) | 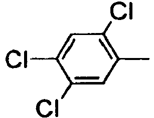 | 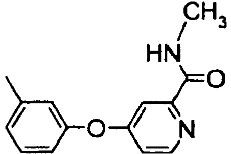 |
| (73) | 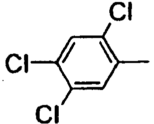 | 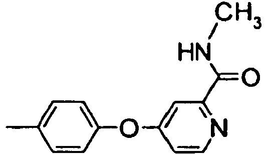 |
| (74) | 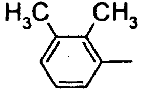 | 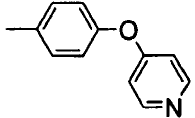 |
| (75) | 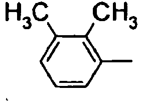 | 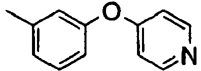 |
| (76) | 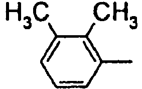 | 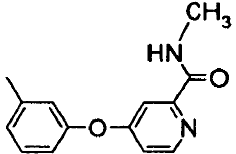 |
| (77) | 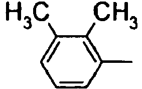 | 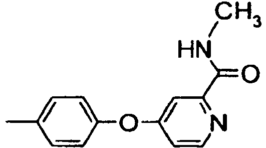 |
| (78) | 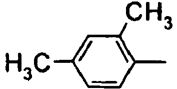 | 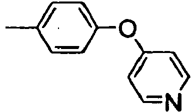 |
| (79) | 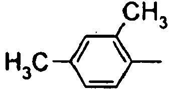 | 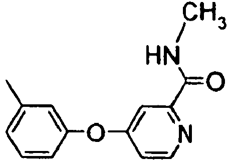 |
| (80) | 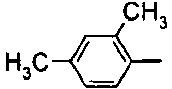 | 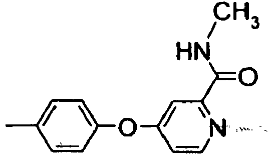 |
| (81) | 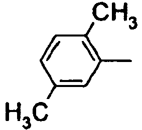 | 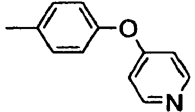 |
| (82) | 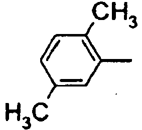 | 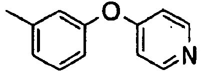 |
| (83) | 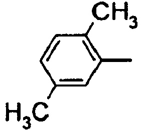 | 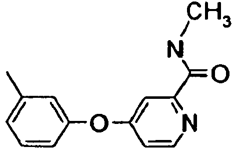 |
| (84) | 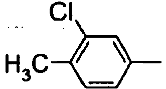 | 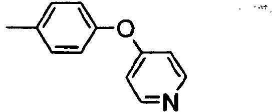 |
| (85) | 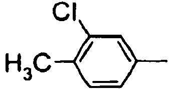 | 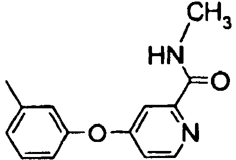 |
| (86) | 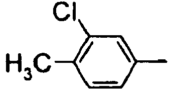 | 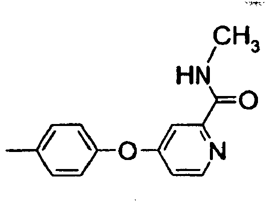 |
| (87) | 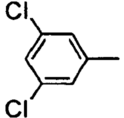 | 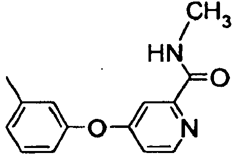 |
| (88) | 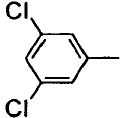 | 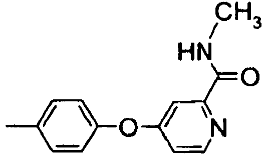 |
| (89) | 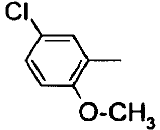 | 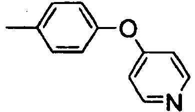 |
| (90) | 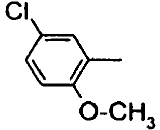 | 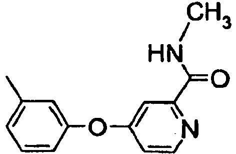 |
| (91) | 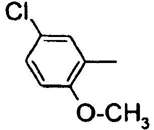 | 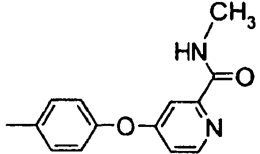 |
| (92) | 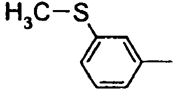 | 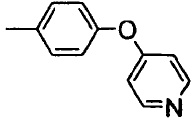 |
| (93) | 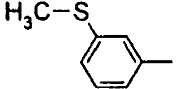 | 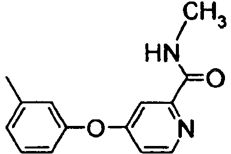 |
| (94) | 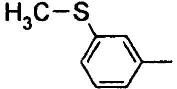 | 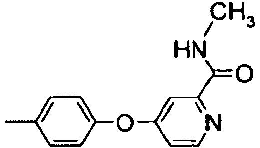 |
| (95) | 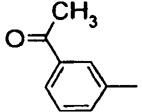 | 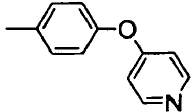 |
| (96) | 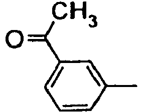 | 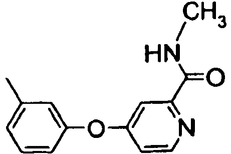 |
| (97) | 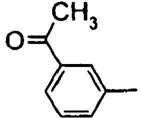 | 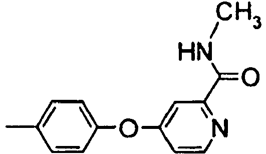 |
| (98) | 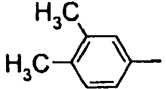 | 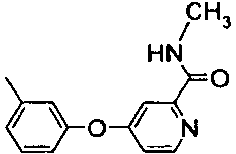 |
| (99) | 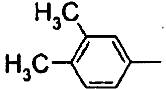 | 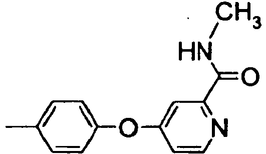 |
| (100) | 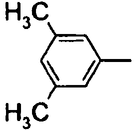 | 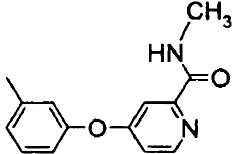 |
| (101) | 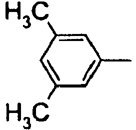 | 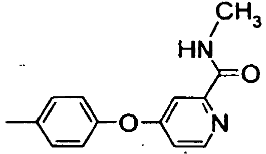 |
| (102) | 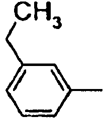 | 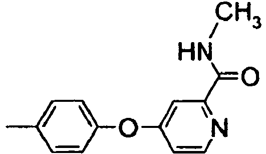 |
| (103) | 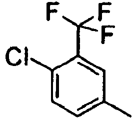 | 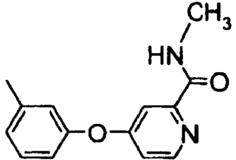 |
| (104) | 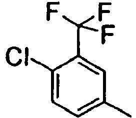 | 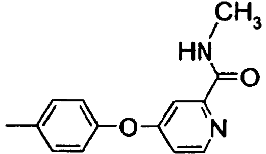 |
| (105) | 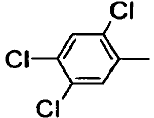 | 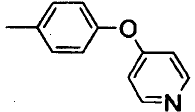 |
| (106) | 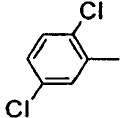 | 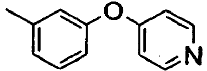 |
| (107) | 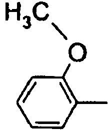 | 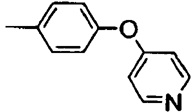 |
| (108) | 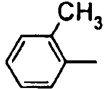 | 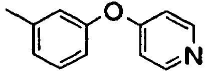 |
| (109) | 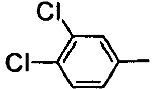 | 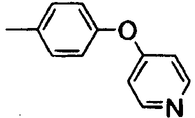 |
| (110) | 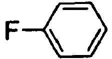 | 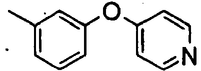 |
| (111) | 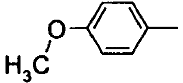 | 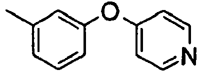 |
| (112) | 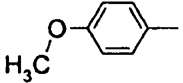 | 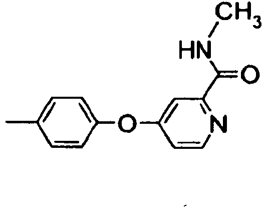 |
| (113) | 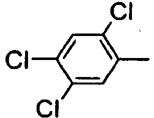 | 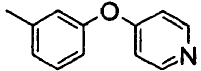 |
| (114) | 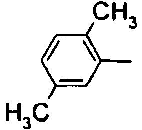 | 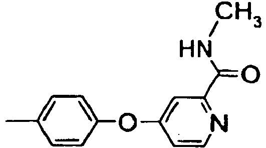 |
| (115) | 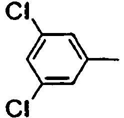 | 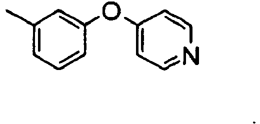 |
| (116) | 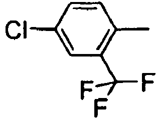 | 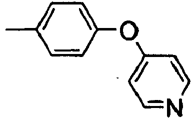 |
| (117) | 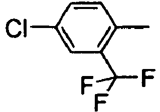 | 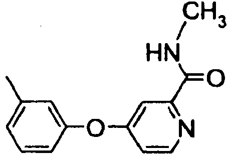 |
| (118) | 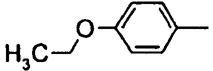 | 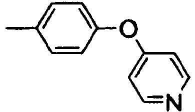 |
| (119) | 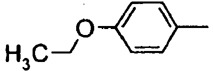 | 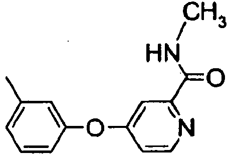 |
| (120) | 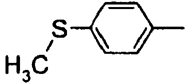 | 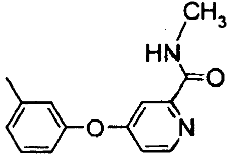 |
| (121) | 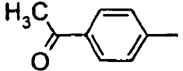 | 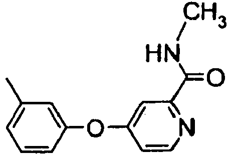 |
| (122) | 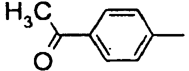 | 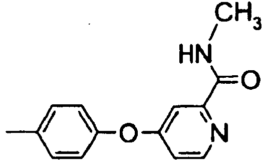 |
| (123) | 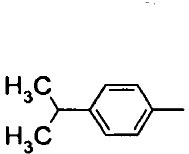 | 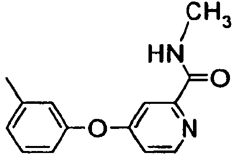 |
| (124) | 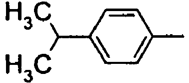 | 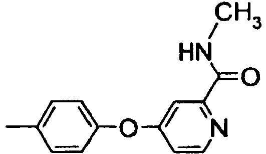 |
| (125) | 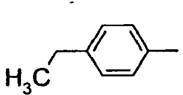 | 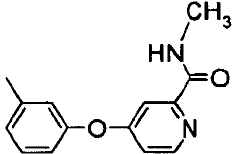 |
| (126) | 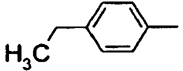 | 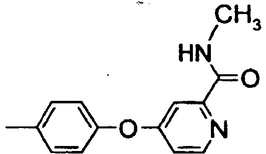 |
| (127) | 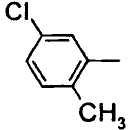 | 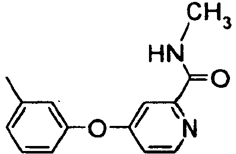 |
| (128) | 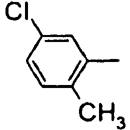 | 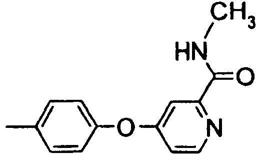 |
| (129) | 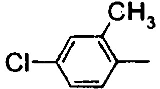 | 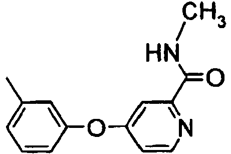 |
| (130) | 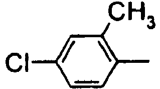 | 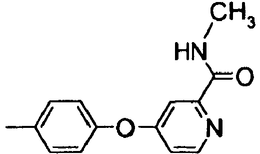 |
| (131) | 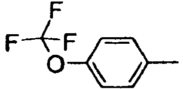 | 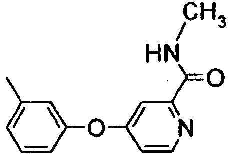 |
| (132) 2 | 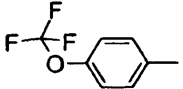 | 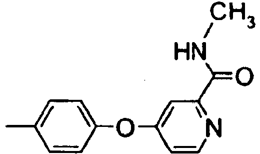 |
| (133) | 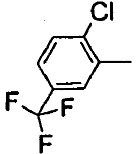 | 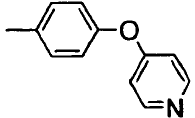 |
| (134) | 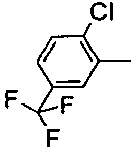 | 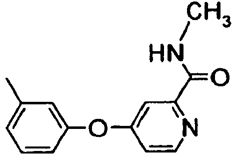 |
| (135) | 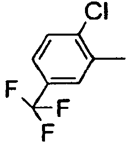 | 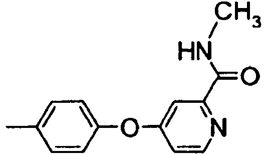 |
| (136) | 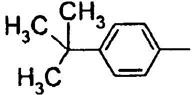 | 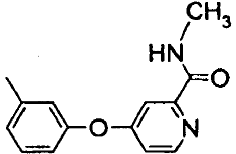 |
| (137) | 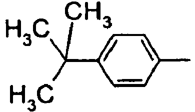 | 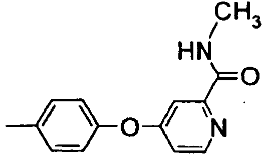 |
| (138) | 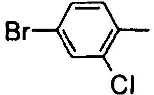 | 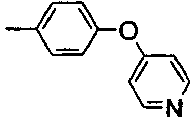 |
| (139) | 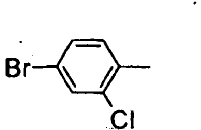 | 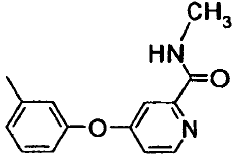 |
| (140) | 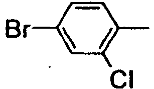 | 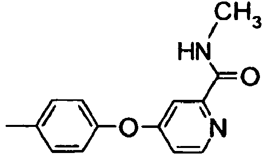 |
| (141) | 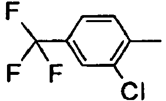 | 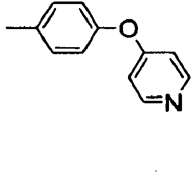 |
| (142) | 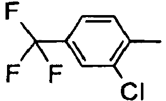 | 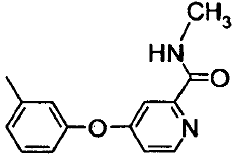 |
| (143) | 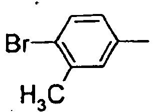 | 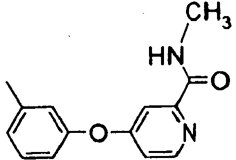 |
| (144) | 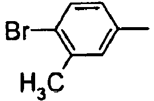 | 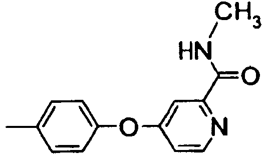 |
| (145) | 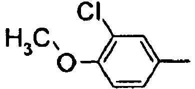 | 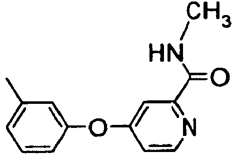 |
| (146) | 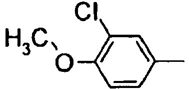 | 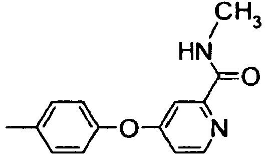 |
| (147) | 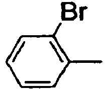 | 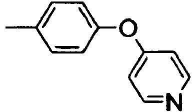 |
| (148) | 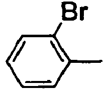 | 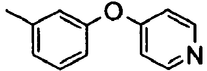 |
| (149) | 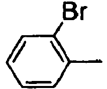 | 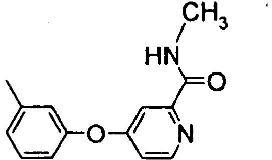 |
| (150) | 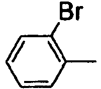 | 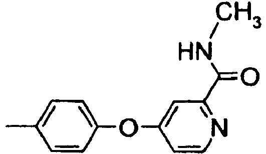 |
| (151) | 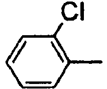 | 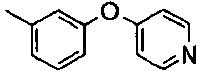 |
| (152) | 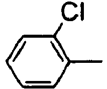 | 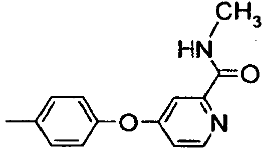 |
| (153) | 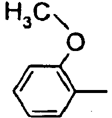 | 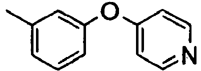 |
| (154) | 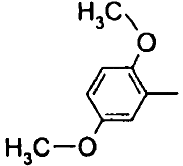 | 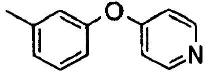 |
| (155) | 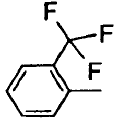 | 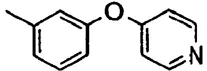 |
| (156) | 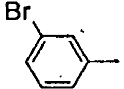 | 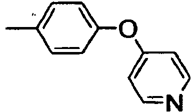 |
| (157) | 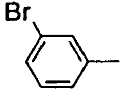 | 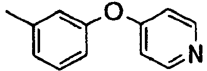 |
| (158) | 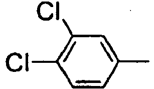 | 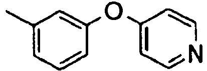 |
| (159) | 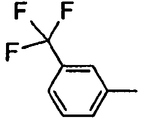 | 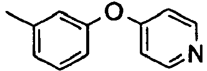 |
| (160) | 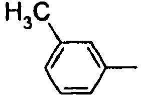 | 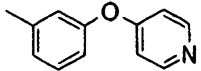 |
| (161) | 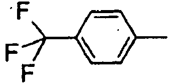 | 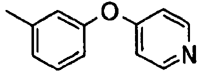 |
| (162) | 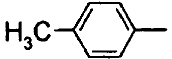 | 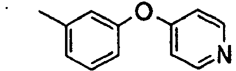 |
| (163) | 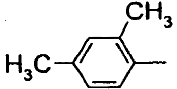 | 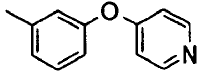 |
| (164) | 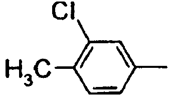 | 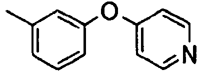 |
| (165) | 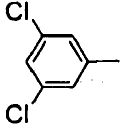 | 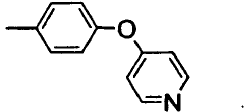 |
| (166) | 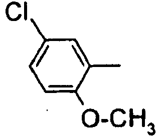 | 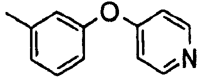 |
| (167) | 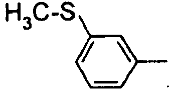 | 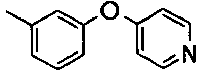 |
| (168) | 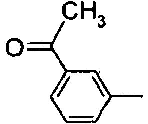 | 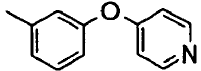 |
| (169) | 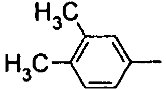 | 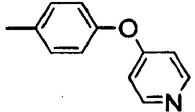 |
| (170) | 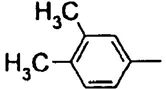 | 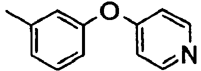 |
| (171) | 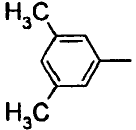 | 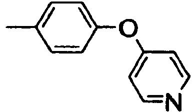 |
| (172) | 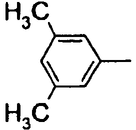 | 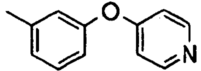 |
| (173) | 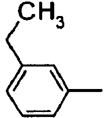 | 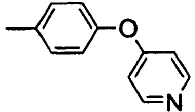 |
| (174) | 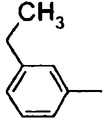 | 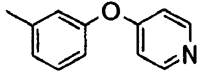 |
| (175) | 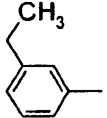 | 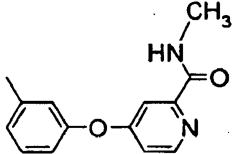 |
| (176) | 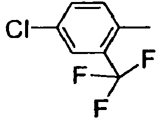 | 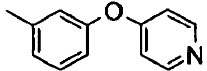 |
| (177) | 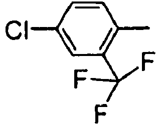 | 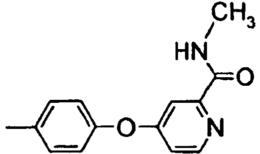 |
| (178) | 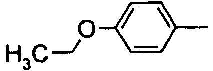 | 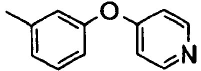 |
| (179) | 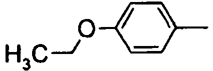 | 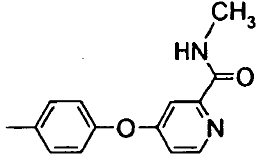 |
| (180) | 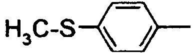 | 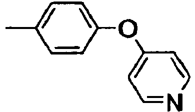 |
| (181) | 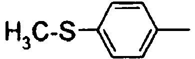 | 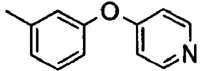 |
| (182) | 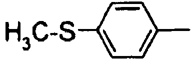 | 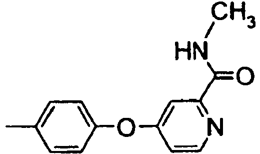 |
| (183) | 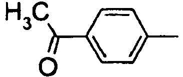 | 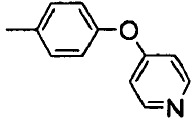 |
| (184) | 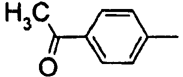 | 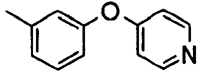 |
| (185) | 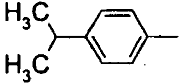 | 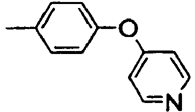 |
| (186) | 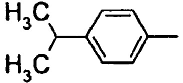 | 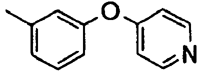 |
| (187) | 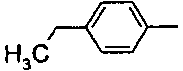 | 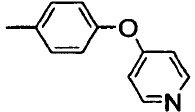 |
| (188) | 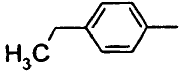 | 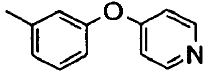 |
| (189) | 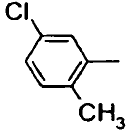 | 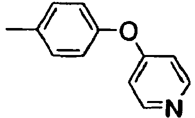 |
| (190) | 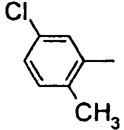 | 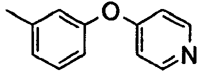 |
| (191) | 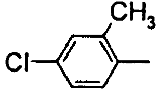 | 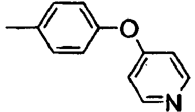 |
| (192) | 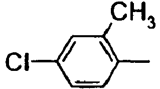 | 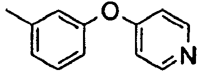 |
| (193) | 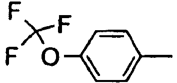 | 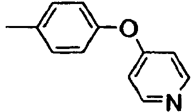 |
| (194) | 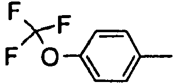 | 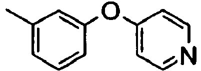 |
| (195) | 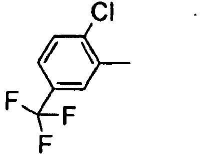 | 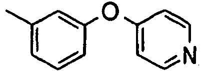 |
| (196) | 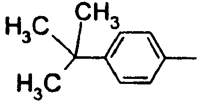 | 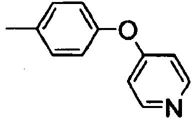 |
| (197) | 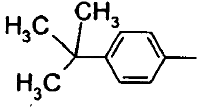 | 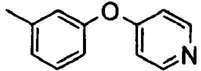 |
| (198) | 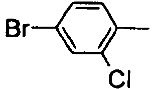 | 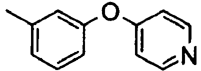 |
| (199) | 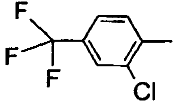 | 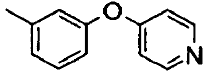 |
| (200) | 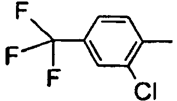 | 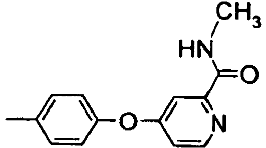 |
| (201) | 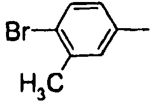 | 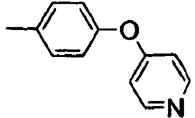 |
| (202) | 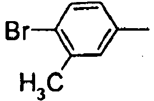 | 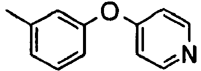 |
| (203) | 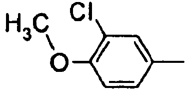 | 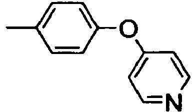 |
| (204) | 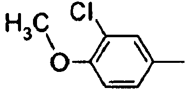 | 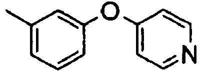 |
| (205) | 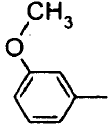 | 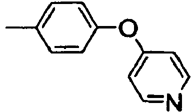 |
| (206) | 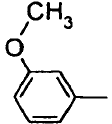 | 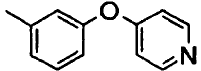 |
| (207) | 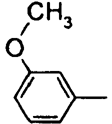 | 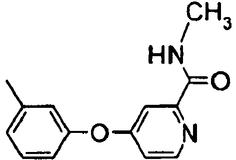 |
| (208) | 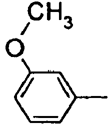 | 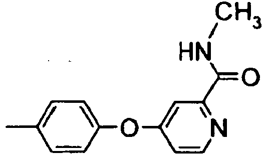 |
| (209) | 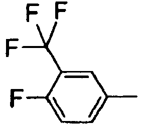 | 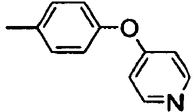 |
| (210) | 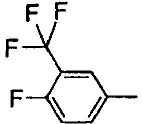 | 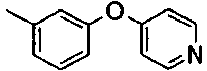 |
| (211) | 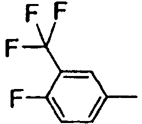 | 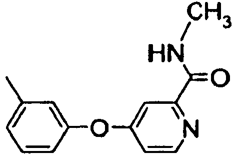 |
| (212) | 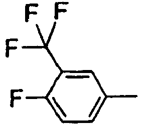 | 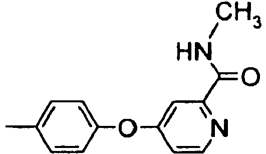 |
| (213) | 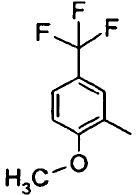 | 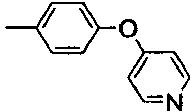 |
| (214) | 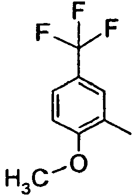 | 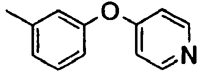 |
| (215) | 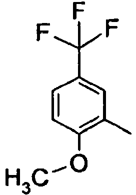 | 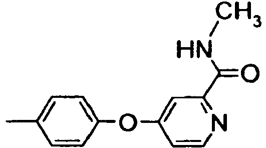 |
| (216) | 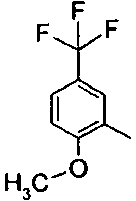 | 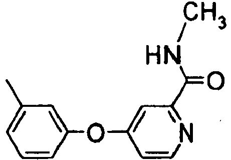 |
| (217) | 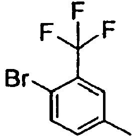 | 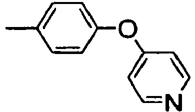 |
| (218) | 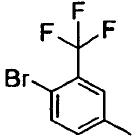 | 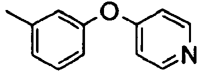 |
| (219) | 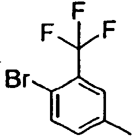 | 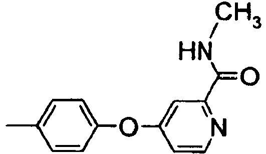 |
| (220) | 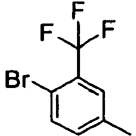 | 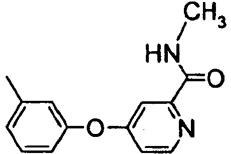 |
| (221) | 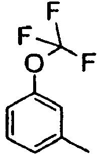 | 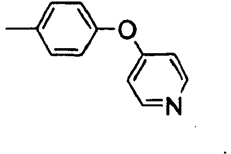 |
| (222) | 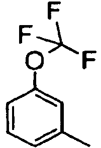 | 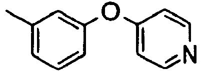 |
| (223) | 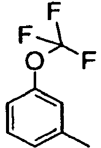 | 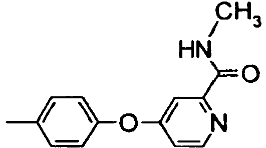 |
| (224) | 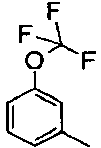 | 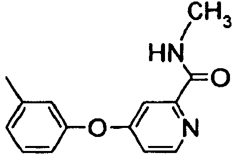 |
| (225) | 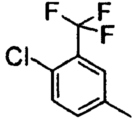 | 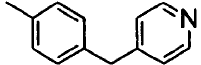 |
| (226) | 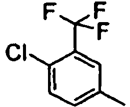 | 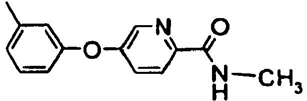 |
| (227) | 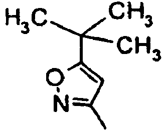 | 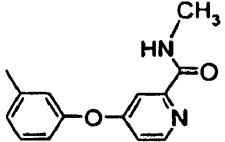 |
| (228) | 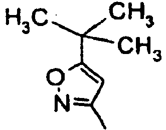 | 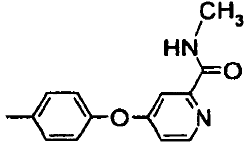 |
and the physiologically acceptable salts and solvates thereof.

4. Malonamide derivative according to one of the claims 1 to 3 as a medicament.

5. Malonamide derivative according to one of the claims 1 to 3 as a kinase inhibitor.

6. Malonamide derivative according to claim 5, **characterized in that** the kinases are selected from raf-kinases and VEGFR kinases.

7. Pharmaceutical composition, **characterized in that** it contains one or more compounds according to one of the claims 1 to 3.

8. Pharmaceutical composition according to claim 7, **characterised in that** it contains one or more additional compounds, selected from the group consisting of physiologically acceptable excipients, auxiliaries, adjuvants, carriers and pharmaceutical active ingredients other than the compounds according to one of the claims 1 to 3.

9. Process for the manufacture of a pharmaceutical composition, **characterised in that** one or more compounds according to one of the claims 1 to 3 and one or more compounds, selected from the group consisting of carriers, excipients, auxiliaries and pharmaceutical active ingredients other than the compounds according to one of the claims 1 to 3, is processed by mechanical means into a pharmaceutical composition that is suitable as dosageform for application and/or administration to a patient.

10. Compound according to one of the claims 1 to 3 for use as a pharmaceutical.

11. Compound according to one of the claims 1 to 3 for use in the treatment and/or prophylaxis of disorders.

12. Use of a compound according to one of the claims 1 to 3 for producing a pharmaceutical composition for the treatment and/or prophylaxis of disorders.

13. Compound for use according to claim 11, **characterised in that** the disorders are caused, mediated and/or propagated by kinases selected from raf-kinases and VEGFR kinases.

14. Compound for use according to claim 11 or 13, **characterised in that** the disorders are selected from the group consisting of hyperproliferative and nonhyperproliferative disorders.

15. Compound for use according to claim 11, 13 or 14, **characterised in that** the disorder is cancer.

16. Compound for use according to claim 11, 13 or 14, **characterised in that** the disorder is noncancerous.

17. Compound for use according to claim 16, **characterised in that** the noncancerous disorders are selected from the group consisting of psioarsis, arthritis, inflammation, endometriosis, scarring, begnin prostatic hyperplasia, immunological diseases, autoimmune diseases and immunodeficiency diseases.

18. Compound for use according to claim 15, **characterised in that** the disorders are selected from the group consisting of brain cancer, lung cancer, squamous cell cancer, bladder cancer, gastric cancer, pancreatic cancer, hepatic cancer, renal cancer, colorectal cancer, breast cancer, head cancer, neck cancer, oesophageal cancer, gynaecological cancer, thyroid cancer, lymphoma, chronic leukaemia and acute leukaemia.

19. Compound for use according to claim 15 or 16, **characterised in that** the disorders are selected from the group consisting of arthritis, restenosis; fibrotic disorders; mesangial cell proliferative disorders, diabetic nephropathy, malignant nephrosclerosis, thrombotic microangiopathy syndromes, organ transplant rejection, glomerulopathies, metabolic disorders, inflammation and neurodegenerative diseases.

20. Use according to claim 12, **characterised in that** the disorders are selected from the group consisting of rheumatoid arthritis, inflammation, autoimmune disease, chronic obstructive pulmonary disease, asthma, inflammatory bowel disease, fibrosis, atherosclerosis, restenosis, vascular disease, cardiovascular disease, inflammation, renal disease and angiogenesis disorders.

21. Compound according to one of the claims 1 to 3 for use as a kinase inhibitor.

22. Compound for use according to claim 21, **characterised in that** the kinase is one or more raf-kinases, selected from the group consisting of A-Raf, B-Raf and Raf-1.

23. Compound according to one of the claims 1 to 3 for use in the treatment of cancerous cell growth mediated by one or more kinases.

24. Method for producing compounds of formula II, **characterised in that**
a) a compound of formula III 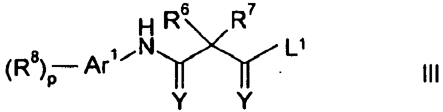 wherein
L¹ is Cl, Br, I, OH, an esterified OH-group or a diazonium moiety, and R⁶, R⁷, R⁸, p, Ar¹, Y are as defined in claim 1,
is reacted
b) with a compound of formula IV, 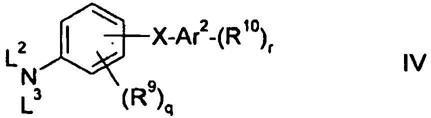 wherein
L², L³ are independently from one another H or a metal ion, and R⁹, q, X, Ar², R¹⁰ and r are as as defined in claim 1,
and optionally
c) isolating and/or treating the compound of formula II obtained by said reaction with an acid, to obtain the salt thereof.

25. Use of a compound of formula III, 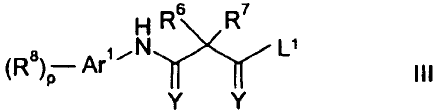 wherein
L¹ is Cl, Br, I, OH, an esterified OH-group or a diazonium
moiety, and R⁶, R⁷, R⁸, p, Ar¹, Y are as defined in claim 1, in a process according to claim. 24, for the manufacture of a compound of formula II according to claim 1.

26. Use of a compound of formula IV, 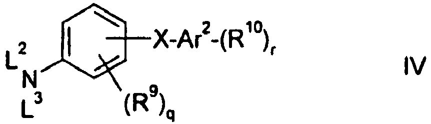 wherein
L², L³ are independently from one another H or a metal ion, and R⁹,
q, X, Ar², R¹⁰ and r are as defined in claim 1, in a process according to Claim 24, for the manufacture of a compound of formula II according to claim 1.

## Patentansprüche

1. Malonamidderivate der Formel II 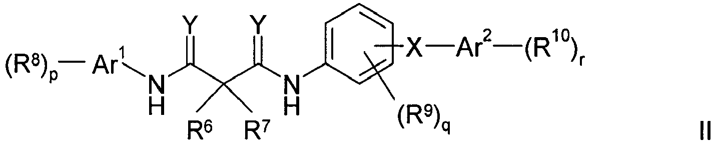 in der
Ar¹ Phenyl, Pyridinyl, Oxazolyl, Isoxazolyl, Pyrazolyl oder Imidazolyl bedeutet,
Ar² Pyridinyl bedeutet,
R¹⁰ aus der Gruppe bestehend aus Alkyl mit 1 bis 4 Kohlenstoffatomen, Alkoxy mit 1 bis 4 Kohlenstoffatomen, Hal, CH₂Hal, CH(Hal)₂, Perhalogenalkyl mit 1 bis 4 Kohlenstoffatomen, NO₂, (CH₂)ₙCN, (CH₂)ₙNR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² und (CH₂)ₙS(O)ᵤR¹³ ausgewählt ist,
k für 0, 1 oder 2 steht,
r für 0, 1 oder 2 steht,
R⁶, R⁷ unabhängig aus den für R⁸, R⁹ angegebenen Bedeutungen ausgewählt sind,
oder R⁶ und R⁷ zusammen einen carbocyclischen Rest mit 3 bis 7 Kohlenstoffatomen oder einen heterocyclischen Rest mit 1, 2 oder 3 aus der Gruppe bestehend aus O, N und S ausgewählten Heteroatomen und 2 bis 6 Kohlenstoffatomen bilden, wobei der carbocyclische oder heterocyclische Rest unsubstituiert ist oder 1, 2 oder 3 Substituenten enthält, die aus den für R⁸, R⁹ und R¹⁰ angegebenen Bedeutungen ausgewählt sind,
R⁸, R⁹ unabhängig aus einer Gruppe bestehend aus H, A, Cycloalkyl mit 3 bis 7 Kohlenstoffatomen, Hal, CH₂Hal, CH(Hal)₂, C(Hal)₃, NO₂, (CH₂)ₙCN, (CH₂)ₙNR¹¹R¹², (CH₂)ₙOR¹¹, (CH₂)ₙO(CH₂)ₖNR¹¹R¹², (CH₂)ₙCOOR¹², (CH₂)ₙCONR¹¹R¹², (CH₂)ₙNR¹¹COR¹³, (CH₂)ₙNR¹¹CONR¹¹R¹², (CH₂)ₙNR¹¹SO₂A, (CH₂)ₙSO₂NR¹¹R¹², (CH₂)ₙS(O)ᵤR¹³, (CH₂)ₙOC(O)R¹³, (CH₂)ₙCOR¹³, (CH₂)ₙSR¹¹, CH=N-OA, CH₂CH=N-OA, (CH₂)ₙNHOA, (CH₂)ₙCH=N-R¹¹, (CH₂)ₙOC(O)NR¹¹R¹², (CH₂)ₙNR¹¹COOR¹², (CH²)ₙN(R¹¹)CH₂CH₂OR¹³, (CH₂)ₙN(R¹¹)CH₂CH₂OCF₃, (CH₂)ₙN(R¹)C(R³)HCOOR², C(R¹³)HCOR¹², (CH₂)ₙN(R¹¹)CH₂CH₂N(R¹²)CH₂COOR¹², (CH₂)ₙN(R¹¹)CH₂CH₂NR¹¹R¹², CH=CHCOOR¹¹, CH=CHCH₂NR¹¹R¹², CH=CHCH₂NR¹¹R¹², CH=CHCH₂OR¹³, (CH₂)ₙN(COOR11)COOR¹², (CH₂)ₙN(CONH₂)COOR¹¹, (CH₂)ₙN(CONH₂)CONH₂, (CH₂)ₙN(CH₂COOR¹¹)COOR¹², (CH₂)ₙN(CH₂CONH₂)COOR¹¹, (CH₂)ₙN(CH₂CONH₂)CONH₂, (CH₂)ₙCHR¹³COR¹¹, (CH₂)ₙCHR¹³COOR¹¹, (CH₂)ₙCHR¹³CH₂OR¹⁴, (CH₂)ₙOCN und (CH₂)ₙNCO ausgewählt sind, in denen
R¹¹, R¹² unabhängig aus einer Gruppe bestehend aus H, A und (CH₂)ₘAr³ ausgewählt sind oder in NR¹¹R¹²
R¹¹ und R¹² zusammen mit dem N-Atom, an das sie gebunden sind, einen 5-, 6- oder 7-gliedrigen Heterocyclus bilden, der gegebenenfalls 1 oder 2 zusätzliche, aus O, N und S ausgewählte Heteroatome enthält,
R¹³, R¹⁴ unabhängig aus einer Gruppe bestehend aus H, Hal, A, (CH₂)ₘAr⁴ und (CH₂)ₘHet ausgewählt sind,
A aus der Gruppe bestehend aus Alkyl, Alkenyl, Cycloalkyl, Alkylencycloalkyl, Alkoxy und Alkoxyalkyl ausgewählt ist,
Ar³, Ar⁴ unabhängig voneinander aromatische Kohlenwasserstoffreste mit 5 bis 12 und bevorzugt 5 bis 10 Kohlenstoffatomen bedeuten, die gegebenenfalls durch einen oder mehrere Substituenten substituiert sind, die aus einer Gruppe bestehend aus A, Hal, NO₂, CN, OR¹⁵, NR¹⁵R¹⁶, COOR¹⁵, CONR¹⁵R¹⁶, NR¹⁵COR¹⁶, NR¹⁵CONR¹⁵R¹⁶, NR¹⁶SO₂A, COR¹⁵, SO₂R¹⁵R¹⁶, S(O)ᵤA und OOCR¹⁵ ausgewählt sind,
Het einen gesättigten, ungesättigten oder aromatischen heterocyclischen Rest bedeutet, der gegebenenfalls durch einen oder mehrere Substituenten substituiert ist, die aus einer Gruppe bestehend aus A, Hal, NO₂, CN, OR¹⁵, NR¹⁵R¹⁶, COOR¹⁵, CONR¹⁵R¹⁶, NR¹⁵COR¹⁶, NR¹⁵CONR¹⁵R¹⁶, NR¹⁶SO₂A, COR¹⁵, SO₂R¹⁵R¹⁶, S(O)ᵤA und OOCR¹⁵ ausgewählt sind,
R¹⁵, R¹⁶ unabhängig aus einer Gruppe bestehend aus H, A und (CH₂)ₘAr⁶ ausgewählt sind, in der
Ar⁶ einen 5- oder 6-gliedrigen aromatischen Kohlenwasserstoff bedeutet, der gegebenenfalls durch einen oder mehrere Substituenten substituiert ist, die aus einer Gruppe bestehend aus Methyl, Ethyl, Propyl, 2-Propyl, tert.-Butyl, Hal, CN, OH, NH₂ und CF₃ ausgewählt sind,
m und n unabhängig voneinander für 0, 1, 2, 3, 4 oder 5 stehen,
X aus der Gruppe bestehend aus O, S, NR¹¹, CHOR¹¹, CH₂, CH₂CH₂, OCH₂, CH₂O, OCH₂CH₂, CH₂CH₂O ausgewählt ist,
h, i unabhängig voneinander für 0, 1, 2, 3, 4, 5 oder 6 stehen, und
j für 1, 2, 3, 4, 5 oder 6 steht,
Y aus O, S, NR²¹, C(R²²)-NO₂, C(R²²)-CN, und C(CN)₂, ausgewählt ist, in denen
R²¹ unabhängig aus den für R¹³, R¹⁴ angegebenen Bedeutungen ausgewählt ist und
R²² unabhängig aus den für R¹¹, R¹² angegebenen Bedeutungen ausgewählt ist,
p für 0, 1, 2, 3, 4 oder 5 steht,
q für 0, 1, 2, 3 oder 4 steht, bevorzugt 0, 1 oder 2,
u für 0, 1, 2 oder 3 steht, bevorzugt 0, 1 oder 2,
und
Hal unabhängig aus einer Gruppe bestehend aus F, Cl, Br und I ausgewählt ist;
und deren pharmazeutisch unbedenkliche Salze und Solvate.

2. Malonamidderivat nach Anspruch 1, ausgewählt aus den Verbindungen der Formel IIa, IIb, IIc, IId, IIe, IIf, IIg und IIh, 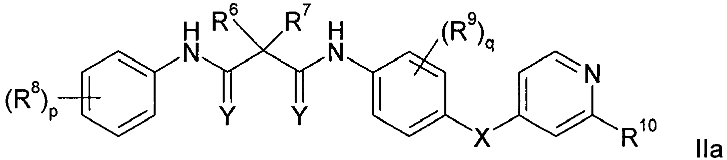 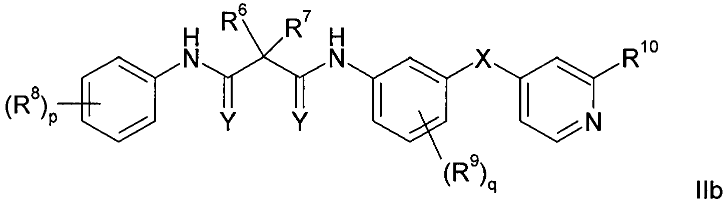 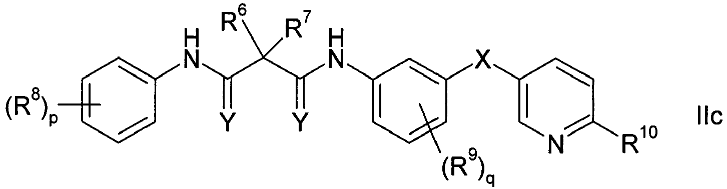 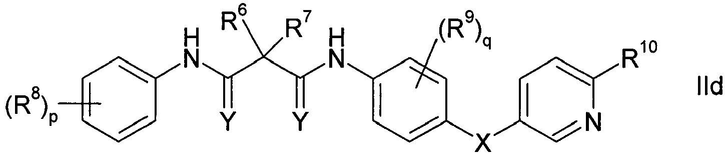 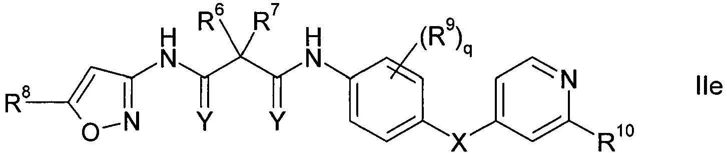 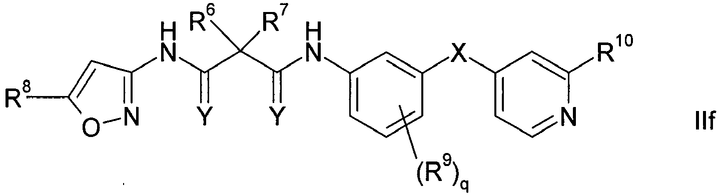 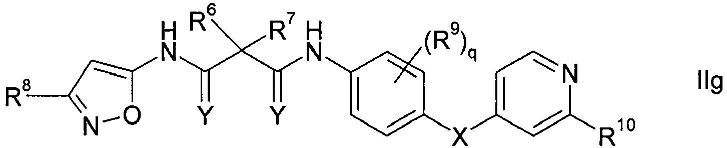 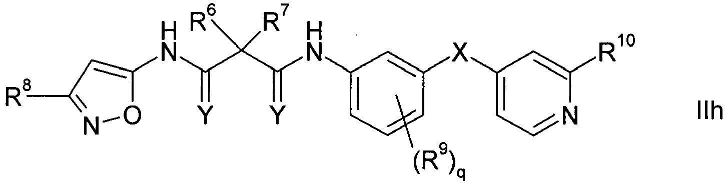 in denen R⁶, R⁷, R⁸, p, X, Y, R⁹, q wie in Anspruch 1 definiert sind und R¹⁰ H bedeutet oder wie in Anspruch 1 definiert ist;
und deren pharmazeutisch unbedenkliche Salze und Solvate.

3. Malonamidderivat nach einem der Ansprüche 1 oder 2, ausgewählt aus den Verbindungen (1) bis (228) der Tabelle 1; Verbindungen (1) bis (228) der Formel A-NH-CO-CH₂-CO-NH-B, in der A und B wie nachstehend angegeben sind:
| | A | B |
|---|---|---|
| (1) | 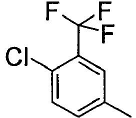 | 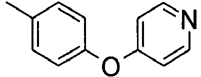 |
| (2) | 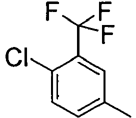 | 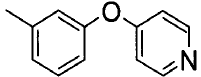 |
| (3) | 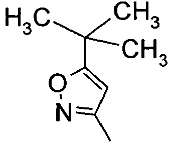 | 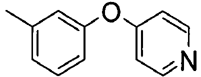 |
| (4) | 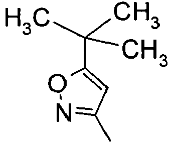 | 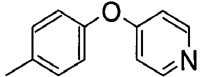 |
| (5) | 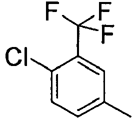 | 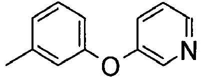 |
| (6) | 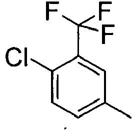 | 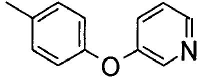 |
| (7) | 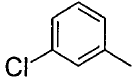 | 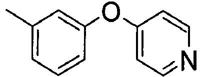 |
| (8) | 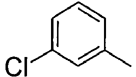 | 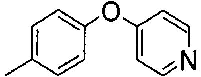 |
| (9) | 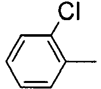 | 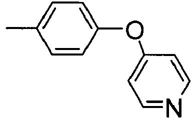 |
| (10) | 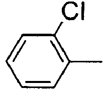 | 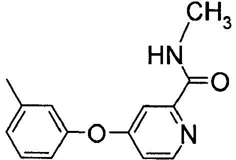 |
| (11) | 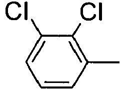 | 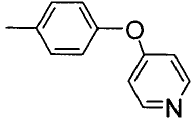 |
| (12) | 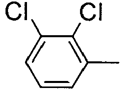 | 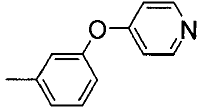 |
| (13) | 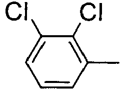 | 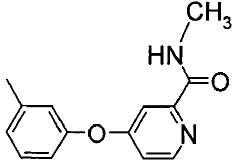 |
| (14) | 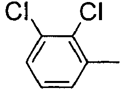 | 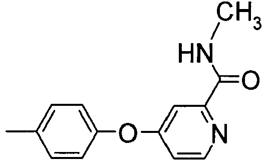 |
| (15) | 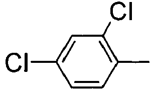 | 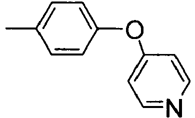 |
| (16) | 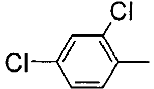 | 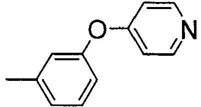 |
| (17) | 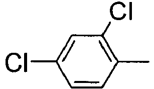 | 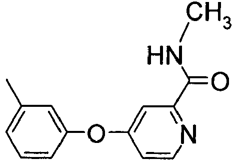 |
| (18) | 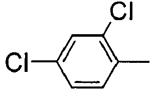 | 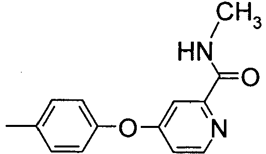 |
| (19) | 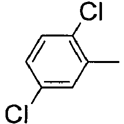 | 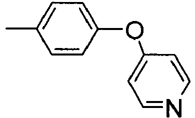 |
| (20) | 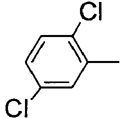 | 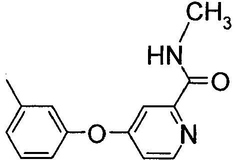 |
| (21) | 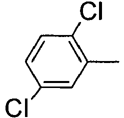 | 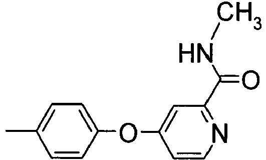 |
| (22) | 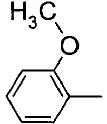 | 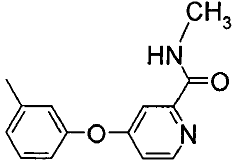 |
| (23) | 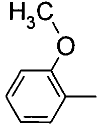 | 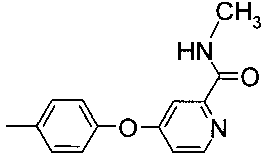 |
| (24) | 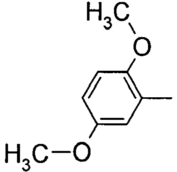 | 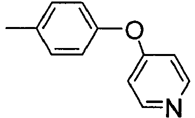 |
| (25) | 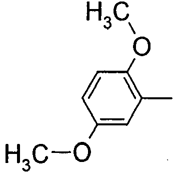 | 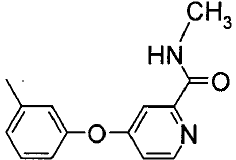 |
| (26) | 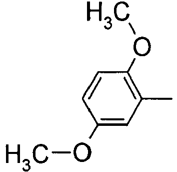 | 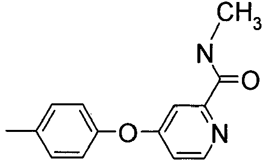 |
| (27) | 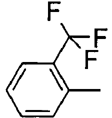 | 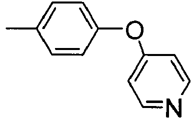 |
| (28) | 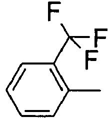 | 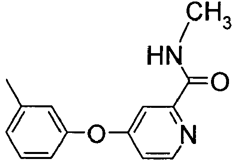 |
| (29) | 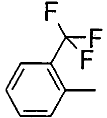 | 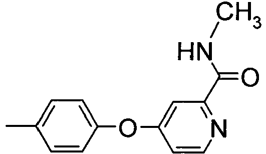 |
| (30) | 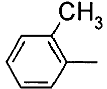 | 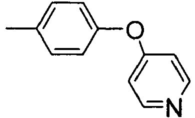 |
| (31) | 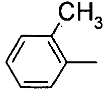 | 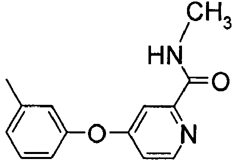 |
| (32) | 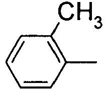 | 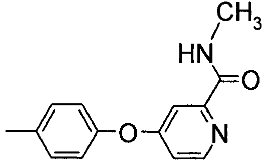 |
| (33) | 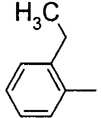 | 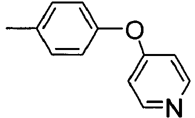 |
| (34) | 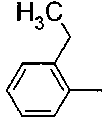 | 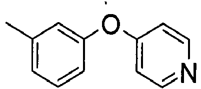 |
| (35) | 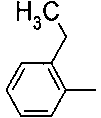 | 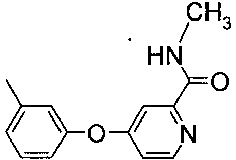 |
| (36) | 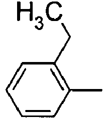 | 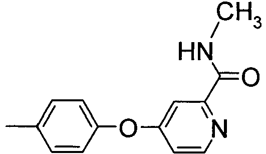 |
| (37) | 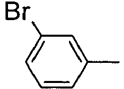 | 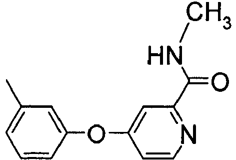 |
| (38) | 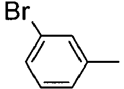 | 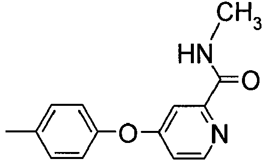 |
| (39) | 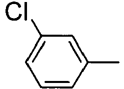 | 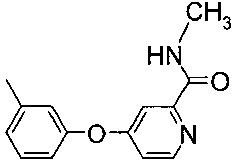 |
| (40) | 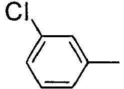 | 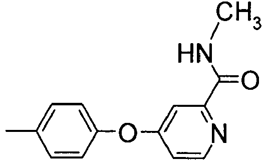 |
| (41) | 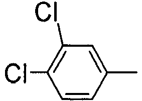 | 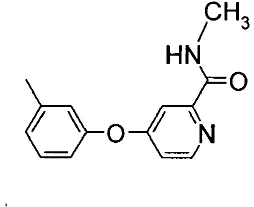 |
| (42) | 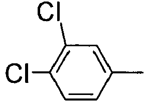 | 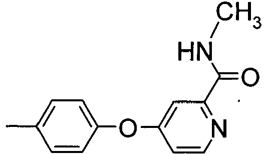 |
| (43) | 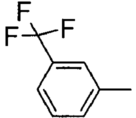 | 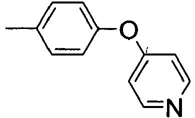 |
| (44) | 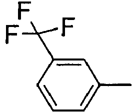 | 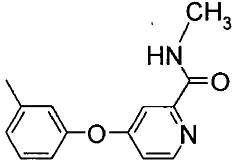 |
| (45) | 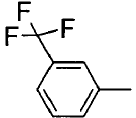 | 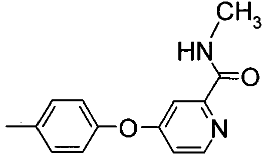 |
| (46) | 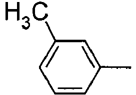 | 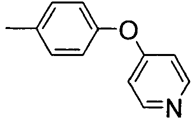 |
| (47) | 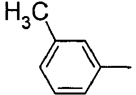 | 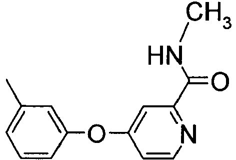 |
| (48) | 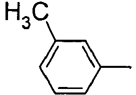 | 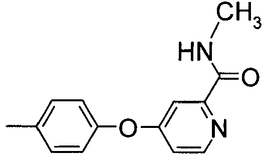 |
| (49) | 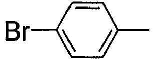 | 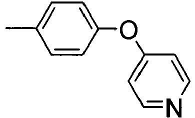 |
| (50) | 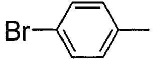 | 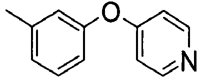 |
| (51) | 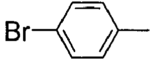 | 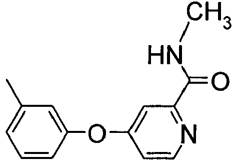 |
| (52) | 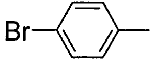 | 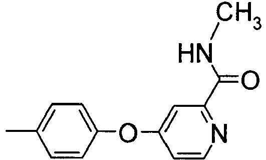 |
| (53) | 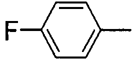 | 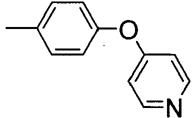 |
| (54) | 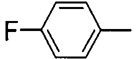 | 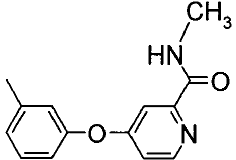 |
| (55) | 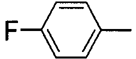 | 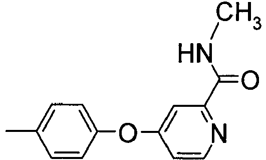 |
| (56) | 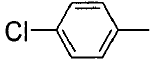 | 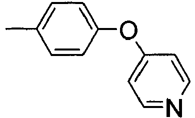 |
| (57) | 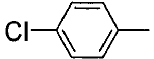 | 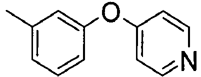 |
| (58) | 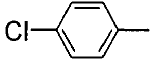 | 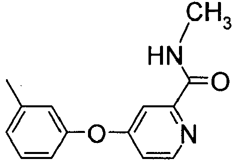 |
| (59) | 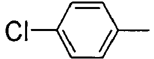 | 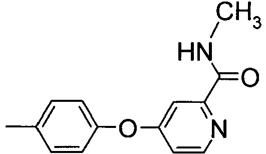 |
| (60) | 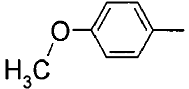 | 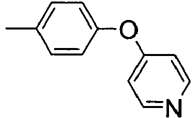 |
| (61) | 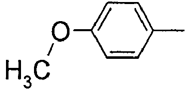 | 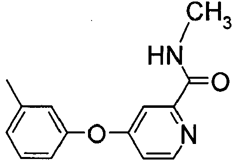 |
| (62) | 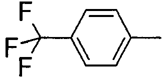 | 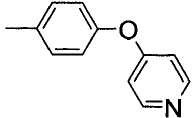 |
| (63) | 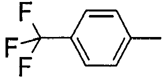 | 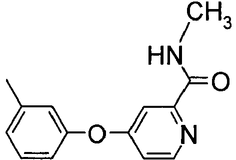 |
| (64) | 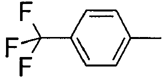 | 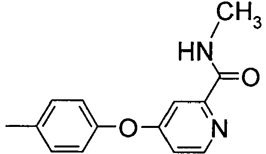 |
| (65) | 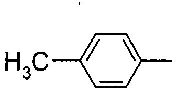 | 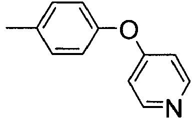 |
| (66) | 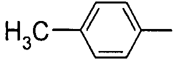 | 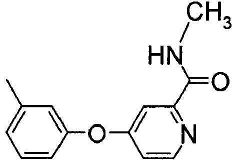 |
| (67) | 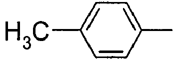 | 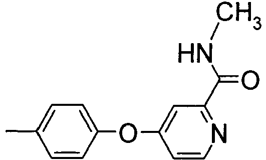 |
| (68) | 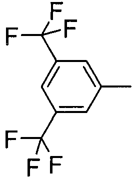 | 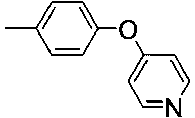 |
| (69) | 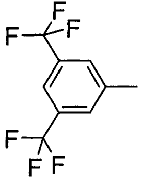 | 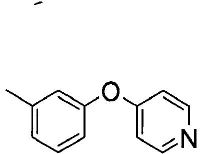 |
| (70) | 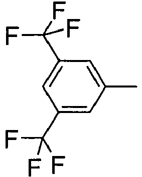 | 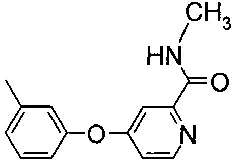 |
| (71) | 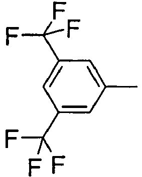 | 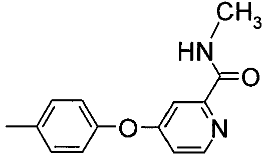 |
| (72) | 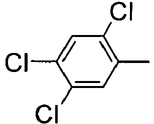 | 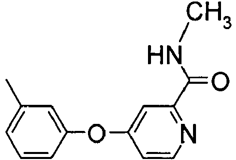 |
| (73) | 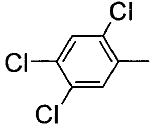 | 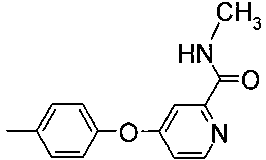 |
| (74) | 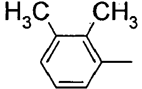 | 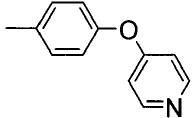 |
| (75) | 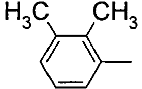 | 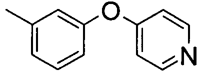 |
| (76) | 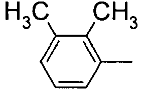 | 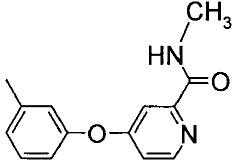 |
| (77) | 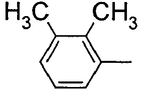 | 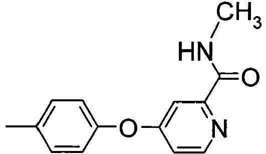 |
| (78) | 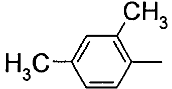 | 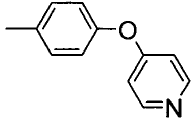 |
| (79) | 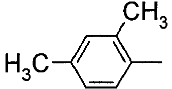 | 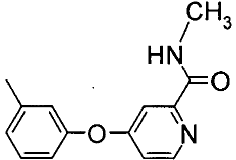 |
| (80) | 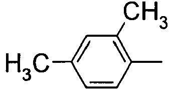 | 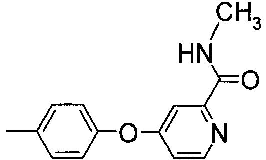 |
| (81) | 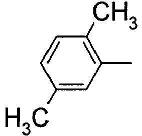 | 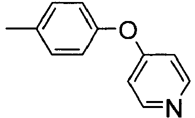 |
| (82) | 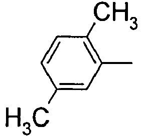 | 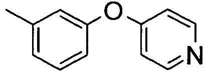 |
| (83) | 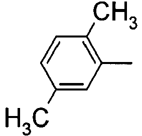 | 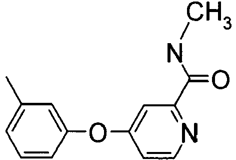 |
| (84) | 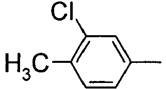 | 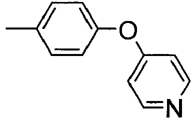 |
| (85) | 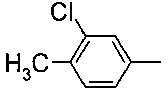 | 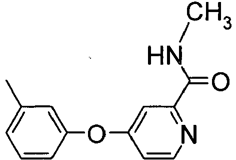 |
| (86) | 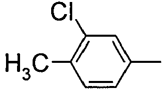 | 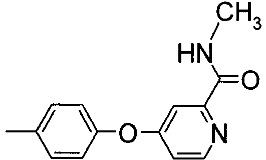 |
| (87) | 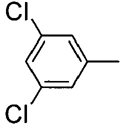 | 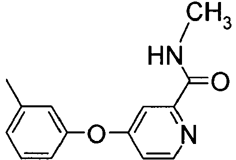 |
| (88) | 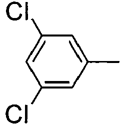 | 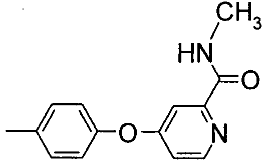 |
| (89) | 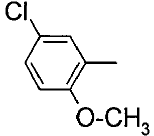 | 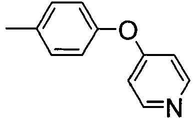 |
| (90) | 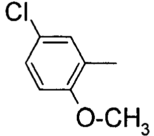 | 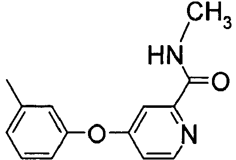 |
| (91) | 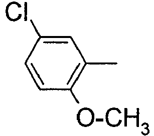 | 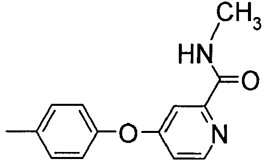 |
| (92) | 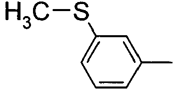 | 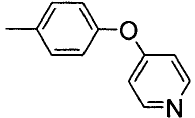 |
| (93) | 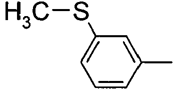 | 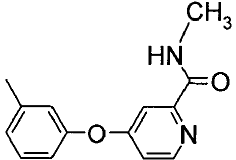 |
| (94) | 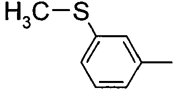 | 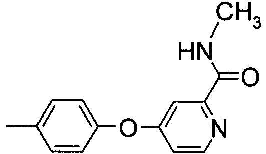 |
| (95) | 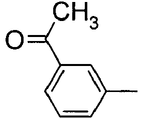 | 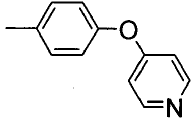 |
| (96) | 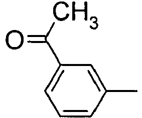 | 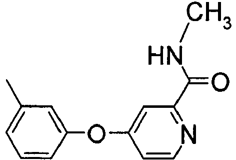 |
| (97) | 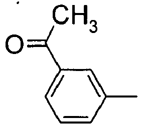 | 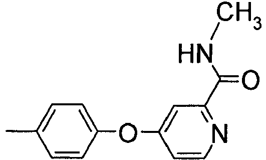 |
| (98) | 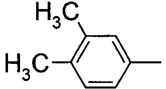 | 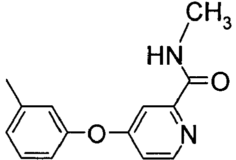 |
| (99) | 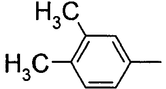 | 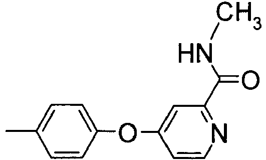 |
| (100) | 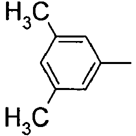 | 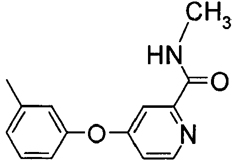 |
| (101) | 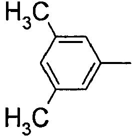 | 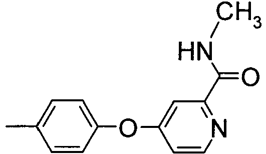 |
| (102) | 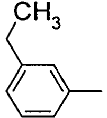 | 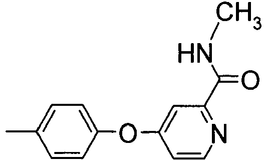 |
| (103) | 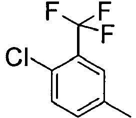 | 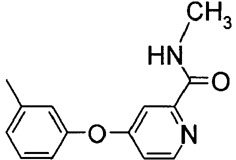 |
| (104) | 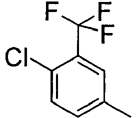 | 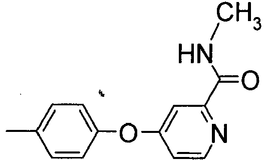 |
| (105) | 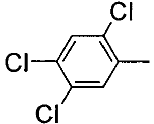 | 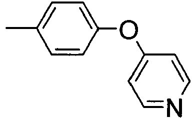 |
| (106) | 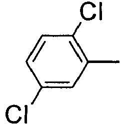 | 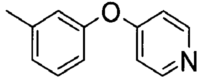 |
| (107) | 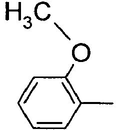 | 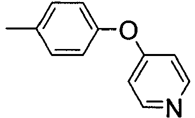 |
| (108) | 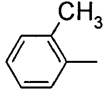 | 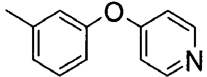 |
| (109) | 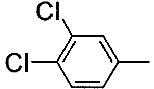 | 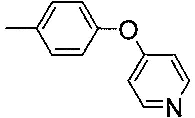 |
| (110) | 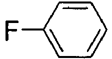 | 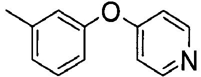 |
| (111) | 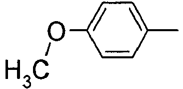 | 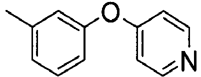 |
| (112) | 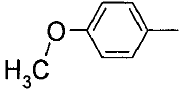 | 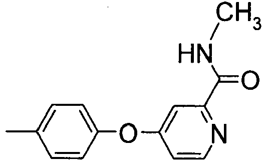 |
| (113) | 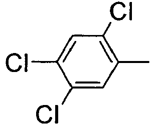 | 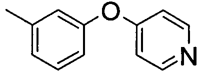 |
| (114) | 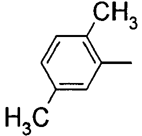 | 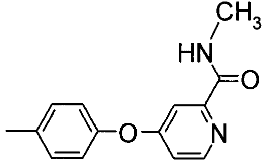 |
| (115) | 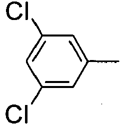 | 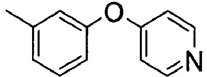 |
| (116) | 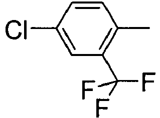 | 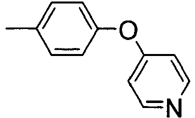 |
| (117) | 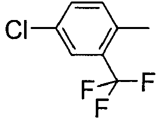 | 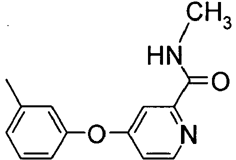 |
| (118) | 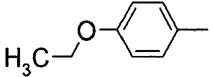 | 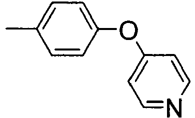 |
| (119) | 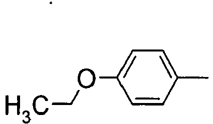 | 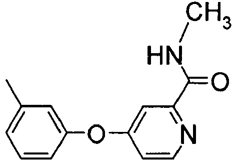 |
| (120) | 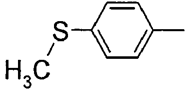 | 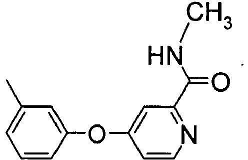 |
| (121) | 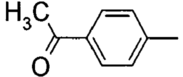 | 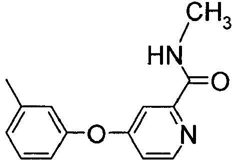 |
| (122) | 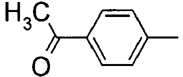 | 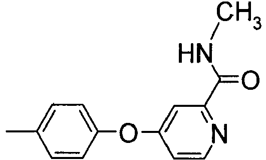 |
| (123) | 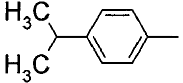 | 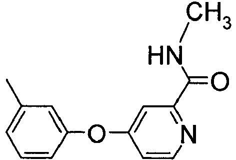 |
| (124) | 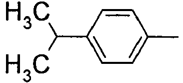 | 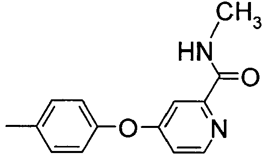 |
| (125) | 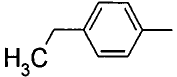 | 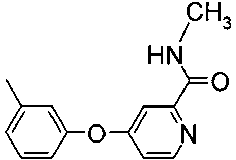 |
| (126) | 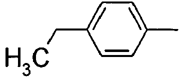 | 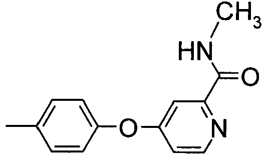 |
| (127) | 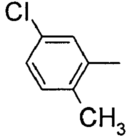 | 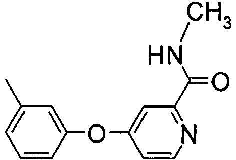 |
| (128) | 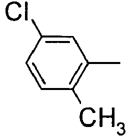 | 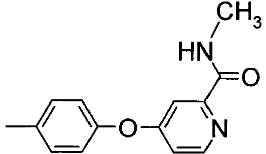 |
| (129) | 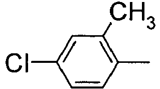 | 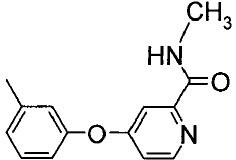 |
| (130) | 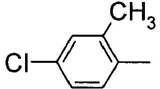 | 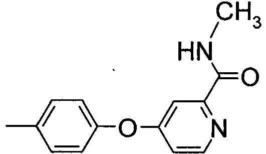 |
| (131) | 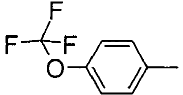 | 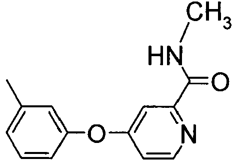 |
| (132) 2 | 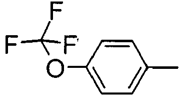 | 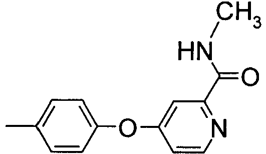 |
| (133) | 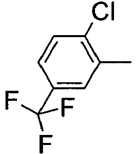 | 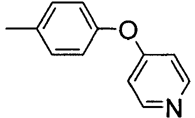 |
| (134) | 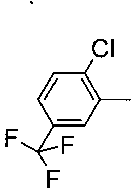 | 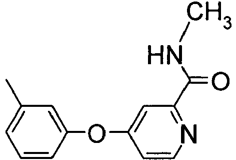 |
| (135) | 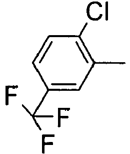 | 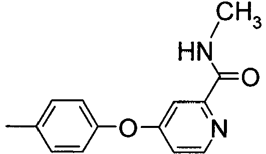 |
| (136) | 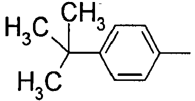 | 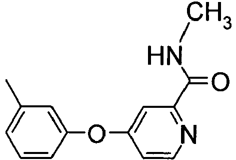 |
| (137) | 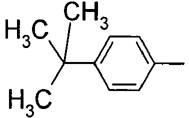 | 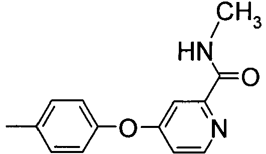 |
| (138) | 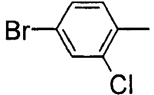 | 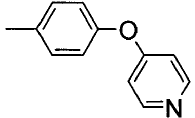 |
| (139) | 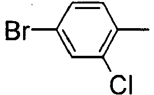 | 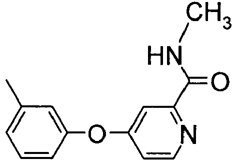 |
| (140) | 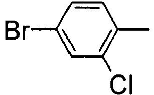 | 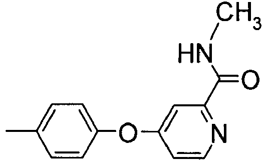 |
| (141) | 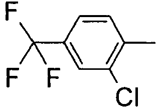 | 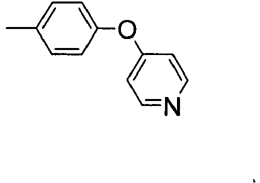 |
| (142) | 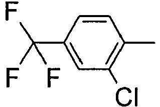 | 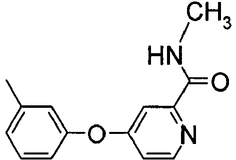 |
| (143) | 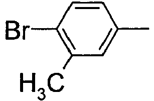 | 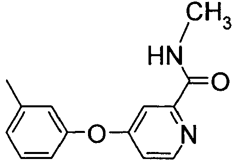 |
| (144) | 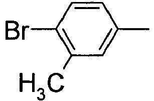 | 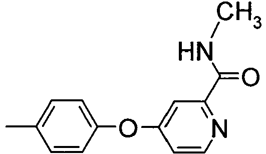 |
| (145) | 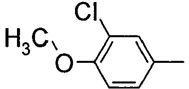 | 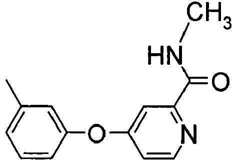 |
| (146) | 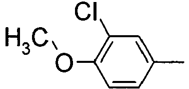 | 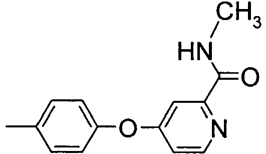 |
| (147) | 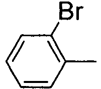 | 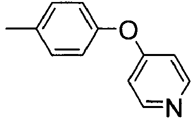 |
| (148) | 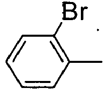 | 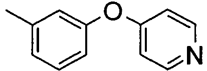 |
| (149) | 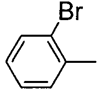 | 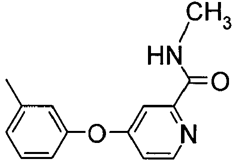 |
| (150) | 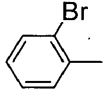 | 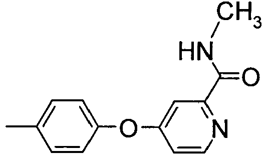 |
| (151) | 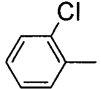 | 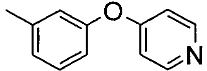 |
| (152) | 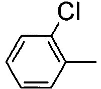 | 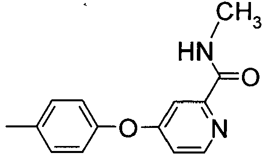 |
| (153) | 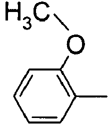 | 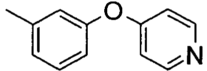 |
| (154) | 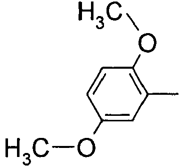 | 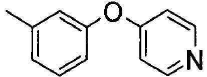 |
| (155) | 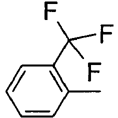 | 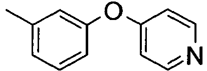 |
| (156) | 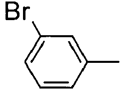 | 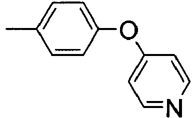 |
| (157) | 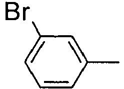 | 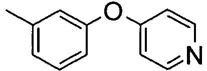 |
| (158) | 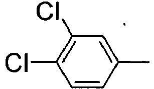 | 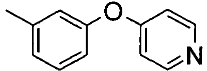 |
| (159) | 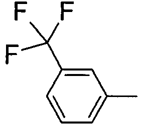 | 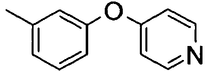 |
| (160) | 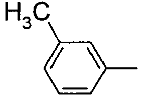 | 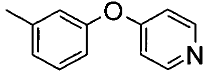 |
| (161) | 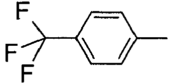 | 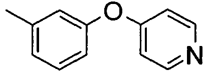 |
| (162) | 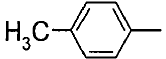 | 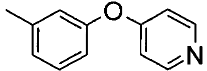 |
| (163) | 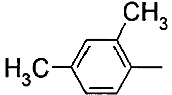 | 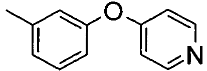 |
| (164) | 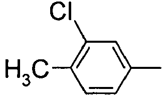 | 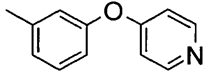 |
| (165) | 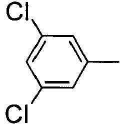 | 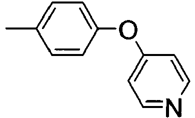 |
| (166) | 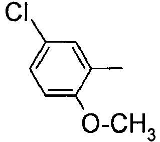 | 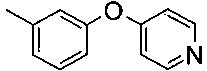 |
| (167) | 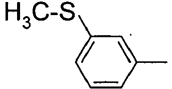 | 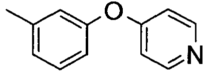 |
| (168) | 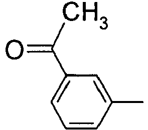 | 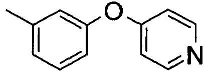 |
| (169) | 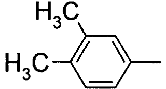 | 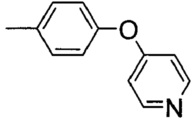 |
| (170) | 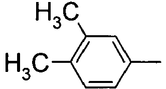 | 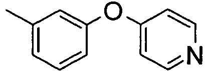 |
| (171) | 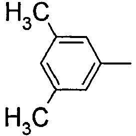 | 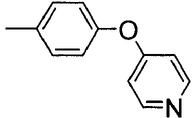 |
| (172) | 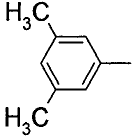 | 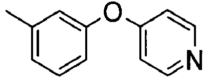 |
| (173) | 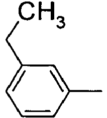 | 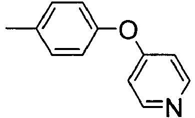 |
| (174) | 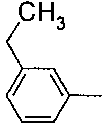 | 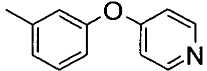 |
| (175) | 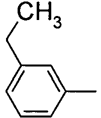 | 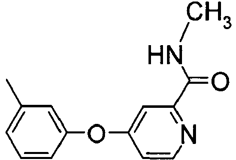 |
| (176) | 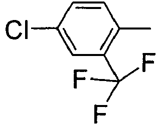 | 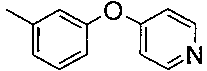 |
| (177) | 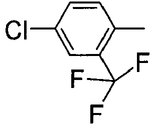 | 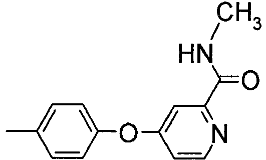 |
| (178) | 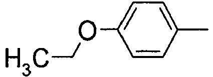 | 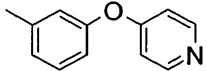 |
| (179) | 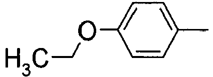 | 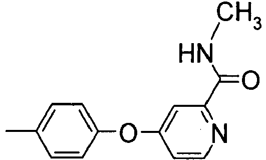 |
| (180) | 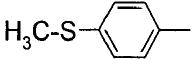 | 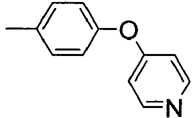 |
| (181) | 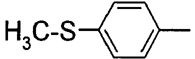 | 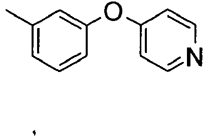 |
| (182) | 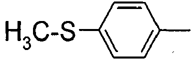 | 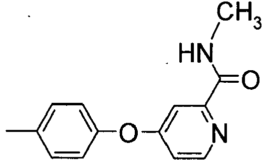 |
| (183) | 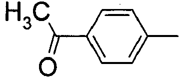 | 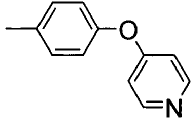 |
| (184) | 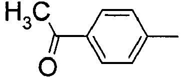 | 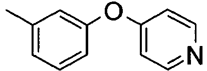 |
| (185) | 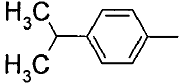 | 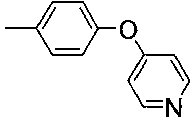 |
| (186) | 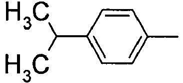 | 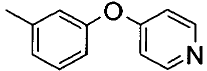 |
| (187) | 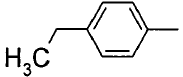 | 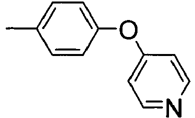 |
| (188) | 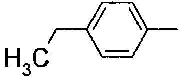 | 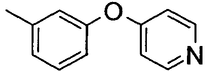 |
| (189) | 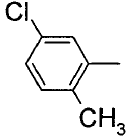 | 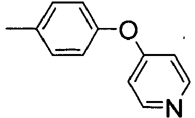 |
| (190) | 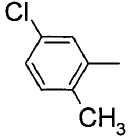 | 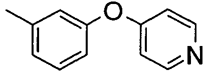 |
| (191) | 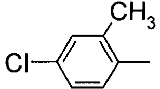 | 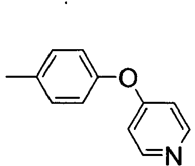 |
| (192) | 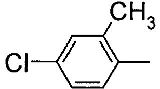 | 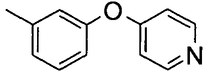 |
| (193) | 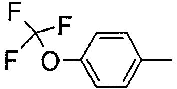 | 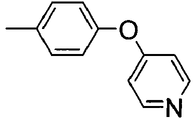 |
| (194) | 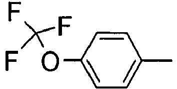 | 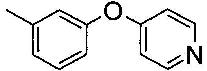 |
| (195) | 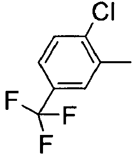 | 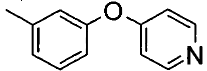 |
| (196) | 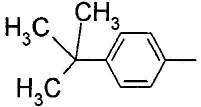 | 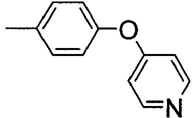 |
| (197) | 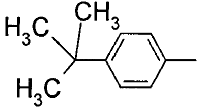 | 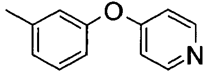 |
| (198) | 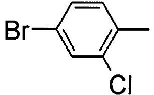 | 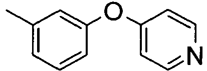 |
| (199) | 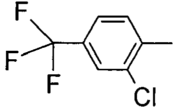 | 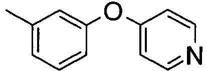 |
| (200) | 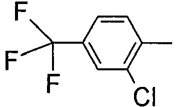 | 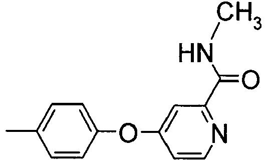 |
| (201) | 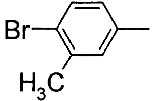 | 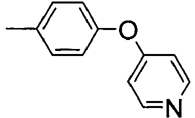 |
| (202) | 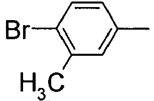 | 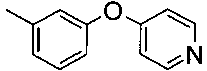 |
| (203) | 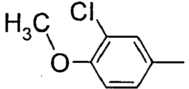 | 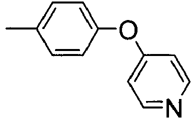 |
| (204) | 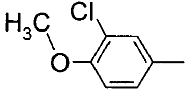 | 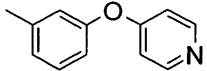 |
| (205) | 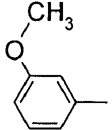 | 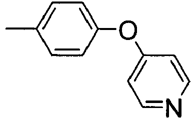 |
| (206) | 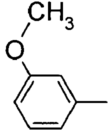 | 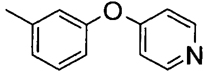 |
| (207) | 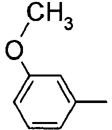 | 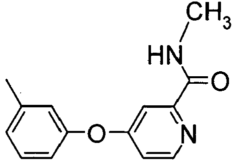 |
| (208) | 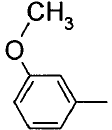 | 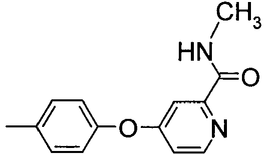 |
| (209) | 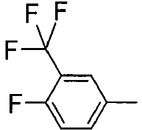 | 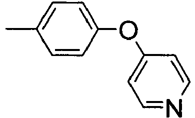 |
| (210) | 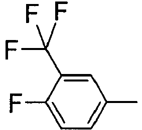 | 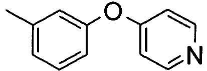 |
| (211) | 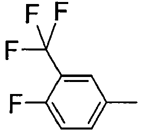 | 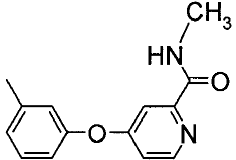 |
| (212) | 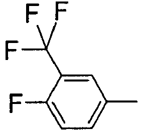 | 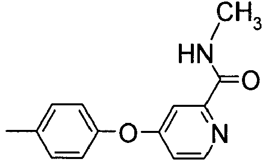 |
| (213) | 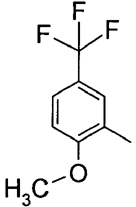 | 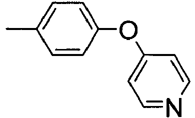 |
| (214) | 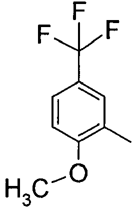 | 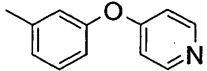 |
| (215) | 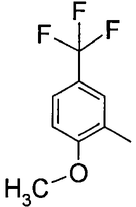 | 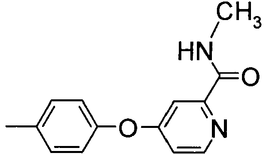 |
| (216) | 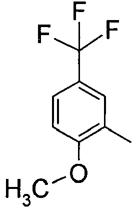 | 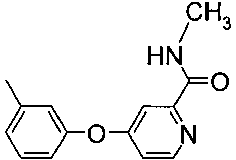 |
| (217) | 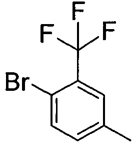 | 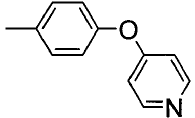 |
| (218) | 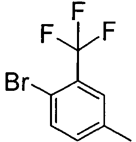 | 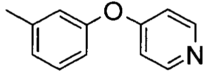 |
| (219) | 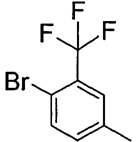 | 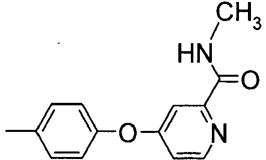 |
| (220) | 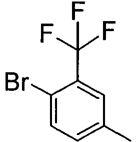 | 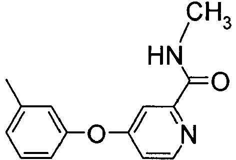 |
| (221) | 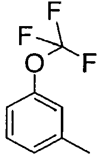 | 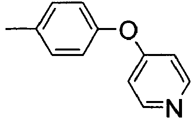 |
| (222) | 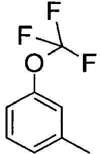 | 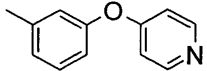 |
| (223) | 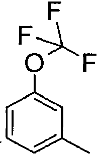 | 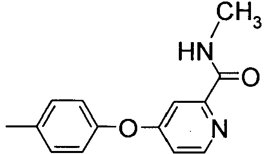 |
| (224) | 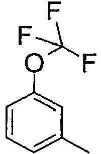 | 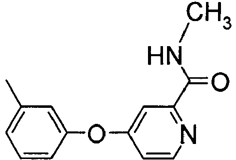 |
| (225) | 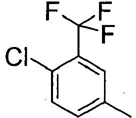 | 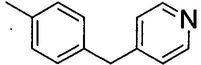 |
| (226) | 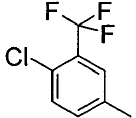 | 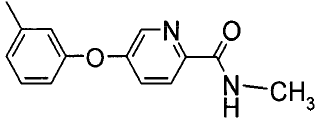 |
| (227) | 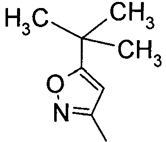 | 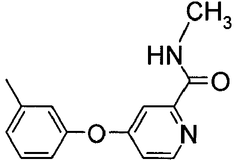 |
| (228) | 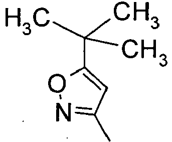 | 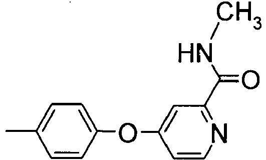 |
und deren physiologisch unbedenkliche Salze und Solvate.

4. Malonamidderivat nach einem der Ansprüche 1 bis 3 als Arzneimittel.

5. Malonamidderivat nach einem der Ansprüche 1 bis 3 als Kinasehemmer.

6. Malonamidderivat nach Anspruch 5, **dadurch gekennzeichnet, dass** die Kinasen aus raf-Kinasen und VEGFR-Kinasen ausgewählt sind.

7. Pharmazeutische Zubereitung, **dadurch gekennzeichnet, dass** sie eine oder mehrere Verbindungen nach einem der Ansprüche 1 bis 3 enthält.

8. Pharmazeutische Zubereitung nach Anspruch 7, **dadurch gekennzeichnet, dass** sie eine oder mehrere weitere Verbindungen enthält, die aus der Gruppe bestehend aus physiologisch unbedenklichen Streckmitteln, Hilfsstoffen, Zusatzstoffen, Trägerstoffen und anderen pharmazeutischen Wirkstoffen als den Verbindungen nach einem der Ansprüche 1 bis 3 ausgewählt sind.

9. Verfahren zur Herstellung einer pharmazeutischen Zubereitung, **dadurch gekennzeichnet, dass** man eine oder mehrere Verbindungen nach einem der Ansprüche 1 bis 3 und eine oder mehrere Verbindungen, die aus der Gruppe bestehend aus Trägerstoffen, Streckmitteln, Hilfsstoffen und anderen pharmazeutischen Wirkstoffen als den Verbindungen nach einem der Ansprüche 1 bis 3 ausgewählt sind, auf mechanische Weise zu einer pharmazeutischen Zubereitung verarbeitet, die sich als Darreichungsform für die Anwendung bei einem und/oder Verabreichung an einen Patienten eignet.

10. Verbindung nach einem der Ansprüche 1 bis 3 zur Verwendung als Pharmazeutikum.

11. Verbindung nach einem der Ansprüche 1 bis 3 zur Verwendung bei der Behandlung und/oder Prophylaxe von Erkrankungen.

12. Verwendung einer Verbindung nach einem der Ansprüche 1 bis 3 zur Herstellung einer pharmazeutischen Zubereitung für die Behandlung und/oder Prophylaxe von Erkrankungen.

13. Verbindung zur Verwendung nach Anspruch 11, **dadurch gekennzeichnet, dass** die Erkrankungen durch Kinasen, die aus raf-Kinasen und VEGFR-Kinasen ausgewählt sind, verursacht, vermittelt und/oder propagiert werden.

14. Verbindung zur Verwendung nach Anspruch 11 oder 13, **dadurch gekennzeichnet, dass** die Erkrankungen aus der Gruppe bestehend aus hyperproliferativen und nicht hyperproliferativen Erkrankungen ausgewählt sind.

15. Verbindung zur Verwendung nach Anspruch 11, 13 oder 14, **dadurch gekennzeichnet, dass** die Erkrankung Krebs ist.

16. Verbindung zur Verwendung nach Anspruch 11, 13 oder 14, **dadurch gekennzeichnet, dass** die Erkrankung nicht krebsartig ist.

17. Verbindung zur Verwendung nach Anspruch 16, **dadurch gekennzeichnet, dass** die nicht krebsartigen Erkrankungen aus der Gruppe bestehend aus Psoriasis, Arthritis, Entzündungen, Endometriose, Vernarbung, gutartiger Prostatahyperplasie, immunologischen Krankheiten, Autoimmunkrankheiten und Immunschwächekrankheiten ausgewählt sind.

18. Verbindung zur Verwendung nach Anspruch 15, **dadurch gekennzeichnet, dass** die Erkrankungen aus der Gruppe bestehend aus Hirnkrebs, Lungenkrebs, Plattenepithelkrebs, Blasenkrebs, gastrischem Krebs, Pankreaskrebs, Leberkrebs, Nierenkrebs, Kolorektalkrebs, Brustkrebs, Kopfkrebs, Halskrebs, Ösophaguskrebs, gynäkologischem Krebs, Schilddrüsenkrebs, Lymphom, chronischer Leukämie und akuter Leukämie ausgewählt sind.

19. Verbindung zur Verwendung nach Anspruch 15 oder 16, **dadurch gekennzeichnet, dass** die Erkrankungen aus der Gruppe bestehend aus Arthritis, Restenose; fibrotischen Erkrankungen; Störungen der Mesangiumzellproliferation, diabetischer Nephropathie, maligner Nephrosklerose, thrombotischen Mikroangiopathie-Syndromen, Organtransplantatabstoßung, Glomerulopathien, Stoffwechselstörungen, Entzündungen und neurodegenerativen Erkrankungen ausgewählt sind.

20. Verwendung nach Anspruch 12, **dadurch gekennzeichnet, dass** die Erkrankungen aus der Gruppe bestehend aus rheumatoider Arthritis, Entzündungen, Autoimmunkrankheiten, chronischer obstruktiver pulmonaler Krankheit, Asthma, Reizdarm, Fibrose, Atherosklerose, Restenose, Gefäßerkrankung, Kardiovaskulärerkrankung, Entzündungen, Nierenerkrankung und Angiogenesestörungen ausgewählt sind.

21. Verbindung nach einem der Ansprüche 1 bis 3 als Kinasehemmer.

22. Verbindung zur Verwendung nach Anspruch 21, **dadurch gekennzeichnet, dass** es sich bei der Kinase um eine oder mehrere raf-Kinasen handelt, die aus der Gruppe bestehend aus A-Raf, B-Raf und Raf-1 ausgewählt sind.

23. Verbindung nach einem der Ansprüche 1 bis 3 zur Verwendung bei der Behandlung von karzinösem Zellwachstum, das durch eine oder mehrere Kinasen vermittelt wird.

24. Verfahren zur Herstellung von Verbindungen der Formel II, **dadurch gekennzeichnet, dass** man
a) eine Verbindung der Formel III 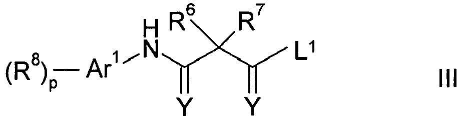 in der
L¹ Cl, Br, I, OH, eine veresterte OH-Gruppe oder einem Diazoniumrest bedeutet und R⁶, R⁷, R⁸, p, Ar¹, Y wie in Anspruch 1 definiert sind,
b) mit einer Verbindung der Formel IV 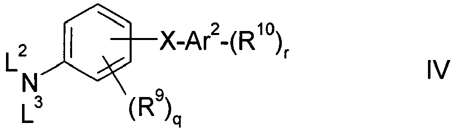 in der
L², L³ unabhängig voneinander H oder ein Metallion bedeuten und R⁹, q, X, Ar², R¹⁰ und r wie in Anspruch 1 definiert sind,
umsetzt und gegebenenfalls
c) die durch diese Reaktion erhaltene Verbindung der Formel II isoliert und/oder mit einer Säure behandelt, um deren Salz zu erhalten.

25. Verwendung einer Verbindung der Formel III 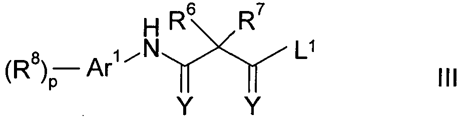 in der
L¹ Cl, Br, I, OH, eine veresterte OH-Gruppe oder einen Diazoniumrest bedeutet und R⁶, R⁷, R⁸, p, Ar¹, Y wie in Anspruch 1 definiert sind,
in einem Verfahren nach Anspruch 24, zur Herstellung einer Verbindung der Formel II nach Anspruch 1.

26. Verwendung einer Verbindung der Formel IV 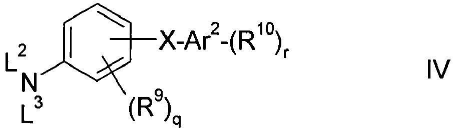 in der
L², L³ unabhängig voneinander H oder ein Metallion bedeuten und R⁹, q, X, Ar², R¹⁰ und r wie in Anspruch 1 definiert sind,
in einem Verfahren nach Anspruch 24, zur Herstellung einer Verbindung der Formel II nach Anspruch 1.

## Revendications

1. Dérivés de malonamide de formule II 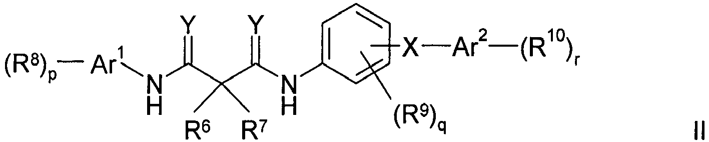 dans laquelle
Ar¹ est phényle, pyridinyle, oxazolyle, isoxazolyle, pyrazolyle ou imidazolyle,
Ar² est pyridinyle,
R¹⁰ est choisi dans le groupe constitué par alkyle comprenant de 1 à 4 atomes de carbone, alcoxy comprenant de 1 à 4 atomes de carbone, Hal, CH₂Hal, CH(Hal)₂, perhalogénoalkyle comprenant de 1 à 4 atomes de carbone, NO₂, (CH₂)ₙCN, (CH₂)ₙ,NR¹¹R¹², (CH₂)ₙO(CH₂)ₖNR¹¹R¹², (CH₂)ₙCOR¹³, (CH₂)ₙCOOR¹³, (CH₂)ₙCONR¹¹R¹², (CH₂)ₙSO₂NR¹¹R¹² et (CH₂)ₙS(O)ᵤR¹³,
k vaut 0, 1 ou 2,
r vaut 0, 1 ou 2,
R⁶, R⁷ sont choisis indépendamment parmi les significations données pour R⁸, R⁹,
ou R⁶ et R⁷ forment ensemble un reste carbocyclique comprenant de 3 à 7 atomes de carbone ou un reste hétérocyclique comprenant 1, 2 ou 3 hétéroatomes, choisis dans le groupe constitué par O, N et S, et de 2 à 6 atomes de carbone, ledit reste carbocyclique ou hétérocyclique étant non substitué ou comprenant 1, 2 ou 3 substituants, choisis parmi les significations données pour R⁸, R⁹ et R¹⁰,
R⁸, R⁹ sont choisis indépendamment dans le groupe constitué par H, A, cycloalkyle comprenant de 3 à 7 atomes de carbone, Hal, CH₂Hal, CH(Hal)₂, C(Hal)₃, NO₂, (CH₂)ₙCN, (CH₂)ₙNR¹¹R¹², (CH₂)ₙOR¹¹, (CH₂)ₙO(CH₂)ₖNR¹¹R¹², (CH₂)ₙCOOR¹², (CH₂)ₙCONR¹¹R¹², (CH₂)ₙNR¹¹COR¹³, (CH₂)ₙNR¹¹CONR¹¹R¹², (CH₂)ₙNR¹¹SO₂A, (CH₂)ₙSO₂NR¹¹R¹², (CH₂)ₙS(O)ᵤR¹³, (CH₂)ₙOC(O)R¹³, (CH₂)ₙCOR¹³, (CH₂)ₙSR¹¹, CH=N-OA, CH₂CH=N-OA, (CH₂)ₙNHOA, (CH₂)ₙCH=N-R¹¹, (CH₂)ₙOC(O)NR¹¹R¹², (CH₂)ₙNR¹¹COOR¹², (CH₂)ₙN(R¹¹)CH₂CH₂OR¹³, (CH₂)ₙN(R¹¹)CH₂CH₂OCF₃, (CH₂)ₙN(R¹¹)C(R¹³)HCOOR¹², C(R¹³)HCOR¹², (CH₂)ₙN(R¹¹)CH₂CH₂N(R¹²)CH₂COOR¹², (CH₂)ₙN(R¹¹)CH₂CH₂NR¹¹R¹², CH=CHCOOR¹¹, CH=CHCH₂NR¹¹R¹², CH=CHCH₂NR¹¹R¹², CH=CHCH₂OR¹³, (CH₂)ₙN(COOR¹¹)COOR¹², (CH₂)ₙN(CONH₂)COOR¹¹, (CH₂)ₙN(CONH₂)CONH₂, (CH₂)ₙN(CH₂COOR¹¹)COOR¹², (CH₂)ₙN(CH₂CONH₂)COOR¹¹, (CH₂)ₙN(CH₂CONH₂)CONH₂, (CH₂)ₙCHR¹³COR¹¹, (CH₂)ₙCHR¹³COOR¹¹, (CH₂)ₙCHR¹³CH₂OR¹⁴, (CH₂)ₙOCN et (CH₂)ₙNCO, où
R¹¹, R¹² sont choisis indépendamment dans le groupe constitué par H, A et (CH₂)ₘAr³ ou parmi NR¹¹R¹²,
R¹¹ et R¹² forment, conjointement avec l'atome de N auquel ils sont liés, un hétérocycle de 5, 6 ou 7 chaînons qui contient éventuellement 1 ou 2 hétéroatomes supplémentaires, choisis parmi N, O et S,
R¹³, R¹⁴ sont choisis indépendamment dans le groupe constitué par H, Hal, A, (CH₂)ₘAr⁴ et (CH₂)ₘHét,
A est choisi dans le groupe constitué par alkyle, alcényle, cycloalkyle, alkylènecycloalkyle, alcoxy et alcoxyalkyle,
Ar³, Ar⁴ sont, indépendamment l'un de l'autre, des restes hydrocarbonés aromatiques comprenant de 5 à 12 et préférablement de 5 à 10 atomes de carbone qui sont éventuellement substitués par un ou plusieurs substituants, choisis parmi le groupe constitué par A, Hal, NO₂, CN, OR¹⁵, NR¹⁵R¹⁶, COOR¹⁵, CONR¹⁵R¹⁶, NR¹⁵COR¹⁶, NR¹⁵CONR¹⁵R¹⁶, NR¹⁶SO₂A, COR¹⁵, SO₂R¹⁵R¹⁶, S(O)ᵤA et OOCR¹⁵,
Hét est un reste hétérocyclique saturé, insaturé ou aromatique qui est éventuellement substitué par un ou plusieurs substituants, choisis parmi le groupe constitué par A, Hal, NO₂, CN, OR¹⁵, NR¹⁵R¹⁶, COOR¹⁵, CONR¹⁵R¹⁶, NR¹⁵COR¹⁶ NR¹⁵CONR¹⁵R¹⁶, NR¹⁶SO₂A, COR¹⁵, SO₂R¹⁵R¹⁶, S(O)ᵤA et OOCR¹⁵,
R¹⁵, R¹⁶ sont choisis indépendamment dans le groupe constitué par H, A, et (CH₂)ₘAr⁶, où
Ar⁶ est un hydrocarbure aromatique de 5 ou 6 chaînons qui est éventuellement substitué par un ou plusieurs substituants choisis parmi le groupe constitué par méthyle, éthyle, propyle, 2-propyle, tertio-butyle, Hal, CN, OH, NH₂ et CF₃,
m et n valent, indépendamment l'un de l'autre, 0, 1, 2, 3, 4, ou 5,
X est choisi dans le groupe constitué par O, S, NR¹¹, CHOR¹¹, CH₂, CH₂CH₂, OCH₂, CH₂O, OCH₂CH₂, CH₂CH₂O,
h, i sont, indépendamment l'un de l'autre, 0, 1, 2, 3, 4, 5, ou 6, et
j vaut 1, 2, 3, 4, 5, ou 6,
Y est choisi parmi O, S, NR²¹, C(R²²)-NO₂, C(R²²)-CN et C(CN)₂, où
R²¹ est choisi indépendamment parmi les significations données pour R¹³, R¹⁴ et
R²² est choisi indépendamment parmi les significations données pour R¹¹, R¹²,
p vaut 0, 1, 2, 3, 4 ou 5,
q vaut 0, 1, 2, 3 ou 4, préférablement 0, 1 ou 2,
u vaut 0, 1, 2 ou 3, préférablement 0, 1 ou 2,
et
Hal est choisi indépendamment parmi le groupe constitué par F, Cl, Br et I;
et les sels et solvats pharmaceutiquement acceptables de ceux-ci.

2. Dérivé de malonamide selon la revendication 1, choisi parmi les composés de formule IIa, IIb, IIc, IId, IIe, IIf, IIg et IIh, 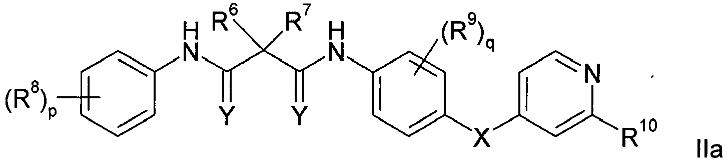 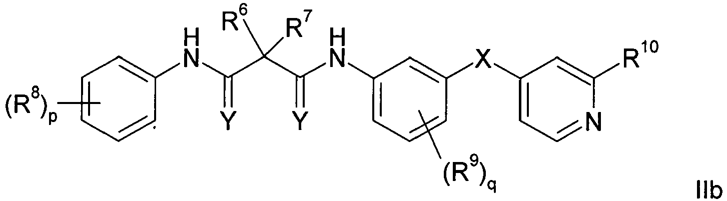 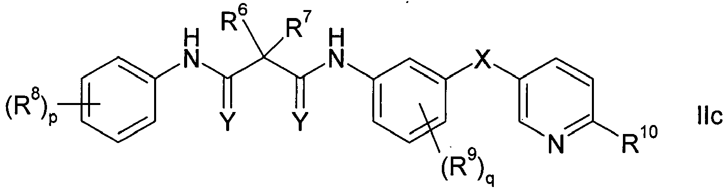 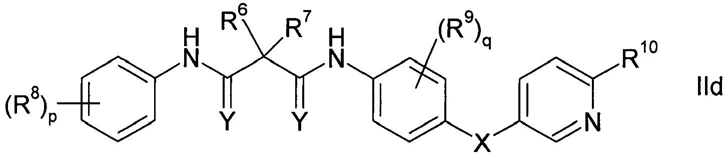 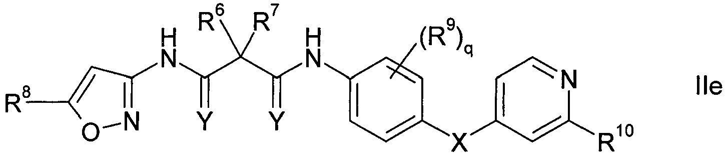 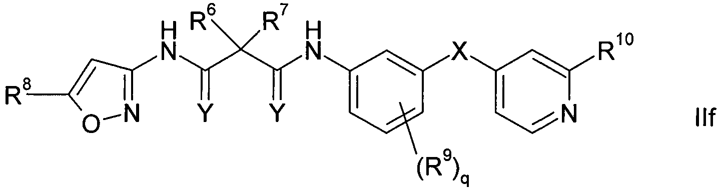 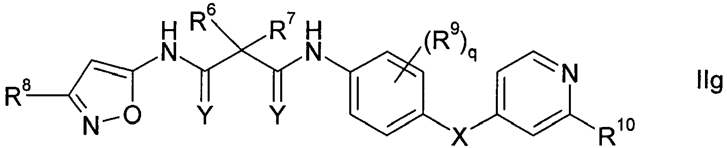 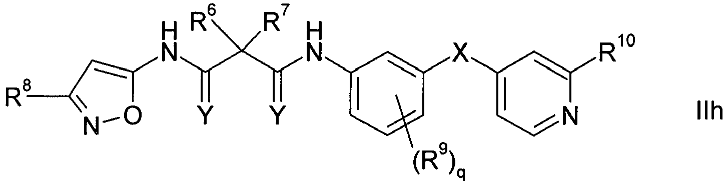 dans laquelle R⁶, R⁷, R⁸, p, X, Y, R⁹, q sont tels que définis selon la revendication 1 et R¹⁰ est H ou tel que défini selon la revendication 1; et les sels et solvats pharmaceutiquement acceptables de celui-ci.

3. Dérivé de malonamide selon l'une des revendications 1 ou 2, choisi parmi les composés (1) à (228) du tableau 1; les composés (1) à (228) de formule A-NH-CO-CH₂-CO-NH-B, où A et B sont tels que donnés ci-après :
| | A | B |
|---|---|---|
| (1) | 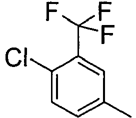 | 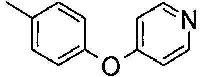 |
| (2) | 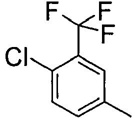 | 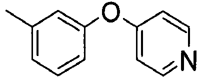 |
| (3) | 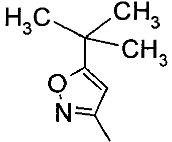 | 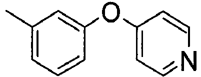 |
| (4) | 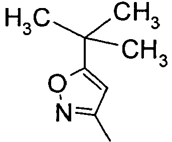 | 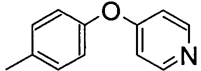 |
| (5) | 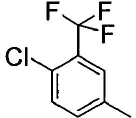 | 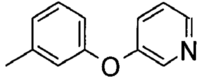 |
| (6) | 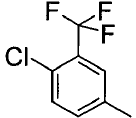 | 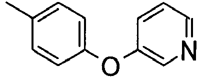 |
| (7) | 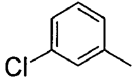 | 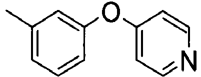 |
| (8) | 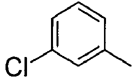 | 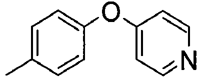 |
| (9) | 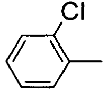 | 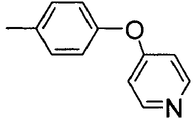 |
| (10) | 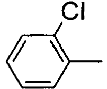 | 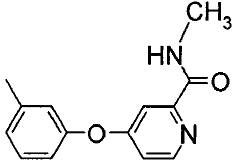 |
| (11) | 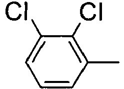 | 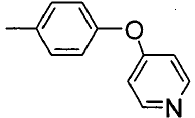 |
| (12) | 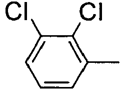 | 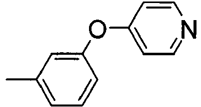 |
| (13) | 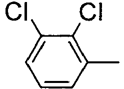 | 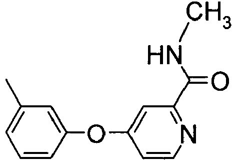 |
| (14) | 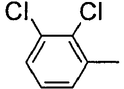 | 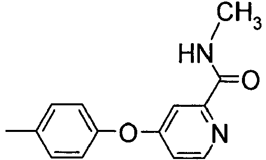 |
| (15) | 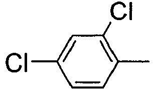 | 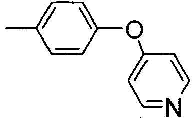 |
| (16) | 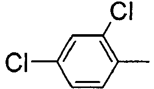 | 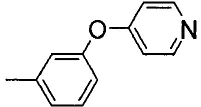 |
| (17) | 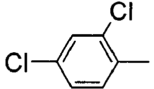 | 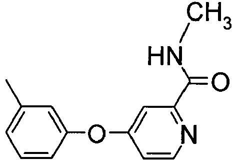 |
| (18) | 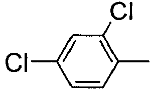 | 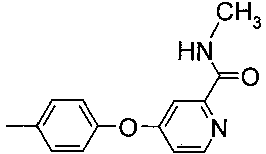 |
| (19) | 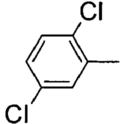 | 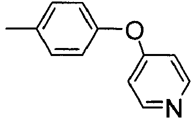 |
| (20) | 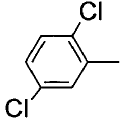 | 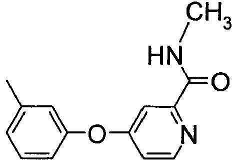 |
| (21) | 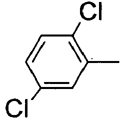 | 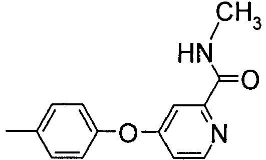 |
| (22) | 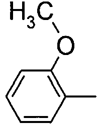 | 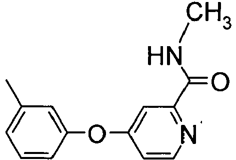 |
| (23) | 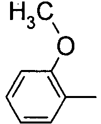 | 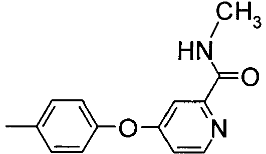 |
| (24) | 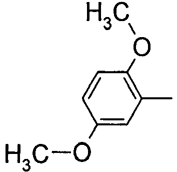 | 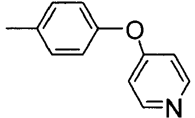 |
| (25) | 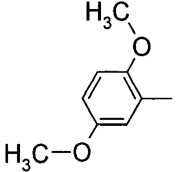 | 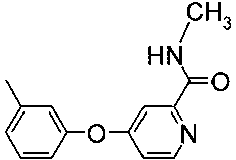 |
| (26) | 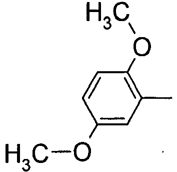 | 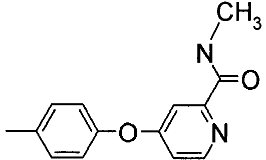 |
| (27) | 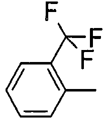 | 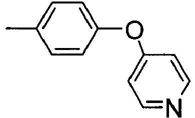 |
| (28) | 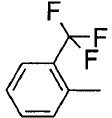 | 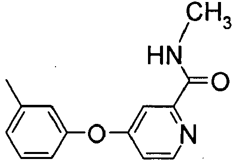 |
| (29) | 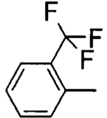 | 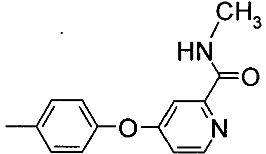 |
| (30) | 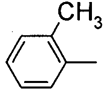 | 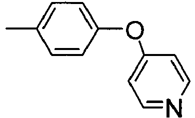 |
| (31) | 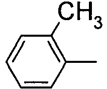 | 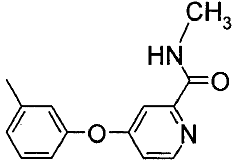 |
| (32) | 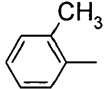 | 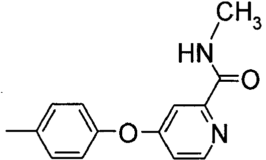 |
| (33) | 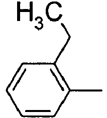 | 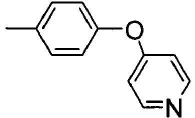 |
| (34) | 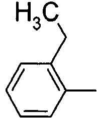 | 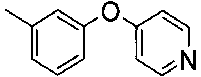 |
| (35) | 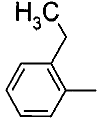 | 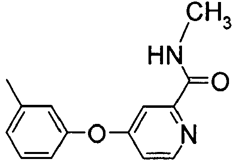 |
| (36) | 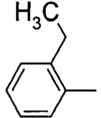 | 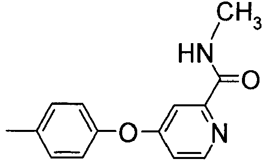 |
| (37) | 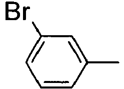 | 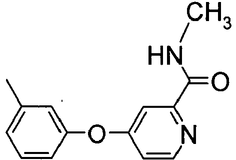 |
| (38) | 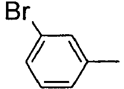 | 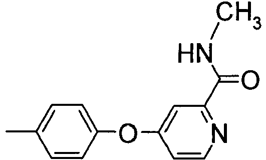 |
| (39) | 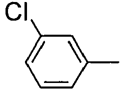 | 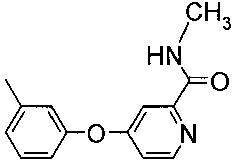 |
| (40) | 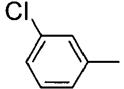 | 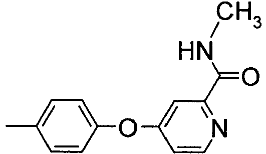 |
| (41) | 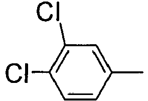 | 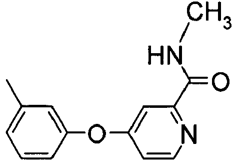 |
| (42) | 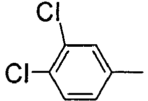 | 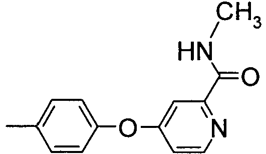 |
| (43) | 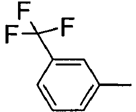 | 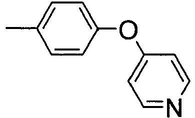 |
| (44) | 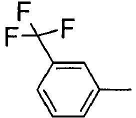 | 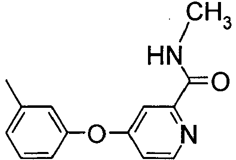 |
| (45) | 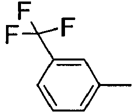 | 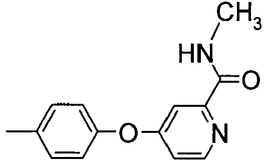 |
| (46) | 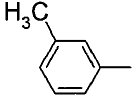 | 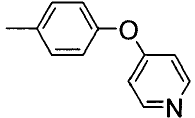 |
| (47) | 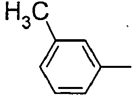 | 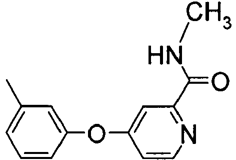 |
| (48) | 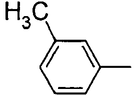 | 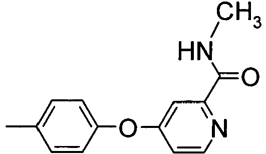 |
| (49) | 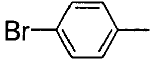 | 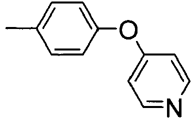 |
| (50) | 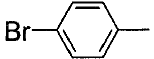 | 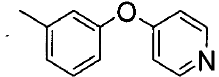 |
| (51) | 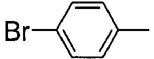 | 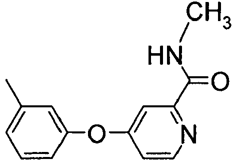 |
| (52) | 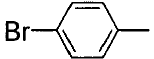 | 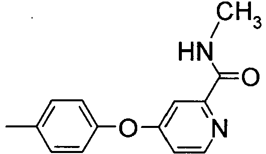 |
| (53) | 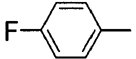 | 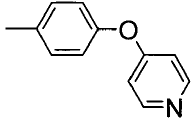 |
| (54) | 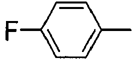 | 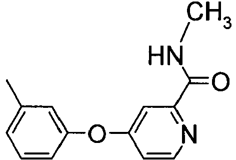 |
| (55) | 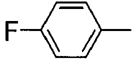 | 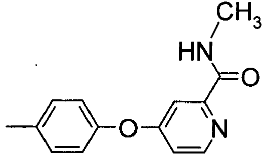 |
| (56) | 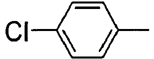 | 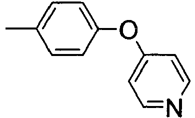 |
| (57) | 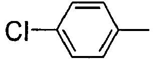 | 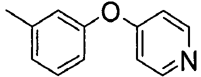 |
| (58) | 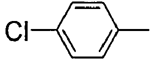 | 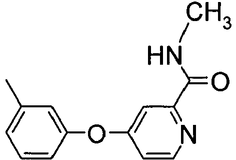 |
| (59) | 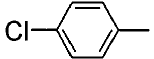 | 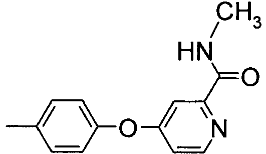 |
| (60) | 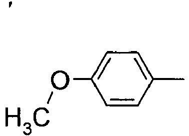 | 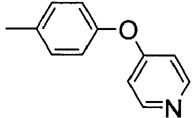 |
| (61) | 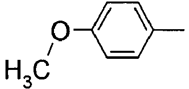 | 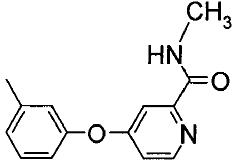 |
| (62) | 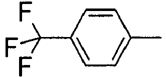 | 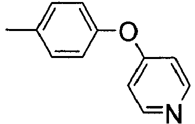 |
| (63) | 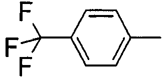 | 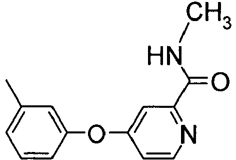 |
| (64) | 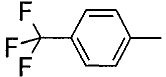 | 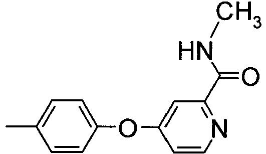 |
| (65) | 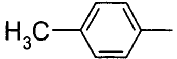 | 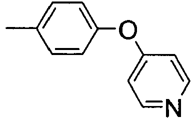 |
| (66) | 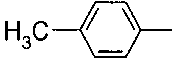 | 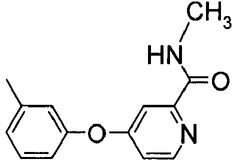 |
| (67) | 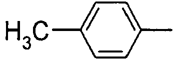 | 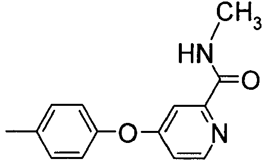 |
| (68) | 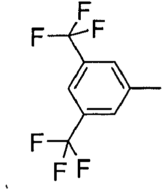 | 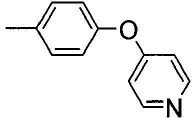 |
| (69) | 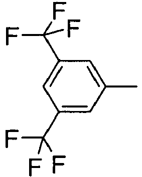 | 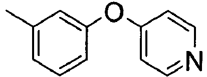 |
| (70) | 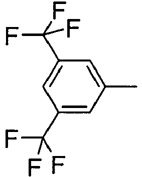 | 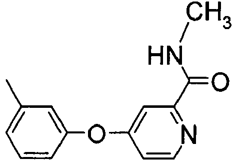 |
| (71) | 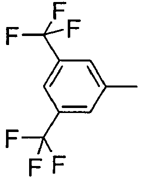 | 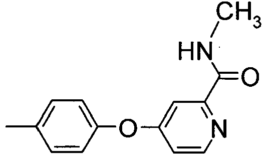 |
| (72) | 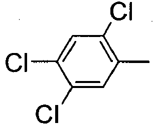 | 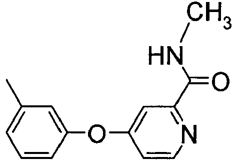 |
| (73) | 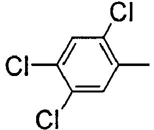 | 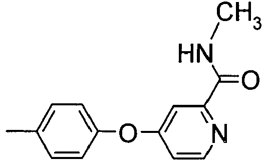 |
| (74) | 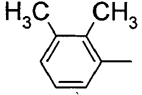 | 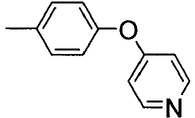 |
| (75) | 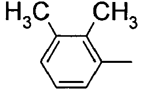 | 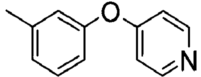 |
| (76) | 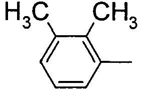 | 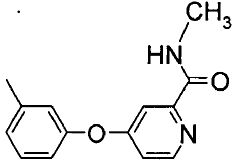 |
| (77) | 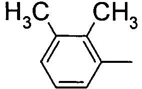 | 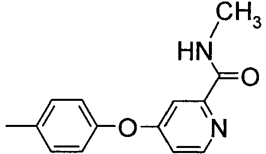 |
| (78) | 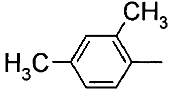 | 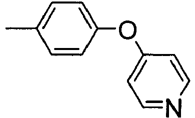 |
| (79) | 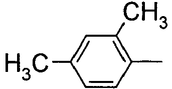 | 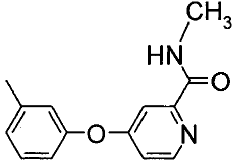 |
| (80) | 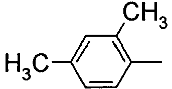 | 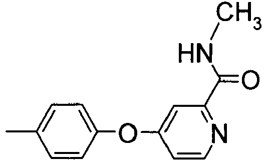 |
| (81) | 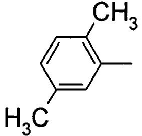 | 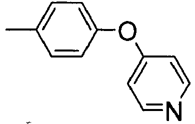 |
| (82) | 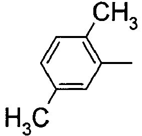 | 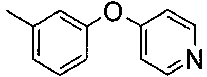 |
| (83) | 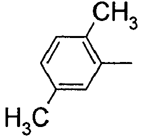 | 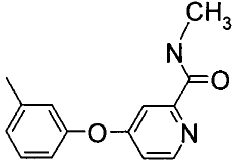 |
| (84) | 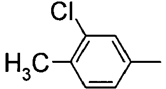 | 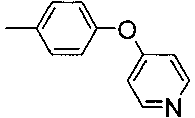 |
| (85) | 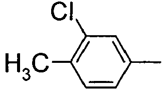 | 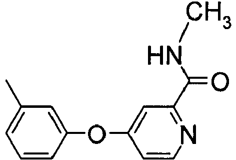 |
| (86) | 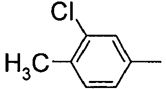 | 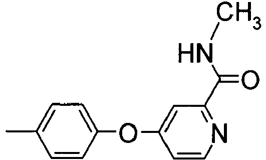 |
| (87) | 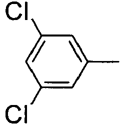 | 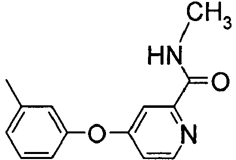 |
| (88) | 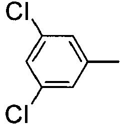 | 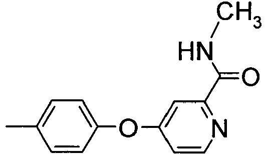 |
| (89) | 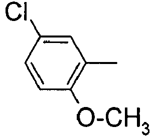 | 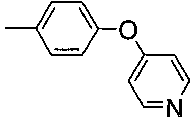 |
| (90) | 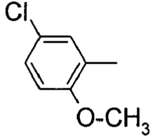 | 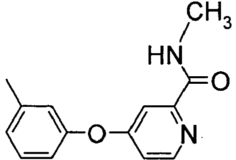 |
| (91) | 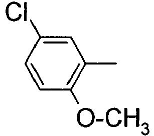 | 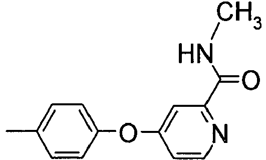 |
| (92) | 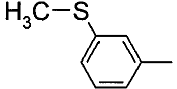 | 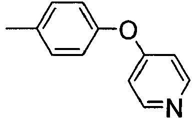 |
| (93) | 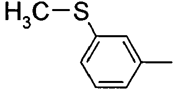 | 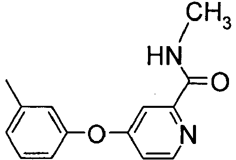 |
| (94) | 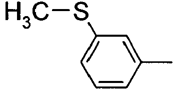 | 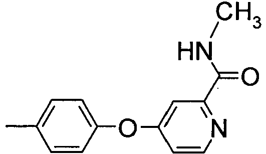 |
| (95) | 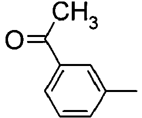 | 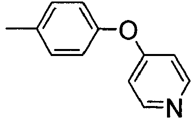 |
| (96) | 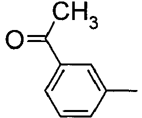 | 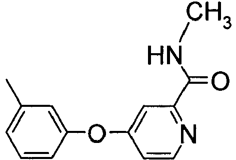 |
| (97) | 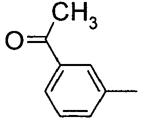 | 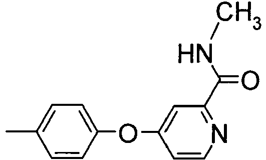 |
| (98) | 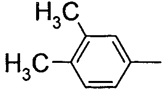 | 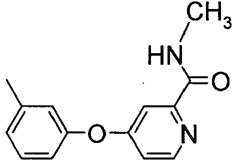 |
| (99) | 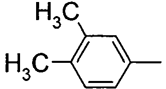 | 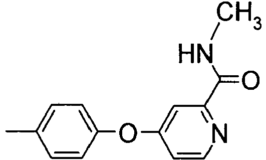 |
| (100) | 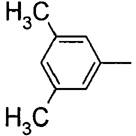 | 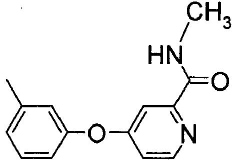 |
| (101) | 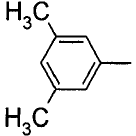 | 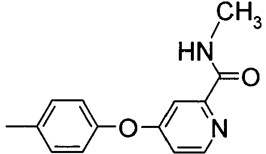 |
| (102) | 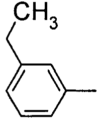 | 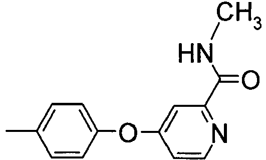 |
| (103) | 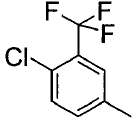 | 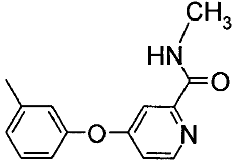 |
| (104) | 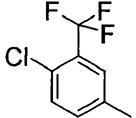 | 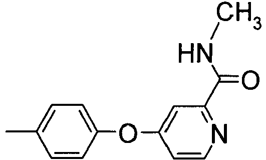 |
| (105) | 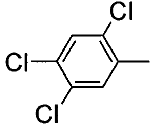 | 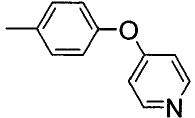 |
| (106) | 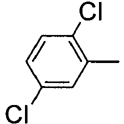 | 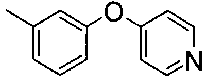 |
| (107) | 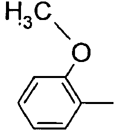 | 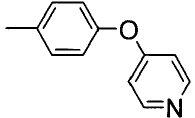 |
| (108) | 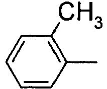 | 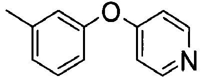 |
| (109) | 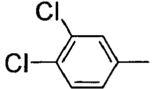 | 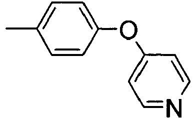 |
| (110) | 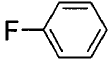 | 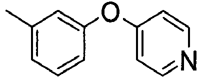 |
| (111) | 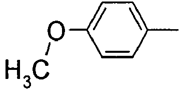 | 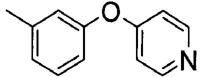 |
| (112) | 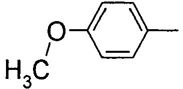 | 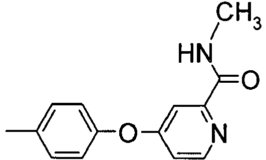 |
| (113) | 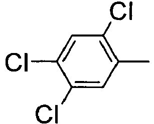 | 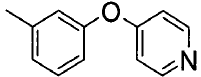 |
| (114) | 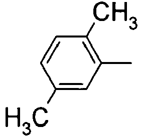 | 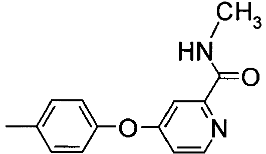 |
| (115) | 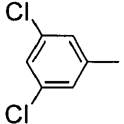 | 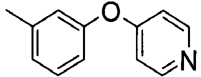 |
| (116) | 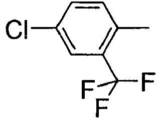 | 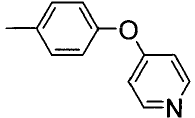 |
| (117) | 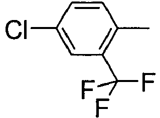 | 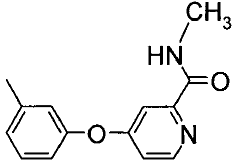 |
| (118) | 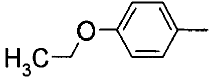 | 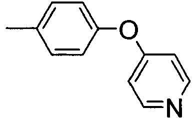 |
| (119) | 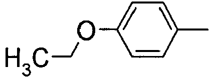 | 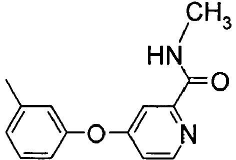 |
| (120) | 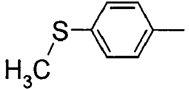 | 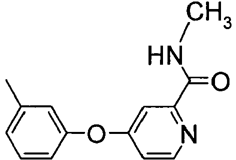 |
| (121) | 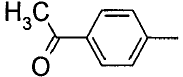 | 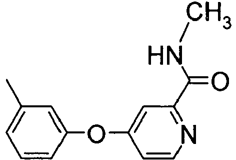 |
| (122) | 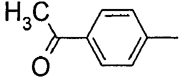 | 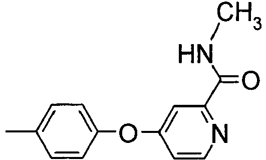 |
| (123) | 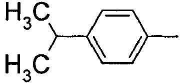 | 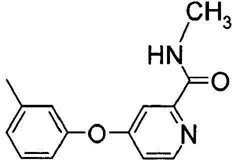 |
| (124) | 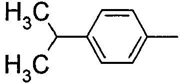 | 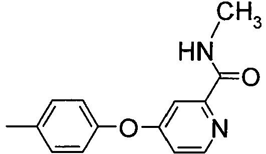 |
| (125) | 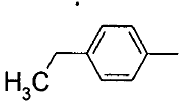 | 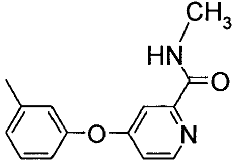 |
| (126) | 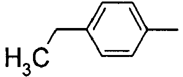 | 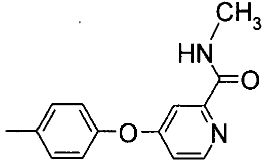 |
| (127) | 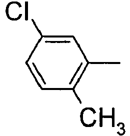 | 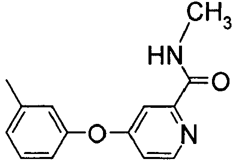 |
| (128) | 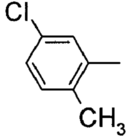 | 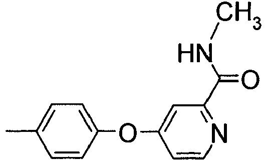 |
| (129) | 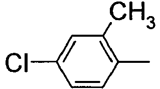 | 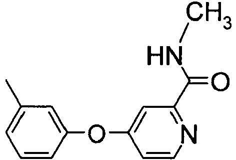 |
| (130) | 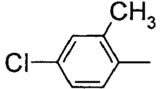 | 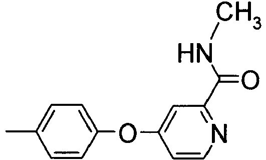 |
| (131) | 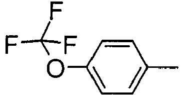 | 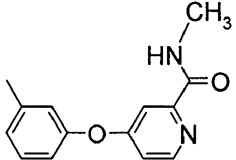 |
| (132) 2 | 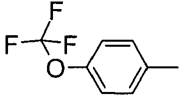 | 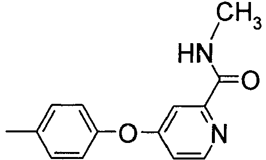 |
| (133) | 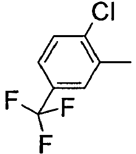 | 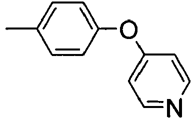 |
| (134) | 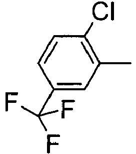 | 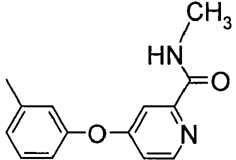 |
| (135) | 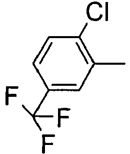 | 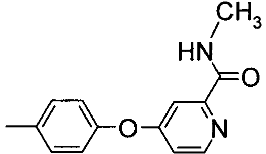 |
| (136) | 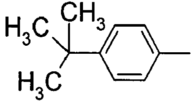 | 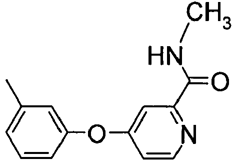 |
| (137) | 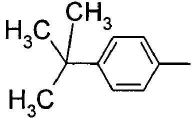 | 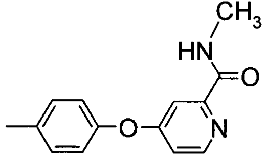 |
| (138) | 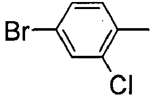 | 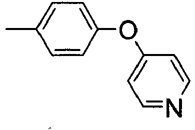 |
| (139) | 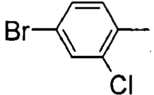 | 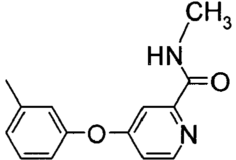 |
| (140) | 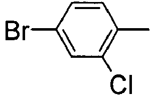 | 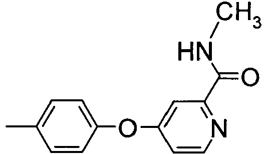 |
| (141) | 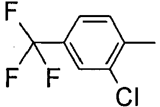 | 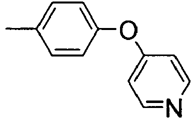 |
| (142) | 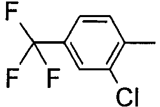 | 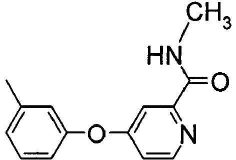 |
| (143) | 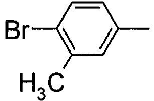 | 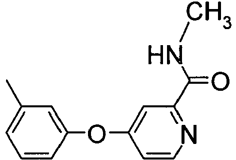 |
| (144) | 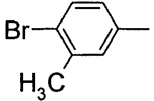 | 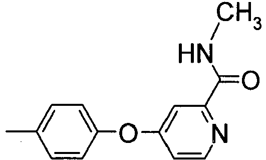 |
| (145) | 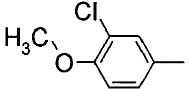 | 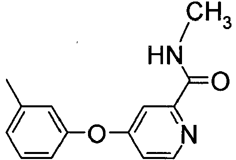 |
| (146) | 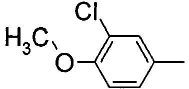 | 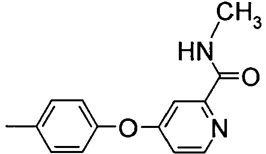 |
| (147) | 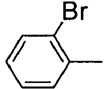 | 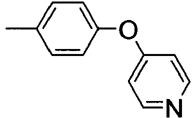 |
| (148) | 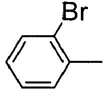 | 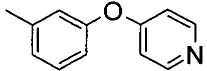 |
| (149) | 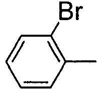 | 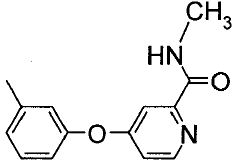 |
| (150) | 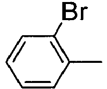 | 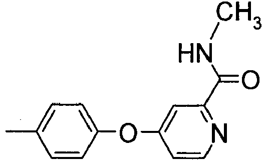 |
| (151) | 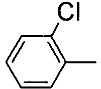 | 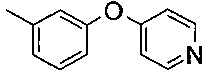 |
| (152) | 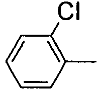 | 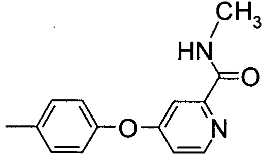 |
| (153) | 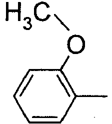 | 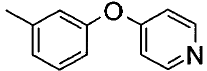 |
| (154) | 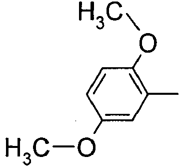 | 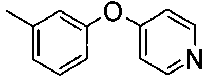 |
| (155) | 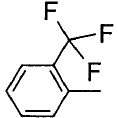 | 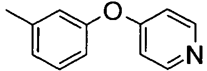 |
| (156) | 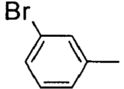 | 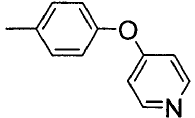 |
| (157) | 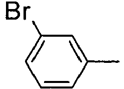 | 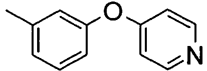 |
| (158) | 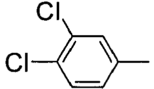 | 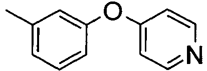 |
| (159) | 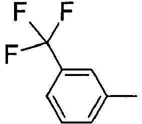 | 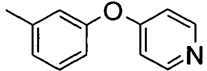 |
| (160) | 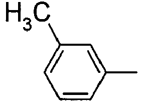 | 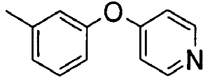 |
| (161) | 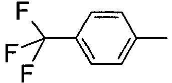 | 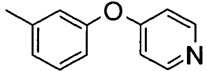 |
| (162) | 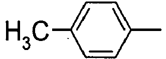 | 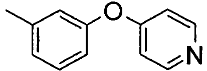 |
| (163) | 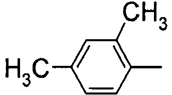 | 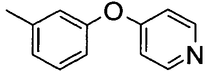 |
| (164) | 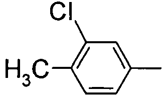 | 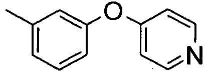 |
| (165) | 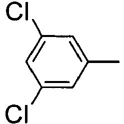 | 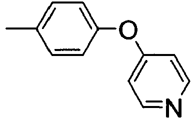 |
| (166) | 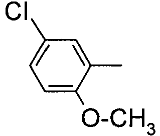 | 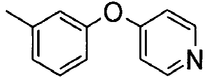 |
| (167) | 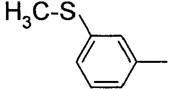 | 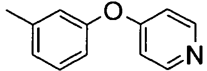 |
| (168) | 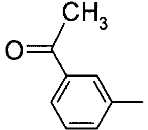 | 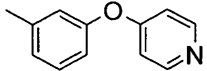 |
| (169) | 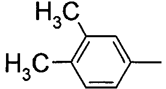 | 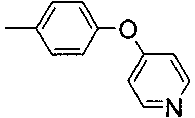 |
| (170) | 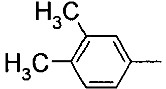 | 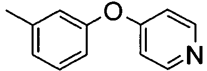 |
| (171) | 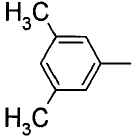 | 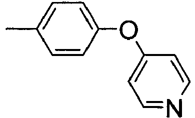 |
| (172) | 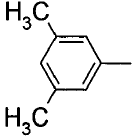 | 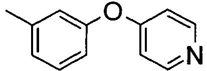 |
| (173) | 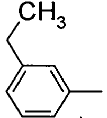 | 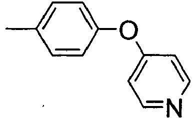 |
| (174) | 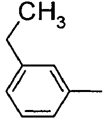 | 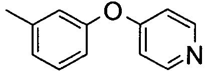 |
| (175) | 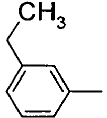 | 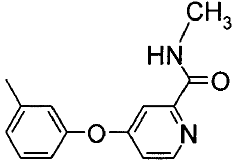 |
| (176) | 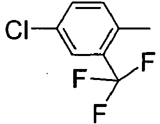 | 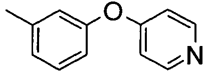 |
| (177) | 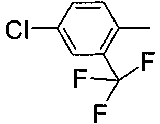 | 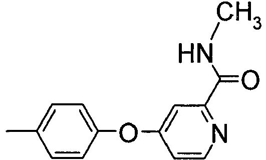 |
| (178) | 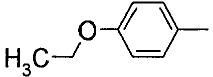 | 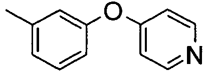 |
| (179) | 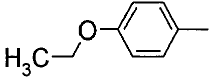 | 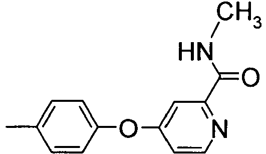 |
| (180) | 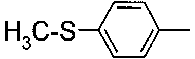 | 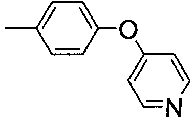 |
| (181) | 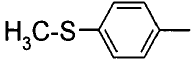 | 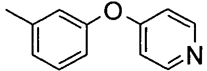 |
| (182) | 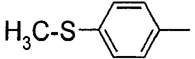 | 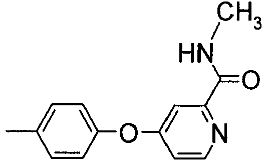 |
| (183) | 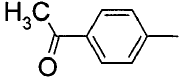 | 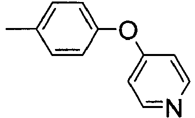 |
| (184) | 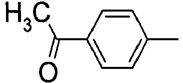 | 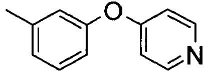 |
| (185) | 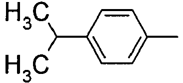 | 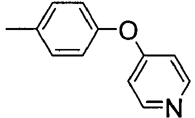 |
| (186) | 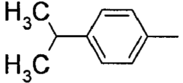 | 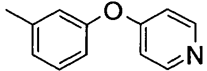 |
| (187) | 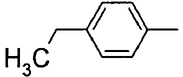 | 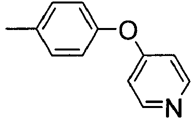 |
| (188) | 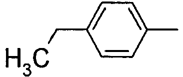 | 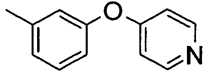 |
| (189) | 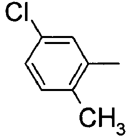 | 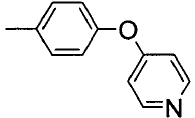 |
| (190) | 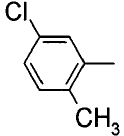 | 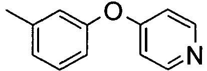 |
| (191) | 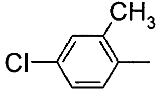 | 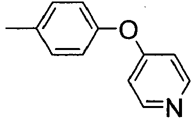 |
| (192) | 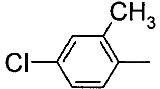 | 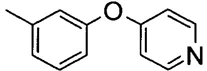 |
| (193) | 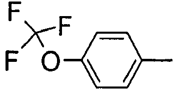 | 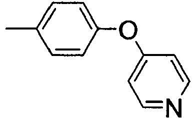 |
| (194) | 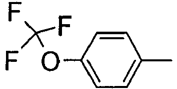 | 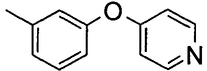 |
| (195) | 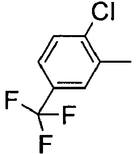 | 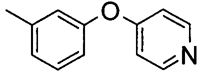 |
| (196) | 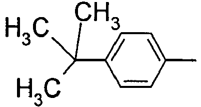 | 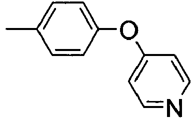 |
| (197) | 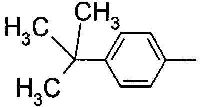 | 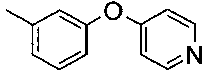 |
| (198) | 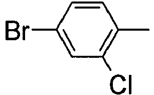 | 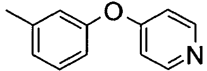 |
| (199) | 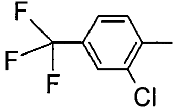 | 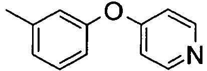 |
| (200) | 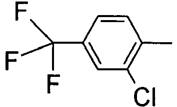 | 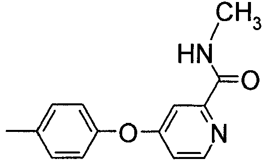 |
| (201) | 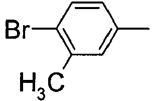 | 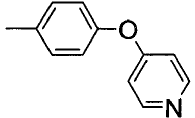 |
| (202) | 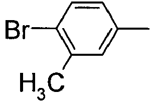 | 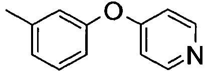 |
| (203) | 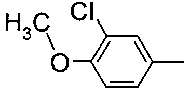 | 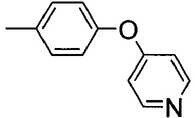 |
| (204) | 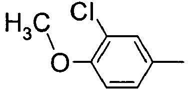 | 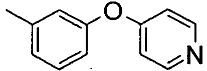 |
| (205) | 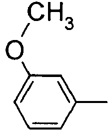 | 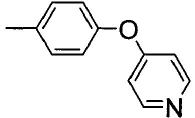 |
| (206) | 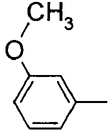 | 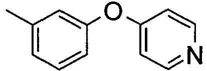 |
| (207) | 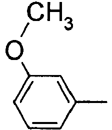 | 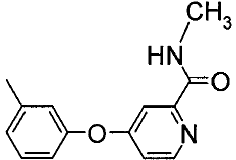 |
| (208) | 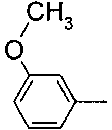 | 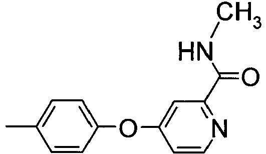 |
| (209) | 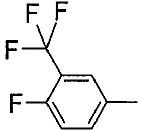 | 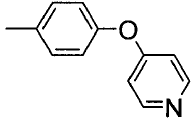 |
| (210) | 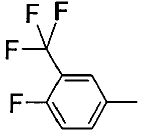 | 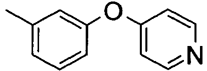 |
| (211) | 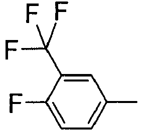 | 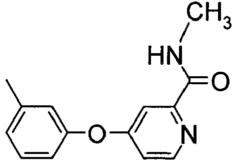 |
| (212) | 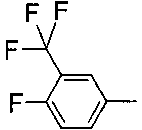 | 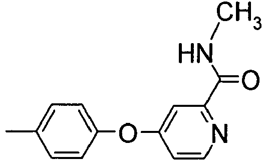 |
| (213) | 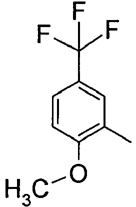 | 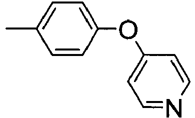 |
| (214) | 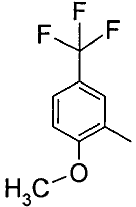 | 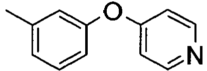 |
| (215) | 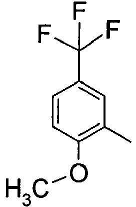 | 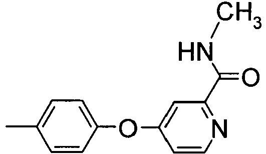 |
| (216) | 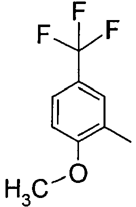 | 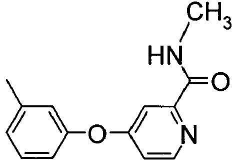 |
| (217) | 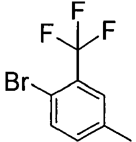 | 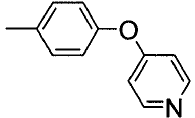 |
| (218) | 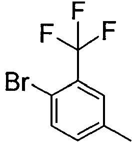 | 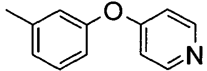 |
| (219) | 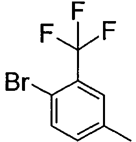 | 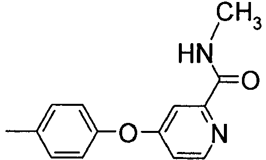 |
| (220) | 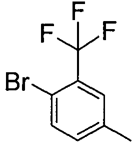 | 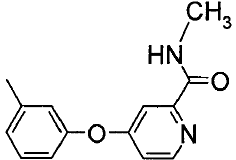 |
| (221) | 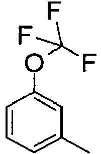 | 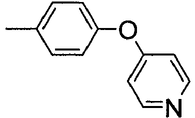 |
| (222) | 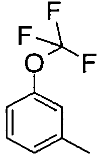 | 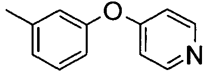 |
| (223) | 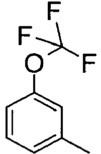 | 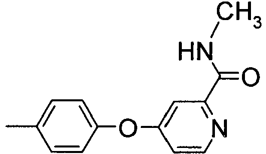 |
| (224) | 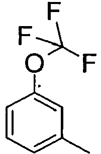 | 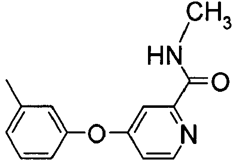 |
| (225) | 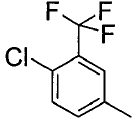 | 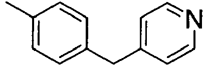 |
| (226) | 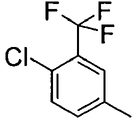 | 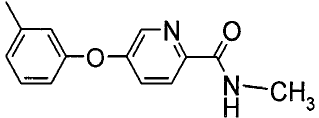 |
| (227) | 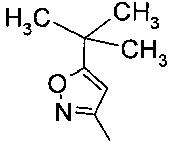 | 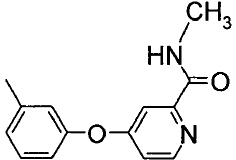 |
| (228) | 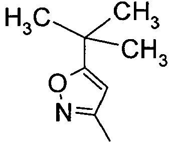 | 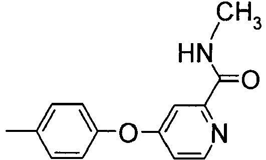 |
et les sels et solvats physiologiquement acceptables de celui-ci.

4. Dérivé de malonamide selon l'une des revendications 1 à 3, comme médicament.

5. Dérivé de malonamide selon l'une des revendications 1 à 3, comme inhibiteur de kinase.

6. Dérivé de malonamide selon la revendication 5, **caractérisé en ce que** les kinases sont choisies parmi les raf-kinases et les kinases VEGFR.

7. Composition pharmaceutique, **caractérisée en ce qu'**elle contient un ou plusieurs composés selon l'une des revendications 1 à 3.

8. Composition pharmaceutique selon la revendication 7, **caractérisée en ce qu'**elle contient un ou plusieurs composés supplémentaires, choisis dans le groupe constitué par les excipients, auxiliaires, adjuvants, véhicules physiologiquement acceptables et des ingrédients actifs pharmaceutiques autres que les composés selon l'une des revendications 1 à 3.

9. Procédé d'élaboration d'une composition pharmaceutique, **caractérisé en ce qu'**un ou plusieurs composés selon l'une des revendications 1 à 3 et un ou plusieurs composés, choisis dans le groupe constitué par les véhicules, excipients, auxiliaires et ingrédients actifs pharmaceutiques autres que les composés selon l'une des revendications 1 à 3, sont transformés par des moyens mécaniques en une composition pharmaceutique qui est convenable comme forme de dosage pour une application et/ou une administration à un patient.

10. Composé selon l'une des revendications 1 à 3, pour une utilisation comme substance pharmaceutique.

11. Composé selon l'une des revendications 1 à 3, pour une utilisation dans le traitement et/ou la prophylaxie de troubles.

12. Utilisation d'un composé selon l'une des revendications 1 à 3, pour la production d'une composition pharmaceutique destinée au traitement et/ou à la prophylaxie de troubles.

13. Composé pour une utilisation selon la revendication 11, **caractérisé en ce que** les troubles sont provoqués, médiés et/ou propagés par des kinases choisies parmi les raf-kinases et les kinases VEGFR.

14. Composé pour une utilisation selon la revendication 11 ou 13, **caractérisé en ce que** les troubles sont choisis dans le groupe constitué par les troubles hyperprolifératifs et non hyperprolifératifs.

15. Composé pour une utilisation selon la revendication 11, 13 ou 14, **caractérisé en ce que** le trouble est le cancer.

16. Composé pour une utilisation selon la revendication 11, 13 ou 14, **caractérisé en ce que** le trouble est non cancéreux.

17. Composé pour une utilisation selon la revendication 16, **caractérisé en ce que** les troubles non cancéreux sont choisis dans le groupe constitué par le psoriasis, l'arthrite, les inflammations, l'endométriose, la cicatrisation, l'hyperplasie bénigne de la prostate, les maladies immunologiques, les maladies auto-immunes et les maladies d'immunodéficience.

18. Composé pour une utilisation selon la revendication 15, **caractérisé en ce que** les troubles sont choisis dans le groupe constitué par le cancer du cerveau, le cancer du poumon, le cancer des cellules squameuses, le cancer de la vessie, le cancer de l'estomac, le cancer du pancréas, le cancer du foie, le cancer du rein, le cancer colorectal, le cancer du sein, le cancer de la tête, le cancer du cou, le cancer de l'oesophage, le cancer gynécologique, le cancer de la thyroïde, les lymphomes, la leucémie chronique et la leucémie aiguë.

19. Composé pour une utilisation selon la revendication 15 ou 16, **caractérisé en ce que** les troubles sont choisis dans le groupe constitué par l'arthrite, la resténose, les troubles fibrotiques, les troubles de prolifération des cellules mésangiales, la néphropathie diabétique, la néphrosclérose maligne, les syndromes de microangiopathie thrombotique, le rejet de greffe d'organes, les glomérulopathies, les troubles métaboliques, les inflammations et les maladies neurodégénératives.

20. Utilisation selon la revendication 12, **caractérisée en ce que** les troubles sont choisis dans le groupe constitué par la polyarthrite rhumatoïde, les inflammations, les maladies auto-immunes, la bronchopneumopathie chronique obstructive, l'asthme, l'affection abdominale inflammatoire, la fibrose, l'athérosclérose, la resténose, les maladies vasculaires, les maladies cardiovasculaires, les inflammations, les maladies rénales et les troubles d'angiogenèse.

21. Composé selon l'une des revendications 1 à 3, comme inhibiteur de kinase.

22. Composé pour une utilisation selon la revendication 21, **caractérisé en ce que** la kinase est une ou plusieurs raf-kinases, choisies dans le groupe constitué par A-Raf, B-Raf et Raf-1.

23. Composé selon l'une des revendications 1 à 3, pour une utilisation dans le traitement d'une croissance cellulaire cancéreuse médiée par une ou plusieurs kinases.

24. Méthode de production de composés de formule II, **caractérisée en ce que**
a) un composé de formule III 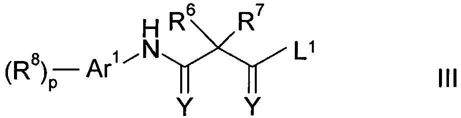 dans laquelle
L¹ est Cl, Br, I, OH, un groupement OH estérifié ou un motif diazonium, et R⁶, R⁷, R⁸, p, Ar¹, Y sont tels que définis selon la revendication 1,
est réagi
b) avec un composé de formule IV, 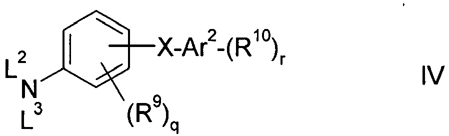 dans laquelle
L², L³ sont, indépendamment l'un de l'autre, H ou un ion métallique, et R⁹, q, X, Ar², R¹⁰ et r sont tels que définis selon la revendication 1,
et éventuellement
c) on isole et/ou on traite le composé de formule II obtenu par ladite réaction par un acide, afin d'obtenir le sel de celui-ci.

25. Utilisation d'un composé de formule III, 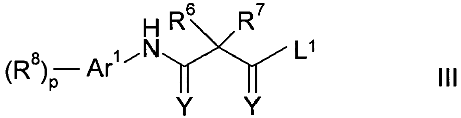 dans laquelle L¹ est Cl, Br, I, OH, un groupement OH estérifié ou un motif diazonium, et R⁶, R⁷, R⁸, p, Ar¹, Y sont tels que définis selon la revendication 1, dans un procédé selon la revendication 24, pour l'élaboration d'un composé de formule II selon la revendication 1.

26. Utilisation d'un composé de formule IV, 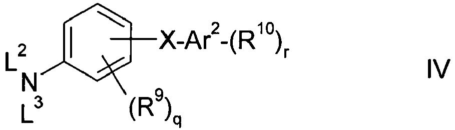 dans laquelle
L², L³ sont, indépendamment l'un de l'autre, H ou un ion métallique, et R⁹, q, X, Ar², R¹⁰ et r sont tels que définis selon la revendication 1, dans un procédé selon la revendication 24, pour l'élaboration d'un composé de formule II selon la revendication 1.
